# EUROPEAN PATENT SPECIFICATION

(11) **EP 1 813 609 B1**
(45) Date of publication and mention of the grant of the patent: **19.06.2013**
(21) Application number: 05799135.8
(22) Date of filing: 25.10.2005
(51) Int. Cl.: C07D 401/12, A61K 31/52, A61K 31/282, A61K 31/675, A61K 31/337, A61K 31/704, A61K 31/407, A61K 31/7048, A61K 31/4745, A61K 31/7068, A61K 31/475

(54) **NOVEL AMINOPYRIDINE DERIVATIVES HAVING SELECTIVE AURORA-A INHIBITORY EFFECT**
NEUE AMINOPYRIDINDERIVATE MIT SELEKTIVA AURORA-A-HEMMENDER WIRKUNG
NOUVEAUX DERIVES D'AMINOPYRIDINE PRESENTANT UN EFFET INHIBITEUR SELECTIF D'AURORA-A

(30) Priority: 29.10.2004 JP 2004315152; 01.06.2005 JP 2005161156
(43) Date of publication of application: 01.08.2007
(73) Proprietor: MSD K.K., Chiyoda-ku Tokyo 102-8667 (JP)
(72) Inventor: OHKUBO, Mitsuru, c/o BANYU PHARMACEUTICAL CO., LTD, Tsukuba-shi, Ibaraki 300-2611 (JP); KATO, Tetsuya, c/o BANYU PHARMACEUTICAL CO., LTD, Tsukuba-shi, Ibaraki 300-2611 (JP); KAWANISHI, N., c/o BANYU PHARMACEUTICAL CO., LTD, Tsukuba-shi, Ibaraki 300-2611 (JP); MITA, Takashi, c/o BANYU PHARMACEUTICAL CO., LTD., Tsukuba-shi, Ibaraki 300-2611 (JP); SHIMOMURA, T., c/o BANYU PHARMACEUTICAL CO., LTD, Tsukuba-shi, Ibaraki 300-2611 (JP)
(74) Representative: Hussain, Deeba
(86) International application number: PCT/JP2005/019957
(87) International publication number: WO 2006/046734

(56) References cited:
- WO-A1-2006/008874
- WO-A2-02/057259
- WO-A2-02/059112
- JP-A- 2003 509 342

### SEQUENCE LISTING

<110> Banyu Pharmaceutical Co., Ltd. Ohkubo, Mitsuru Kato, Tetsuya Kawanishi, Nobuhiko Mita, Takashi Shimomura, Toshiyasu
<120> NOVEL AMINOPYRIDINE DERIVATIVES HAVING
   AURORA A SELECTIVE INHIBITORY ACTION
<130> BY0045Y
<150> JP2004-315152
   <151> 2004-10-29
<150> JP2005-161156
   <151> 2005-06-01
<160> 1
<170> FastSEQ for Windows Version 4.0
<210> 1
   <211> 7
   <212> PRT
   <213> Artificial Sequence
<220>
   <223> Completely Synthetic Amino Acid Sequence
<400> 1
   Lys Arg Arg Ala Ser Lys Gly
    1               5

## Description

### Technical Field

The present invention relates to novel aminopyridine derivatives which are useful in the pharmaceutical field, and more particularly, to those which inhibit the growth of tumor cells based on an Aurora A selective inhibitory action and exhibit an antitumor effect, and also to an Aurora A selective inhibitor and an antitumor agent containing them.

### Background Art

Aurora kinase is a serine/threonine kinase involved in cell division. With regard to the Aurora kinase, three subtypes of A, B and C are known at present, and they have very high homology to each other. Aurora A participates in the maturation and distribution of centrosome or in the formation of spindle body. On the other hand, it is believed that Aurora B participates in the aggregation and pairing of chromosome, a spindle checkpoint and cytoplasm division *[*Nat. Rev. Mol. Cell Biol., No. 4, pp. 842-854]. Also, it is believed that Aurora C acts similarly as a result of interaction with Aurora B *[*J. Biol. Chem., Epub ahead (2004)]. From the fact that high expression of Aurora A has been hitherto confirmed in many cancer cells; that high expression of Aurora A in normal cells leads to transformation of normal cell strains of rodent; and the like, Aurora A, being one of oncogenes, is recognized to be an adequate target for an antitumor agent *[*EMBO J., No. 17, pp. 3052-3065 (1998)].

There is another report that cancer cells in which Aurora A is highly expressed have a resistance to paclitaxel [Cancer Cell, Vol. 3, pp. 51-62 (2003)]. Meanwhile, with regard to the Aurora kinase inhibitor, development of subtype-selective drugs has been thought to be difficult in view of high homology among subtypes, protein structure analysis and the like; and although there have been known reports on drugs such as ZM447439 which inhibit both Aurora A and Aurora B at the same time *[*J. Cell Biol., No. 161, pp. 267-280 (2003); J. Cell Biol., No. 161, pp. 281-294, (2003); Nat. Med., No. 10, pp. 262-267, (2004)], no report concerning Aurora A selective drugs have been known. Thus, in those reports, disclosed is the antitumor effect only for the case where a drug which inhibits both Aurora A and Aurora B at the same time is solely administered. In addition, there has been also reported a result that in a drug which inhibits both Aurora A and Aurora B at the same time, the Aurora kinase inhibiting action attenuates the action of paclitaxel *[*J. Cell Biol., No. 161, pp. 281-294, (2003)].

Now, patent applications concerning compounds having an Aurora kinase inhibiting action have been previously filed (WO 02/057259, U.S. Patent No. 6,664,247, etc.), and patent applications concerning aminopyridine derivatives has been filed as well (U.S. Patent No. 6,586,424, etc.). However, there has been no report on an aminopyridine derivative having an excellent Aurora A selective inhibitory action thus far. WO-A-02059112 (Vertex Pharmaceuticals Incorporated) discloses pyrazole compounds as protein kinase inhibitors, particularly Aurora-2 and GSK-3, for treating cancer, diabetes and Alzheimer's Disease.

### Disclosure of the Invention

The problems that the present invention should solve are to create novel aminopyridine derivatives which show an excellent Aurora A selective inhibitory action and cell-growth inhibitory action based on the foregoing, as well as achieve a synergistic action by a combined use with other antitumor agent(s).

In order to solve the above problems, the present inventors have synthesized a variety of novel aminopyridine derivatives and found that the compound represented by the following Formula (I) shows an excellent Aurora A selective inhibitory action and cell-growth inhibitory action based on the foregoing, and also achieves a synergistic action by a combined use with other antitumor agents, thus completing the invention. With regard to those cancers which have been unable to be completely treated with known antitumor agents such as paclitaxel because it has been impossible to use a sufficient amount of the agents owing to side-effects or drug resistance thereof, the administration of the compound according to the invention or the combined administration of the compound according to the invention with other antitumor agent is expected to exhibit an excellent antitumor effect (including potentiation of action due to the other antitumor agent) and an effect of attenuating side-effects.

Thus, the invention relates to a compound represented by Formula (I): 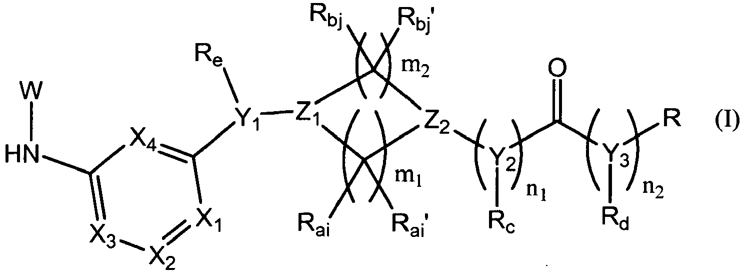 wherein:
m₁ is 2 or 3;
m₂ is 2;
n₁ is 0;
n₂ is 0;
i is an integer of any of 1 to m₁;
j is an integer of any of 1 to m₂;
R is phenyl of which 2^{nd} and 3^{rd} positions are substituted with the same or different halogen atoms or alternatively substituted with halogen atom and methyl substituted with one to three of the same or different halogen atoms, respectively;
Rₐᵢ and Rₐᵢ' are each independently hydrogen atom and lower alkyl;
R_{bj} and R_{bj}' are each independently hydrogen atom and lower alkyl;
wherein:
i is i₀ wherein i₀ is an integer of any 1 to m₁, and j is j₀ wherein j₀ is an integer of any 1 to m₂, so that one of Rₐᵢ₀ and Rₐᵢ₀' and one of R_{bj0} and R_{bj0}' may be combined to form -(CH₂)ₙ- wherein n is 1 or 2; and
Rₑ is a hydrogen atom or lower alkyl;
X₁ is CH, CX₁ₐ, or N wherein X₁ₐ is lower alkyl;
X₂ is CH or N;
X₃ is CH;
X₄ is N;
the number of nitrogen atoms among X₁, X₂ and X₄ is one or two;
Y₁, is CH or N; however, if Y₁ is CH and Rₑ is hydrogen atom, then the two hydrogen atoms may be substituted with oxo;
Z₁ is N and Z₂ is CH or N;

W is selected from: 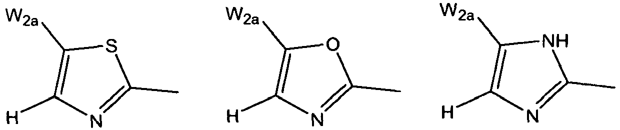 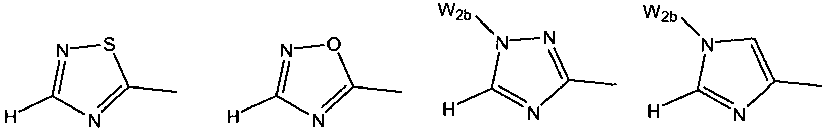 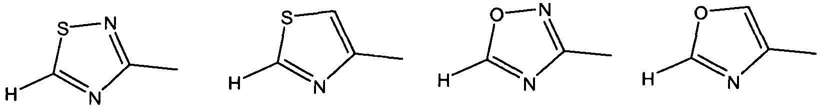 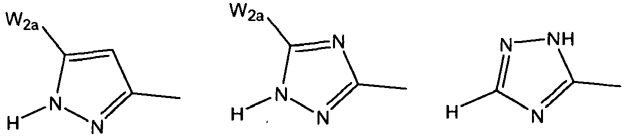 wherein W₂ₐ and W_{2b} are each independently hydrogen atom, halogen atom, cyano, C₁₋₂ lower alkyl, C₃₋₅ cycloalkyl, or C₁₋₂ lower alkyl which may be substituted with one or more halogen atoms; and wherein 'lower alkyl' means a linear or branched alkyl group having 1 to 6 carbon atoms;
with the proviso that when W is: 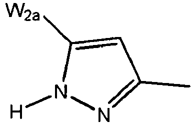 then X, is CH or CX₁ₐ and X₂ is N;
or a pharmaceutically acceptable salt thereof.

The invention also relates to a combined preparation for simultaneous, separate or sequential administration in the treatment of cancer, comprising two separate preparations which are:
* a preparation comprising, together with a pharmaceutically acceptable carrier or diluent, a compound represented by the above-described Formula (I) or a pharmaceutically acceptable salt thereof; and
* a preparation comprising, together with a pharmaceutically acceptable carrier or diluent, one antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, interferons, biological response modifiers and other antitumor agents as well as pharmaceutically acceptable salt(s) thereof, wherein:
   the antitumor alkylating agent is nitrogen mustard N-oxide, cyclophosphamide, ifosfamide, melphalan, busulfan, mitobronitol, carboquone, thiotepa, ranimustine, nimustine, temozolomide or carmustin;
   the antitumor antimetabolite is methotrexate, 6-mercaptopurine riboside, mercaptopurine, 5-fluorouracil, tegafur, doxyfluridine, carmofur, cytarabine, cytarabine ocfosfate, enocitabine, S-1, gemcitabine, fludarabine orpemetrexed disodium;
   the antitumor antibiotic is actinomycin D, doxorubicin, daunorubicin, neocarzinostatin, bleomycin, peplomycine, mitomycin C, aclarubicin, pirarubicin, epirubicin, zinostatin stimalamer, idarubicin, sirolimus or valrubicin;
   the plant-derived antitumor agent is vincristine, vinblastine, vindesine, etoposide, sobuzoxane, docetaxel, paclitaxel or vinorelbine;
   the antitumor platinum coordination compound is cisplatin, carboplatin, nedaplatin or oxaliplatin;
   the antitumor camptothecin derivative is irinotecan, topotecan or camptothecin;
   the antitumor tyrosine kinase inhibitor is gefitinib, imatinib or erlotinib;
   the monoclonal antibody is cetuximab, bevacizumab, rituximab, bevacizumab, alemtuzumab or trastuzumab;
   the interferon is interferon α, interferon α-2a, interferon α-2b, interferon β, interferon γ-1a or interferon γ-n1;
   the biological response modifier is krestin, lentinan, sizofiran, picibanil or ubenimex; and
   the other antitumor agent is mitoxantrone, L-asparaginase, procarbazine, dacarbazine; hydroxycarbamide, pentostatin, tretinoin, alefacept, darbepoetin alfa, anastrozole, exemestane, bicalutamide, leuprolelin, flutamide, fulvestrant, pegaptanib octasodium, denileukin diftitox, aldesleukin, thyrotropin alfa, arsenic trioxide, bortezomib, capecitabine or goserelin;

The invention further relates to a pharmaceutical composition comprising, together with a pharmaceutically acceptable carrier or diluent, a compound represented by the above-described Formula (I) or a a pharmaceutically acceptable salt thereof, and an antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, biological response modifiers and other antitumor agents (here, the definition of each antitumor agent is the same as that defined hereinabove) or a pharmaceutically acceptable salt thereof.

The invention still further considers a method for the treatment of cancer, comprising administering simultaneously, separately or sequentially a therapeutically effective amount of a compound represented by the above-described Formula (I) or a pharmaceutically acceptable salt thereof in combination with a therapeutically effective amount of an antitumor agent selected from the group consisting of antitumor alkylating agents, antitumor antimetabolites, antitumor antibiotics, plant-derived antitumor agents, antitumor platinum coordination compounds, antitumor camptothecin derivates, antitumor tyrosine kinase inhibitors, monoclonal antibodies, interferons, biological response modifiers and other antitumor agents (here, definition of each antitumor agent is the same as that defined hereinabove) or a pharmaceutically acceptable salt thereof.

Furthermore, the invention considers the use of an Aurora selective A inhibitor for the manufacture of a medicament for the treatment of cancer; and the use of an Aurora selective A inhibitor in combination with an antitumor agent for the manufacture of a medicament for the treatment of cancer; and also relates to a method of treating cancer to a mammal (particularly a human) which comprises administering to said mammal a therapeutically effective amount of an Aurora selective A inhibitor; and a method of treating cancer in a mammal (particularly a human) which comprises administering to said mammal a therapeutically effective amount of an Aurora selective A inhibitor in combination with a therapeutically effective amount of an antitumor agent.

The invention relates to a pharmaceutical composition comprsing as active ingredient an Aurora selective A inhibitor; and a pharmaceutical composition comprsing as active ingredient an Aurora selective A inhibitor, together with an antitumor agent.

Next, symbols and terms used in the present specification will be explained.

The term "lower alkyl" in the above Formula (I) denotes a linear or branched alkyl group having 1 to 6 carbon atoms, and examples thereof include, for example, methyl, ethyl, propyl, isopropyl, butyl, isobutyl, sec-butyl, tert-butyl, pentyl and hexyl, among these methyl being preferred.

The term "cycloalkyl" in the above Formula (I) denotes a 3- to 5-membered aliphatic cyclic group such as, for example, cyclopropyl, cyclobutyl and cyclopentyl.

The term "halogen atom" in the above Formula (I) is, for example, fluorine atom, chlorine atom, bromine atom or iodine atom. Among them, for example, fluorine atom, chlorine atom or bromine atom is preferred.

The term "selective inhibitor of Aurora A" used in the present specification is a compound or a drug which selectively inhibits Aurora A as compared with Aurora B. The "selective inhibitor of Aurora A" is preferably a compound or a drug of which inhibitory activities against Aurora A are at least ten times the activities against Aurora B; and more preferably a compound or a drug of which inhibitory activities against Aurora A are at least hundread times the activities against Aurora B.

Explanation for the term "pharmaceutically acceptable salt thereof" or the term "pharmaceutically acceptable carrier or diluent" used in the specification still will be given later.

The term "treatment of cancer" as used in the specification means inhibition of cancer cell growth by administering an antitumor agent to a cancer patient. Preferably, this treatment enables retrogression of cancer growth, that is, reduction in the measurable cancer size. More preferably, such treatment completely eliminates cancer.

The term "cancer" as used in the specification refers to solid cancer and hematopoietic cancer. Here, examples of solid cancer include cerebral tumor, head and neck cancer, esophageal cancer, thyroid cancer, small cell lung cancer, non-small cell lung cancer, breast cancer, stomach cancer, gallbladder and bile duct cancer, liver cancer, pancreas cancer, colon cancer, rectal cancer, ovarian cancer, chorioepithelioma, uterine cancer, cervical cancer, renal pelvic and ureteral cancer, bladder cancer, prostate cancer, penile cancer, testicular cancer, embryonal cancer, wilms tumor, skin cancer, malignant melanoma, neuroblastoma; osteosarcoma, Ewing's tumor and soft tissue sarcoma. On the other hand, examples of hematopoietic cancer include acute leukemia, chronic lymphatic leukemia, chronic myelocytic leukemia, polycythemia vera, malignant lymphoma, multiple myeloma and non-Hodgkins' lymphoma.

The term "preparation" as used in the specification includes oral preparations and parenteral preparations. Examples of oral preparations include tablets, capsules, powders and granules, while examples of parenteral preparations include sterilized liquid preparations such as solutions or suspensions, specifically injections or drip infusions. Preferably, they are intravenous injections or intravenous drip infusions, and more preferably intravenous drip infusions.

The term "combined preparation" as used in the specification refers to those comprising two or more preparations for simultaneous, separate or sequential administration in the treatment, and such preparation may be a so-called kit type preparation or pharmaceutical composition. The term "combined preparation" also includes those having one or more preparations further combined with the combined preparation comprising two separate preparations used in the treatment of cancer.

The two separate preparations described above can be further combined with, in combination with a pharmaceutically acceptable carrier or diluent, at least one preparation comprising at least one antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, interferons, biological response modifiers and other antitumor agents (here, definition of each antitumor agent is the same as that defined above), or a pharmaceutically acceptable salt thereof. In this case, the above-mentioned at least one preparation that has been further combined can be administered simultaneously, separately or sequentially with respect to the two separate preparations, For example, a combined preparation comprising three preparations may include that is comprised of a preparation including a preparation containing the compound represented by the above Formula (I), a preparation containing 5-fluorouracil and a preparation containing leucovorin.

Here, in the above-mentioned combined preparation, either or both of the two separate preparations may be parenteral preparations, preferably injections or drip infusions, and more preferably intravenous drip infusions.

The term "preparation" according to the invention may usually comprise a therapeutically effective amount of a compound according to the invention, together with a pharmaceutically acceptable carrier or diluent. This technique of formulation is considered to be a technical common knowledge to those having ordinary skill in the pertinent art and is well known. Preferably, intravenous drip infusions or injections can be prepared in combination with a pharmaceutically acceptable carrier or diluent, by various methods that are well known in the art.

In the case of using the combined preparation according to the invention, the term "administration" as used in the present specification refers to parenteral administration and/or oral administration, and preferably parenteral administration. Thus, when a combined preparation is administered, both administrations may be parenteral; one administration may be parenteral while the other may be oral; or both administrations may be oral. Preferably, both preparations in the combined preparation are administered parenterally. Here, the term "parenteral administration" is, for example, intravenous administration, subcutaneous administration or intramuscular administration, and preferably it is intravenous administration. Even when three or more preparations are combined and administered, at least one preparation may be parenterally administered, preferably intravenously administered, and more preferably intravenously infused or intravenously injected.

In the embodiment of the present invention, a compound represented by the above Formula (I) may be administered simultaneously with other antitumor agent(s). Further, it is possible to administer the compound represented by the above Formula (I) first and then another antitumor agent consecutively, or alternatively it is possible to administer another antitumor agent first and then the compound represented by the above Formula (I) consecutively. It is also possible to administer the compound represented by the above Formula (I) first and then separately administer another antitumor agent after a while, or alternatively it is possible to administer another antitumor agent first and then separately administer the compound represented by the above Formula (I) after a while. The order and the time interval for the administration may be appropriately selected by a person skilled in the art in accordance with, for example, a preparation containing the compound represented by the above Formula (I) used and a preparation containing an antitumor agent that is used in combination therewith, the type of the cancer cells to be treated and the condition of the patient. For example, in the case of administering the compound represented by the above Formula (I) and paclitaxel, preferably paclitaxel is administered first, and then the compound represented by the above Formula (I) is administered sequentially or separately after a while.

The term "simultaneously" as used in the specification refers to the use of preparations for the treatment substantially at the same time, whereas the term "separately" refers to the separate use of preparations for the treatment at different times such that, for example, one agent is used on the first day and another agent is used on the second day for the treatment. The term "sequentially" refers to the use of preparations in such an order that, for example, one agent is first used and another agent is used after a predetermined period of time for the treatment.

The term "antitumor alkylating agent" as used in the present specification refers to an alkylating agent having antitumor activity, and the term "alkylating agent" herein generally refers to an agent giving an alkyl group in the alkylation reaction in which a hydrogen atom of an organic compound is substituted with an alkyl group. The term "antitumor alkylating agent" may be exemplified by nitrogen mustard N-oxide, cyclophosphamide, ifosfamide, melphalan, busulfan, mitobronitol, carboquone, thiotepa, ranimustine, nimustine, temozolomide or carmustine.

The term "antitumor antimetabolite" as used in the specification refers to an antimetabolite having antitumor activity, and the term "antimetabolite" herein includes, in a broad sense, substances which disturb normal metabolism and substances which inhibit the electron transfer system to prevent the production of energy-rich intermediates, due to their structural or functional similarities to metabolites that are important for living organisms (such as vitamins, coenzymes, amino acids and saccharides). The term "antitumor antimetabolites" may be exemplified methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine or pemetrexed disodium, and preferred are 5-fluorouracil, S-1, gemcitabine and the like.

The term "antitumor antibiotic" as used in the specification refers to an antibiotic having antitumor activity, and the "antibiotic" herein includes substances that are produced by microorganisms and inhibit cell growth and other functions of microorganisms and of other living organisms. The term "antitumor antibiotic" may be exemplified by actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus or valrubicin.

The term "plant-derived antitumor agent" as used in the specification includes compounds having antitumor activities which originate from plants, or compounds prepared by applying chemical modification to the foregoing compounds. The term "plant-derived antitumor agent" may be exemplified by vincristine, vinblastine, vindesine, etoposide, sobuzoxane, docetaxel, paclitaxel and vinorelbine, and preferred and docetaxel and paclitaxel.

The term "antitumor camptothecin derivative" as used in the specification refers to compounds that are structurally related to camptothecin and inhibit cancer cell growth, including camptothecin *per* se. The term "antitumor camptothecin derivative" is not particularly limited to, but may be exemplified by, camptothecin, 10-hydroxycamptothecin, topotecan, irinotecan or 9-aminocamptothecin, with camptothecin, topotecan and irinotecan being preferred. Further, irinotecan is metabolized in vivo and exhibits antitumor effect as SN-38. The action mechanism and the activity of the camptothecin derivatives are believed to be virtually the same as those of camptothecin (e.g., Nitta, et al., Gan to Kagaku Ryoho, 14, 850-857 (1987)).

The term "antitumor platinum coordination compound" as used in the specification refers to a platinum coordination compound having antitumor activity, and the term "platinum coordination compound" herein refers to a platinum coordination compound which provides platinum in ion form. Preferred platinum compounds include cisplatin; cis-diamminediaquoplatinum (II)-ion; chloro(diethylenetriamine)-platinum (11) chloride; dichloro(ethylenediamine)-platinum (II); diammine(1,1-cyclobutanedicarboxylato) platinum (II) (carboplatin); spiroplatin; iproplatin; diammine(2-ethylmalonato)platinum (II); ethylenediaminemalonatoplatinum (II); aqua(1,2-diaminodicyclohexane)sulfatoplatinum (II); aqua(1,2-diaminodicyclohexane)malonatoplatinum (II); (1,2-diaminocyclohexane)malonatoplatinum (II); (4-carboxyphthalato)(1,2-diaminocyclohexane) platinum (II); (1,2-diaminocyclohexane)-(isocitrato)platinum (II); (1,2-diaminocyclohexane)oxalatoplatinum (II); ormaplatin; tetraplatin; carboplatin, nedaplatin and oxaliplatin, and preferred is carboplatin or oxaliplatin. Further, other antitumor platinum coordination compounds mentioned in the specification are known and are commercially available and/or producible by a person having ordinary skill in the art by conventional techniques.

The term "antitumor tyrosine kinase inhibitor" as used in the specification refers to a tyrosine kinase inhibitor having antitumor activity, and the term "tyrosine kinase inhibitor" herein refers to a chemical substance inhibiting "tyrosine kinase" which transfers a γ-phosphate group of ATP to a hydroxyl group of a specific tyrosine in protein. The term "antitumor tyrosine kinase inhibitor" may be exemplified by gefitinib, imatinib or erlotinib.

The term "monoclonal antibody" as used in the specification, which is also known as single clonal antibody, refers to an antibody produced by a monoclonal antibody-producing cell, and examples thereof include cetuximab, bevacizumab, rituximab, alemtuzumab and trastuzumab.

The term "interferon" as used in the specification refers to an interferon having antitumor activity, and it is a glycoprotein having a molecular weight of about 20,000 which is produced and secreted by most animal cells upon viral infection. It has not only the effect of inhibiting viral growth but also various immune effector mechanisms including inhibition of growth of cells (in particular, tumor cells) and enhancement of the natural killer cell activity, thus being designated as one type of cytokine. Examples of "interferon" include interferon α, interferon α-2a, interferon α-2b, interferon β, interferon γ-1a and interferon γ-n1.

The term "biological response modifier" as used in the specification is the so-called biological response modifier or BRM and is generally the generic term for substances or drugs for modifying the defense mechanisms of living organisms or biological responses such as survival, growth or differentiation of tissue cells in order to direct them to be useful for an individual against tumor, infection or other diseases. Examples of the "biological response modifier" include krestin, lentinan, sizofiran, picibanil and ubenimex.

The term "other antitumor agent" as used in the specification refers to an antitumor agent which does not belong to any of the above-described agents having antitumor activities. Examples of the "other antitumor agent" include mitoxantrone, L-asparaginase, procarbazine, dacarbazine, hydroxycarbamide, pentostatin, tretinoin, alefacept, darbepoetin alfa, anastrozole, exemestane, bicalutamide, leuprorelin, flutamide, fulvestrant, pegaptanib octasodium, denileukin diftitox, aldesleukin, thyrotropin alfa, arsenic trioxide, bortezomib, capecitabine, and goserelin.

The above-described terms "antitumor alkylating agent", "antitumor antimetabolite", "antitumor antibiotic", "plant-derived antitumor agent", "antitumor platinum coordination compound", "antitumor camptothecin derivative", "antitumor tyrosine kinase inhibitor", "monoclonal antibody", "interferon", "biological response modifier" and "other antitumor agent" are all known and are either commercially available or producible by a person skilled in the art by methods known *per se* or by well-known or conventional methods. The process for preparation of gefitinib is described, for example, in USP No. 5,770,599; the process for preparation of cetuximab is described, for example, in WO 96/40210; the process for preparation of bevacizumab is described, for example, in WO 94/10202; the process for preparation of oxaliplatin is described, for example, in USP Nos. 5,420,319 and 5,959,133; the process for preparation of gemcitabine is described, for example, in USP Nos. 5,434,254 and 5,223,608; and the process for preparation of camptothecin is described in USP Nos. 5,162,532, 5,247,089, 5,191,082, 5,200,524, 5,243,050 and 5,321,140; the process for preparation of irinotecan is described, for example, in USP No. 4,604,463; the process for preparation of topotecan is described, for example, in USP No. 5,734,056; the process for preparation of temozolomide is described, for example, in JP-B No. 4-5029; and the process for preparation of rituximab is described, for example, in JP-W No. 2-503143.

The above-mentioned antitumor alkylating agents are commercially available, as exemplified by the following: nitrogen mustard N-oxide from Mitsubishi Pharma Corp. as Nitromin (tradename); cyclophosphamide from Shionogi & Co., Ltd. as Endoxan (tradename); ifosfamide from Shionogi & Co., Ltd. as Ifomide (tradename); melphalan from GlaxoSmithKline Corp. as Alkeran (tradename); busulfan from Takeda Pharmaceutical Co., Ltd. as Mablin (tradename); mitobronitol from Kyorin Pharmaceutical Co., Ltd. as Myebrol (tradename); carboquone from Sankyo Co., Ltd. as Esquinon (tradename); thiotepa from Sumitomo Pharmaceutical Co., Ltd. as Tespamin (tradename); ranimustine from Mitsubishi Pharma Corp. as Cymerin (tradename); nimustine from Sankyo Co., Ltd. as Nidran (tradename); temozolomide from Schering Corp. as Temodar (tradename); and carmustine from Guilford Pharmaceuticals Inc. as Gliadel Wafer (tradename).

The above-mentioned antitumor antimetabolites are commercially available, as exemplified by the following: methotrexate from Takeda Pharmaceutical Co., Ltd. as Methotrexate (tradename); 6-mercaptopurine riboside from Aventis Corp. as Thioinosine (tradename); mercaptopurine from Takeda Pharmaceutical Co., Ltd. as Leukerin (tradename); 5-fluorouracil from Kyowa Hakko Kogyo Co., Ltd. as 5-FU (tradename); tegafur from Taiho Pharmaceutical Co., Ltd. as Futraful (tradename); doxyfluridine from Nippon Roche Co., Ltd. as Furutulon (tradename); carmofur from Yamanouchi Pharmaceutical Co., Ltd. as Yamafur (tradename); cytarabine from Nippon Shinyaku Co., Ltd. as Cylocide (tradename); cytarabine ocfosfate from Nippon Kayaku Co., Ltd. as Strasid(tradename); enocitabine from Asahi Kasei Corp. as Sanrabin (tradename); S-1 from Taiho Pharmaceutical Co., Ltd. as TS-1 (tradename); gemcitabine from Eli Lilly & Co. as Gemzar (tradename); fludarabine from Nippon Schering Co., Ltd. as Fludara (tradename); and pemetrexed disodium from Eli Lilly & Co. as Alimta (tradename).

The above-mentioned antitumor antibiotics are commercially available, as exemplified by the following: actinomycin D from Banyu Pharmaceutical Co., Ltd. as Cosmegen (tradename); doxorubicin from Kyowa Hakko Kogyo Co., Ltd. as adriacin (tradename); daunorubicin from Meiji Seika Kaisha Ltd. as Daunomycin; neocarzinostatin from Yamanouchi Pharmaceutical Co., Ltd. as Neocarzinostatin (tradename); bleomycin from Nippon Kayaku Co., Ltd. as Bleo (tradename); pepromycin from Nippon Kayaku Co, Ltd. as Pepro (tradename); mitomycin C from Kyowa Hakko Kogyo Co., Ltd. as Mitomycin (tradename); aclarubicin from Yamanouchi Pharmaceutical Co., Ltd. as Aclacinon (tradename); pirarubicin from Nippon Kayaku Co., Ltd. as Pinorubicin (tradename); epirubicin from Pharmacia Corp. as Pharmorubicin (tradename); zinostatin stimalamer from Yamanouchi Pharmaceutical Co., Ltd. as Smancs (tradename); idarubicin from Pharmacia Corp. as Idamycin (tradename); sirolimus from Wyeth Corp. as Rapamune (tradename); and valrubicin from Anthra Pharmaceuticals Inc. as Valstar (tradename).

The above-mentioned plant-derived antitumor agents are commercially available, as exemplified by the following: vincristine from Shionogi & Co., Ltd. as Oncovin (tradename); vinblastine from Kyorin Pharmaceutical Co., Ltd. as Vinblastine (tradename); vindesine from Shionogi & Co., Ltd. as Fildesin (tradename); etoposide from Nippon Kayaku Co., Ltd. as Lastet (tradename); sobuzoxane from Zenyaku Kogyo Co., Ltd. as Perazolin (tradename); docetaxel from Aventis Corp. as Taxsotere (tadename); paclitaxel from Bristol-Myers Squibb Co. as Taxol (tradename); and vinorelbine from Kyowa Hakko Kogyo Co., Ltd. as Navelbine (tradename).

The above-mentioned antitumor platinum coordination compounds are commercially available, as exemplified by the following: cisplatin from Nippon Kayaku Co., Ltd. as Randa (tradename); carboplatin from Bristol-Myers Squibb Co. as Paraplatin (tradename); nedaplatin from Shionogi & Co., Ltd. as Aqupla (tradename); and oxaliplatin from Sanofi-Synthelabo Co. as Eloxatin (tradename).

The above-mentioned antitumor camptothecin derivatives are commercially available, as exemplified by the following: irinotecan from Yakult Honsha Co., Ltd. as Campto (tradename); topotecan from GlaxoSmithKline Corp. as Hycamtin (tradename); and camptothecin from Aldrich Chemical Co., Inc., U.S.A.

The above-mentioned antitumor tyrosine kinase inhibitors are commercially available, as exemplified by the following: gefitinib from AstraZeneca Corp. as Iressa (tradename); imatinib from Novartis AG as Gleevec (tradename); and erlotinib from OSI Pharmaceuticals Inc. as Tarceva (tradename).

The above-mentioned monoclonal antibodies are commercially available, as exemplified by the following: cetuximab from Bristol-Myers Squibb Co. as Erbitux (tradename); bevacizumab from Genentech, Inc. as Avastin (tradename); rituximab from Biogen Idec Inc. as Rituxan (tradename); alemtuzumab from Berlex Inc. as Campath (tradename); and trastuzumab from Chugai Pharmaceutical Co., Ltd. as Herceptin (tradename).

The above-mentioned interferons are commercially available, as exemplified by the following: interferon α from Sumitomo Pharmaceutical Co., Ltd. as Sumiferon (tradename); interferon α-2a from Takeda Pharmaceutical Co., Ltd. as Canferon-A (tradename); interferon α-2b from Schering-Plough Corp. as Intron A (tradename); interferon β from Mochida Pharmaceutical Co., Ltd. as IFNβ (tradename); interferon γ-1a from Shionogi & Co., Ltd. as Imunomax-γ (tradename); and interferon γ-n1 from Otsuka Pharmaceutical Co., Ltd. as Ogamma (tradename).

The above-mentioned biological response modifiers are commercially available, as exemplified by the following: krestin from Sankyo Co., Ltd. as krestin (tradename); lentinan from Aventis Corp. as Lentinan (tradename); sizofiran from Kaken Seiyaku Co., Ltd. as Sonifiran (tradename); picibanil from Chugai Pharmaceutical Co., Ltd. as Picibanil (tradename); and ubenimex from Nippon Kayaku Co., Ltd. as Bestatin (tradename).

The above-mentioned other antitumor agents are commercially available, as exemplified by the following: mitoxantrone from Wyeth Lederle Japan, Ltd. as Novantrone (tradename); L-asparaginase from Kyowa Hakko Kogyo Co., Ltd. as Leunase (tradename); procarbazine from Nippon Roche Co., Ltd. as Natulan (tradename); dacarbazine from Kyowa Hakko Kogyo Co., Ltd. as Dacarbazine (tradename); hydroxycarbamide from Bristol-Myers Squibb Co. as Hydrea (tradename); pentostatin from Kagaku Oyobi Kessei Ryoho Kenkyusho as Coforin (tradename); tretinoin from Nippon Roche Co., Ltd. As Vesanoid (tradename); alefacept from Biogen Idec Inc. as Amevive (tradename); darbepoetin alfa from Amgen Inc. as Aranesp (tradename); anastrozole from AstraZeneca Corp. as Arimidex (tradename); exemestane from Pfizer Inc. as Aromasin (tradename); bicalutamide from AstraZeneca Corp. as Casodex (tradename); leuprorelin from Takeda Pharmaceutical Co., Ltd. as Leuplin (tradename); flutamide from Schering-Plough Corp. as Eulexin (tradename); fulvestrant from AstraZeneca Corp. as Faslodex (tradename); pegaptanib octasodium from Gilead Sciences, Inc. as Macugen (tradename); denileukin diftitox from Ligand Pharmaceuticals Inc. as Ontak (tradename); aldesleukin from Chiron Corp. as Proleukin (tradename); thyrotropin alfa from Genzyme Corp. as Thyrogen (tradename); arsenic trioxide from Cell Therapeutics, Inc. as Trisenox (tradename); bortezomib from Millennium Pharmaceuticals, Inc. as Velcade (tradename); capecitabine from Hoffmann-La Roche, Ltd. as Xeloda (tradename); and goserelin from AstraZeneca Corp. as Zoladex (tradename).

The term "antitumor agent" as used in the specification includes the above-described "antitumor alkylating agent", "antitumor antimetabolite", "antitumor antibiotic", "plant-derived antitumor agent", "antitumor platinum coordination compound", "antitumor camptothecin derivative", "antitumor tyrosine kinase inhibitor", "monoclonal antibody", "interferon", "biological response modifier" and "other antitumor agent".

The term "aminopyridine derivative" as used in the specification includes, but is not limited to, any compound having a pyridyl group substituted with an amino group. It is exemplified by a compound of the above General Formula (I), and preferably any one compound of the below-mentioned (a) to (cc).

Embodiments of the compound represented by the above General Formula (I) will be illustrated in more detail.

Preferably m₁ is 2. For example, with regard to Rₐᵢ and Rₐᵢ' (wherein i is an integer of 1 to m₁), and R_{bj} and R_{bj}' (wherein j is an integer of 1 to m₂), if m₁ is 2 and m₂ is 2, then either of Rₐ₂ and Rₐ₂' and either of R_{b1} and R_{b1}' are combined to form -CH₂-.

With regard to Rₐᵢ and Rₐᵢ' (wherein i is an integer of 1 to m₁), and R_{bj} and R_{bj}' (wherein j is an integer of 1 to m₂), if m₁ is 2 or 3 and m₂ is 2, then Rₐᵢ, Rₐᵢ', R_{bj} and R_{bj}', preferably all of Rₐᵢ, Rₐᵢ', R_{bj} and R_{bj}', are hydrogen atom, or any one of Rₐᵢ, Rₐᵢ', R_{bj} and R_{bj}' is methyl while the others are hydrogen atom; more preferably, all of Rₐᵢ, Rₐᵢ', R_{bj} and R_{bj}' are hydrogen atom. For example, if m₁ is 2 and m₂ is 2, R_{a1,} Rₐ₁', Rₐ₂, Rₐ₂', R_{b1}, R_{b1}', R_{b2} and R_{b2}' are preferably all hydrogen atoms.

Rₑ is a hydrogen atom or lower alkyl.

Rₑ is preferably a hydrogen atom. X₁ is preferably CH.

Preferably X₁ and X₃ are each CH, X₂ is CH or N, and X₄ is N; and particularly preferably X₁, X₂ and X₃ are all CH, while X₄ is N.

Y₁ is preferably CH.

Preferably, both Z₁ and Z₂ are N.

W is preferably selected from the following: 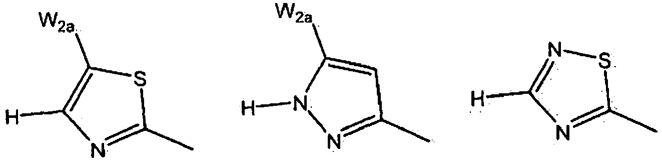 wherein W₂ₐ is hydrogen atom, halogen atom, cyano, or methyl which may be substituted with one to three fluorine atoms.

W is particularly preferably selected from the following:

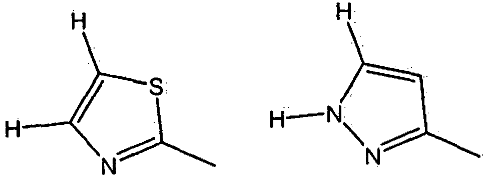

The compound of the above General Formula (I) is preferably:
(a) 6-((4-(3-chlor6-2-fluorobenzoyl)piperazin-1-yl) methyl)-N-thiazol-2-ylpyridin-2-amine,
(b) 6-((4-(2,3-dichlorobenzoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine,
(d) 6-((4-(3-chloro-2-fluorobenzoyl)piperazin-1-yl)methyl)-N-(1H-pyrazol-3-yl)pyridin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine; .
(g) 6-((4-(3-chloro-2-fluorobenzoyl)piperazin-1-yl)methyl)-N-(5-methyl-1H-pyrazol-3-yl): pyridin-2-amine,
(h) 6-((4-(2-fluoro-3-(trifluoromethyl)benzoyl)piperazin-1-yl)methyl)-N-(5-methyl-1H-pyrazol-3-yl)pyridin-2-amine,
(i) 6-((4-(2-chloro-3-(trifluoromethyl)benzoyl)piperazin-1-yl)methyl)-N-(5-methyl-1 H-pyrazol-3-yl)pyridin-2-amine,
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluoromethyl)benzoyl)piperazin-1-yl)methyl)-N-1,2,4-thiadiazol-5-ylpyridin-2-amine,
(l) 6-((4-(3-chloro-2-fluorobenzoyl)piperazin-1-yl)methyl)-N-1H-pyrazol-3-ylpyrazin-2-amine,
(m) 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine,
(q) 6-((4-(3-chloro-2-fluorobenzoyl)piperazin-1-yl)methyl)-N-thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 2-((4-(3-chloro-2-fluorobenzoyl)piperazin-1-yl)methyl)-N,N-dimethyl-6-(thiazol-2-yl)aminoisonicotinic amide,
(t) (2-((4-(3-chloro-2-fluorobenzoyl)piperazin-1-yl)methyl)-6-(thiazol-2-ylamino)pyridin-4-yl)methanol,
(u) 6-((4-(3-chloro-2-fluorobenzoyl)piperazin-1-yl)methyl)-4-(methoxymethyl)-N-thiazol-2-ylpyridin-2-amine,
(v) 1-(2-((4-(3-chloro-2-fluorobenzoyl)piperazin-1-yl)methyl)-6-(thiazol-2-ylamino) pyridin-4-yl)-3-methylimidazolidin-2-one,
(w) 2-((4-(3-chloro-2-fluorobenzoyl)piperazin-1-yl)methyl)-6-(thiazol-2-ylamino) isonicotinonitrile,
(x) 6-((4-(3-chloro-2-fluorobenzoyl)piperazin-1-yl)methyl)-4-(1-methyl-1H-tetrazol-5-yl)-N-thiazol-2-ylpyridin-2-amine,
(y) 6-((4-(3-chloro-2-fluorobenzoyl)piperazin-1-yl)methyl)-4-(2-methyl-2H-tetrazol-5-yl)-N-thiazol-2-ylpyridin-2-amine,
(z) 6-((4-(3-chloro-2-fluorobenzoyl)piperazin-1-yl)methyl)-N-thiazol-2-yl-4-(1H-1,2,4-triazol-5-yl)pyridin-2-amine,
(aa) 6-((4-(3-chloro-2-fluorobenzoyl)piperazin-1-yl)methyl)-N-(5-fluorothiazol-2-yl) pyridin-2-amine;
(bb) 6-((4-(2-fluoro-3-trifluoromethylbenzoyl)piperazin-1-yl)methyl)-N-(5-methyl-1 H-pyrazol-3-yl)pyrazin-2-amine; or
(cc) 2-{[4-(3-chloro-2-fluorobenzoyl)piperazin-1-yl]methyl}-N,N-dimethyl-6-(1H-pyrazol-3-ylamino)isonicotinamide,
or a pharmaceutically acceptable salt thereof.

In another embodiment, the present invention relates to a compound which is:
(a) 6-((4-(3-chloro-2-fluorobenzoyl)piperazin-1-yl) methyl)-N-thiazol-2-ylpyridin-2-amine,
(b) 6-((4-(2,3-dichlorobenzoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine,
(d) 6-((4-(3-chloro-2-fluorobenzoyl)piperazin-1-yl)methyl)-N-(1H-pyrazol-3-yl)pyrazin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine,
(g) 6-((4-(3-chloro-2-fluorobenzoyl)piperazin-1-yl)methyl)-N-(5-methyl-1H-pyrazol-3-yl) pyridin-2-amine;
(h) 6-((4-(2-fluoro-3-(trifluoromethyl)benzoyl)piperazin-1-yl)methyl)-N-(5-methyl-1H-pyrazol-3-yl)pyridin-2-amine,
(i) 6-((4-(2-chloro-3-(trifluoromethyl)benzoyl)piperazin-1-yl)methyl)-N-(5-methyl-1H-pyrazol-3-yl)pyridin-2-amine,
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluoromethyl)benzoyl)piperazin-1-yl)methyl)-N-1,2,4-thiadiazol-5-ylpyridin-2-amine,
(l) 6-((4-(3-chloro-2-fluorobenzoyl)piperazin-1-yl)methyl)-N-1H-pyrazol-3-ylpyrazin-2-amine,
(m) 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine,
(q) 6-((4-(3-chloro-2-fluorobenzoyl)piperazin-1-yl)methyl)-N-thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 6-((4-(3-chloro-2-fluorobenzoyl)piperazin-1-yl)methyl)-N-(5-fluorothiazol-2-yl) pyridin-2-amine; or
(t) 6-((4-(2-fluoro-3-trifluoromethylbenzoyl)piperazin-1-yl)methyl)-N-(5-methyl-1 H-pyrazol-3-yl)pyrazin-2-amine;
or a pharmaceutically acceptable salt thereof.

Further, in the combined preparation comprising two separate preparations according to the invention, preferably either or both of the two separate preparations are parenteral preparations, and more preferably either or both of the two separate preparations are injections or drip infusions.

The combined preparation comprising two separate preparations according to the invention is preferably such that one of the preparations is a preparation containing the following:
(a) 6-((4-(3-chloro-2-fluorobenzoyl)piperazin-1-yl) methyl)-N-thiazol-2-ylpyridin-2-amine,
(b) 6-((4-(2,3-dichlorobenzoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine,
(d) 6-((4-(3-chloro-2-fluorobenzoyl)piperazin-1-yl)methyl)-N-(1H-pyrazol-3-yl)pyridin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine,
(g) 6-((4-(3-chloro-2-fluorobenzoyl)piperazin-1-yl)methyl)-N-(5-methyl-1H-pyrazol-3-yl) pyridin-2-amine,
(h) 6-((4-(2-fluoro-3-(trifluoromethyl)benzoyl)piperazin-1-yl)methyl)-N-(5-methyl-1H-pyrazol-3-yl)pyridin-2-amine,
(i) 6-((4-(2-chloro-3-(trifluoromethyl)benzoyl)piperazin-1-yl)methyl)-N-(5-methyl-1 H-pyrazol-3-yl)pyridin-2-amine,
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluoromethyl)benzoyl)piperazin-1-yl)methyl)-N-1,2,4-thiadiazol-5-ylpyridin-2-amine,
(l) 6-((4-(3-chloro-2-fluorobenzoyl)piperazin-1-yl)methyl)-N-1H-pyrazol-3-ylpyrazin-2-amine,
(m) 6-(((1S,4S)-5-(3-chloro-2-fluorobenzoyl) 2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine,
(q) 6-((4-(3-chloro-2-fluorobenzoyl)piperazin-1-yl)methyl)-N-thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 2-((4-(3-chloro-2-fluorobenzoyl)piperazin-1-yl)methyl)-N,N-dimethyl-6-(thiazol-2-yl)aminoisonicotinic amide,
(t) (2-((4-(3-chloro-2-fluorobenzoyl)piperazin-1-yl)methyl)-6-(thiazol-2-ylamino)pyridin-4-yl)methanol,
(u) 6-((4-(3-chloro-2-fluorobenzoyl)piperazin-1-yl)methyl)-4-(methoxymethyl)-N-thiazol-2-ylpyridin-2-amine;
(v) 1-(2-((4-(3-chloro-2-fluorobenzoyl)piperazin-1-yl)methyl)-6-(thiazol-2-ylamino) pyridin-4-yl)-3-methylimidazolidin-2-one,
(w) 2-((4-(3-chloro-2-fluorobenzoyl)piperazin-1-yl)methyl)-6-(thiazol-2-ylamino) isonicotinonitrile,
(x) 6-((4-(3-chloro-2-fluorobenzoyl)piperazin-1-yl)methyl)-4-(1-methyl-1H-tetrazol-5-yl)-N-thiazol-2-ylpyridin-2-amine,
(y) 6-((4-(3-chloro-2-fluorobenzoyl)piperazin-1-yl)methyl)-4-(2-methyl-2H-tetrazol-5-yl)-N-thiazol-2-ylpyridin-2-amine,
(z) 6-((4-(3-chloro-2-fluorobenzoyl)piperazin-1-yl)methyl)-N-thiazol-2-yl-4-(1H-1,2,4-triazol-5-yl)pyridin-2-amine,
(aa) 6-((4-(3-chloro-2-fluorobenzoyl)piperazin-1-yl)methyl)-N-(5-fluorothiazol-2-yl) pyridin-2-amine;
(bb) 6-((4-(2-fluoro-3-trifluoromethylbenzoyl)piperazin-1-yl)methyl)-N-(5-methyl-1H-pyrazol-3-yl)pyrazin-2-amine; or
(cc) 2-{[4-(3-chloro-2-fluorobenzoyl)piperazin-1-yl]methyl}-N,N-dimethyl-6-(1H-pyrazol-3-ylamino)isonicotinamide,
or a pharmaceutically acceptable salt thereof; and
the other preparation is a preparation containing paclitaxel or docetaxel, or a pharmaceutically acceptable salt thereof, together with a pharmaceutically acceptable carrier or diluent.

In another embodiment, the combined preparation comprising two separate preparations according to the invention is more preferably such that one of the preparations is a preparation containing the following:
(a) 6-((4-(3-chloro-2-fluorobenzoyl)piperazin-1-yl) methyl)-N-thiazol-2-ylpyridin-2-amine,
(b) 6-((4-(2,3-dichlorobenzoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine,
(d) 6-((4-(3-chloro-2-fluorobenzoyl)piperazin-1-yl)methyl)-N-(1H-pyrazol-3-yl)pyridin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine,
(g) 6-((4-(3-chloro-2-fluorobenzoyl)piperazin-1-yl)methyl)-N-(5-methyl-1H-pyrazol-3-yl) pyridin-2-amine,
(h) 6-((4-(2-fluoro-3-(trifluoromethyl)benzoyl)piperazin-1-yl)methyl)-N-(5-methyl-1H-pyrazol-3-yl)pyridin-2-amine,
(i) b-((4-(2-chloro-3-(trifluoromethyl)benzoyl)piperazin-1-yl)methyl)-N-(5-methyl-1H-pyrazol-3-yl)pyridin-2-amine,
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluoromethyl)benzoyl)piperazin-1-yl)methyl)-N-1,2,4-thiadiazol-5-ylpyridin-2-amine;
(l) 6-((4-(3-chloro-2-fluorobenzoyl)piperazin-1-yl)methyl)-N-1H-pyrazol-3-ylpyrazin-2-amine;
(m) 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine,
(q) 6-((4-(3-chloro-2-fluorobenzoyl)piperazin-1-yl)methyl)-N-thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 6-((4-(3-chloro-2-fluorobenzoyl)piperazin-1-yl)methyl)-N-(5-fluorothiazol-2-yl) pyridin-2-amine; or
(t) 6-((4-(2-fluoro-3-trifluoromethylbenzoyl)piperazin-1-yl)methyl)-N-(5-methyl-1H-pyrazol-3-yl)pyrazin-2-amine;
or a pharmaceutically acceptable salt thereof; and
the other preparation is a preparation containing paclitaxel or docetaxel, or a pharmaceutically acceptable salt thereof, together with a pharmaceutically acceptable carrier or diluent.

Moreover, the combined preparation comprising, together with a pharmaceutically acceptable carrier or diluent, two separate preparations according to the invention may be further combined with at least one preparation containing an antitumor agent selected from the group consisting of antitumor alkylating agents, antitumor antimetabolites, antitumor antibiotics, plant-derived antitumor agents, antitumor platinum coordination compounds, antitumor camptothecin derivatives, antitumor tyrosine kinase inhibitors, monoclonal antibodies, interferons, biological response modifiers and other antitumor agents (here, definition of each antitumor agent is the same as that defined above), or a pharmaceutically acceptable salt thereof.

Also, the pharmaceutical composition according to the invention preferably contains the following:
(a) 6-((4-(3-chloro-2-fluorobenzoyl)piperazin-1-yl) methyl)-N-thiazol-2-ylpyridin-2-amine,
(b) 6-((4-(2,3-dichlorobenzoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine,
(d) 6-((4-(3-chloro-2-fluorobenzoyl)piperazin-1-yl)methyl)-N-(1H-pyrazol-3-yl)pyridin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine,
(g) 6-((4-(3-chloro-2-fluorobenzoyl)piperazin-1-yl)methyl)-N-(5-methyl-1H-pyrazol-3-yl) pyridin-2-amine,
(h) 6-((4-(2-fluoro-3-(trifluormethyl)benzyl)piperazin-1-yl)methyl)-N-(5-methyl-1H-pyrazol-3-yl)pyridin-2-amine,
(i) 6-((4-(2-chloro-3-(trifluoromethyl)benzoyl)piperazin-1-yl)methyl)-N-(5 -methyl-1H-pyrazol-3-yl)pyridin-2-amine,
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluoromethyl)benzoyl)piperazin-1-yl)methyl)-N-1,2,4-thiadiazol-5-ylpyridin-2-amine,
(l) 6-((4-(3-chloro-2-fluorobenzoyl)piperazin-1-yl)methyl)-N-1H-pyrazol-3-ylpyrazin-2-amine,
(m) 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine;
(q) 6-((4-(3-chloro-2-fluorobenzoyl)piperazin-1-yl)methyl)-N=thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 2-((4-(3-chloro-2-fluorobenzoyl)piperazin-1-yl)methyl)-N,N-dimethyl-6-(thiazol-2-yl)aminoisonicotinic amide,
(t) (2-((4-(3-chloro-2-fluorobenzoyl)piperazin-1-yl)methyl)-6-(thiazol-2-ylamino)pyridin-4-yl)methanol,
(u) 6-((4-(3-chloro-2-fluorobenzoyl)piperazin-1-yl)methyl)-4-(methoxymethyl)-N-thiazol-2-ylpyridin-2-amine,
(v) 1-(2-((4-(3-chloro-2-fluorobenzoyl)piperazin-1-yl)methyl)-6-(thiazol-2-ylamino) pyridin-4-yl)3-methylimidazolidin-2-one,
(w) 2-((4-(3-chloro-2-fluorobenzoyl)piperazin-1-yl)methyl)-6-(thiazol-2-ylamino) isonicotinonitrile,
(x) 6-((4-(3-chloro-2-fluorobenzoyl)piperazin-1-yl)methyl)-4-(1-methyl-1H-tetrazol-5-yl)-N-thiazol-2-ylpyridin-2-amine,
(y) 6-((4-(3-chloro-2-fluorobenzoyl)piperazin-1-yl)methyl)-4-(2-methyl-2H-tetrazol-5-yl)-N-thiazol-2-ylpyridin-2-amine,
(z) 6-((4-(3-chloro-2-fluorobenzoyl)piperazin-1-yl)methyl)-N-thiazol-2-yl-4-(1H-1,2,4-triazol-5-yl)pyridin-2-amine,
(aa) 6-((4-(3-chloro-2-fluorobenzoyl)piperazin-1-yl)methyl)-N-(5-fluorothiazol-2-yl) pyridin-2-amine;
(bb) 6-((4-(2-fluoro-3-trifluoromethylbenzoyl)piperazin-1-yl)methyl)-N-(5-methyl-1H-pyrazol-3-yl)pyrazin-2-amine; or
(cc) 2- {[4-(3-chloro-2-fluorobenzoyl)piperazin-1-yl]methyl}-N,N-dimethyl-6-(1H-pyrazol-3-ylamino)isonicotinamide;
or a pharmaceutically acceptable salt thereof; and paclitaxel or docetaxel, or a pharmaceutically acceptable salt thereof, together with a pharmaceutically acceptable carrier or diluent.

### Description of the process for preparation of compound of General Formula (I)

Among the compounds represented by the General Formula (I): 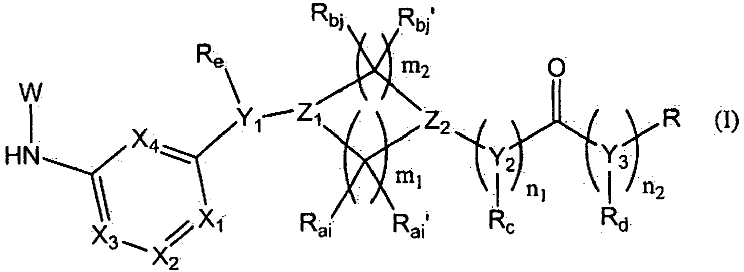 (wherein m₁, m₂, n₁, n₂, i, j, R, Rₐᵢ, Rₐᵢ', R_{bj}, R_{bj}', R_{c}, R_{d}, Rₑ, X₁, X₂, X₃, X₄, Y₁, Y₂, Y₃, Z₁, Z₂ and W have the same meaning as defined in the above) according to the invention, the compound of Formula (I-1) in which Y₁ is CH and Z₁ is N: 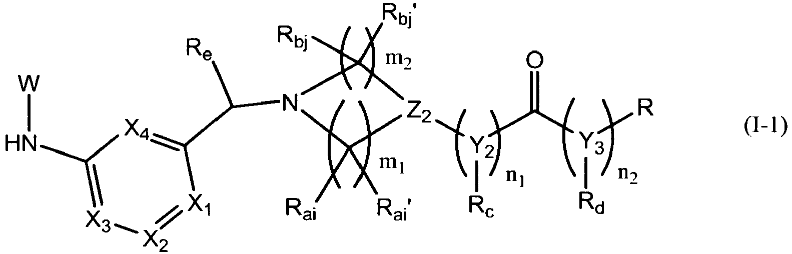 (wherein the symbols have the same meaning as the symbols for the above Formula (I)) can be prepared by, for example, the following method. Hereinafter, it is meant by the term "symbols for the above Formula (I)" in the phrase "same meaning as the symbols for the above Formula (I)," that "the respective symbols as described for General Formula (I) initially described in the present specification."

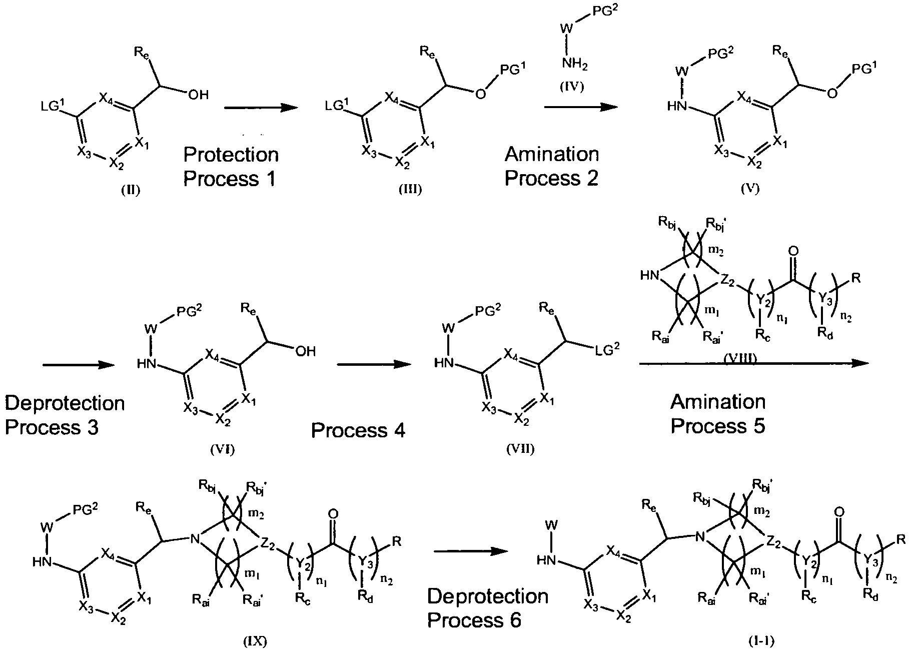

(Process 1) The present process is a method of introducing a protective group PG¹ such as a tert-butyldimethylsilyl group to Compound (II) (wherein LG¹ represents a leaving group such as halogen, and X₁, X₂, X₃, X₄ and Rₑ have the same meaning as the symbols for the above Formula (I)), to produce Compound (III) (wherein LG¹ and PG¹ have the same meaning as defined above, and X₁, X₂, X₃, X₄ and Rₑ have the same meaning as the symbols for the above Formula (I)).

The above-mentioned Compound (II) used in this process may be exemplified by (6-bromopyridin-2-yl)methanol, 1-(6-bromopyridin-2-yl)ethanol or (3-iodophenyl)methanol. The above-mentioned Compound (II) is commercially available or can be prepared by known methods.

As to the protective group PG¹, a method of protection may vary depending on the type of the protective group, but methods described in the literature [See T.W. Greene, Protective Groups in Organic Synthesis, John Wiley & Sons (1981)] or methods equivalent thereto can be utilized. For example, the Compound (II) can be synthesized by using tert-butyldimethylsilyl chloride in a solvent such as N,N-dimethylformamide in the presence of a base such as imidazole. When tert-butyldimethylsilyl chloride is used in a protection reaction, tert-butyldimethylsilyl chloride is used in an amount of from 1 to 10 mol, preferably from 1 to 3 mol, and the base is used in an amount of from 1 to 20 mol, preferably from I to 5 mol, relative to 1 mol of Compound (II). In this case, the reaction temperature may be appropriately selected by a person skilled in the art in accordance with the starting compound or reaction solvent used, but it is typically from 0° to the boiling point of the solvent. Also, the reaction is typically completed within 1 hour to 24 hours, but the reaction time can be appropriately extended or reduced.

Thus obtained Compound (III) is subjected to isolation and purification by known separation and purification means such as, for example, concentration, concentration under reduced pressure, crystallization, solvent extraction, reprecipitation or chromatography, or is subjected to the next process without isolation and purification.

(Process 2) The present process is a method of subjecting Compound (III) obtained by the above-described Process 1 (wherein LG¹ and PG¹ have the same meaning as defined above, and X₁, X₂, X₃, X₄ and Rₑ have the same meaning as the symbols for the above Formula (I)) and Compound (IV) (wherein PG² may be absent, or if present, it is a protective group such as 4-methoxybenzyl, 2,4-dimethoxybenzyl, benzyl, methoxymethyl, (2-(trimethylsilyl)ethoxy)methyl or tert-butyl, preferably (2-(trimethylsilyl)ethoxy)methyl, methoxymethyl or tert-butyl, and W has the same meaning as the symbol for the above Formula (I)) to an amination reaction to produce Compound (V) (wherein PG¹ and PG² have the same meaning as defined above, and X₁, X₂, X₃, X₄ and Rₑ have the same meaning as the symbols for the above Formula (I)).

The above-mentioned Compound (IV) used in this process may be exemplified by 2-aminothiazol-5-carbonitrile, 2-aminothiazole, 2-amino-5-methylthiazole, 5-amino-1,2,4-thiadiazole, 5-methyl-1-((2-(trimethylsilyl)ethoxy)methyl)-1H-pyrazol-3-amine, 1-((2-(trimethylsilyl)ethoxy)methyl)-1H-pyrazol-3-amine, or 1-tert-butyl-3-methyl-1H-pyrazol-5-amine. The Compound (IV) is commercially available or can be prepared by known methods (e.g., Phosphorus, Sulfur and Silicon and the Related Elements, Vol.177, No.11, pages 2651-2659 (2002), and Journal of Chemical Research, Synopses, Vol.6, page 198 (1979)).

The amination reaction used in this process employs methods well known to those skilled in the art. In the amination reaction used in the process, specifically, for example, synthesis can be performed by reacting the above-mentioned Compound (III) and Compound (IV) in a solvent such as 1,4-dioxane, 1,2-dimethoxyethane, tetrahydrofuran, methylene chloride, chloroform or toluene, using a palladium catalyst such as trisdibenzylideneacetone dipalladium (0) or palladium acetate, a ligand such as 2,2'-bisdiphenylphosphino-1,1'-binaphthyl or 4,5-bis(diphenylphosphino)-9,9-dimethylxanthene, and a base such as cesium carbonate or sodium t-butoxide. In the reaction, with respect to 1 mol of compound (III), 0.5 to 3 mol, preferably 1 mol, of Compound (IV) is used; 0.001 to 1 mol, preferably 0.05 to 0.5 mol, of palladium catalyst is used; 0.002 to 2 mol, preferably 0.1 to 1.0 mol, of ligand is used; and 1 to 10 mol, preferably 1 to 3 mol, of base is used. The reaction temperature is appropriately selected by a person skilled in the art in accordance with the starting compound or reaction solvent used, but it is typically from 50°C to the boiling point of the solvent used in the reaction. Also, the reaction is typically completed within 1 hour to 24 hours, but the reaction time can be appropriately extended or reduced.

Thus obtained Compound (V) is subjected to isolation and purification by known separation and purification means such as, for example, concentration, concentration under reduced pressure, crystallization, solvent extraction, reprecipitation or chromatography, or subjected to the next process without isolation and purification.

(Process 3) The present process is a method of deprotecting Compound (V) obtained in the above-described Process 2 (wherein PG¹ and PG² have the same meaning as defmed above, and X₁, X₂, X₃, X₄, Rₑ and W have the same meaning as the symbols for the above Formula (I)) by removing protective group PG¹ to produce Compound (VI) (wherein PG² has the same meaning as defined above, and X₁, X₂, X₃, X₄, Rₑ and W have the same meaning as the symbols for the above Formula (I)).

For removal of the protective group PG¹ used in this process, the method of removal may vary depending on the type of the protective group and stability of the compound, but methods described in the literature [See T.W. Greene, Protective Groups in Organic Synthesis, John Wiley & Sons (1981)] or methods equivalent thereto can be carried out. For example, Compound (V) in which PG¹ is tert-butyldimethylsilyl can be deprotected in a solvent such as tetrahydrofuran using tetrabutylammonium fluoride. When tetrabutylammonium fluoride is used in the deprotection reaction, tetrabutylammonium fluoride is used in an amount of from 1 to 10 mol, preferably from 1 to 3 mol, relative to 1 mol of Compound (V). The reaction temperature can be appropriately selected by a person having ordinary skill in the art in accordance with the starting compound or reaction solvent used, but it is typically from 0°C to the boiling point of the solvent. Also, the reaction is typically completed within 1 hour to 24 hours, but the reaction time can be appropriately extended or reduced.

Thus obtained Compound (VI) is subjected to isolation and purification by known separation and purification means such as, for example, concentration, concentration under reduced pressure, crystallization, solvent extraction, reprecipitation or chromatography, or subjected to the next process without isolation and purification.

(Process 4) The present process is a method of converting a hydroxyl group of Compound (VI) obtained in the above-described Process 3 (wherein PG² has the same meaning as defined above, and X₁, X₂, X₃, X₄, Rₑ and W have the same meaning as the symbols for the above Formula (I)) to a leaving group such as methanesulfonyloxy or chloro to produce Compound (VII) (wherein LG² represents a leaving group such as methanesulfonyloxy or halogen atom, PG² has the same meaning as defined above, and X₁, X₂, X₃, X₄, Rₑ and W have the same meaning as the symbols for the above Formula (I)).

The reaction used in this process employs methods well known to those skilled in the art. In the reaction used in this process, specifically, for example, Compound (VII) in which LG² is methanesulfonyloxy can be obtained by reacting Compound (VI) with methanesulfonyl chloride in a solvent such as chloroform, methylene chloride, tetrahydrofuran, N,N-dimethylformamide, diethyl ether or ethyl acetate, in the presence of a base such as triethylamine or diisopropylethylamine. In this case, with respect to 1 mol of Compound (VI), methanesulfonyl chloride is used in an amount of from 1 to 10 mol, preferably from 1 to 3 mol; and the base is used in an amount of from 1 to 20 mol, preferably from 1 to 6 mol. The reaction temperature can be appropriately selected by a person having ordinary skill in the art in accordance with the starting compound or reaction solvent used, but it is typically from 0°C to room temperature. Also, the reaction is typically completed within 10 minutes to 2 hours, but the reaction time can be appropriately extended or reduced.

Thus obtained Compound (VII) is subjected to isolation and purification by known separation and purification means such as, for example, concentration, concentration under reduced pressure, crystallization, solvent extraction, reprecipitation or chromatography, or subjected to the next process without isolation and purification.

(Process 5) The present process is a method of subjecting Compound (VII) obtained in the above-described Process 4 (wherein LG² and PG² have the same meaning as defined above, and X₁, X₂, X₃, X₄, Rₑ and W have the same meaning as the symbols for the above Formula (I)) and Compound (VIII) (wherein m₁, m₂, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)) to an amination reaction to produce Compound (IX) (wherein PG² has the same meaning as defined above, and X₁, X₂, X₃, X₄, Rₑ and W, and m₁, m₂, n₁, n₂, R, Rₐᵢ, Rₐᵢ', R_{bj}, R_{bj}', Rₑ, R_{d}, Y₂, Y₃ and Z₂ have the same meaning as the symbols for the above Formula (I)).

The aforementioned Compound (VIII) used in this process may be exemplified by 1-(3-chloro-2-fluorobenzoyl)piperazine, 1-(3-(trifluoromethyl)-2-fluorobenzoyl)piperazine, 1-((6-fluoropyridin-2-yl)carbonyl)piperazine, phenyl(piperidin-4-yl)methanone, 2-benzoyl-2,5-diazabicyclo[2.2.1]heptane or 1-benzoyl-1,4-diazepan. Compound (VIII) is commercially available or can be prepared by known methods (e.g., Journal of Medicinal Chemistry, Vol.29, No.5, pages 630-634 (1986)).

The amination reaction used in this process employs methods well known to those skilled in the art. In the amination reaction used in this process, specifically, for example, synthesis can be performed by reacting Compound (VII) and Compound (VIII) in a solvent such as tetrahydrofuran, dimethylsulfoxide, N,N-dimethylformamide, 1,4-dioxane, dichloromethane or chloroform, using a base such as sodium hydrogen carbonate, triethylamine, diisopropylethylamine or sodium hydroxide. In this case, with respect to 1 mol of Compound (VII), Compound (VIII) is used in an amount of from 1 to 10 mol, preferably from 1 to 3 mol; and the base is used in an amount of from 1 to 20 mol, preferably from 1 to 5 mol. The reaction temperature can be appropriately selected by a person having ordinary skill in the art in accordance with the starting compound or reaction solvent used, but it is typically from room temperature to the boiling point of the solvent. Also, the reaction is typically completed within 1 hour to 24 hours, but the reaction time can be appropriately extended or reduced.

Thus obtained Compound (IX) is subjected to isolation and purification by known separation and purification means such as, for example, concentration, concentration under reduced pressure, crystallization, solvent extraction, reprecipitation or chromatography, or subjected to the next process without isolation and purification.

In addition, if Compound (IX) does not necessitate deprotection, this Compound (IX) is used as the compound according to the invention without further performing the following Process 6.

(Process 6) The present process is a method of subjecting Compound (IX) obtained in the above-described Process 5 (wherein PG² has the same meaning as defined above, and X₁, X₂, X₃, X₄, Rₑ and W, and m₁, m₁, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)) to a deprotection reaction to produce Compound (I-1) (wherein X₁, X₂, X₃, X₄, Rₑ and W, and m₁, m₂, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)).

For the deprotection reaction of PG², the method may vary depending on the type of the protective group or stability of the compound, but methods described in the literature [See T.W. Greene, Protective Groups in Organic Synthesis, John Wiley & Sons (1981)] or methods equivalent thereto can be carried out, for example, by solvolysis using acid.

For example, specifically, synthesis can be performed by subjecting Compound (IX) (wherein W is 1H-pyrazol-3-yl, PG² is (2-(trimethylsilyl)ethoxy)methyl, the pyrazole of W is substituted with PG² at the 1-position, and X₁, X₂, X₃, X₄, Rₑ and W, and m₁, m₂, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)) to deprotection reaction by solvolysis using a solvent mixture of trifluoroacetic acid and water, to produce the corresponding Compound (1-1) (wherein W has the same meaning as defined above, and X₁, X₂, X₃, X₄, and Rₑ, and m₁, m₂, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)). In this case, the reaction temperature can be appropriately selected by a person having ordinary skill in the art in accordance with the starting compound or reaction solvent used, but it is typically from 0°C to the boiling point of the solvent. Also, the reaction is typically completed within 1 hour to 24 hours, but the reaction time can be appropriately extended or reduced.

Thus obtained Compound (I-1) is subjected to isolation and purification by known separation and purification means such as, for example, concentration, concentration under reduced pressure, crystallization, solvent extraction, reprecipitation or chromatography, or subjected to the next process without isolation and purification.

Among the Compounds (VIII) (wherein m₁, m₂, n₁, n₂, R, Rₐᵢ, Rₐᵢ', R_{bj}, R_{bj}', R_{c}, R_{d}, Y₂, Y₃ and Z₂ have the same meaning as the symbols for the above Formula (I)) according to the invention, Compound (VIII-1) (wherein Z₂ is N, n₁ is 0, and m₁, m₂, n₂, R, Rₐᵢ, Rₐᵢ', R_{bj}, R_{bj}', R_{d} and Y₃ have the same meaning as the symbols for the above Formula (I)) can be prepared, for example, by the following method.

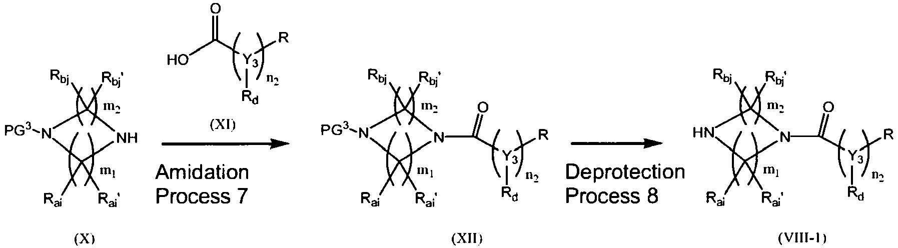

(Process 7) The present process is a method of subjecting Compound (X) (wherein PG³ is a protective group such as tert-butyloxycarbonyl, and m₁, m₂, Rₐᵢ, Rₐᵢ', R_{bj} and R_{bj}' have the same meaning as the symbols for the above Formula (I)) and Compound (XI) (wherein n₂, R, R_{d} and Y₃ have the same meaning as the symbols for the above Formula (I)) to an amidation reaction to produce Compound (XII) (wherein PG³ has the same meaning as defined above, and m₁, m₂, Rₐᵢ, Rₐᵢ', R_{bj} and R_{bj}', and n₂, R, R_{d} and Y₃ have the same meaning as the symbols for the above Formula (I)).

The aforementioned Compound (X) used in this process may be exemplified by tert-butylpiperazin-1-carboxylic acid ester, tert-butyl-2-methylpiperazin-1-carboxylic acid ester, tert-butyl-2,5-diazabicyclo[2.2.1]heptane-2-carboxylic acid ester, or tert-butyl-1,4-diazepan-1-carboxylic acid ester. This Compound (X) is commercially available or can be prepared by known methods (e.g., Journal of Medicinal Chemistry, Vol.29, No.5, pages 630-634 (1986)).

The aforementioned Compound (XI) used in this process may be exemplified by 6-fluoropyridine-2-carboxylic acid, thiophene-2-carboxylic acid, 2,3-dichlorobenzoic acid, 3-chloro-2-fluorobenzoic acid, 3-(trifluoromethyl)-2-fluorobenzoic acid, furan-3-carboxylic acid or 2-fluoropyridine-3-carboxylic acid. This Compound (XI) is commercially available or can be produced by known methods.

The amidation reaction used in this process can be carried out by using a carboxylic acid represented by the above-described Compound (XI) or its reactive derivatives and the above-described Compound (X). Examples of the "reactive derivatives" of Compound (XI) may include mixed acid anhydrides, active esters and active amides, and these can be obtained according to the method described in, for example, WO 98/05641. Specifically, for example, synthesis can be performed by condensing the above Compound (X) and Compound (XI) in a solvent such as tetrahydrofuran, dimethylsulfoxide, N,N-dimethylformamide, 1,4-dioxane, dichloromethane or chloroform, using a condensing agent such as 1-(3-dimethylaminopropyl)-3-ethylcarbodiimide, and 1-hydroxybenzotriazole. In this case, with respect to 1 mol of Compound (X), Compound (XI) is used in an amount of from 1 to 3 mol, preferably 1 mol, and the condensing agent is used in an amount from 1 to 10 mol, preferably from 1 to 3 mol. The reaction temperature is appropriately selected by a person skilled in the art in accordance with the starting compound or reaction solvent used, but it is typically from room temperature to the boiling point of the solvent used in the reaction. Also, the reaction is typically completed within 1 hour to 24 hours, but the reaction time can be appropriately extended or reduced.

Thus obtained Compound (XII) is subjected to isolation and purification by known separation and purification means such as, for example, concentration, concentration under reduced pressure, crystallization, solvent extraction, reprecipitation or chromatography, or subjected to the next process without isolation and purification.

(Process 8) The present process is a method of deprotecting Compound (XII) obtained in the above-described Process 7 (wherein PG³ has the same meaning as defined above, and m₁, m₂, Rₐᵢ, Rₐᵢ', R_{bj} and R_{bj}', and n₂, R, R_{d} and Y3 have the same meaning as the symbols for the above Formula (I)) by removing protective group PG³ to produce a compound represented by Formula (VIII-1) (wherein m₁, m₂, Rₐᵢ, Rₐᵢ', R_{bj} and R_{bj}', and n₂, R, R_{d} and Y₃ have the same meaning as the symbols for the above Formula (I)).

The deprotection reaction used in this process employs methods well known to those skilled in the art. For removal of the protective group of the above-mentioned Compound (XII) in this process, the method of removal may vary depending on the type of the protective group and stability of the compound, but methods described in the literature [See T.W. Greene, Protective Groups in Organic Synthesis, John Wiley & Sons (1981)] or methods equivalent thereto can be carried out. For example, the deprotection reaction for the compound represented by Formula (XII) (wherein PG³ is tert-butyloxycarbonyl) can be carried out by solvolysis using acid.

Thus obtained Compound (V1II-1) is subjected to isolation and purification by known separation and purification means such as, for example, concentration, concentration under reduced pressure, crystallization, solvent extraction, reprecipitation or chromatography, or subjected to the next process without isolation and purification.

Among the Compounds (VI) (wherein PG² has the same meaning as defined above, and Rₑ, X₁, X₂, X₃, X₄ and W have the same meaning as the symbols for the above Formula (I)) according to the invention, Compound (VI-I) (wherein Rₑ is hydrogen atom, PG² has the same meaning as defined above, and X₁, X₂, X₃, X₄ and W have the same meaning as the symbols for the above Formula (I)) can be also prepared, for example, by the following method.

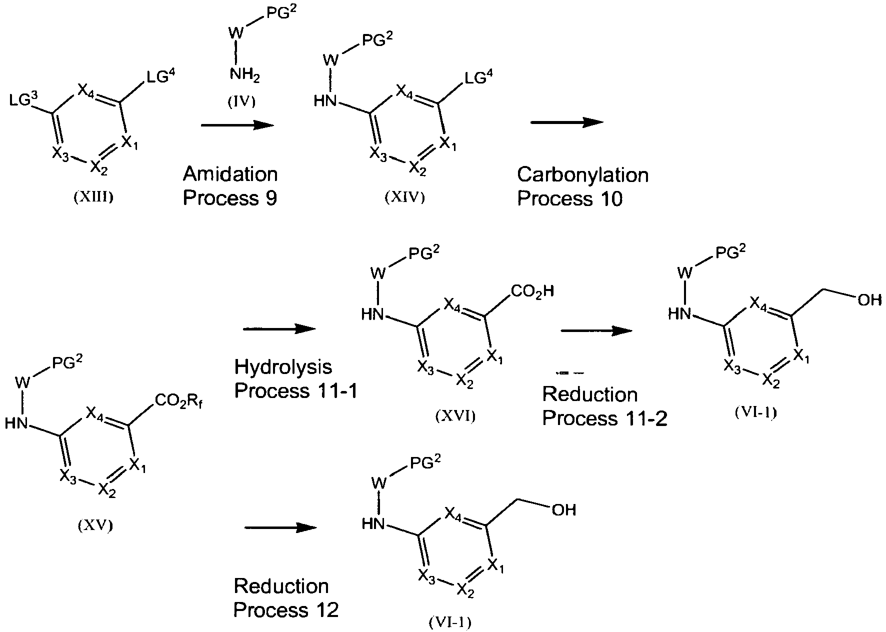

(Process 9) The present process is a method of subjecting Compound (XIII) (wherein LG³ and LG⁴ each represent a leaving group such as halogen atom, and X₁, X₂, X₃ and X₄ have the same meaning as the symbols for the above Formula (I)) and Compound (IV) (wherein PG² have the same meaning as defined above, and W has the same meaning as the symbol for the above Formula (I)) to an amination reaction to produce Compound (XIV) (wherein PG² and LG⁴ have the same meaning as defined above, and X₁, X₂, X₃, X₄ and W have the same meaning as the symbols for the above Formula (I)).

The above-described Compound (IV) used in this process may be exemplified by 2-aminothiazole, 5-amino-1,2,4-thiadiazole, 5-methyl-1-((2-(trimethylsilyl)ethoxy)methyl)-1H-pyrazol-3-amine, 1-((2-(trimethylsilyl)ethoxy)methyl)-1H-pyrazol-3-amine, or 1-tert-butyl-3-methyl-1H-pyrazol-5-amine. The Compound (IV) is commercially available or can be prepared by known methods (e.g., Phosphorus, Sulfur and Silicon and the Related Elements, Vol.177, No.11, pages 2651-2659 (2002), and Journal of Chemical Research, Synopses, Vol.6, page 198 (1979)).

The above-described Compound (XIII) used in this process may be exemplified by 2,6-dichloropyridine, 2,4-dichloropyrimidine or 2,6-dichloropyrazine. Compound (XIII) is commercially available or can be prepared by known methods.

This process can be carried out according to a method similar to the aforementioned Process 2, a method equivalent to that, or a combination of these methods with conventional methods.

Thus obtained Compound (XIV) (wherein PG² and LG⁴ have the same meaning as defined above, and X₁, X₂, X₃, X₄ and W have the same meaning as the symbols for the above Formula (I)) is subjected to isolation and purification by known separation and purification means such as, for example, concentration, concentration under reduced pressure, crystallization, solvent extraction, reprecipitation or chromatography, or subjected to the next process without isolation and purification.

(Process 10) The present process is a method of subjecting Compound (XIV) obtained in the above-described Process 9 (wherein PG² and LG⁴ have the same meaning as defined above, and X₁, X₂, X₃, X₄ and W have the same meaning as the symbols for the above Formula (I)) to a carbonylation reaction to produce Compound (XV) (wherein R_{f} is lower alkyl, PG² has the same meaning as defined above, and X₁, X₂, X₃, X₄ and W have the same meaning as the symbols for the above Formula (I)).

The carbonylation reaction used in this process employs methods well known to those skilled in the art. In the carbonylation reaction used in this process, specifically, for example, Compound (XV) can be synthesized by reacting Compound (XIV) with carbon monoxide in a solvent mixture in which alcohol such as methanol or ethanol is added to a solvent such as N,N-dimethylacetamide, N-methylpyrrolidone or N,N-dimethylformamide, in the presence of a ligand such as 1,1'-bis(diphenylphosphino)ferrocene, a palladium catalyst such as palladium (II) acetate, and a base such as sodium hydrogen carbonate or triethylamine. In this case, with respect to 1 mol of Compound (XIV), the palladium catalyst is used in an amount of from 0.01 to 1 mol, preferably from 0.05 to 0.5 mol; the ligand is used in an amount of from 0.02 to 1 mol, preferably from 0.1 to 1 mol; and the base is used in an amount of from 1 to 10 mol, preferably from 1 to 3 mol. The reaction temperature can be appropriately selected by a person skilled in the art in accordance with the starting compound and reaction solvent used, but it is typically from 50°C to the boiling point of the solvent used in the reaction. Also, the reaction is typically completed within 1 hour to 24 hours, but the reaction time can be appropriately extended or reduced.

Thus obtained Compound (XV) is subjected to isolation and purification by known separation and purification means such as, for example, concentration, concentration under reduced pressure, crystallization, solvent extraction, reprecipitation or chromatography, or subjected to the next process without isolation and purification.

(Process 11-1) The present process is a method of subjecting Compound (XV) obtained in the above-described Process 10 (wherein R_{f} and PG² have the same meaning as defined above, and X₁, X₂, X₃, X₄ and W have the same meaning as the symbols for the above Formula (I)) to a hydrolysis reaction to produce Compound (XVI) (wherein PG² has the same meaning as defined above and X₁, X₂, X₃, X₄ and W have the same meaning as the symbols for the above Formula (I)).

The hydrolysis reaction used in this process employs methods well known to those skilled in the art. In the hydrolysis reaction used in this process, specifically, for example, Compound (XVI) can be synthesized by hydrolyzing Compound (XV) in a solvent such as methanol, ethanol or tetrahydrofuran, using an aqueous solution of sodium hydroxide as the base. In this case, with respect to 1 mol of Compound (XV), the base is used in an amount of from 1 to 1000 mol, preferably from 1 to 100 mol. The reaction temperature can be appropriately selected by a person skilled in the art in accordance with the starting compound and reaction solvent used, but it is typically from room temperature to the boiling point of the solvent. Also, the reaction is typically completed within 1 hour to 24 hours, but the reaction time can be appropriately extended or reduced.

Thus obtained Compound (XVI) is subjected to isolation and purification by known separation and purification means such as, for example, concentration, concentration under reduced pressure, crystallization, solvent extraction, reprecipitation or chromatography, or subjected to the next process without isolation and purification.

(Process 11-2) The present process is a method of subjecting Compound (XVI) obtained in the above-described Process 11-1 (wherein PG² has the same meaning as defined above, and X₁, X₂, X₃, X₄ and W have the same meaning as the symbols for the above Formula (I)) to a reduction reaction to produce Compound (VI-1) (wherein PG² has the same meaning as defined above, and X₁, X₂, X₃, X₄ and W have the same meaning as the symbols for the above Formula (I)).

The reduction reaction used in this process employs methods well known to those skilled in the art. In the reduction reaction used in this process, specifically, for example, Compound (VI-1) can be synthesized by reacting Compound (XVI) with N,N'-carbonyldiimidazole in a solvent such as N,N-dimethylformamide or tetrahydrofuran at room temperature for 12 to 24 hours, and then reacting again with a reducing agent such as sodium borohydride. In this case, with respect to 1 mol of Compound (XVI), N,N'-carbonyldiimidazole is used in an amount of from 1 to 10 mol, preferably from 1 to 3 mol; and the reducing agent is used in an amount of from 1 to 20 mol, preferably from 1 to 5 mol. The reaction temperature can be appropriately selected by a person skilled in the art in accordance with the starting compound and reaction solvent used, but it is typically from 0°C to room temperature. Also, the reaction is typically completed within 10 minutes to 24 hours, but the reaction time can be appropriately extended or reduced.

Thus obtained Compound (VI-1) is subjected to isolation and purification by known separation and purification means such as, for example, concentration, concentration under reduced pressure, crystallization, solvent extraction, reprecipitation or chromatography, or subjected to the next process without isolation and purification.

(Process 12) The present process is a method of subjecting Compound (XV) obtained in the above-described Process 10 (wherein R_{f} and PG² have the same meaning as defined above, and X₁, X₂, X₃, X₄ and W have the same meaning as the symbols for the above Formula (I)) to a reduction reaction to produce Compound (VI-1) (wherein PG² has the same meaning as defined above, and X₁, X₂, X₃, X₄ and W have the same meaning as the symbols for the above Formula (I)).

The reduction reaction used in this process employs methods well known to those skilled in the art. In the reduction reaction used in this process, specifically, for example, Compound (VI-1) can be synthesized by reacting Compound (XV) with a reducing agent such as lithium borohydride or lithium aluminum hydride in a solvent such as tetrahydrofuran or 1,4-dioxane. In this case, with respect to 1 mol of Compound (XV), the reducing agent is used in an amount of from 1 to 20 mol, preferably from 1 to 5 mol. The reaction temperature can be appropriately selected by a person skilled in the art in accordance with the starting compound and reaction solvent used, but it is typically from 0°C to the boiling point of the solvent used in the reaction. Also, the reaction is typically completed within 10 minutes to 24 hours, but the reaction time can be appropriately extended or reduced.

Thus obtained Compound (VI-1) is subjected to isolation and purification by known separation and purification means such as, for example, concentration, concentration under reduced pressure, crystallization, solvent extraction, reprecipitation or chromatography, or subjected to the next process without isolation and purification.

Compound (XV) (wherein R_{f} is a lower alkyl group, PG² has the same meaning as defined above, and X₁, X₂, X₃, X₄ and W have the same meanings as the symbols for the above Formula (I)) according to the invention can be also prepared by, for example, the following method.

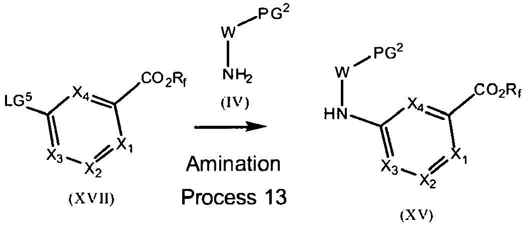

(Process 13) The present process is a method of subjecting Compound (XVII) (wherein R_{f} is a lower alkyl group, LG⁵ is a leaving group such as halogen atom, and X₁, X₂, X₃ and X₄ have the same meanings as the symbols for the above Formula (I)) and Compound (IV) (wherein PG² has the same meaning as defined above, and W has the same meaning as the symbol for the above Formula (I)) to an amination reaction to produce Compound (XV) (wherein R_{f} is a lower alkyl group, PG² has the same meaning as defined above, and X₁, X₂, X₃, X₄ and W have the same meanings as the symbols for the above Formula (I)).

The above-described Compound (IV) that is used in this process may be exemplified by 2-aminothiazole, 5-amino-1,2,4-thiadiazole, 5-methyl-1-((2-(trimethylsilyl)ethoxy)methyl)-1H-pyrazol-3-amine, 1-((2-(trimethylsilyl)ethoxy)methyl)-1H-pyrazol-3-amine, 1-tert-butyl-3-methyl-1H-pyrazol-5-amine and the like. The above-described Compound (IV) is commercially available or can be produced by known methods (for example, Phosphorus, Sulfur and Silicon and the Related Elements, Vol. 177 (11) pp. 2651-2659 (2002); and Journal of Chemical Research, Synopses, Vol.6, p. 198 (1979)).

The above-mentioned Compound (XVII) that is used in the present process may be exemplified by 6-chloro-2-pyridinecarboxylic acid methyl ester, 6-chloro-4-methoxy-2-pyridinecarboxylic acid methyl ester or the like. Compound (XVII) is commercially available or can be produced by known methods.

This process can be carried out by a method similar to the above-described Process 2, a method equivalent to this, or a combination of these methods and conventional methods.

Thus obtained, aforementioned Compound (XV) (wherein R_{f} is a lower alkyl group, PG² has the same meaning as defined above, and X₁, X₂, X₃, X₄ and W have the same meanings as the symbols for the above Formula (I)) can be either subjected to isolation and purification by known separation and purification means such as, for example, concentration, concentration under reduced pressure, crystallization, solvent extraction, reprecipitation or chromatography, or subjected to the next process without isolation and purification.

The aforementioned introduction of a protective group into a compound can be carried out in a number of stages for producing the above-described synthetic intermediates as needed. In obtaining the protection product, reaction can be carried out in a manner similar to the corresponding process as described above. Further, such compound can be deprotected by removing the introduced protective group according to a method similar to the aforementioned Process 6, a method equivalent to that, or a combination of these methods and conventional methods.

Hereinunder, examples of introducing protective groups to Compound (IV) and to Compound (XV) will be illustrated. In addition, a person having ordinary skill in the art can perform introduction of protective groups into the above-mentioned synthetic intermediates by using commercially available, known compounds and using any appropriate, known method, and/or the below-described methods or methods equivalent to these.

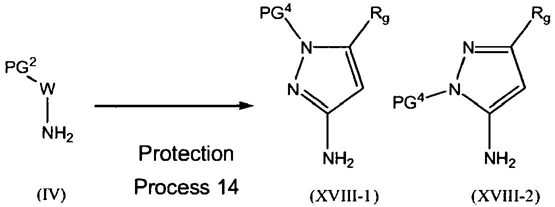

(Process 14) The present process is a method of producing Compound (XVIII-1) or Compound (XVIII-2) (wherein PG⁴ is a protective group such as methoxymethyl or (2-(trimethylsilyl)ethoxy)methyl, and Rg is a substituent such as hydrogen atom, methyl or cyclopropyl) by introducing a protective group into Compound (IV) (wherein -W-PG² is 5-methyl-1H-pyrazol-3-yl, 5-cyclopropyl-1H-pyrazol-3-yl or 1-H-pyrazol-3-yl).

In the protection reaction used in this process, for example, Compound (IV) is protected in a solvent such as tetrahydrofuran, N,N-dimethylformamide, 1,4-dioxane, toluene, dichloromethane or chloroform, using a base such as sodium hydride together with chloromethyl methyl ether, chloromethyl 2-(trimethylsilyl)ethyl ether or the like, to synthesize the corresponding Compound (XVIII-1) or Compound (XVIII-2). In this case, with respect to 1 mol of Compound (IV), the base is used in an amount of from I to 20 mol, preferably from 1 to 5 mol; and the protective reagent is used in an amount of from 1 to 10 mol, preferably from 1 to 3 mol. The reaction temperature can be appropriately selected by a person skilled in the art in accordance with the starting compound or reaction solvent used, but it is typically from 0°C to room temperature. Also, the reaction is typically completed within 10 minutes to 24 hours, but the reaction time can be appropriately extended or reduced.

Thus obtained Compound (XVIII-1) or Compound (XVIII-2) is subjected to isolation and purification by known separation and purification means such as, for example, concentration, concentration under reduced pressure, crystallization, solvent extraction, reprecipitation or chromatography, or subjected to the next process without isolation and purification.

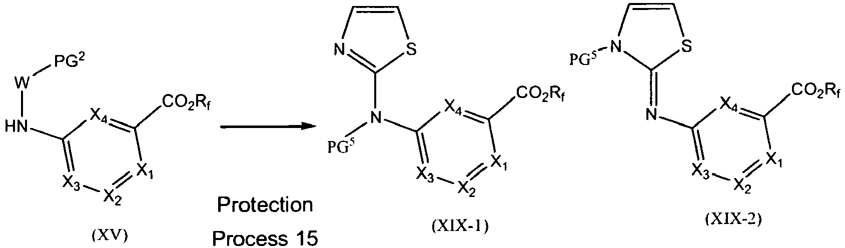

(Process 15) The present process is a method of producing Compound (XIX-1) or Compound (XIX-2) (wherein R_{f} and PG⁵ have the same meaning as defined above, and X₁, X₂, X₃, and X₄ have the same meaning as the symbols for the above Formula (I)) by introducing a protective group PG⁵ such as methoxymethyl or (2-(trimethylsilyl)ethoxy)methyl into Compound (XV) (wherein R_{f} and PG² have the same meaning as defined above, and X₁, X₂, X₃, X₄ and W have the same meaning as the symbols for the above Formula (I)).

The protection reaction used in this process can be carried out, for example, by protecting Compound (XV) in a solvent such as tetrahydrofuran, N,N-dimethylformamide, 1,4-dioxane, toluene, dichloromethane or chloroform, using a base such as sodium hydride or diisopropylethylamine together with chloromethyl methyl ether, chloromethyl 2-(trimethylsilyl)ethyl ether or the like. In this case, with respect to 1 mol of Compound (XV), the base is used in an amount of from 1 to 20 mol, preferably from 1 to 5 mol, and the protective reagent is used in an amount of from 1 to 10 mol, preferably from 1 to 3 mol. The reaction temperature can be appropriately selected by a person skilled in the art in accordance with the starting compound or reaction solvent used, but it is typically from 0°C to room temperature. Also, the reaction is typically completed within 10 minutes to 24 hours, but the reaction time can be appropriately extended or reduced.

Thus obtained Compound (XIX-1) or Compound (XIX-2) is subjected to isolation and purification by known separation and purification means such as, for example, concentration, concentration under reduced pressure, crystallization, solvent extraction, reprecipitation or chromatography, or subjected to the next process without isolation and purification.

Furthermore, introduction or conversion of X₁ₐ, X₂ₐ or X₃ₐ can be carried out at any step for producing the above-mentioned synthetic intermediates which may have appropriate protective groups. Hereinafter, examples of introduction or conversion of a substituent for X₂ₐ in the compound represented by Formula (I) (wherein X₁ is CH, X₂ is CX₂ₐ, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meanings as the symbols for the above Formula (I)), the above-mentioned Compound (XV) (wherein R_{f}, PG², X₁, X₂, X₃, X₄ and W have the same meanings as defined above) and the above-mentioned Compound (V) (wherein PG¹, PG², X₁, X₂, X₃, X₄, Rₑ and W have the same meanings as defined above) will be illustrated. Here, the compound of Formula (I) mentioned in the description of the following Processes (16-1) to (16-7), the compound of Formula (XV) mentioned in the description of Process (17), and the compound of Formula (V) mentioned in the description of Processes (18-1) and (18-2) may have an appropriate protective group at a substitutable position to which a protective group can be introduced. Further, a person skilled in the art can perform introduction or conversion of a substituent for X₁ₐ, X₂ₐ or X₃ₐ by using commercially available, known compounds and using any appropriate, known method, and/or the below-described methods or methods equivalent to these.

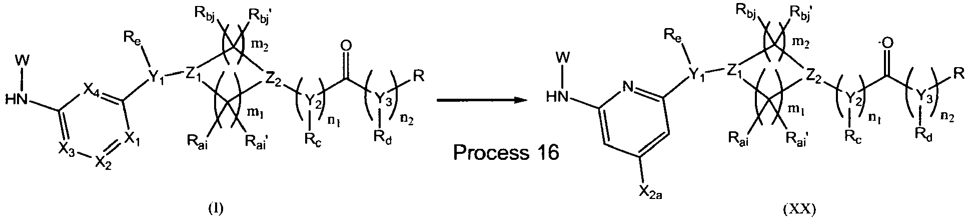

Process 16 relates to a method of synthesizing Compound (XX) from Compound (I). Hereafter, it is exemplified in Processes 16-1 to 16-7.

(Process 16-1) The present process is a method of subjecting Compound (I) (wherein X₁ is CH, X₂ is CX₂ₐ, X₂ₐ is bromine atom, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a carbonylation reaction to produce Compound (XX) (wherein X₁ is CH, X₂ is CX₂ₐ, X₂ₐ is alkoxycarbonyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)).

This process can be carried out by a method similar to the above-described Process 10, a method equivalent to this, or a combination of these methods and conventional methods.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-2) The present process is a method of subjecting Compound (I) (wherein X₁ is CH, X₂ is CX₂ₐ, X₂ₐ is alkoxycarbonyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a hydrolysis reaction to produce Compound (XX) (wherein X₁ is CH, X₂ is CX₂ₐ, X₁ₐ is carboxyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)).

This process can be carried out by a method similar to the above-described Process 11, a method equivalent to this, or a combination of these methods and conventional methods.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-3) The present process is a method of subjecting Compound (I) (wherein X₁ is CH, X₂ is CX₂ₐ, X₂ₐ is carboxyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to an amidation reaction to produce Compound (XX) (wherein X₂ₐ is carbamoyl, and m₁, m₂, n₁, n₂, i, j, R, Rₐᵢ, Rₐᵢ', R_{bj}, R_{bj}', R_{c} R_{d}, Rₑ, Y₁, Y₂, Y₃, Z₁, Z₂ and W have the same meaning as the symbols for the above Formula (I)).

This process can be carried out by a method similar to the above-described Process 7, a method equivalent to this, or a combination of these methods and conventional methods. The amine used in this process may be exemplified by dimethylamine, methylamine, pyrrolidine and 2-hydroxyethylamine.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-4) The present process is a method of subjecting Compound (I) (wherein X₁ is CH, X₂ is CX₂ₐ, X₂ₐ is carboxyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a reduction reaction to produce Compound (XX) (wherein X₂ₐ is hydroxymethyl, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)).

This process can be carried out by a method similar to the above-described Process 11-2, a method equivalent to this, or a combination of these methods and conventional methods.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-5) The present process is a method of subjecting Compound (I) (wherein X₁ is CH, X₂ is CX₂ₐ, X₂ₐ is hydroxymethyl, X₃ is CH, X₄ is N, and m,, m₁, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a reaction to produce Compound (XX) (wherein X₂ₐ is methanesulfonyloxymethyl, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)).

This process can be carried out by a method similar to the above-described Process 4, a method equivalent to this, or a combination of these methods and conventional methods.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-6) The present process is a method of subjecting Compound (I) (wherein X₁ is CH, X₂ is CX₂ₐ, X₂ₐ is methanesulfonyloxymethyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a substitution reaction to produce Compound (XX) [wherein X₂ₐ is RᵢRₕNCH₂- (wherein Rᵢ and Rₕ, which may be identical or different, are each hydrogen atom or lower alkyl which may be substituted, or Rᵢ and Rₕ may be combined to form an aliphatic heterocyclic group which may be substituted), and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)].

In the substitution reaction used in this process, for example, synthesis can be performed by reacting Compound (I) (wherein X₁ is CH, X₂ is CX₂ₐ, X₂ₐ is methanesulfonyloxymethyl, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) with a nucleophilic agent represented by RᵢRₕNH such as dimethylamine, 1,2,3-triazole or 1,2,4-triazole, in a solvent such as chloroform, methylene chloride, tetrahydrofuran, N,N-dimethylformamide or dimethylsulfoxide, in the presence of a base such as potassium carbonate, triethylamine, diisopropylethylamine, or 1,8-diazabicyclo[5.4.0]undec-7-ene. In this case, with respect to 1 mol of Compound (I), the base is used in an amount of from 1 to 20 mol, preferably from 1 to 5 mol; and the nucleophilic agent is used in an amount of from 1 to 10 mol, preferably from 1 to 3 mol. The reaction temperature can be appropriately selected by a person skilled in the art in accordance with the starting compound or reaction solvent used, but it is typically from room temperature to the boiling point of the solvent used in the reaction. Also, the reaction is typically completed within 1 hour to 24 hours, but the reaction time can be appropriately extended or reduced.

Thus obtained Compound (XX) according to the invention can be subjected to isolation and purification by known separation and purification means such as, for example, concentration, concentration under reduced pressure, crystallization, solvent extraction, reprecipitation or chromatography.

(Process 16-7) The present process is a method of subjecting Compound (I) (wherein X₁ is CH, X₂ is CX₂ₐ, X₂ₐ is bromine atom, X₃ is CH, X₄ is N, and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (I)) to a coupling reaction to produce Compound (XX) [wherein X₂ₐ is RⱼRₖN- (wherein Rⱼ and Rₖ, which may be identical or different, are each hydrogen atom, lower alkyl which may be substituted, lower acyl, lower carbamoyl or lower alkoxycarbonyl, or Rⱼ and Rₖ may be combined to form a heterocyclic group which may be substituted), and m₁, m₂, n₁, n₂, i, j, R, Rₐᵢ, Rₐᵢ', R_{bj}, R_{bj}', R_{c}, R_{d}, Rₑ, Y₁, Y₂, Y₃, Z₁, Z₂ and W have the same meaning as the symbols for the above Formula (1)].

This process can be carried out by a method similar to the above-described Process 2, a method equivalent to this, or a combination of these methods and conventional methods. The nucleophilic agent used in this process may be exemplified by amine represented by RⱼRₖNH (such as 1-methyl-2-imidazolidinone, 2-pyrrolidone, 2-oxazolidone, piperazine or morpholine), amide, urea or carbamate.

Thus obtained Compound (XX) according to the invention can be subjected to isolation and purification by known separation and purification means such as, for example, concentration, concentration under reduced pressure, crystallization, solvent extraction, reprecipitation or chromatography.

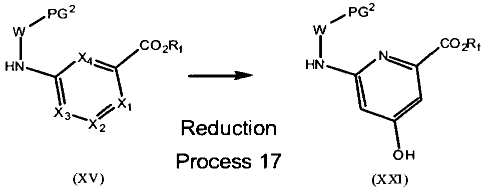

(Process 17) The present process is a method of removing a benzyl group that is a protective group of the hydroxyl group of Compound (XV) (wherein R_{f} is a lower alkyl group, PG² has the same meaning as defined above, X₁ is CH, X₂ is CX₂ₐ, X₂ₐ is a benzyloxy group, X₃ is CH, X₄ is N, and W has the same meaning as the symbol for the above Formula (I)) to produce Compound (XXI) (wherein R_{f}, PG² and W have the same meanings as defined above).

Removal of a protective group in this process can be carried out by methods described in the literature (for example, T.W. Green, Protective Groups in Organic Synthesis, 2nd Ed., John Wiley & Sons (1991), etc.), methods equivalent to these or combinations of these methods and conventional methods, for example, by catalytic hydrogenation using a palladium hydroxide-carbon catalyst, or the like.

In the case of using a palladium hydroxide-carbon catalyst in removal of the benzyl group, the amount of the catalyst is usually 0.01 to 1000 equivalents, and preferably 0.1 to 10 equivalents.

The reaction solvent used in the present process is not particularly limited as long as it does not affect the reaction, and may be exemplified by methanol, ethanol or the like.

Thus obtained, above-described Compound (XXI) according to the invention can be subjected to isolation and purification by known separation and purification means such as, for example, concentration, concentration under reduced pressure, crystallization, solvent extraction, reprecipitation or chromatography.

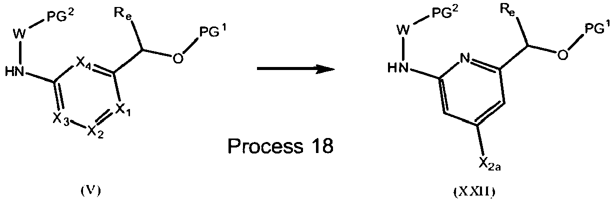

Process 18 relates to a method of synthesizing Compound (XXII) from Compound (V). Hereafter, it is exemplified in Processes 18-1 and 18-2.

(Process 18-1) The present process is a method of producing Compound (XXII) (wherein Rₑ, W, PG¹ and PG² have the same meanings as defined above, and X₂ₐ is a trifluoromethanesulfonyloxy group) from Compound (V) (wherein Rₑ, W, PG¹ and PG² have the same meanings as defined above, X₁ is CH, X₂ is CX₂ₐ, X₂ₐ is a hydroxyl group, X₃ is CH, and X₄ is N).

The reaction used in this process employs a method well-known to a person skilled in the art. In the reaction used in this process, specifically, for example, the above-described Compound (V) can be reacted with anhydrous trifluoromethanesulfonic acid in a solvent such as chloroform, methylene chloride, tetrahydrofuran, N,N-dimethylformamide, diethyl ether and ethyl acetate, in the presence of a base such as 4-dimethylaminopyridine, triethylamine and diisopropylethylamine, to obtain Compound (XXII) (wherein Rₑ, W, PG¹ and PG² have the same meanings as defined above, and X₂ₐ is a trifluoromethanesulfonyloxy group). In this case, with respect to 1 mole of Compound (V), anhydrous trifluoromethanesulfonic acid is used in an amount of 1 to 10 moles, and preferably 1 to 3 moles, and the base is used in an amount of 1 to 20 moles, and preferably 1 to 6 moles. The reaction temperature can be appropriately selected by a person skilled in the art in accordance with the starting compound used, and it is usually 0°C to room temperature. Also, the reaction is typically completed in 10 minutes to 2 hours, but the reaction time can be appropriately extended or reduced.

Thus obtained, above-mentioned Compound (XXII) can be either subjected to isolation and purification by known separation and purification means such as, for example, concentration, concentration under reduced pressure, crystallization, solvent extraction, reprecipitation and chromatography, or subjected to the next process without isolation and purification.

(Process 18-2) The present process is a method of subjecting Compound (V) (wherein Rₑ, W, PG¹ and PG² have the same meanings as defined above, X₁ is CH, X₂ is CX₂ₐ, X₂ₐ is a trifluoromethanesulfonyloxy group, X₃ is CH, and X₄ is N) to a carbonylation reaction to produce Compound (XXII) (wherein Rₑ, W, PG¹ and PG² have the same meanings as defined above, and X₂ₐ is an alkoxycarbonyl group).

The present process can be carried out by a method similar to the above-described Process 10, a method equivalent to this, or a combination of these methods and conventional methods.

Thus obtained, above-described Compound (XXII) according to the invention can be subjected to isolation and purification by known separation and purification means such as, for example, concentration, concentration under reduced pressure, crystallization, solvent extraction, reprecipitation and chromatography.

Introduction or conversion of substituent for W can be carried out in any one of stages for producing the above-mentioned synthetic intermediates or protection products thereof. Hereinunder, an example of introducing a substituent for W in the compound represented by Formula (V) (wherein W is thiazol-2-yl, PG¹ and PG² have the same meaning as defined above, and Rₑ, X₁, X₂, X₃, X₄ and W have the same meaning as the symbols for the above Formula (I)) will be illustrated. In addition, a person having ordinary skill in the art can carry out introduction or conversion of substituent for W in a number of stages for producing the above-mentioned synthetic intermediates or protection products thereof by using commercially available, known compounds and using any appropriate, known method, and/or the below-described method or a method equivalent to this.

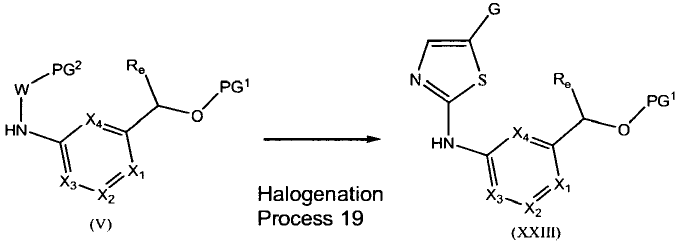

(Process 19) The present process is a method of subjecting Compound (V) (wherein-W-PG² is thiazol-2-yl, PG¹ has the same meaning as defined above, and Rₑ, X₁, X₂, X₃ and X₄ have the same meaning as the symbols for the above Formula (I)) to a halogenation reaction to produce Compound (XXIII) (wherein G is halogen atom such as chlorine, bromine or iodine, PG¹ has the same meaning as defined above, and Rₑ, X₁, X₂, X₃ and X₄ have the same meaning as the symbols for the above Formula (I)).

The halogenation reaction used in this process can be carried out, for example, by reacting Compound (V) (wherein -W-PG² is thiazol-2-yl, PG¹ has the same meaning as defined above, and Rₑ, X₁, X₂, X₃ and X₄ have the same meaning as the symbols for the above Formula (I)) with a halogenating reagent such as N-chlorosuccinimide, N-bromosuccinimide or N-iodosuccinimide, in a solvent such as tetrahydrofuran, water, acetic acid, methanol, ethanol, 1,4-dioxane, methylene chloride, chloroform or toluene. In the reaction, with respect to 1 mol of Compound (V), the halogenating reagent is used in an amount of from 1 to 3 mol, preferably 1 mol. In this case, the reaction temperature is appropriately selected in accordance with the starting compound or reaction solvent used, but it is typically from 0°C to the boiling point of the solvent. Also, the reaction is typically completed within 1 hour to 24 hours, but the reaction time can be appropriately extended or reduced.

Thus obtained Compound (XXIII) is subjected to isolation and purification by known separation and purification means such as, for example, concentration, concentration under reduced pressure, crystallization, solvent extraction, reprecipitation or chromatography, or subjected to the next process without isolation and purification.

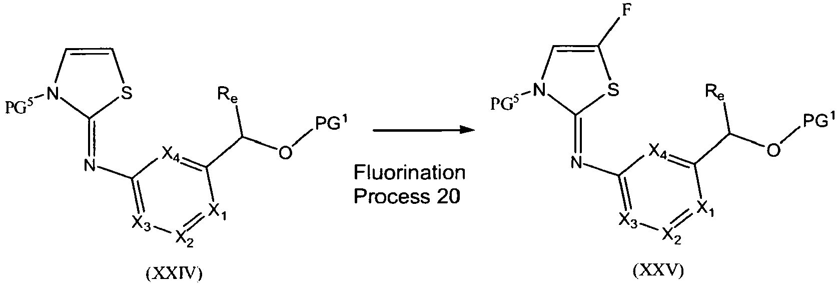

(Process 20) The present process is a method of subjecting Compound (XXIV) (wherein PG⁵ is a protective group such as methoxymethyl or (2-(trimethylsilyl)ethoxy)methyl, PG¹ has the same meaning as defined above, and Rₑ, X₁, X₂, X₃ and X₄ have the same meaning as the symbols for the above Formula (I)) to a fluorination reaction to produce Compound (XXV) (wherein PG⁵ is a protective group such as methoxymethyl or (2-(trimethylsilyl)ethoxy)methyl, PG¹ has the same meaning as defined above, and Rₑ, X₁, X₂, X₃ and X₄ have the same meaning as the symbols for the above Formula (I)).

The fluorination reaction used in this process can be carried out, for example, by adding dropwise a hexane solution of n-butyllithium to a solution of Compound (XXIV) (wherein PG⁵ is a protective group such as methoxymethyl or (2-(trimethylsilyl)ethoxy)methyl, PG¹ has the same meaning as defined above, and Rₑ, X₁, X₂, X₃ and X₄ have the same meaning as the symbols for the above Formula (I)) in tetrahydrofuran or toluene, and then adding dropwise a tetrahydrofuran solution of N-fluorobenzenesulfonimide again. In the reaction, with respect to 1 mol of Compound (XXIV), the fluorinating reagent is used in an amount of from 1 to 3 mol, preferably 1 mol. In this case, the reaction temperature is appropriately selected in accordance with the starting compound or reaction solvent used, but it is typically from -78°C to -20°C. Also, the reaction is typically completed within 15 minutes to 2 hours, but the reaction time can be appropriately extended or reduced.

Thus obtained Compound (XXV) is subjected to isolation and purification by known separation and purification means such as, for example, concentration, concentration under reduced pressure, crystallization, solvent extraction, reprecipitation or chromatography, or subjected to the next process without isolation and purification.

Next, the Aurora A and Aurora B inhibitory actions of the compound of General Formula (I) according to the invention will be explained below.

### Aurora A Inhibitory Action

### (1) Purification of Aurora A

cDNA of Aurora A having histidine tag fused at the amino terminal was integrated into an expression vector, which was then highly expressed in Escherichia coli BL21-CodonPlus(DE3)-RIL cells. The Escherichia coli cells were recovered and solubilized, and then the histidine-tagged Aurora A protein was adsorbed onto a nickel chelate column and eluted from the column with imidazole. The active fraction was desalted with a desalting column to give a pure enzyme.

### (2) Measurement of activity of Aurora A

For measurement of the activity of Aurora A, the substrate used was Kemptide (Lys-Arg-Arg-Ala-Ser-Lys-Gly) (SEQ.ID.NO.: 1), a synthetic peptide purchased from Sigma-Aldrich, Inc. [Certificate of analysis (Upstate, Inc.)].

Reaction was conducted by a partial modification of a method by Upstate, Inc. [Kinase Profiler™ Assay Protocols]. The amount of the reaction liquid was 21.1 µL, and the composition of the reaction buffer (R2 buffer) was 50 mM Tris-hydrochloride buffer (pH 7.4)/15 mM magnesium acetate/0.2 mM ethylenediamine-N,N,N',N'-tetraacetate (EDTA). To this, purified Aurora A, 100 µM of a substrate peptide, 20 µM of unlabeled adenosine triphosphate (ATP) and 0.5 µCi of [γ-³³P] labeled ATP (2,500 Ci/mmole or more) were added, and the mixture was reacted at 30°C for 20 minutes. Then, 10 µL of 350 mM phosphate buffer was added to the reaction system to stop the reaction. The substrate peptide was adsorbed on a P81 paper filter 96-well plate and then washed with 130 mM phosphate buffer for several times. The radiation activity of the peptide was measured with a liquid scintillation counter. The [µ-³³P] labeled ATP was purchased from Amersham Biosciences Co., Ltd.

The compound to be tested was added to the reaction system such that a dilution series of the compound in dimethylsulfoxide was first prepared, and 1.1 µL of this solution was added. A control was provided by adding 1.1 µL of DMSO to the reaction system.

### Aurora B Inhibitory Action

### (1) Purification of Aurora B

cDNA of Aurora B having histidine tag fused at the amino terminal was integrated into an expression vector, which was then highly expressed in Escherichia coli BL21-CodonPlus(DE3)-RIL cells. The Escherichia coli cells were recovered and solubilized, and then the histidine-tagged Aurora A protein was adsorbed onto a nickel chelate column and eluted from the column with imidazole. The active fraction was desalted with a desalting column to give a pure enzyme.

### (2) Measurement of activity of Aurora B

For measurement of the activity of Aurora B, the substrate used was Kemptide (Lys-Arg-Arg-Ala-Ser-Lys-Gly) (SEQ.ID.NO.: 1), a synthetic peptide purchased from Sigma-Aldrich, Inc. [Certificate of analysis (Upstate, Inc.)].

Reaction was conducted by a partial modification of the method of activity measurement for Aurora A. The amount of the reaction liquid was 21.1 µL, and the composition of the reaction buffer (R2 buffer) was 50 mM Tris-hydrochloride buffer (pH 7.4)/15 mM magnesium acetate/0.2 mM ethylenediamine-N,N,N',N'-tetraacetate (EDTA). To this, purified Aurora B, 100 µM of a substrate peptide, 100 µM of unlabeled adenosine triphosphate (ATP) and 1 µCi of [γ_^{33p}] labeled ATP (2,500 Ci/mmole or more) were added, and the mixture was reacted at 30°C for 20 minutes. Then, 10 µL of 350 mM phosphate buffer was added to the reaction system to stop the reaction. The substrate peptide was adsorbed on a P81 paper filter 96-well plate and then washed with 130 mM phosphate buffer for several times. The radiation activity of the peptide was measured with a liquid scintillation counter. The [γ-³³P] labeled ATP was purchased from Amersham Biosciences Co., Ltd.

The compound to be tested was added to the reaction system such that a dilution series of the compound in dimethylsulfoxide was first prepared, and 1.1 µL of this solution was added. A control was provided by adding 1.1 µL of DMSO to the reaction system.

The compound according to the invention exhibits excellent Aurora A selective inhibitory activity, as shown in Table 1.

**[Table 1]**

| Example | Aurora A inhibitory action (IC50, nM) | Aurora B inhibitory action (IC50, nM) | Example | Aurora A inhibitory action (IC50, nM) | Aurora B inhibitory action (IC50, nM) |
|---|---|---|---|---|---|
| Example 5 | 0.67 | 440 | Example 61 | 17 | 530 |
| Example 6 | 0.5 | 200 | Example 62 | 12 | 380 |
| Example 8 | 1.9 | 1400 | Example 63 | 1.3 | 570 |
| Example 9 | 1.3 | 760 | Example 64 | 110 | 2500 |
| Example 10 | 0.49 | 92 | Example 65 | 2.4 | 160 |
| Example 11 | 1.3 | 570 | Example 66 | 3.4 | 250 |
| Example 12 | 0.52 | 400 | Example 67 | 17 | 320 |
| Example 13 | 0.89 | 380 | Example 68 | 11 | 670 |
| Example 15 | 1.4 | 1000 | Example 69 | 32 | 920 |
| Example 16 | 1.8 | 1300 | Example 70 | 2.4 | 96 |
| Example 17 | 1.2 | 3200 | Example 71 | 3 | 370 |
| Example 18 | 1.8 | 830 | Example 72 | 27 | 170 |
| Example 19 | 0.9 | 530 | Example 73 | 17 | 410 |
| Example 20 | 1.1 | 1800 | Example 74 | 47 | 850 |
| Example 21 | 16 | 6800 | Example 75 | 0.44 | 89 |
| Example 22 | 2.9 | 1500 | Example 76 | 0.44 | 47 |
| Example 23 | 3.6 | 1200 | Example 77 | 4.1 | 1300 |
| Example 24 | 23 | 26000 | Example 78 | 2 | 240 |
| Example 25 | 1.1 | 770 | Example 79 | 26 | 2200 |
| Example 26 | 1.1 | 450 | Example 81 | 0.33 | 300 |
| Example 27 | 3.3 | 1700 | Example 82 | 0.51 | 210 |
| Example 28 | 0.52 | 310 | Example 83 | 0.64 | 800 |
| Example 29 | 0.97 | 590 | Example 84 | 0.72 | 400 |
| Example 30 | 1.1 | 320 | Example 85 | 1 | 610 |
| Example 31 | 37 | 760 | Example 86 | 0.66 | 560 |
| Example 32 | 3.7 | 4800 | Example 87 | 1.2 | 1400 |
| Example 33 | 50 | 3000 | Example 88 | 0.72 | 1000 |
| Example 35 | 31 | 3400 | Example 89 | 0.38 | 200 |
| Example 36 | 2.6 | 3200 | Example 90 | 1.5 | 860 |
| Example 37 | 4.9 | 8800 | Example 91 | 1.4 | 1200 |
| Example 38 | 9.1 | 9500 | Example 92 | 0.93 | 830 |
| Example 39 | 67 | 9100 | Example 93 | 0.36 | 250 |
| Example 40 | 0.99 | 1900 | Example 94 | 1.1 | 1100 |
| Example 41 | 2.9 | 5000 | Example 95 | 0.59 | 250 |
| Example 42 | 1 | 530 | Example 96 | 0.57 | 690 |
| Example 43 | 1.1 | 460 | Example 97 | 0.62 | 830 |
| Example 44 | 0.89 | 1100 | Example 98 | 0.36 | 230 |
| Example 45 | 2.5 | 4800 | Example 99 | 0.77 | 460 |
| Example 46 | 6.1 | 1400 | Example 100 | 2.6 | 1300 |
| Example 47 | 5.5 | 2200 | Example 103 | 0.76 | 250 |
| Example 48 | 2.8 | 2900 | Example 104 | 1.1 | 1400 |
| Example 49 | 1.4 | 4400 | Example 105 | 1.4 | 1000 |
| Example 50 | 2.2 | 5400 | Example 106 | 0.88 | 400 |
| Example 51 | 0.98 | 930 | Example 107 | 0.32 | 190 |
| Example 55 | 20 | 7400 | Example 108 | 0.59 | 800 |
| Example 56 | 2.1 | 2900 | Example 109 | 0.42 | 520 |
| Example 57 | 15 | 28000 | Example 110 | 0.59 | 750 |
| Example 58 | 4.8 | 9900 | Example 112 | 0.81 | 230 |

Next, the cell growth suppressive action of the compound of the General Formula (I) according to the invention will be explained below.

### Method for judging the pharmaceutical effect using cells

### a) Reagent

Fetal calf serum (FCS) was purchased from Moregate Biotech, and DMEM medium was purchased from Invitrogen Corp. WST-8 was purchased from Kishida Chemical Co., Ltd.

### b) Cells

Human cervical cancer cells (HeLa S3) were obtained from the American Type Culture Collection (ATCC).

### c) Method of judging the effect

Cells were suspended in a DMEM medium containing 10% FCS, and the cell suspension was dispensed to a 96-well plastic plate at a rate of 750 cells/100 microliters per well. The plate was incubated overnight in 5% CO₂-95% air at 37°C. A drug was subjected to graded dilution in dimethylsulfoxide and further diluted with a DMEM medium containing 10% FCS. Then, the dilution was dispensed to the plate on which cells had been disseminated, at a rate of 100 microliters per well. The plate was incubated for further three days in 5% CO₂-95% air at 37°C. Cell growth after incubation was measured by the WST-8 method (H. Tominaga, et al., Anal. Commun., 36, 47-50 (1999)). Here, the WST-8 method refers to a method in which 20 microliters of a WST-8 reagent solution is added to each well, incubation is conducted at 37°C for 45 minutes, the plate is stirred, and the amount of formazan produced is measured by a colorimetric method to determine the inhibitory rate of the drug. The concentration for 50% growth inhibition (EC₅₀, µM) of the compound was determined.

The compound according to the invention exhibits excellent cell growth inhibitory effect against human-derived cancer cells (HeLa S3), as shown in Table 2.

**[Table 2]**

| Example | Cell growth inhibitory effect (IC₅₀, µM) | Example | Cell growth inhibitory effect (IC₅₀, µM) |
|---|---|---|---|
| Example 5 | 11.00 | Example 56 | 1.40 |
| Example 6 | 0.40 | Example 58 | 3.00 |
| Example 8 | 0.25 | Example 62 | 0.86 |
| Example 17 | 1.10 | Example 66 | 2.90 |
| Example 19 | 0.92 | Example 68 | 5.10 |
| Example 22 | 3.50 | Example 71 | 3.00 |
| Example 25 | 0.80 | Example 75 | 11.00 |
| Example 28 | 1.10 | Example 77 | 1.60 |
| Example 29 | 3.30 | Example 86 | 0.51 |
| Example 30 | 2.50 | Example 89 | 0.36 |
| Example 36 | 6.80 | Example 95 | 0.22 |
| Example 39 | 11.00 | Example 104 | 0.99 |
| Example 40 | 6.50 | Example 106 | 0.40 |
| Example 44 | 2.40 | Example 107 | 0.21 |
| Example 46 | 4.10 | Example 108 | 1.20 |
| Example 50 | 3.60 | | |

### Method for judging the effect by combined use of drugs in cells

### a) Reagent

Fetal calf serum (FCS) was purchased from Moregate Biotech, DMEM medium from Invitrogen Corp., paclitaxel (tradename: Taxol) from Sigma-Aldrich, Inc., and WST-8 from Kishida Chemical Co., Ltd.

### b) Cells

Human cervical cancer cells (HeLa S3) were obtained from the American Type Culture Collection (ATCC).

### c) Method of judging the effect

Cells were suspended in a DMEM medium containing 10% FCS, and the cell suspension was dispensed to two 96-well plastic plates at a rate of 750 cells/100 microliters per well. The plates were incubated overnight in 5% CO₂-95% air at 37°C. A drug was subjected to graded dilution in dimethylsulfoxide and further diluted with DMSO or with a DMEM medium containing 10% FCS and also containing 2 nM paclitaxel. Then, the dilutions were each dispensed to one of the plates on which cells had been disseminated, at a rate of 100 microliters per well. The final concentration of paclitaxel at this stage was 1 nM. Also, the concentrations in the case of sole administration of the compound according to the invention were 0.03, 0.1, 0.3, 1 and 3 µM. The plates were incubated for further three days in 5% CO₂-95% air at 37°C. Cell growth after incubation was measured by the WST-8 method (H. Tominaga, et al., Anal. Commun., 36, 47-50 (1999)). Here, the WST-8 method refers to a method in which 20 microliters of a WST-8 reagent solution is added to each well, incubation is conducted at 37°C for 45 minutes, the plate is stirred, and the amount of formazan produced is measured by a colorimetric method to determine the inhibitory rate of the drug. The growth inhibitory effects of paclitaxel and of the compound according to the invention were determined, with the value obtained in sole treatment of DMSO being defined as 0%.

The compound according to the invention exhibits excellent cell growth inhibitory effect as well as a synergistic action with paclitaxel against human-derived cancer cells (HeLa S3), as shown in Table 3.

**[Table 3]**

| Example | Cell growth inhibitory effect by sole administration of paclitaxel (1 nM) (%) | Conc. of the compound of Example (µM) | Cell growth inhibitory effect by sole administration of the compound of Example (%) | Cell growth inhibitory effect by combined administration of paclitaxel and the compound of Example (%) |
|---|---|---|---|---|
| Example 5 | 44.1 | 0.1 | 0.0 | 72.8 |
| Example 6 | 44.1 | 0.3 | 19.6 | 89.0 |
| Example 8 | 37.8 | 0.1 | 4.6 | 87.1 |
| Example 17 | 45.4 | 0.3 | 0.0 | 73.8 |
| Example 19 | 44.1 | 0.1 | 0.0 | 77.6 |
| Example 22 | 43.3 | 1.0 | 18.5 | 80.9 |
| Example 25 | 45.4 | 0.1 | 0.0 | 65.1 |
| Example 28 | 45.4 | 0.3 | 0.0 | 84.5 |
| Example 29 | 45.4 | 0.3 | 0.0 | 77.3 |
| Example 30 | 36.8 | 1.0 | 29.1 | 90.1 |
| Example 36 | 45.4 | 3.0 | 17.2 | 83.4 |
| Example 39 | 43.3 | 3.0 | 5.4 | 72.2 |
| Example 40 | 43.3 | 1.0 | 6.5 | 76.9 |
| Example 44 | 44.1 | 0.3 | 7.1 | 86.5 |
| Example 46 | 36.8 | 1.0 | 8.5 | 75.0 |
| Example 50 | 45.4 | 1.0 | 6.0 | 82.0 |
| Example 56 | 37.8 | 0.3 | 6.5 | 81.8 |
| Example 58 | 45.4 | 1.0 | 0.0 | 81.4 |
| Example 62 | 36.8 | 0.1 | 2.9 | 68.0 |
| Example 66 | 36.8 | 1.0 | 7.2 | 60.9 |
| Example 68 | 36.8 | 3.0 | 27.3 | 71.8 |
| Example 71 | 36.8 | 1.0 | 13.2 | 60.7 |
| Example 75 | 45.4 | 0.3 | 27.1 | 91.5 |
| Example 77 | 36.8 | 0.3 | 2.2 | 51.8 |
| Example 86 | 43.3 | 0.03 | 6.2 | 71.6 |
| Example 89 | 43.3 | 0.1 | 19.1 | 85.8 |
| Example 95 | 44.1 | 0.03 | 0.0 | 73.5 |
| Example 104 | 43.3 | 0.3 | 6.3 | 81.6 |
| Example 106 | 43.3 | 0.1 | 6.1 | 76.7 |
| Example 107 | 43.3 | 0.03 | 11.5 | 74.1 |
| Example 108 | 44.1 | 1.0 | 17.2 | 86.4 |

From the above, the compound according to the invention is believed to be useful as an antitumor agent since it exhibits not only excellent cell growth inhibitory action based on Aurora A selective inhibitory activity, but also a synergistic action in combined use with other antitumor agent. Thus, it is believed that a pharmaceutical composition or Aurora A selective inhibitor containing the novel aminopyridine derivative according to the invention or a pharmaceutically acceptable salt or ester thereof, or an antitumor agent containing the compound according to the invention or a pharmaceutically acceptable salt or ester thereof is effective in the treatment of cancer patients.

The above-mentioned pharmaceutical composition and inhibitor, and the above-mentioned antitumor agent may contain a pharmaceutically acceptable carrier or diluent. Here, the "pharmaceutically acceptable carrier or diluent" refers to excipients [e.g., fats, beeswax, semi-solid and liquid polyols, natural or hydrogenated oils, etc.]; water (e.g., distilled water, particularly distilled water for injection, etc.), physiological saline, alcohol (e.g., ethanol), glycerol, polyols, aqueous glucose solution, mannitol, plant oils, etc.); additives [e.g., extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant], and the like.

A suitable tumor for which the therapeutic effect of the compound according to the invention is expected may be exemplified by human solid cancer. Examples of human solid cancer include brain cancer, head and neck cancer, esophageal cancer, thyroid cancer, small cell carcinoma, non-small cell carcinoma, breast cancer, stomach cancer, gallbladder and bile duct cancer, liver cancer, pancreas cancer, colon cancer, rectal cancer, ovarian cancer, chorioepithelioma, uterine cancer, cervical cancer, renal pelvic and ureteral cancer, bladder cancer, prostate cancer, penile cancer, testicular cancer, embryonal cancer, Wilms' tumor, skin cancer, malignant melanoma, neuroblastoma, osteosarcoma, Ewing's tumor, soft tissue sarcoma, and the like.

Next, the above-described "pharmaceutically acceptable salt or ester" will be explained below.

When the compound according to the invention is used as an antitumor agent or the like, it may be also used in a form of pharmaceutically acceptable salt. Typical examples of the pharmaceutically acceptable salt include a salt with an alkali metal such as sodium and potassium; a salt with an inorganic acid, such as hydrochloride, sulfate, nitrate, phosphate, carbonate, hydrogen carbonate, and perchlorate; a salt with an organic acid, such as acetate, propionate, lactate, maleate, fumarate, tartrate, malate, citrate, and ascorbate; a salt with sulfonic acid, such as methanesulfonate, isethionate, benzenesulfonate, and toluenesulfonate; a salt with acidic amino acid, such as aspartate and glutamate; and the like. A pharmaceutically acceptable salt of the Compound (I) is preferably a salt with an inorganic acid, such as hydrochloride, sulfate, nitrate, phosphate, carbonate, hydrogen carbonate, and perchlorate; more preferably hydrochloride.

The process for preparation of a pharmaceutically acceptable salt of the compound according to the invention may be carried out by an appropriate combination of those methods that are conventionally used in the field of organic synthetic chemistry. A specific example thereof is a method in which a solution of the compound according to the invention in its free form is subjected to neutralization titration with an alkaline solution or an acidic solution.

Examples of the ester of the compound according to the invention include methyl ester and ethyl ester. Such esters can be prepared by esterification of a free carboxyl group according to a conventional method.

With regard to each preparation of the combined preparation according to the invention, various preparation forms can be selected, and examples thereof include oral preparations such as tablets, capsules, powders, granules or liquids, or sterilized liquid parenteral preparations such as solutions or suspensions, suppositories, ointments and the like.

Solid preparations can be prepared in the forms of tablet, capsule, granule and powder without any additives, or prepared using appropriate carriers (additives). Examples of such carriers (additives) may include saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose; and other conventionally used additives such as gelatin, talc, plant oil and gum arabic.

These solid preparations such as tablets, capsules, granules and powders may generally contain, for example, 0.1 to 100% by weight, and preferably 5 to 98% by weight, of the compound of the above Formula (I) as an active ingredient, based on the total weight of the preparation.

Liquid preparations are produced in the forms of suspension, syrup, injection and drip infusion (intravenous fluid) using appropriate additives that are conventionally used in liquid preparations, such as water, alcohol or a plant-derived oil such as soybean oil, peanut oil and sesame oil.

In particular, when the preparation is administered parenterally in a form of intramuscular injection, intravenous injection or subcutaneous injection, appropriate solvent or diluent may be exemplified by distilled water for injection, an aqueous solution of lidocaine hydrochloride (for intramuscular injection), physiological saline, aqueous glucose solution, ethanol, polyethylene glycol, propylene glycol, liquid for intravenous injection (e.g., an aqueous solution of citric acid, sodium citrate and the like) or an electrolytic solution (for intravenous drip infusion and intravenous injection), or a mixed solution thereof.

Such injection may be in a form of a preliminarily dissolved solution, or in a form of powder *per se* or powder associated with a suitable carrier (additive) which is dissolved at the time of use. The injection liquid may contain, for example, 0.1 to 10% by weight of an active ingredient based on the total weight of the preparation.

Liquid preparations such as suspension or syrup for oral administration may contain, for example, 0.1 to 10% by weight of an active ingredient based on the total weight of the preparation.

Each preparation of the combined preparation according to the invention can be prepared by a person having ordinary skill in the art according to conventional methods or common techniques. For example, a preparation containing another antitumor agent that is used in combination with the compound represented by the above General Formula (I), can be prepared, if the preparation is an oral preparation, for example, by mixing an appropriate amount of the antitumor agent with an appropriate amount of lactose and filling this mixture into hard gelatin capsules which are suitable for oral administration. On the other hand, preparation can be carried out, if the preparation containing the antitumor agent is an injection, for example, by mixing an appropriate amount of the antitumor agent with an appropriate amount of 0.9% physiological saline and filling this mixture in vials for injection.

Also, in the case of a combination preparation containing the compound represented by the above General Formula (I) according to the invention and another antitumor agent, a person having ordinary skill in the art can easily prepare the preparation according to conventional methods or common techniques.

In the process according to the invention, preferred therapeutic unit may vary in accordance with, for example, the administration route of the compound represented by the General Formula (I), the type of the compound represented by the General Formula (I) used, and the dosage form of the compound represented by the General Formula (I) used; the type, administration route and dosage form of the other antitumor agent used in combination; and the type of cells to be treated, the condition of patient, and the like. The optimal treatment under the given conditions can be determined by a person skilled in the art, based on the set conventional therapeutic unit and/or based on the content of the present specification.

In the process according to the invention, the therapeutic unit for the compound represented by the above General Formula (I) may vary in accordance with, specifically, the type of compound used, the type of compounded composition, application frequency and the specific site to be treated, seriousness of the disease, age of the patient, doctor's diagnosis, the type of cancer, or the like. However, as an exemplary reference, the daily dose for an adult may be within a range of, for example, 1 to 1,000 mg in the case of oral administration. In the case of parenteral administration, preferably intravenous administration, and more preferably intravenous drip infusion, the daily dose may be within a range of, for example, 1 to 100 mg/m² (body surface area). Here, in the case of intravenous drip infusion, administration may be continuously carried out for, for example, 1 to 48 hours. Moreover, the administration frequency may vary depending on the administering method and symptoms, but it is, for example, once to five times a day. Alternatively, periodically intermittent administration such as administration every other day, administration every two days or the like may be employed as well in the administering method. The period of withdraw from medication in the case of parenteral administration is, for example, 1 to 6 weeks.

Although the therapeutic unit for the other antitumor agent used in combination with the compound represented by the General Formula (I) is not particularly limited, it can be determined, if needed, by those skilled in the art according to known literatures. Examples may be as follows.

The therapeutic unit of 5-fluorouracil (5-FU) is such that, in the case of oral administration, for example, 200 to 300 mg per day is administered in once to three times consecutively, and in the case of injection, for example, 5 to 15 mg/kg per day is administered once a day for the first 5 consecutive days by intravenous injection or intravenous drip infusion, and then 5 to 7.5 mg/kg is administered once a day every other day by intravenous injection or intravenous drip infusion (the dose may be appropriately increased or decreased).

The therapeutic unit of S-1 (Tegafur, Gimestat and Ostat potassium) is such that, for example, the initial dose (singe dose) is set to the following standard amount in accordance with the body surface area, and it is orally administered twice a day, after breakfast and after dinner, for 28 consecutive days, followed by withdrawal from medication for 14 days. This is set as one course of administration, which is repeated. The initial standard amount per unit body surface area (Tegafur equivalent) is 40 mg in one administration for an area less than 1.25 m²; 50 mg in one administration for an area of 1.25 m² to less than 1.5 m²; 60 mg in one administration for an area of 1.5 m² or more. This dose is appropriately increased or decreased depending on the condition of the patient.

The therapeutic unit for gemcitabine is, for example, 1 g as gemcitabine/m² in one administration, which is administered by intravenous drip infusion over a period of 30 minutes, and one administration per week is continued for 3 weeks, followed by withdrawal from medication on the fourth week. This is set as one course of administration, which is repeated. The dose is appropriately decreased in accordance with age, symptom or development of side-effects.

The therapeutic unit for doxorubicin (e.g., doxorubicin hydrochloride) is such that, for example, in the case of intravenous injection, 10 mg (0.2 mg/kg) (titer) is administered once a day by intravenous one-shot administration for 4 to 6 consecutive days, followed by withdrawal from medication for 7 to 10 days. This is set as one course of administration, which is repeated two or three times. Here, the total dose is preferably 500 mg (titer)/m² (body surface area) or less, and it may be appropriately increased or decreased within the range.

The therapeutic unit for etoposide is such that, for example, in the case of intravenous injection, 60 to 100 mg/m² (body surface area) per day is administered for 5 consecutive days, followed by withdrawal from medication for three weeks (the dose may be appropriately increased or decreased). This is set as one course of administration, which is repeated. Meanwhile, in the case of oral administration, for example, 175 to 200 mg per day is administered for 5 consecutive days, followed by withdrawal from medication for three weeks (the dose may be appropriately increased or decreased). This is set as one course of administration, which is repeated.

The therapeutic unit for docetaxel (docetaxel hydrate) is such that, for example, 60 mg as docetaxel/m² (body surface area) is administered once a day by intravenous drip infusion over a period of 1 hour or longer at an interval of 3 to 4 weeks (the dose may be appropriately increased or decreased).

The therapeutic unit of paclitaxel is such that, for example, 210 mg/m² (body surface area) is administered once a day by intravenous drip infusion over a period of 3 hours, followed by withdrawal from medication for at least 3 weeks. This is set as one course of administration, which is repeated. The dose may be appropriately increased or decreased.

The therapeutic unit for cisplatin is such that, for example, in the case of intravenous injection, 50 to 70 mg/m² (body surface area) is administered once a day, followed by withdrawal from medication for 3 weeks or longer (the dose may be appropriately increased or decreased). This is set as one course of administration, which is repeated.

The therapeutic unit for carboplatin is such that, for example, 300 to 400 mg/m² is administered once a day by intravenous drip infusion over a period of 30 minutes or longer, followed by withdrawal from medication for at least 4 weeks (the dose may be appropriately increased or decreased). This is set as one course of administration, which is repeated.

The therapeutic unit for oxaliplatin is such that 85 mg/m² is administered once a day by intravenous injection, followed by withdrawal from medication for two weeks. This is set as one course of administration, which is repeated.

The therapeutic unit for irinotecan (e.g., irinotecan hydrochloride) is such that, for example, 100 mg/m² is administered once a day by intravenous drip infusion for 3 or 4 times at an interval of one week, followed by withdrawal from medication for at least two weeks.

The therapeutic unit for topotecan is such that, for example, 1.5 mg/m² is administered once a day by intravenous drip infusion for 5 days, followed by withdrawal from medication for at least 3 weeks.

The therapeutic unit for cyclophosphamide is such that, for example, in the case of intravenous injection, 100 mg is administered once a day by intravenous injection for consecutive days. If the patient can tolerate, the daily dose may be increased to 200 mg. The total dose is 3,000 to 8,000 mg, which may be appropriately increased or decreased. If necessary, it may be injected or infused intramuscularly, intrathoracically or intratumorally. On the other hand, in the case of oral administration, for example, 100 to 200 mg is administered a day.

The therapeutic unit for gefitinib is such that 250 mg is orally administered once a day.

The therapeutic unit for cetuximab is such that, for example, 400 mg/m² is administered on the first day by intravenous drip infusion, and then 250 mg/m² is administered every week by intravenous drip infusion.

The therapeutic unit for bevacizumab is such that, for example, 3 mg/kg is administered every week by intravenous drip infusion.

The therapeutic unit for trastuzumab is such that, for example, typically for an adult, once a day, 4 mg as trastuzumab/kg (body weight) is administered initially, followed by intravenous drip infusion of 2 mg/kg over a period of 90 minutes or longer every week from the second administration.

The therapeutic unit for exemestane is such that, for example, typically for an adult, 25 mg is orally administered once a day after meal.

The therapeutic unit for leuprorelin (e.g., leuprorelin acetate) is such that, for example, typically for an adult, 11.25 mg is subcutaneously administered once in 12 weeks.

The therapeutic unit for imatinib is such that, for example, typically for an adult in the chronic phase of chronic myelogenous leukemia, 400 mg is orally administered once a day after meal.

The therapeutic unit for a combination of 5-FU and leucovorin is such that, for example, 425 mg/m² of 5-FU and 200 mg/m² of leucovorin are administered from the first day to the fifth day by intravenous drip infusion, and this course is repeated at an interval of 4 weeks.

### Best Mode for Carrying Out the Invention

### Examples

In a thin-layer chromatography of Examples and Referential Examples, Silica gel60F254 (Merck) was used as a plate and a UV detector was used in a detecting method. As silica gel for the column, WakogelTM C-300 or C-200 (Wako Pure Chemical) or NH (FUJI SILYSIA CHEMICAL) was used. In a reversed phase preparative liquid chromatography, CombiPrep Pro C18 (YMC) was used as a column and a 0.1% aqueous trifluoroacetic acid solution and a 0.1% solution of trifluoroacetic acid in acetonitrile were used in a mobile phase. MS spectra were measured using JMS-SX102A (JEOL) or QUATTROII (Micro Mass). NMR spectra were measured using a spectrometer in a type of Gemini-200 (200 MHz; Varian), Gemini-300 (300 MHz; Varian), VXR-300 (300 MHz; Varian), Mercury 400 (400 MHz; Varian) or Inova 400 (400 MHz; Varian) and all δ values are represented in ppm.

Meanings of abbreviations used in the NMR measurement are as follows.
- s:: singlet
- d:: doublet
- dd:: double doublet
- t:: triplet
- dt:: double triplet
- q:: quartet
- qui:: quintet
- m:: multiplet
- br:: broad
- J:: coupling constant
- Hz:: Hertz
- DMSO-d6:: dimethylsulfoxide-d6
- TBS:: tert-butyldimethylsilyl group
- Ms:: methanesulfonyl group
- SEM:: 2-(trimethylsilyl)ethoxymethyl group
- MOM:: methoxymethyl group
- THP:: tetrahydropyran-2-yl group
- Boc:: tert-butoxycarbonyl group

### Example 1

### Synthesis of (5-bromo-thiazol-2-yl)-(6-(4-benzoyl-piperazin-1-ylmethyl)-pyridin-2-yl)-amine

### (1) Synthesis of (6-chloro-pyridin-2-yl)-thiazol-2-yl-amine

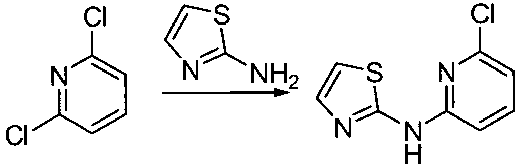

A mixture of 1.37 g (9.26 mmol) of 2-aminothiazole, 0.74 g (7.39 mmol) of 2,6-dichloropyridine, 387 mg (0.621 mmol) of (S)-(-)-2,2'-(bisdiphenylphosphino)-1,1'-binaphthyl, 322 mg (0.311 mmol) of tris(dibenzylideneacetone)dipalladium(O)-chloroform complex, 2.77 g (8.50 mmol) of cesium carbonate and 10 ml of toluene was heated under reflux for 1 hour and 30 minutes, cooled to room temperature and diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography to give 990 mg (4.68 mmol) of the title compound as a white solid.

### (2) Synthesis of methyl 6-(thiazol-2-ylamino)-pyridine-2-carboxylate

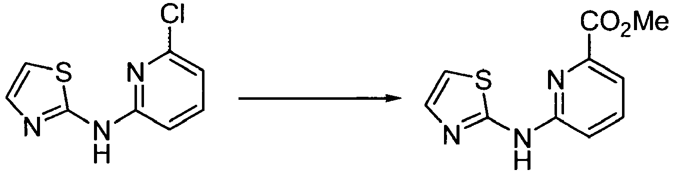

A mixture of 1.94 g (9.17 mmol) of (6-chloro-pyridin-2-yl)-thiazol-2-yl-amine, 206 mg (0.918 mmol) of palladium acetate, 508 mg (0.916 mmol) of 1,1'-bisdiphenylphosphinoferrocene, 2.40 ml (13.8 mmol) of N,N-diisopropylethylamine, 10 ml of methanol and 15 ml of N,N-dimethylformamide was stirred at 100°C for 3 hours and 15 minutes under 3 atmospheric pressure of carbon monoxide and cooled on an ice bath. The resulting solid was filtered and washed with ether to give 1.53 g (6.50 mmol) of the title compound as a light brown solid.

### (3) Synthesis of methyl 6-(3-methoxymethyl-3H-thiazol-2-ylideneamino)-pyridine-2-carboxylate

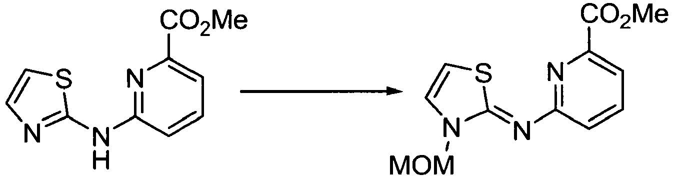

To a mixture of 4.85 g (20.6 mmol) of methyl 6-(thiazol-2-ylamino)-pyridine-2-carboxylate, 7.20 ml (41.2 mmol) of N,N-diisopropylethylamine and 100 ml of chloroform was added 2.35 ml (30.9 mmol) of chloromethylmethyl ether followed by stirring at room temperature for 1 hour. The reaction mixture was washed with 100 ml of water for three times and the organic layer was dried over anhydrous magnesium sulfate and filtered. The filtrate was concentrated to about 20 ml in total and the resulting solid was filtered. The resulting solid was washed with ether and dried *in vacuo* to give 6.38 g (20.6 mmol) of the title compound as a white solid.

### (4) Synthesis of methyl 6-(5-bromo-3-methoxymethyl-3H-thiazol-2-ylideneamino)-pyridine-2-carboxylate

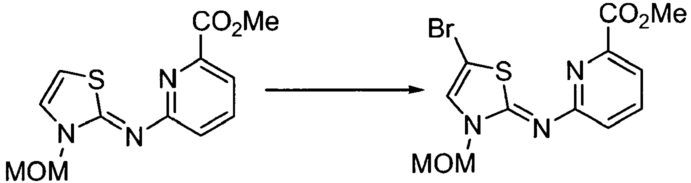

6.38 g (20.6 mmol) of methyl 6-(3-methoxymethyl-3H-thiazol-2-ylideneamino)-pyridine-2-carboxylate was dissolved in 60 ml of 1,4-dioxane and 3.67 g (20.6 mmol) ofN-bromosuccinimide was added thereto at room temperature. The reaction mixture was heated under reflux for 30 minutes, cooled to room temperature, diluted with 200 ml of ethyl acetate and washed with 150 ml of water for three times. After that, the reaction mixture was washed with 150 ml of brine, dried over anhydrous magnesium sulfate and filtered, and the filtrate was concentrated *in vacuo* to give 7.10 g (19.8 mmol) of the title compound as a white solid.

### (5) Synthesis of (6-(5-bromo-3-methoxymethyl-3H- thiazol-2-ylideneamino)-pyridin-2-yl)-methanol

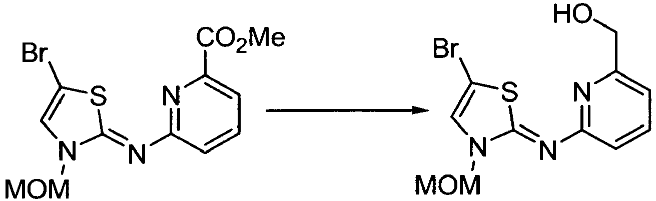

6.50 g (18.0 mmol) of methyl 6-(5-bromo-3-methoxymethyl-3H-thiazol-2-ylideneamino)-pyridine-2-carboxylate was dissolved in 80 ml of tetrahydrofuran, 790 mg (36.0 mmol) of lithium borohydride was added thereto and the mixture was heated under reflux. After 1 hour, 790 mg (36.0 mmol) of lithium borohydride was added thereto followed by further heating under reflux for 1 hour, the reaction mixture was cooled to room temperature and water was added thereto. After water was added until no more bubbling took place, the mixture was extracted with ethyl acetate. The resulting ethyl acetate solution was washed with 100 ml of water for two times and then washed with 100 ml of brine. The organic layer was dried over anhydrous magnesium sulfate and filtered, and the filtrate was concentrated *in vacuo.* The resulting residue was purified by a silica gel column chromatography to give 2.67 g (8.09 mmol) of the title compound as a white solid.

### (6) Synthesis of 1-(6-(5-bromo-3-methoxymethyl-3H-thiazol-2-ylideneamino)-pyridin-2-ylmethyl)-piperazine

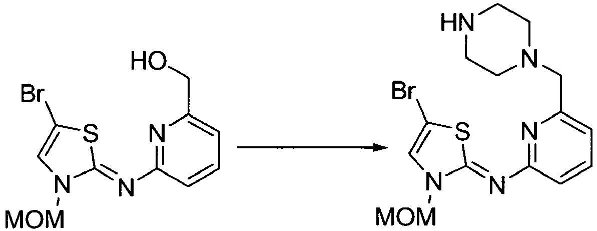

To a mixture of 330 mg (1.00 mmol) of (6-(5-bromo-3-methoxymethyl-3H-thiazol-2-ylideneamino)-pyridin-2-yl)-methanol, 0.348 ml (2.00 mmol) of N,N-diisopropylethylamine and 15 ml of chloroform was added 0.116 ml (1.50 mmol) of methanesulfonyl chloride at room temperature followed by stirring for 1 hour. The reaction mixture was washed with brine, the organic layer was dried over anhydrous magnesium sulfate and filtered, and the filtrate was concentrated *in vacuo.* The resulting residue was dissolved in 4 ml of dimethyl sulfoxide and dropped into 2 ml of a solution of 861 mg (10.0 mmol) of piperazine in dimethyl sulfoxide and the mixture was stirred at room temperature for 4 hours and 30 minutes. The reaction solution was filtered and purified by a reversed phase preparative liquid chromatography to give 281 mg (0.706 mmol) of the title compound as a white solid.

### (7) Synthesis of 1-(6-(5-bromo-3-methoxymethyl-3H-thiazol-2-ylideneamino)-pyridin-2-ylmethyl)-4-benzoyl-piperazine

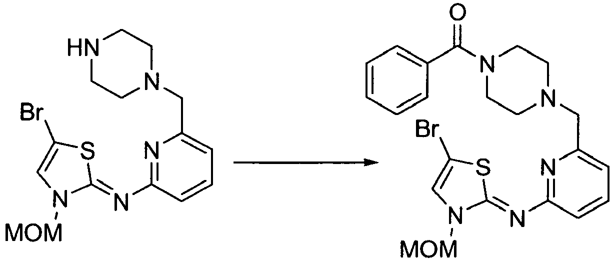

77 mg (0.217 mmol) of 1-(6-(5-bromo-3-methoxymethyl-3H-thiazol-2-ylideneamino)-pyridin-2-ylmethyl)-piperazine was dissolved in 10 ml of chloroform, 0.363 ml (2.60 mmol) of triethylamine was added thereto, 0.126 ml (1.09 mmol) of benzoyl chloride was gradually dropped thereinto and the mixture was stirred at room temperature for 30 minutes. The resulting mixture was diluted with ethyl acetate, washed with brine, dried over anhydrous magnesium sulfate and filtered, and the filtrate was concentrated *in vacuo.* The resulting residue was purified by a silica gel column chromatography to give 81 mg (0.161 mmol) of the title compound as a white solid.

### (8) Synthesis of (5-bromo-thiazol-2-yl)-(6-(4-benzoyl-piperazin-1-ylmethyl)-pyridin-2-yl)-amine

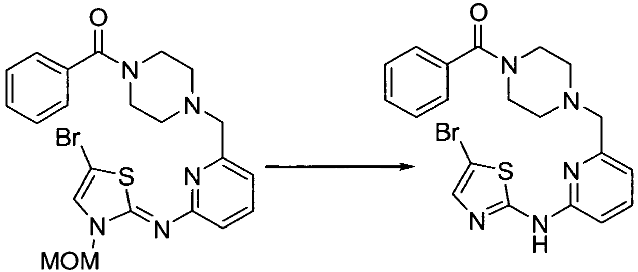

81 mg (0.161 mmol) of 1-(6-(5-bromo-3-methoxymethyl-3H-thiazol-2-ylideneamino)-pyridin-2-ylmethyl)-4-benzoyl-piperazine was dissolved in 10 ml of chloroform, a hydrochloric acid-1,4-dioxane solution (4 M, 4 ml) was added thereto and the mixture was stirred at room temperature for 18 hours. The reaction mixture was concentrated *in vacuo,* the residue was recrystallized from methanol-ether and filtered and the resulting white solid was dissolved in water (10 ml) and neutralized with sodium bicarbonate. The resulting precipitate was filtered to give 32 mg (0.070 mmol) of the title compound as a white solid.
Spectral data of the title compound are as follows.
¹H-NMR (DMSO-d₆) δ: 11.50 (brs, 1H), 7.78-7.62 (m, 1H), 7.50-7.30 (m, 6H), 7.02 (d, J = 6.9 Hz, 1H), 6.90 (d, J = 8.6 Hz, 1H), 3.80-3.20 (m, 10H)
Mass: 458, 460 (M+1)⁺

### Example 2

### Synthesis of (5-methyl- H-pyrazol-3-yl)-(6-(4-benzoyl-piperazin-1-yl-methyl)-pyridin-2-yl)-amine

### (1) Synthesis of 2-amino-6-(tert-butyldimethylsilyloxymethyl)pyridine

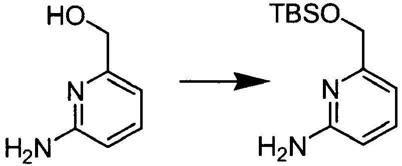

1.26 g (10.2 mmol) of 2-amino-6-hydroxymethylpyridine (Journal of Heterocyclic Chemistry, 2001, 38, 173) was dissolved in 5.1 mL of dimethylformamide and 1.7 g (25 mmol) of imidazole was added thereto. Under cooling with ice, 1.8 g (12 mmol) of tert-butyldimethylsilyl chloride was added thereto followed by stirring at room temperature for 1 hour. The reaction solution was diluted with ethyl acetate and the organic layer was washed with water and brine. This was dried over magnesium sulfate and filtered, and the filtrate was concentrated. The resulting residue was purified by a silica gel column chromatography to give 1.9 g of the title compound as an orange-colored oil.
Spectral data of the title compound are as follows.
¹H-NMR (CDCl₃) δ: 7.45 (dd, J = 8.0, 7.6 Hz, 1H), 6.86 (dd, J = 7.6, 0.8 Hz, 1H), 6.37 (dd, J = 8.0, 0.8 Hz, 1H), 4.65 (s, 2H), 4.34 (brs, 2H), 0.95 (s, 9H), 0.11 (s, 6H)
Mass: 239 (M + 1)⁺

### (2) Synthesis of N-(6-(tert-butyldimethylsilyloxymethyl)pyridin-2-yl)-3-oxobutanamide

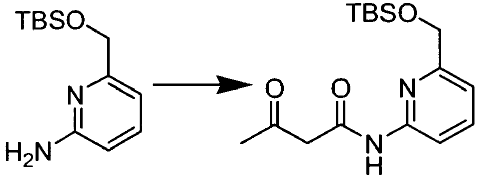

6.6 mL of tert-butyl acetoacetate was dissolved in 10 mL of toluene and the mixture was stirred at 100°C for 1 hour. To the reaction solution was added a solution of 1.9 g (8.1 mmol) of 2-amino-6-(tert-butyldimethylsilyloxymethyl)pyridine in 5 mL of toluene and the mixture was stirred at the same temperature for 15 hours. The reaction solution was concentrated and the resulting residue was purified by a silica gel column chromatography to give 2.2 g of the title compound as a yellow oil.
Spectral data of the title compound are as follows.
¹H-NMR (CDCl₃) δ: 9.03 (brs, 1H), 7.99 (brd, J = 7.6 Hz, 1H), 7.69 (dd, J = 8.0, 7.6 Hz, 1H), 7.25 (brd, J = 8.0 Hz, 1H), 4.72 (s, 2H), 3.58 (s, 2H), 2.33 (s, 3H), 0.96 (s, 9H), 0.12 (s, 6H)
Mass: 323 (M + 1)⁺

### (3) Synthesis of 3-(6-hydroxymethylpyridin-2-yl)amino-5-1H-methylpyrazole

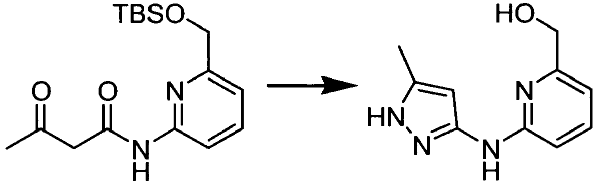

2.2 g (6.8 mmol) of N-(6-(tert-butyldimethylsilyloxymethyl)pyridin-2-yl)-3-oxobutanamide was dissolved in 68 mL of 1,2-dimethoxyethane and 8.9 mL (140 mmol) of methanesulfonic acid and 3.3 mL (68 mmol) of hydrazine monohydrate were added thereto successively at 0°C. After stirring at room temperature for 15 hours, the mixture was stirred at 80°C for 6 hours. Under cooling with an ice bath, 30 mL of 25% aqueous ammonia and 30 mL of water were added to the reaction solution and the mixture was extracted with chloroform. The organic layer was concentrated and the resulting residue was purified by a silica gel column chromatography to give 630 mg of the title compound as an orange-colored oil.
Spectral data of the title compound are as follows.
¹H-NMR (CD₃OD) δ: 7.53 (t, J = 7.6 Hz, 1H), 6.83 (brd, J = 7.6 Hz, 2H), 4.57 (s, 2H), 2.23 (s, 3H)
Mass: 205 (M+1)⁺

### (4) Synthesis of 3-(6-chloromethylpyridin-2-yl)amino-5-methyl-1H-pyrazole

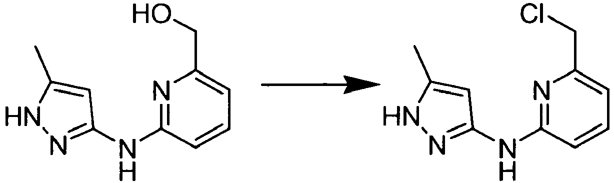

68 mg (340 µmol) of 3-(6-hydroxymethylpyridin-2-yl)amino-5-methyl-1H-pyrazole was dissolved in 4 mL of a 6:1 mixed solvent of chloroform and dimethylformamide. Under cooling on an ice bath, 250 µL (3.4 mmol) of thionyl chloride was added thereto. After stirring at room temperature for 18 hours, the reaction solution was concentrated. The resulting residue was purified by a silica gel thin-layer chromatography to give 87 mg of a brown oil. This oil was used as it was for the next reaction.

### (5) Synthesis of 3-(6-(4-benzoyl-]H-piperazin-1-ylmethyl)pyridin-2-yl)amino-5-methylpyrazole

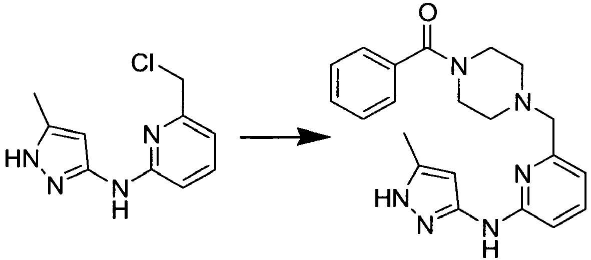

87 mg of the above-mentioned oil was dissolved in 2 mL of dimethyl sulfoxide and then 340 µL (1.95 mmol) of N,N-diisopropylethylamine and 134 mg (706 µmol) of N-benzoylpiperazine were added thereto successively. After the mixture was made to react at 90°C for 30 minutes, the reaction solution was purified by a reversed phase preparative column chromatography to give 52 mg of the title compound as a light yellow solid.
Spectral data of the title compound are as follows.
¹H-NMR (CD₃OD) δ: 7.93 (dd, J = 8.8, 7.6 Hz, 1H), 7.52-7.44 (m, 5H), 7.10 (d, J = 7.6 Hz, 1H), 7.09 (d, J = 8.8 Hz, 1H), 5.99 (s, 1H), 4.22 (s, 2H), 4.06-3.60 (m, 4H), 3.15-2.85 (m, 4H), 2.39 (s, 3H)
Mass: 377 (M+1)⁺

### Example 3

### Synthesis of (5-bromo-thiazol-2-yl)-(6-(4-(2,3-difluorobenzoyl)-piperazin-1-yl-methyl)-pyridin-2-yl)-amine

### (1) Synthesis of (5-bromo-thiazol-2-yl)-(6-(piperazin-1-ylmethyl)-pyridin-2-yl)-amine

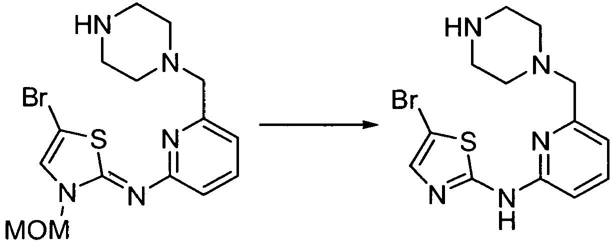

A hydrochloric acid-1,4-dioxane solution (4 M, 5 ml) was added to a mixture of 509 mg (1.28 mmol) of the compound obtained in Example 1-(6), 5 ml of chloroform and 14 ml of methanol and the mixture was stirred at room temperature for 10 hours. To the reaction solution was added 1 ml of a hydrochloric acid-1,4-dioxane solution followed by further stirring at room temperature for 18 hours. The reaction mixture was concentrated *in vacuo* and the residue was diluted with ethyl acetate and washed with a 1 M aqueous sodium hydroxide solution and brine. This was dried over magnesium sulfate, filtered and concentrated *in vacuo* to give 367 mg (1.04 mmol) of the title compound as a white solid.

### (2) Synthesis of (5-bromo-thiazol-2-yl)-(6-(4-(2,3-difluorobenzoyl)-piperazin-1-ylmethyl)-pyridin-2-yl)-amine

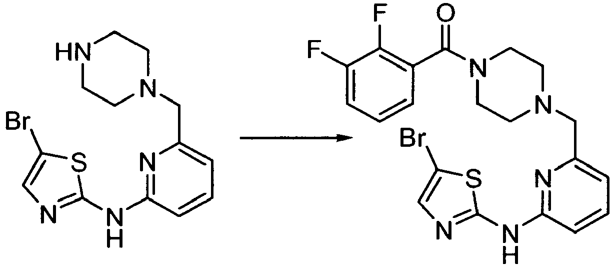

A mixture of 20 mg (0.056 mmol) of the compound obtained in the above (1), 32.5 mg (0.169 mmol) of 1-(3-dimethyl-aminopropyl)-3-ethylcarbodiimide hydrochloride, 22.9 mg (0.169 mmol) of 1-hydroxybenzotriazole, 26.8 mg (0.169 mmol) of 2,3-difluorobenzoic acid and 1 ml of chloroform was stirred at room temperature for 4 hours. Water was added thereto and the resulting mixture was extracted with ethyl acetate followed by washing with brine. The resulting mixture was dried over magnesium sulfate and filtered, the filtrate was concentrated *in vacuo* and the resulting residue was purified by a preparative thin-layer chromatography to give 17.0 mg (0.034 mmol) of the title compound as a colorless amorphous substance.
Spectral data of the title compound are as follows.
¹H-NMR (CDCl₃) δ: 7.63 (dd, J = 7.8, 7.6 Hz, 1H), 7.34 (s, 1H), 7.32-7.09 (m, 3H), 7.06 (d, J = 7.2 Hz, 1H), 6.76 (d, J = 8.0 Hz, 1H), 3.90-3.82 (m, 2ml), 3.76 (s, 2H), 3.47-3.37 (m, 2H), 2.76-2.63 (m, 2H), 2.63-2.50 (m, 2H)
Mass: 494, 496 (M+1)⁺

### Example 4

### Synthesis of (thiazol-2-yl)-(6-(4-(2,3-difluorobenzoyl)-piperazin-1-ylmethyl)-pyridin-2-yl)-amine

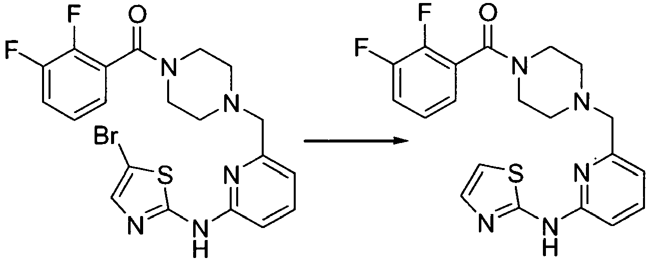

8.5 mg (0.017 mmol) of the compound obtained in Example 3-(2) was dissolved in 1 ml of methanol, 10 mg of 10% palladium-carbon was added thereto and the mixture was stirred under a hydrogen atmosphere at ordinary pressure and room temperature for 1 hour. The reaction solution was filtered, the solvent was concentrated *in vacuo* and the resulting residue was purified by a preparative thin-layer chromatography to give 4.1 mg (0.010 mmol) of the title compound as a colorless amorphous substance.
Spectral data of the title compound are as follows.
¹H-NMR (CD₃OD) δ: 7.65 (t, J = 7.4 Hz, 1H), 7.32 (d, J = 3.7 Hz, 1H), 7.40-7.30 (m, 1H), 7.28-7.22 (m, 1H), 7.19-7.14 (m, 1H), 7.04 (d, J = 7.2 Hz, 1H), 6.92-6.85 (m, 2H), 3.82 (dd, J = 5.3, 5.1 Hz, 2H), 3.74 (s, 2H), 3.39 (t, J = 4.7 Hz, 2H), 2.69 (t, J = 5.1 Hz, 2H), 2.58 (dd, J = 5.7, 4.1 Hz, 2H)
Mass: 416 (M + 1)⁺

### Example 5

### Synthesis of 6-((4-(3-chloro-2-fluorobenzoyl)piperazin-1-yl)methyl)-N-thiazol-2-ylpyridin-2-amine

### (1) Synthesis of 2-bromo-6-(((tert-butyl(dimethyl)silyl)oxy)methyl)pyridine

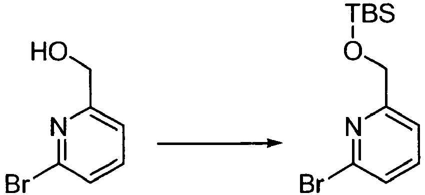

5.00 g of (6-bromo-pyridin-2-yl)-methanol was dissolved in 50 mL of dimethylformamide and 2.72 g of imidazole was added thereto. Under cooling with ice, 5.21 g of tert-butyldimethylsilyl chloride was added thereto followed by stirring at room temperature for 1 hour. The reaction solution was diluted with ethyl acetate and washed with water and brine. The resulting organic layer was dried over magnesium sulfate and filtered, and the filtrate was concentrated. The resulting residue was purified by a silica gel column chromatography (eluent: hexane/ethyl acetate = 10/1 to 1/1) to give the title compound.

### (2) Synthesis of 6-(((tert-butyl(dimethyl)silyl)oxy)methyl)-N-thiazol-2-ylpyridin-2-amine

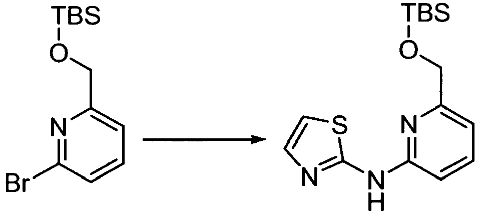

A mixture of 7.85 g of 2-bromo-6-(((tert-butyl(dimethyl)silyl)oxy)methyl)pyridine, 3.12 g of 2-aminothiazole, 1.50 g of 9,9-dimethyl-4,5-bis(diphenylphosphino)xanthene, 1.35 g of tris(dibenzylideneacetone)dipalladium(O)-chloroform complex, 13.8 g of potassium phosphate and 80 ml of 1,4-dioxane was stirred at 100°C for 4.5 hours, cooled to room temperature and diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (3) Synthesis of (6-(thiazol-2-ylamino)pyridin-2-yl)methanol

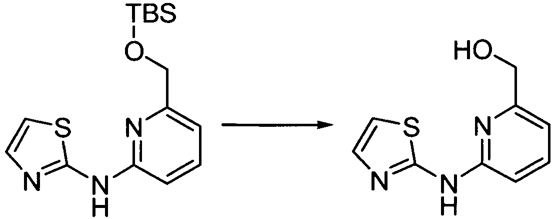

6.48g of 6-(((tert-butyl(dimethyl)silyl)oxy)methyl)-N-thiazol-2-ylpyridin-2-amine was dissolved in 100ml of tetrahydrofuran. Under cooling with ice, a tetrabutylammonium fluoride-tetrahydrofuran solution (1.0 M, 20.2 ml) was added followed by stirring at room temperature for 1 hour. The reaction solution was diluted with ethyl acetate and then washed with phosphate buffer (pH 6.8). The organic layer was dried over anhydrous magnesium sulfate and filtered, and the filtrate was concentrated to give the title compound.

### (4) Synthesis of 6-(chloromethyl)-N-thiazol-2-ylpyridin-2-amine

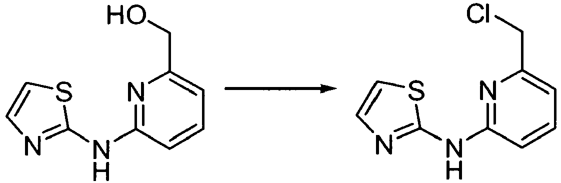

The entire amount of (6-(thiazol-2-ylamino)pyridin-2-yl)methanol obtained by the above operation was suspended in 150 ml of chloroform, and 7.37 ml of thionyl chloride was added thereto. After stirring at room temperature for 2 hours, the reaction solution was concentrated. The resulting residue was diluted with ethyl acetate, and then washed with a 2 M aqueous sodium hydroxide solution and brine. The organic layer was dried over anhydrous magnesium sulfate and filtered, and the filtrate was concentrated. The residue was purified by a silica gel column chromatography (eluent: chloroform to chloroform/methanol = 20/1) to give the title compound.

### (5) Synthesis of 6-((4-(3-chloro-2-fluorobenzoyl)piperazin-1-yl)methyl)-N-thiazol-2-ylpyridin-2-amine

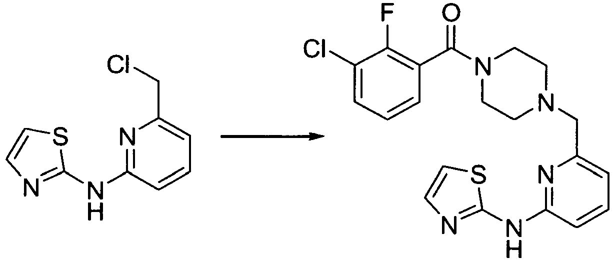

A mixture of 2.70 g of 6-(chloromethyl)-N-thiazol-2-ylpyridin-2-amine, 4.00 g of 1-(3-chloro-2-fluorobenzoyl)piperazine obtained in Reference Example 1, 6.25 ml of N,N-diisopropylethylamine and 30 ml of dimethylformamide was stirred at 90°C for 2 hours. The reaction solution was diluted with ethyl acetate and then washed with water and brine. The resulting organic layer was dried over magnesium sulfate and filtered, and the filtrate was concentrated. The resulting residue was purified by a silica gel column chromatography (eluent: chloroform to chloroform/methanol = 10/1). Then, the obtained compound was suspended in ethyl acetate, and filtered and collected to give the title compound as a colorless amorphous substance.
Spectral data of the title compound are as follows.
¹H-NMR (DMSO-d₆) δ: 11.20 (s, 1H), 7.65 (t, J = 7.8 Hz, 2H), 7.41-7.33 (m, 2H), 7.29 (t, J = 7.8 Hz, 1H), 7.00-6.87 (m, 3H), 3.72-3.61 (m, 4H), 3.27-3.20 (m, 2H), 2.60-2.36 (m, 4H).
Mass: 432 (M + 1)⁺

Examples 6 to 15, 32 to 43 and 63 were synthesized in the same manner as in Example 5 as follows.

### Example 6

### Synthesis of 6-((4-(2,3-dichlorobenzoyl)piperazin-1-yl)methyl)-N-thiazol-2-ylpyridin-2-amine

¹H-NMR (DMSO-d₆) δ: 11.21 (s, 1H), 7.72-7.61 (m, 2H), 7.42 (t, J = 7.8 Hz, 1H), 7.39-7.29 (m, 2H), 7.02-6.90 (m, 3H), 3.73-3.61 (m, 4H), 3.19-3.12 (m, 2H), 2.60-2.38 (m, 4H)
Mass: 448 (M+1)⁺

### Example 7

### Synthesis of 6-((4-(3-chlorobenzoyl)piperazin-1-yl)methyl)-N-thiazol-2-ylpyridin-2-amine

¹H-NMR (DMSO-d₆) δ: 11.20 (s, 1H), 7.65 (t, J = 7.8 Hz, 1H), 7.54-7.39 (m, 3H), 7.38-7.28 (m, 2H), 7.00-6.88 (m, 3H), 3.64 (s, 4H), 3.48-3.19 (m, 2H), 2.62-2.34 (m, 4H)
Mass: 414 (M+1)⁺

### Example 8

### Synthesis of 6-((4-(2-fluoro-3-methylbenzoyl)piperazin-1-yl)methyl)-N-thiazol-2-ylpyridin-2-amine

¹H-NMR (DMSO-d₆) δ: 11.21 (s, 1H), 7.65 (t, J = 7.8 Hz, 1H), 7.39-7.31 (m, 2H), 7.20-7.10 (m, 2H), 7.00-6.89 (m, 3H), 3.73-3.62 (m, 4H), 3.22 (t, J = 4.7 Hz, 2H), 2.56-2.34 (m, 4H), 2.24 (d, J = 1.6 Hz, 3H)
Mass: 412 (M+1)⁺

### Example 9

### Synthesis of 6-((4-(2-chloro-3-fluorobenzoyl)piperazin-1-yl)methyl)-N-thiazol-2-ylpyridin-2-amine

¹H-NMR (DMSO-d₆) δ: 11.21 (brs, 1H), 7.65 (t, J = 7.8 Hz, 1H), 7.51-7.42 (m, 2H), 7.36 (d, J = 3.9 Hz, 1H), 7.25-7.18 (m, 1H), 7.02-6.81 (m, 3H), 3.75-3.60 (m, 4H), 3.16 (t, J = 4.3 Hz, 2H), 2.61-2.40 (m, 4H)
Mass: 432 (M + 1)⁺

### Example 10

### Synthesis of 6-(((1 S,4S)-5-(3-chloro-2-fluorobenzoyl)-2,5-diazabicyclo[2.2.1]hept-2-yl)methyl)-N-thiazol-2-ylpyridin-2-amine trifluoroacetate

¹H-NMR (DMSO-d₆) δ: 7.84-7.71 (m, 2H), 7.48-7.40 (m, 2H), 7.38-7.30 (m, 1H), 7.19-7.07 (m, 3H), 5.00-3.36 (m, 8H), 2.54-2.14 (m, 2H)
Mass: 444 (M + 1)⁺

### Example 11

### Synthesis of 6-(((1R,4R)-5-(3-chloro-2-fluorobenzoyl)-2,5-diazabicyclo[2.2.1]hept-2-yl)methyl)-N-thiazol-2-ylpyridin-2-amine

¹H-NMR (CDCl₃) δ: 7.63-7.57 (m, 1H), 7.50-7.44 (m, 2H), 7.37-7.31 (m, 1H), 7.19-7.13 (m, 1H), 7.04-6.99 (m, 1 H), 6.87-6.77 (m, 2H), 4.93-2.82 (m, 8H), 2.05 and 1.99 (each d, J = 10.0 Hz, total 1H), 1.83 (d, J = 10.0 Hz, 1H)
Mass: 444, 446 (M + 1)⁺

### Example 12

### Synthesis of 6-((4-(3-bromo-2-fluorobenzoyl)piperazin-1-yl)methyl)-N-thiazol-2-ylpyridin-2-amine trifluoroacetate

¹H-NMR (CD₃OD) δ: 7.94 (dd, J = 8.4,7.2 Hz, 1H), 7.80-7.74 (m, 1H), 7.53 (d, J = 3.6 Hz, 1H), 7.46-7.40 (m, 1H), 7.32 (d, J = 7.2 Hz, 1H), 7.26 (d, J = 8.4 Hz, 1H), 7.23 (t, J = 8.0 Hz, 1H), 7.19 (d, J = 3.6 Hz, 1H), 4.53 (s, 2H), 4.15-4.00 (m, 2H), 3.72-3.64 (m, 2H), 3.59-3.52 (m, 2H), 3.48-3.41 (m, 2H)
Mass: 478 (M+1)⁺

### Example 13

### Synthesis of 6-((4-(2-fluoro-3-(trifluoromethyl)benzoyl)piperazin-1-yl)methyl)-N-thiazol-2-ylpyridin-2-amine trifluoroacetate

¹H-NMR (CD₃OD) δ: 7.95 (dd, J = 8.4,7.2 Hz, 1H), 7.86 (brt, J = 7.6 Hz, 1H), 7.75 (brt, J = 6.4 Hz, 1H), 7.55 (d, J = 4.4 Hz, 1H), 7.49 (t, J = 8.0 Hz, 1H), 7.34 (d, J = 7.2 Hz, 1H), 7.27 (d, J = 8.4 Hz, 1H), 7.20 (d, J = 4.4 Hz, 1H) 4.54 (s, 2H), 4.16-4.00 (m, 2H), 3.74-3.66 (m, 2H), 3.61-3.53 (m, 2H), 3.50-3.42 (m, 2H)
Mass: 466 (M + 1)⁺

### Example 14

### Synthesis of 6-(((3R)-4-(3-chloro-2-fluorobenzoyl)-3-methylpiperazin-1-yl)methyl)-N-thiazol-2-ylpyridin-2-amine

¹H-NMR (CDCl₃) δ: 7.60 (t, J = 7.6 Hz, 1H), 7.49 (d, J = 3.7 Hz, 1H), 7.43 (t, J = 7.6 Hz, 1H), 7.26-7.11 (m, 2H), 7.06-7.01 (m, 1H), 6.85 (d, J = 3.1 Hz, 1H), 6.80 (d, J = 8.0 Hz, 1H), 5.00-2.20 (m, 9H), 1.50-1.25 (m, 3H)
Mass: 446, 448 (M + 1)⁺

### Example 15

### Synthesis of 6-((4-(3-(difluoromethyt)-2-fluorobenzoyl)piperazin-1-yl)methyl)-N-thiazol-2-ylpyridin-2-amine

¹H-NMR (CDCl₃) δ: 7.65 (brt, J = 7.6 Hz, 1H), 7.60 (dd, J = 8.0,7.2 Hz, 1H), 7.51 (brt, J = 7.8 Hz, 1H), 7.48 (d, J = 3.6 Hz, 1H), 7.31 (t, J = 7.6 Hz, 1H), 7.00 (d, J = 7.2 Hz, 1H), 6.89 (t, J = 54.8 Hz, 1H), 6.85 (d, J = 3.6 Hz, 1H), 6.82 (d, J = 8.0 Hz, 1H), 3.90-3.83 (m, 2H), 3.76 (s, 2H), 3.41-3.33 (m, 2H), 2.71 (brt, J = 4.8 Hz, 2H), 2.61-2.52 (m, 2H)
Mass: 448 (M+ 1)⁺

### Example 16

### Synthesis of 6-((4-(3-chloro-2-fluorobenzoyl)piperazin-1-yl)methyl)-N-1H-pyrazol-3-ylpyridin-2-amine

### (1) Synthesis of(6-((1-((2-(trimethylsilyl)ethoxy)methyl)-1H-pyrazol-3-yl)amino)pyridin-2-yl)methanol

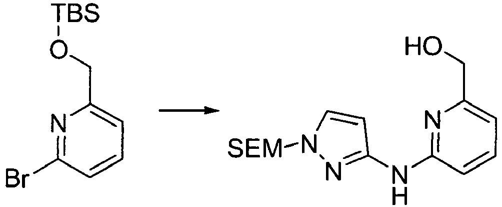

In the same manner as in Example 5-(2) to (3), the title compound was obtained using 2-bromo-6-(((tert-butyl(dimethyl)silyl)oxy)methyl)pyridine obtained in Example 5-(1) and 1-((2-(trimethylsilyl)ethoxy)methyl)-1-H-pyrazol-3-amine obtained in Reference Example 2.

### (2) Synthesis of 6-(methanesulfonyloxyrnethyl)-N-(1-((2-(trimethylsilyl)ethoxy)methyl)-1H-pyrazol-3-yl)pyridin-2-amine

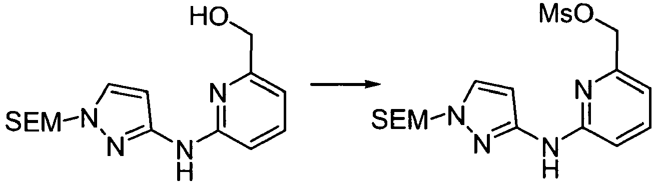

To a mixture of 10 mg of(6-((1-((2-(trimethylsilyl)ethoxy)methyl)-1H-pyrazol-3-yl)amino)pyridin-2-yl)methanol, 27 µl of N,N-diisopropylethylamine and 1 ml of chloroform was added 7.3 µl of methanesulfonyl chloride at room temperature followed by stirring for 1 hour. The reaction mixture was washed with brine, and the organic layer was dried over anhydrous magnesium sulfate and filtered. The filtrate was then concentrated *ᵢₙ vacuo* to give the title compound.

### (3) Synthesis of 6-((4-(3-chloro-2-fluorobenzoyl)piperazin-1-yl)methyl)-N-(1-((2-(trimethylsilyl)ethoxy)methyl)-1H-pyrazol-3-yl)pyiidin-2-amine

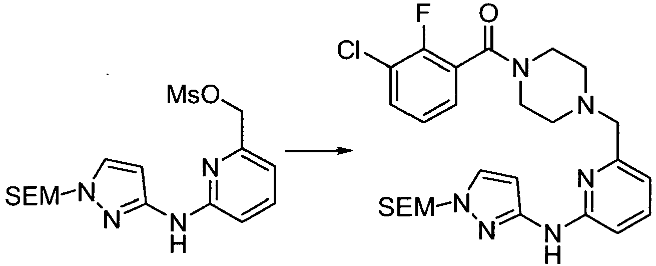

A mixture of 10 mg of 6-(methanesulfonyloxymethyl)-N-(1-((2-(trimethylsilyl)ethoxy)methyl)-1H-pyrazol-3-yl)pyridin-2-amine, 40 mg of 1-(3-chloro-2-fluorobenzoyl)piperazine hydrochlorideobtained in Reference Example 1, 27 µl of N,N-diisopropylethylamine and 1 ml of chloroform was stirred at 60°C for 5 hours. The reaction solution was diluted with chloroform, and then washed with water and brine. The resulting organic layer was dried over magnesium sulfate and filtered, and the filtrate was concentrated. The resulting residue was purified by a silica gel column chromatography(eluent: chloroform to chloroform/methanol = 20/1) to give the title compound.

### (4) Synthesis of 6-((4-(3-chloro-2-ftuorobenzoyl)piperazin-1-yl)methyl)-N-1-H-pyrazol-3-ylpyridin-2-amine

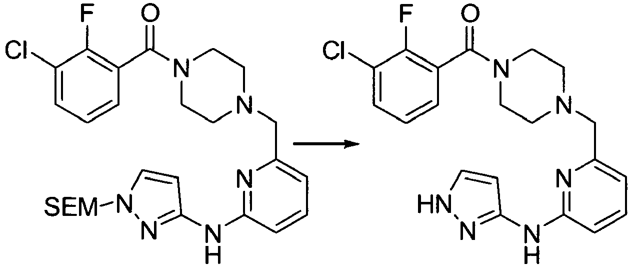

18 mg of (6-((4-(3-Chloro-2-fluorobenzoyl)piperazin-1-yl)methyl)-N-(1-((2-(trimethylsilyl)ethoxy)methyl)-1H-pyrazol-3-yl)pyridin-2-amine was dissolved in 900 µl of trifluoroacetate and 100 µl of water followed by stirring at room temperature for 5 hours. The reaction solution was concentrated *in vacuo,* diluted with ethyl acetate, and then washed with saturated sodium bicarbonate, water and brine. The resulting organic layer was dried over magnesium sulfate and filtered, and the filtrate was concentrated. The resulting residue was purified by a silica gel column chromatography (eluent: chloroform to chloroform/methanol = 10/1) to give the title compound.
Spectral data of the title compound are as follows.
¹H-NMR (CDCl₃) δ: 7.51 (dd, J = 8.2,7.4 Hz, 1H), 7.48-7.39 (m, 2H), 7.36-7.20 (m, 2H) 7.17-7.10 (m, 1H), 6.90 (d, J = 8.2 Hz, 1H), 6.82 (d, J = 7.2 Hz, 1H), 5.98 (s, 1H), 3.87 (brs, 2H), 3.62 (s, 2H), 3.37 (brs, 2H), 2.63 (t, J = 5.2 Hz, 2H), 2.51 (brs, 2H)
Mass: 415 (M + 1)⁺

Examples 17 to 31 were synthesized in the same manner as in Example 16 as follows.

### Example 17

### Synthesis of 6-((4-(2,3-dichlorobenzoyl)piperazin-1-yl)methyl)-N-1H-pyrazol-3-ylpyridin-2-amine

¹H-NMR (CDCl₃) δ: 7.57-7.47 (m, 3H), 7.31-7.10 (m, 4H), 6.90 (d, J = 8.0 Hz, 1H), 6.82 (d, J = 7.4 Hz, 1H), 5.98 (d, J = 2.0 Hz, 1H), 3.97-3.80 (m, 2H), 3.62 (s, 2H), 3.38-3.23 (m, 2H), 2.68-2.59 (m, 2H), 2.59-2.40 (m, 2H)
Mass: 431 (M + 1)⁺

### Example 18

### Synthesis of 6-((4-(2-chloro-3-(trifluoromethyl)benzoyl)piperazin-1-yl)methyl)-N-1H-pyrazol-3-ylpyridin-2-amine trifluoroacetate

¹H-NMR (CD₃OD) δ: 8.01 (dd, J = 8.8, 7.2 Hz, 1H), 7.89 (dd, J = 7.2, 2.4 Hz, 1H), 7.79 (d, J = 2.4 Hz, 1H), 7.67-7.58 (m, 2H), 7.14 (d, J = 8.8 Hz, 1H), 7.04 (brd, J = 7.2 Hz, 1H), 6.16 (d, J = 2.4 Hz, 1H), 4.13-4.03 (m, 1H), 4.00-3.89 (m, 1H), 3.97 (s, 2H), 3.44 (brt, J = 5.2 Hz, 2H), 2.88-2.80 (m, 2H), 2.73-2.66 (m, 2H)
Mass: 465 (M+1)⁺

### Example 19

### Synthesis of 6-((4-(2-fluoro-3-(trifluoromethyl)benzoyl)piperazin-1-yl)methyl)-N-1H-pyrazol-3-ylpyridin-2-amine trifluoroacetate

¹H-NMR (CD₃OD) δ: 8.01 (dd, J = 8.8, 7.2 Hz, 1H), 7.84 (brt, J = 7.2 Hz, 1H), 7.79 (d, J = 2.4 Hz, 1H), 7.71 (brt, J = 7.2 Hz, 1H), 7.49 (brt, J = 7.6 Hz, 1H), 7.14 (d, J = 8.8 Hz, 1H), 7.04 (dd, J = 7.2, 1.2 Hz, 1H), 6.16 (d, J = 2.4 Hz, 1H), 4.05-3.97 (m, 2H), 3.97 (s, 2H), 3.58-3.51 (m, 2H), 2.82 (brt, J = 4.8 Hz, 2H), 2.70 (brt, J = 4.8 Hz, 2H)
Mass: 449 (M+1)⁺

### Example 20

### Synthesis of 6-((4-(3-bromo-2-fluorobenzoyl)piperazin-1-yl)methyl)-N-1H-pyrazol-3-ylpyridin-2-amine trifluoroacetate

¹H-NMR (CD₃OD) δ: 8.01 (dd, J = 8.8, 7.2 Hz, 1H), 7.81-7.73 (m, 2H), 7.43-7.37 (m, 1H), 7.23 (t, J = 7.6 Hz, 1H), 7.14 (d, J = 8.8 Hz, 1H), 7.04 (d, J = 7.2 Hz, 1H), 6.16 (d, J = 2.4 Hz, 1H), 4.05-3.93 (m, 4H), 3.57-3.50 (m, 2H), 2.82 (brt, J = 4.8 Hz, 2H), 2.70 (brt, J = 4.8 Hz, 2H)
Mass: 459 (M + 1)⁺

### Example 21

### Synthesis of 6-((4-((3,4-dichlorophenyl)acetyl)piperazin-1-yl)methyl)-N-1H-pyrazol-3-ylpyridin-2-amine trifluoroacetate

¹H-NMR (CD₃OD) δ: 7.97 (dd, J = 8.8, 7.6 Hz, 1H), 7.80 (d, J = 2.4 Hz, 1H), 7.47 (d, J = 8.4 Hz, 1H) 7.44 (d, J = 2.0 Hz, 1H), 7.19 (dd, J = 8.4,2.0 Hz, 1H), 7.13 (brd, J = 8.8 Hz, 1H), 7.04 (brd, J = 7.6 Hz, 1H), 6.17 (d, J = 2.4 Hz, 1H), 4.02 (s, 2H), 3.97 (s, 1H), 3.86-3.76 (m, 6H), 2.83-2.74 (m, 4H)
Mass: 445 (M + 1)⁺

### Example 22

### Synthesis of 6-((4-(3-(difluoromethyl)-2-fluorobenzoyl)piperazin-1-yl)methyl)-N-1H-pyrazol-3-ylpyridin-2-amine

¹H-NMR (CDCl₃) δ: 7.64 (brt, J = 7.2 Hz, 1H), 7.55-7.44 (m, 3H), 7.31 (t, J = 8.0 Hz, 1H), 6.91 (d, J = 8.0 Hz, 1H), 6.89 (t, J = 54.8 Hz, 1H), 6.82 (d, J = 7.6 Hz, 1H), 5.99 (s, 1H), 3.92-3.84 (m, 2H), 3.62 (s, 2H), 3.42-3.34 (m, 2H), 2.66-2.60 (m, 2H), 2.55-2.47 (m, 2H)
Mass: 431 (M + 1)⁺

### Example 23

### Synthesis of 6-(((1S,4S)-5-(3-chloro-2-fluorobenzoyl)-2,5-diazabicyclo[2.2.1]hept-2-yl)methyl)-N-1H-pyrazol-3-ylpyridin-2-amine

¹H-NMR (CDCl₃) δ: 7.58-7.42 (m, 3H), 7.36-7.28 (m, 1H), 7.28-7.10 (m, 2H), 6.97-6.81 (m, 2H), 6.06-5.96 (m, 1H), 4.95-2.72 (m, 8H), 2.10-2.00 (m, 1H), 1.86-1.78 (m, 1H)
Mass: 427 (M + 1)⁺

### Example 24

### Synthesis of 6-(1-(4-(3-chloro-2-fluorobenzoyl)piperazin--yl)ethyl)-N-1H-pyrazol-3-ylpyridin-2-amine

¹H-NMR (CDCl₃) δ: 7.52 (dd, J = 8.2,7.6 Hz, 1H), 7.50-7.40 (m, 2H), 7.27-7.21 (m, 1H) 7.17-7.11 (m, 1H), 6.87 (d, J = 8.4 Hz, 1H), 6.80 (d, J = 7.2 Hz, 1H), 6.01 (d, J = 1.8 Hz, 1H), 3.83 (brs, 2H), 3.58-3.52 (m, 1H), 3.34 (brs, 2H), 2.72-2.40 (m, 4H), 1.42 (d, J = 6.8 Hz, 3H)
Mass: 429 (M + 1)⁺

### Example 25

### Synthesis of 6-((4-(3-chloro-2-fluorobenzoyl)piperazin-1-yl)methyl)-N-(5-methyl-1H-pyrazol-3-yl)pyridin-2-amine

¹H-NMR (CD₃OD) δ: 7.99 (dd, J = 8.8, 7.2 Hz, 1H), 7.64-7.57 (m, 1H), 7.38-7.26 (m, 2H), 7.11 (d, J = 8.8 Hz, 1H), 7.01 (d, J = 7.2 Hz, 1H), 5.91 (s, 1H), 4.03-3.95 (m, 2H), 3.94 (s, 2H), 3.56-3.50 (m, 2H), 2.82-2.75 (m, 2H), 2.69-2.64 (m, 2H), 2.35 (s, 3H)
Mass: 429 (M + 1)⁺

### Example 26

### Synthesis of 6-((4-(2,3-dichlorobenzoyl)piperazin-1-yl)methyl)-N-(5-methyl-1H-pyrazol-3-yl)pyridin-2-amine trifluoroacetate

¹H-NMR (CD₃OD) δ: 7.96 (dd, J = 8.8, 7.2 Hz, 1H), 7.64 (dd, J = 8.0, 1.6 Hz, 1H), 7.42 (t, J = 8.0 Hz, 1H), 7.32 (dd, J = 8.0, 1.6 Hz, 1H), 7.09 (dd, J = 8.0, 0.8 Hz, 1H), 7.05 (dd, J = 7.2, 0.8 Hz, 1H), 5.95 (d, J = 0.8 Hz, 1H), 4.13-4.03 (m, 1H), 4.07 (s, 2H), 3.98-3.88 (m, 1H), 3.47 (brt, J = 5.2 Hz, 2H), 3.03-2.89 (m, 2H), 2.82 (brt, J = 5.2 Hz, 2H), 2.37 (s, 3H)
Mass: 445 (M+ 1)⁺

### Example 27

### Synthesis of 6-(((1S,4S)-5-(3-chloro-2-fluorobenzoyl)-2,5-diazabicyclo[2.2.1]hept-2-yl)methyl)-N-(5-methyl-1H-pyrazol-3-yl)pyridin-2-amine

¹H-NMR (CD₃OD) δ: 7.85-7.81 (m, 1H), 7.67-7.60 (m, 1H), 7.45-7.38 (m, 1H), 7.33-7.25 (m, 1H), 7.15-7.08 (m, 1H), 7.04 (d, J = 8.8 Hz, 1H), 6.02 (s, 1H), 4.63-4.51 (m, 1H), 4.49-4.36 (m, 3H), 4.00-3.93 (m, 1H), 3.81-3.75 (m, 1H), 3.68-3.58 (m, 1H), 3.56-3.50 (m, 1H), 3.38-3.31 (m, 1H), 2.40 (s, 3H), 2.30-2.20 (m, 1H)
Mass: 441 (M + 1)⁺

### Example 28

### Synthesis of 6-((4-(3-bromo-2-fluorobenzoyl)piperazin-1-yl)methyl)-N-(5-methyl-1H-pyrazol-3-yl)pyridin-2-amine

¹H-NMR (CD₃OD) δ: 7.90 (d, J = 0.8 Hz, 1H), 7.75-7.69 (m, 1H), 7.53 (dd, J = 8.0,7.2 Hz, 1H), 7.40-7.33 (m, 1H), 7.20 (t, J = 8.0 Hz, 1H), 6.82 (d, J = 7.2 Hz, 1H), 3.90-3.78 (m, 2H), 3.67-3.55 (m, 2H), 3.44-3.34 (m, 2H), 2.64 (brt, J = 4.8 Hz, 2H), 2.53 (brt, J = 4.8 Hz, 2H), 2.23 (s, 3H)
Mass: 473 (M + 1)⁺

### Example 29

### Synthesis of 6-((4-(2-fluoro-3-(trifluoromethyl)benzoyl)piperazin-1-yl)methyl)-N-(5-methyl-1 H-pyrazol-3-yl)pyridin-2-amine

¹H-NMR (CD₃OD) δ: 7.90 (s, 1H), 7.81 (brt, J = 7.2 Hz, 1H), 7.68 (brt, J = 6.8 Hz, 1H), 7.53 (t, J = 8.0 Hz, 1H), 7.46 (t, J = 7.6 Hz, 1H), 6.82 (d, J = 7.2 Hz, 1H), 3.89-3.82 (m, 2H), 3.65-3.57 (m, 2H), 3.43-3.37 (m, 2H), 2.65 (brt, J = 4.8 Hz, 2H), 2.54 (brt, J = 4.8 Hz, 2H), 2.23 (s, 3H)
Mass: 463 (M + 1)⁺

### Example 30

### Synthesis of 6-((4-(2-chloro-3-(trifluoromethyl)benzoyl)piperazin-1-yl)methyl)-N-(5-methyl-1H-pyrazol-3-yl)pyridin-2-amine

¹H-NMR (CD₃OD) δ: 7.91-7.84 (m, 2H), 7.64-7.50 (m, 3H), 6.82 (brd, J = 7.2 Hz, 1H), 3.90-3.80 (m, 2H), 3.65-3.56 (m, 2H), 2.66 (brt, J = 4.8 Hz, 2H), 2.63-2.49 (m, 2H), 2.23 (s, 3H)
Mass: 479 (M + 1)⁺

### Example 31

### Synthesis of 6-((4-benzoylpiperazin-1-yl)methyl)-N-(5-cyclopropyl-1H-pyrazol-3-yl)pyridin-2-amine

¹H-NMR (CD₃OD) δ: 7.96 (dd, J = 8.8, 7.6 Hz, 1H), 7.53-7.43 (m, 5H), 7.09 (brd, J = 8.8 Hz, 1H), 7.02 (brd, J = 7.6 Hz, 1H), 5.76 (s, 1H), 4.04-3.60 (m, 4H), 3.99 (s, 2H), 2.95-2.65 (m, 4H), 2.02-1.93 (m, 1H), 1.10-1.05 (m, 2H), 0.82-0.77 (m, 2H)
Mass: 403 (M + 1)⁺

### Example 32

### Synthesis of 6-((4-(3-chloro-2-fluorobenzoyl)piperazin-1-yl)methyl)-N-1,2,4-thiadiazol-5-ylpyridin-2-amine

Mass: 433 (M + 1)⁺

### Example 33

Synthesis of 6-(((3R)-4-benzoyl-3-methylpiperazin-1-yl)methyl)-N-1,2,4-thiadiazol-5-ylpyridin-2-amine trifluoroacetate
¹H-NMR (DMSO-d₆) δ: 12.31 (brs, 1H), 8.34 (s, 1H), 7.99-7.82 (m, 1H), 7.50-7.10 (m, 7H), 4.60-2.80 (m, 9H), 1.31 (d, J = 7.0 Hz, 3H)
Mass: 395 (M + 1)⁺

### Example 34

Synthesis of phenyl (1-((6-(1,2,4-thiadiazol-5-ylamino)pyridin-2-yl)methyl)piperidin-4-yl)methanone trifluoroacetate
¹H-NMR (DMSO-d₆) δ: 12.39 (s, 1H), 9.94 (brs, 1H), 8.36 (s, 1H), 8.02-7.90 (m, 3H), 7.70-7.61 (m, 1H), 7.60-7.50 (m, 2H), 7.38-7.20 (m, 2H), 4.60-4.20 (m, 2H), 4.00-3.20 (m, 5H), 2.10-1.75 (m, 4H)
Mass: 380 (M + 1)⁺

### Example 35

### Synthesis of 6-((4-benzoyl-1,4-diazepan-1-yl)methyl)-N-1,2,4-thiadiazol-5-ylpyridin-2-amine Mass: 395 (M + 1)⁺

### Example 36

### Synthesis of 6-((4-(2,3-dichlorobenzoyl)piperazin-1-yl)methyl)-N-1,2,4-thiadiazol-5-ylpyridin-2-amine trifluoroacetate

¹H-NMR (DMSO-d₆) δ: 12.35 (brs, 1H), 8.35 (s, 1H), 7.99-7.90 (m, 1H), 7.75-7.70 (m, 1H), 7.55-7.40 (m, 2H), 7.38-7.20 (m, 2H), 4.60-3.10 (m, 10H)
Mass: 449 (M + 1 )⁺

### Example 37

### Synthesis of 6-((4-(3-(trifluoromethyl)benzoyl)piperazin-1-yl)methyl)-N-1,2,4-thiadiazol-5-ylpyridin-2-amine

Mass: 449 (M + 1)⁺

### Example 38

### Synthesis of 6-((4-(3-bromobenzoyl)piperazin-1-yl)methyl)-N-1,2,4-thiadiazol-5-ylpyridin-2-amine

Mass: 459, 461 (M + 1)⁺

### Example 39

### Synthesis of 6-((4-(2-(trifluoromethyl)benzoyl)piperazin-1-yl)methyl)-N-1,2,4-thiadiazol-5-ylpyridin-2-amine

Mass: 449 (M + 1)⁺

### Example 40

### Synthesis of 6-((4-(2-fluoro-3-(trifluoromethyl)benzoyl)piperazin-1-yl)methyl)-N-1,2,4-thiadiazol-5-ylpyridin-2-amine

¹H-NMR (DMSO-d₆) δ: 12.18 (brs, 1H), 8.30 (s, 1H), 7.83-7.75 (m, 3H), 7.55-7.45 (m, 1H), 7.13 (d, J = 7.3 Hz, 1H), 7.05 (d, J = 8.3 Hz, 1H), 3.78-3.65 (m, 4H), 3.32-3.22 (m, 2H), 2.62-2.52 (m, 2H), 2.51-2.43 (m, 2H)
Mass: 467 (M + 1)⁺

### Example 41

### Synthesis of 6-((4-(2-fluoro-3-(difluoromethyl)benzoyl)piperazin-1-yl)methyl)-N-1,2,4-thiadiazol-5-ylpyridin-2-amine trifluoroacetate

¹H-NMR (CD₃OD) δ: 8.27 (s, 1H), 7.94 (dd, J = 8.0,7.2 Hz, 1H), 7.76 (brt, J = 7.2 Hz, 1H), 7.64 (brt, J = 6.8 Hz, 1H), 7.44 (t, J = 7.6 Hz, 1H), 7.32 (d, J = 7.2 Hz, 1H), 7.24 (d, J = 8.0 Hz, 1H), 7.02 (t, J = 54.8 Hz, 1H), 4.58 (s, 2H), 3.73-3.46 (m, 6H)
Mass: 449 (M + 1)⁺

### Example 42

### Synthesis of 6-((4-(3-chloro-2-fluorobenzoyl)piperazin-1-yl)methyl)-N-(5-methyl-thiazol-2-yl)pyridin-2-amine trifluoroacetate

¹H-NMR (CD₃OD) δ: 7.98 (dd, J = 8.4,7.2 Hz, 1H), 7.62 (dt, J = 8.0,1.2 Hz, 1H), 7.44-7.37 (m, 2H), 7.32-7.25 (m, 3H), 4.58 (s, 2H), 4.20-3.92 (m, 2H), 3.69 (brs, 2H), 3.57 (brs, 2H), 3.45 (brs, 2H), 2.45 (d, J = 1.2 Hz, 3H)
Mass: 446 (M + 1)⁺

### Example 43

### Synthesis of 6-((4-(3-chloro-2-fluorobenzoyl)piperazin-1-yl)methyl)-N-(5-cyano-thiazol-2-yl)pyridin-2-yl)amine

¹H-NMR (CDCl₃) δ: 11.06 (brs, 1H), 8.00 (s, 1H), 7.72 (dd, J = 8.0,7.2 Hz, 1H), 7.47-7.42 (m, 1H), 7.33-7.25 (m, 1H), 7.20-7.12 (m, 2H), 6.82 (d, J = 8.0 Hz, 1H), 3.92-3.83 (m, 2H), 3.79 (s, 2H), 3.45-3.29 (m, 2H), 2.71-2.45 (m, 4H)
Mass: 457 (M + 1)⁺

### Example 44

### Synthesis of 6-((4-(2-fluoro-3-(trifluoromethyl)benzoyl)piperazin-1-yl)methyl)-N-1H-pyrazol-3-ylpyrazin-2-amine

### (1) Synthesis of 6-chloro-N-(1-((2-(trimethylsilyl)ethoxy)methyl)-1H-pyrazol-3-yl)pyrazin-2-amine

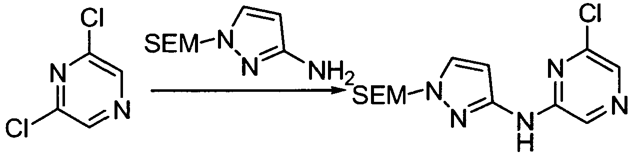

A mixture of 1.78 g of 2,6-dichloropyrazine, 2.84 g of 1-((2-(trimethylsilyl)ethoxy)methyl)-1H-pyrazol-3-amine obtained in Reference Example 2, 690 mg of 9,9-dimethyl-4,5-bis(diphenylphosphino)xanthene, 620 mg of tris(dibenzylideneacetone)dipalladium(0)-chloroform complex, 5.07 g of potassium phosphate, 25ml of 1,4-dioxane was stirred at 100°C for 2 hours, cooled to room temperature, and then diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (2) Synthesis of methyl 6-((1-((2-(trimethylsilyl)ethoxy)methyl)-1H-pyrazol-3-yl)amino)pyrazine-2-carboxylate

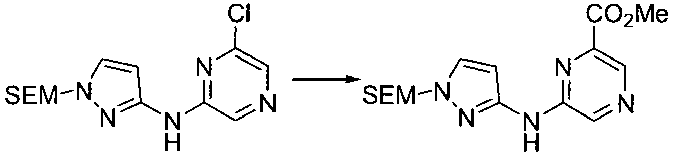

A mixture of 2.41 g of 6-chloro-N-(1-((2-(trimethylsilyl)ethoxy)methyl)-1H-pyrazol-3-yl)pyrazin-2-amine, 320 mg of palladium acetate, 790 mg of 1,1'-bisdiphenylphosphinoferrocene, 890 mg of sodium hydrogen carbonate, 10 ml of methanol and 10ml of N,N-dimethylformamide was stirred at 100°C for 15 hours under 3 atmospheric pressure of carbon monoxide, cooled to room temperature, and then diluted with ethyl acetate. An insoluble matter was filtered off using Celite and the resulting ethyl acetate solution was washed with water and brine. The organic layer was dried over anhydrous magnesium sulfate and filtered, and the filtrate was concentrated. The residue was purified by a silica gel column chromatography (eluent: hexane/ethyl acetate = 10/1 to 1/1) to give the title compound.

### (3) Synthesis of 6-((1-((2-(trimethylsilyl)ethoxy)methyl)-1H-pyrazol-3-yl)amino)pyrazine-2-carboxylic acid

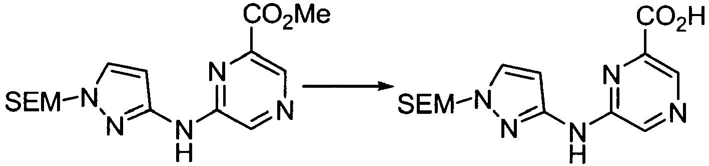

To a mixture of 52 mg of methyl 6-((1-((2-(trimethylsilyl)ethoxy)methyl)-1H-pyrazol-3-yl)amino)pyrazine-2-carboxylate, 0.5 ml of tetrahydrofuran and 1 ml of methanol was added an aqueous sodium hydroxide solution (1.0 M, 0.5 ml), followed by stirring at room temperature for 15 hours. The obtained reaction solution was diluted with ethyl acetate, and then washed with aqueous ammonium chloride and brine. The organic layer was dried over anhydrous magnesium sulfate and filtered, and filtrate was concentrated to give the title compound.

### (4) Synthesis of (6-((1-((2-(trimethylsilyl)ethoxy)methyl)-1H-pyrazol-3-yl)amino)pyrazin-2-yl)methanol

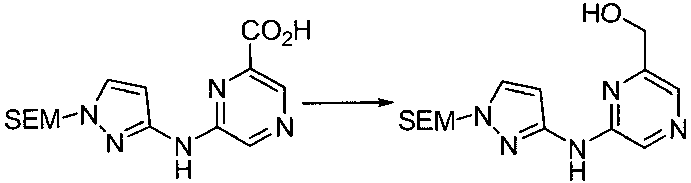

To a mixture of 28 mg of 6-((1-((2-(trimethylsilyl)ethoxy)methyl)-1H-pyrazol-3-yl)amino)pyrazine-2-carboxylic acid and 1 ml of N,N-dimethylformamide was added 84 mg of N,N'-carbonyldiimidazole, followed by stirring at room temperature for 15 hours. Then, 200 µl of an aqueous solution of 20 mg of sodium borohydride was added thereto and the resulting mixture was stirred. Water was added to the reaction mixture, and then the mixture was extracted with ethyl acetate. The resulting ethyl acetate solution was dried over anhydrous magnesium sulfate and filtered, and the filtrate was concentrated. The residue was purified by a silica gel column chromatography (eluent: chloroform to chloroform/methanol = 10/1) to give the title compound.

### (5) Synthesis of 6-((4-(2-fluoro-3-(trifluoromethyl)benzoyl)piperazin-1-yl)methyl)-N-(1-((2-(trimethylsilyl)ethoxy)methyl)-1H-pyrazol-3-yl)pyrazin-2-amine

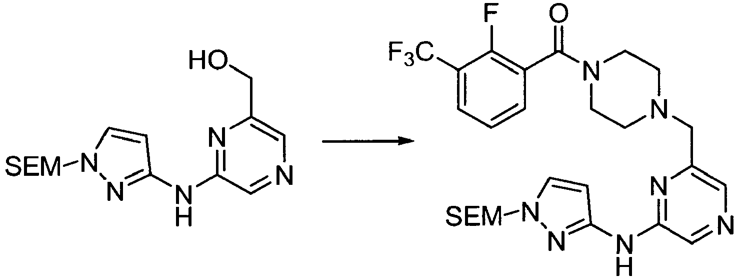

To a mixture of 2.14 g of (6-((1-((2-(trimethylsilyl)ethoxy)methyl)-1H-pyrazol-3-yl)amino)pyrazin-2-yl)methanol, 2.32 ml of N,N-diisopropylethylamine and 40 ml of chloroform was added 619 µl of methanesulfonyl chloride at room temperature followed by stirring for 1 hour. To the reaction mixture was added 2.32 ml ofN,N-diisopropylethylamine, and then 3.13 g of 1-(2-fluoro-3-(trifluoromethyl)benzoyl)piperazine hydrochloride obtained in Reference Example 7 was added thereto followed by stirring at 50°C for 2 hours. The resulting reaction mixture was washed with aqueous sodium bicarbonate and brine. The resulting organic layer was dried over anhydrous magnesium sulfate and filtered, and then the filtrate was concentrated *in vacuo.* The resulting residue was purified by a silica gel column chromatography (eluent: chloroform to chloroform/methanol = 20/1) to obtain the title compound.

### (6) Synthesis of 6-((4-(2-fluoro-3-(trifluoromethyl)benzoyl)piperazin-1-yl)methyl)-N-1H-pyrazol-3-ylpyrazin-2-amine

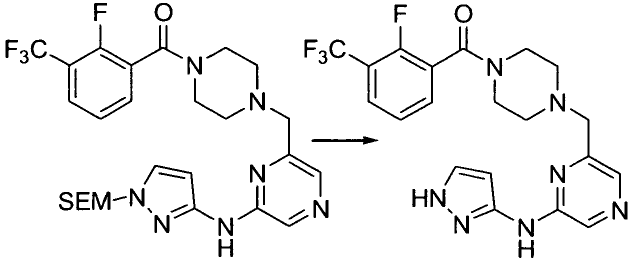

2.49 g of 6-((4-(2-fluoro-3-(trifluoromethyl)benzoyl)piperazin-1-yl)methyl)-N-(1-((2-(trimethylsilyl)ethoxy)methyl)-1H-pyrazol-3-yl)pyrazin-2-amine was dissolved in 25 ml of trifluoroacetic acid and 2.5 ml of water followed by stirring at room temperature for 2 hours. The reaction solution was concentrated *in vacuo,* diluted with ethyl acetate, and then washed with saturated sodium bicarbonate, water and brine. The resulting organic layer was dried over magnesium sulfate and filtered, and the filtrate was concentrated. The resulting residue was purified by a silica gel column chromatography (eluent: chloroform to chloroform/methanol = 5/1) to give the title compound as a solid. The resulting solid was dissolved in ethanol by heating to 80°C. The solution was charged with heptane, and heating was stopped. Then the solution was cooled slowly to room temperature. After heptane was further added, the precipitate was filtered and dried to give a crystal of the title compound.
Spectral data of the title compound are as follows.
¹H-NMR (CDCl₃) δ: 8.51 (s, 1H), 8.09 (s, 1H), 7.70-7.56 (m, 2H), 7.52 (s, 1H), 7.37-7.20 (m, 2H), 6.26 (s, 1H), 3.89 (brs, 2H), 3.64 (s, 2H), 3.37 (brs, 2H), 2.66 (dd, J = 5.1,4.9 Hz, 2H), 2.54 (brs, 2H)
Mass: 450 (M + 1)⁺
m.p.:160-163°C

Examples 45 to 54, 56 to 60 and 113 to 115 were synthesized in the same manner as in Example 44 as follows.

### Example 45

### Synthesis of 6-((4-(2,3-dichlorobenzoyl)piperazin-1-yl)methyl)-N-1H-pyrazol-3-ylpyrazin-2-amine

¹H-NMR (CDCl₃) δ: 8.45 (s, 1H), 8.06 (s, 1H), 7.77 (s, 1H), 7.51-7.47 (m, 2H), 7.28-7.17 (m, 2H), 6.26 (s, 1H), 3.96-3.78 (m, 4H), 3.63 (s, 2H), 2.72-2.43 (m, 4H)
Mass: 432 (M + 1)⁺

### Example 46

Synthesis of 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine
¹H-NMR (CDCI₃) δ: 8.44 (s, 1H), 8.11-8.03 (m, 1H), 7.87-7.65 (m, 1H), 7.57-7.42 (m, 2H), 7.40-7.30 (m, 1H), 7.22-7.13 (m, 1H), 6.34-6.22 (m, 1H), 4.97-2.70 (m, 8H), 2.10-1.80 (m, 2H)
Mass: 428 (M + 1)⁺

### Example 47

### Synthesis of 6-(((1S,4S)-5-(2-fluoro-3-(trifluoromethyl)benzoyl)-2,5-diazabicyclo[2.2.1]hept-2-yl)methyl)-N-1H-pyrazol-3-ylpyrazin-2-amine

¹H-NMR (CDCl₃) δ: 8.46 (s, 1H), 8.08 and 8.05 (each s, total 1H), 8.02-7.80 (m, 1H), 7.72-7.61 (m, 2H), 7.51 and 7.48 (each d, J = 2.3 Hz, total 1H), 7.33 (t, J = 7.8 Hz, 1H), 6.30 and 6.26 (each s, total 1H), 4.95-2.72 (m, 8H), 2.07 and 2.02 (each d, J = 10.0 Hz, total 1H), 1.85 (d, J = 10.0 Hz, 1H)
Mass: 462 (M + 1)⁺

### Example 48

### Synthesis of 6-(((1R,4R)-5-(2-fluoro-3-(trifluoromethyl)benzoyl)-2,5-diazabicyclo[2.2.1]hept-2-yl)methyl)-N-1H-pyrazol-3-ylpyrazin-2-amine

¹H-NMR (CDCl₃) δ: 8.46 (s, 1H), 8.08 and 8.05 (each s, total 1H), 8.02-7.80 (m, 1H), 7.72-7.61 (m, 2H), 7.51 and 7.48 (each d, J = 2.3 Hz, total 1H), 7.33 (t, J = 7.8 Hz, 1H), 6.30 and 6.26 (each s, total 1H), 4.95-2.72 (m, 8H), 2.07 and 2.02 (each d, J = 10.0 Hz, total 1H), 1.85 (d, J = 10.0 Hz, 1H)
Mass: 462 (M + 1)⁺

### Example 49

### Synthesis of 6-((4-(3-bromo-2-fluorobenzoyl)piperazin-1-yl)methyl)-N-1H-pyrazol-3-ylpyrazin-2-amine trifluoroacetate

¹H-NMR (DMSO-d₆) δ: 10.05 (s, 1H), 8.45 (s, 1H), 8.01 (s, 1H), 7.85-7.80 (m, 1H), 7.64 (s, 1H), 7.47 (dd, J = 6.3, 6.0 Hz, 1H), 7.27 (dd, J = 8.0, 7.6 Hz, 1H), 6.52 (s, 1H), 4.40 (s, 2H), 3.58-3.24 (m, 4H), 2.55-2.48 (m, 4H)
Mass: 460, 462 (M + 1)⁺

### Example 50

### Synthesis of 6-((4-(3-chloro-2-fluorobenzoyl)piperazin-1-yl)methyl)-N-1H-pyrazo-3-ylpyrazin-2-amine

¹H-NMR (CDCl₃) δ: 8.51 (s, 1H), 8.09 (s, 1H), 7.52 (s, 1H), 7.45 (dd, J = 7.0, 6.3 Hz, 1H), 7.37-7.24 (m, 2H), 7.15 (t, J = 7.8 Hz, 1H), 6.26 (s, 1H), 3.86 (brs, 2H), 3.64 (s, 2H), 3.38 (brs, 2H), 2.65 (dd, J = 5.3, 4.7 Hz, 2H), 2.53 (brs, 2H)
Mass: 415 (M + 1)⁺

### Example 51

### Synthesis of 6-((4-(2-chloro-3-(trifluoromethyl)benzoyl)piperazin-1-yl)methyl)-N-1H-pyrazol-3-ylpyrazin-2-amine trifluoroacetate

¹H-NMR (DMSO-d₆) δ: 10.05 (s, 1H), 8.45 (s, 1H), 8.01 (s, 1H), 7.98-7.93 (m, 1H), 7.80-7.74 (m, 1H), 7.70-7.62 (m, 2H), 6.52 (s, 1H), 4.41 (s, 2H), 3.60-3.20 (m, 4H), 2.55-2.40 (m, 4H)
Mass: 466 (M + 1)⁺

### Example 52

### Synthesis of 6-(((3R)-4-(2-fluoro-3-(trifluoromethyl)benzoyl)-3-methylpiperazin-1-yl)methyl)-N-1H-pyrazol-3-ylpyrazin-2-amine

¹H-NMR (CDCl₃) δ: 8.42 (s, 1H), 8.12 (s, 1H), 7.79 (s, 1H), 7.66 (t, J = 7.0 Hz, 1H), 7.63-7.51 (m, 1H), 7.50 (d, J = 2.4 Hz, 1H), 7.31 (t, J = 7.8 Hz, 1H), 6.31 (s, 1H), 5.00-2.20 (m, 9H), 1.50-1.25 (m, 3H)
Mass: 464 (M + 1)⁺

### Example 53

### Synthesis of 6-((4-(3-cyclopropyl-2-fluorobenzoyl)piperazin-1-yl)methyl)-N-1H-pyrazol-3-ylpyrazin-2-amine

¹H-NMR (CDCl₃) δ: 8.50 (s, 1H), 8.10 (s, 1H), 7.51 (d, J = 2.0Hz), 7.30-7.05 (m, 4H), 6.94-6.90 (m, 1H), 6.24 (s, 1H), 4.00-3.80 (m, 2H), 3.64 (s, 2H), 3.50-3.30 (m, 2H), 2.65 (t, J = 5.1 Hz, 2H), 2.60-2.40 (m, 2H), 2.11-2.07 (m, 1H), 1.00 (dd, J = 8.5, 1.7 Hz, 2H), 0.72 (d, J = 5.9 Hz, 2H)
Mass: 422 (M + 1)⁺

### Example 54

### Synthesis of 6-((4-(2-fluoro-3-(trifluoromethyl)benzoyl)-1,4-diazepan-1-yl)methyl)-N-1H-pyrazol-3-ylpyrazin-2-amine

¹H-NMR (CDCl₃) δ: 8.47-8.42 (m, 1H), 8.12-8.02 (m, 1H), 7.92-7.79 (m, 1H), 7.68-7.50 (m, 3H), 7.35-7.25 (m, 1H), 6.27 (s, 1H), 3.93-3.71 (m, 4H), 3.48-3.36 (m, 2H), 2.93 (t, J = 4.8 Hz, 1H), 2.83 (dd, J = 5.6, 5.2 Hz, 1H), 2.78-2.68 (m, 2H), 2.07-1.98 (m, 1H), 1.91-1.80 (m, 1H)
Mass: 464 (M + 1)⁺

### Example 55

### Synthesis of 6-((4-(2-fluoro-3-(trifluoromethyl)benzoyl)-6-hydroxy-1,4-diazepan-1-yl)methyl)-N- 1H-pyrazol-3-ylpyrazin-2-amine

### (1) Synthesis of 6-((6-(tetrahydro-2H-pyran-2-yloxy)-1,4-diazepan-1-yl)methyl)-N-(1-((2-(trimethylsilyl)ethoxy)methyl)-1H-pyrazol-3-yl)pyrazin-2-amine

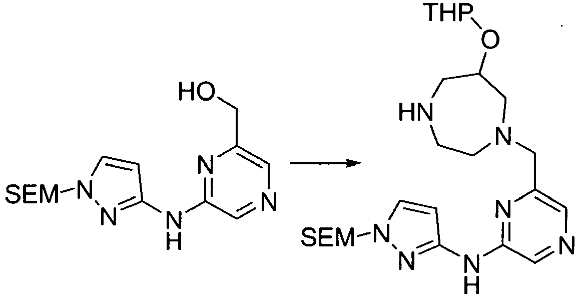

To a mixture of 44.9 mg of (6-((1-((2-(trimethylsilyl)ethoxy)methyl)-1H-pyrazol-3-yl)amino)pyrazin-2-yl)methanol, 73 µl of N,N-diisopropylethylamine and 4 ml of chloroform was added 16 µl of methanesulfonyl chloride at room temperature followed by stirring for 1 hour. The reaction mixture was dropped into a solution of 6-(tetrahydro-2H-pyran-2-yloxy)-1,4-diazepane obtained in Reference Example 5 in 2 ml of chloroform, and the reaction mixture was stirred at room temperature for 15 hours. The resulting reaction mixture was washed with water and brine. The resulting organic layer was dried over anhydrous magnesium sulfate and filtered, and the filtrate was concentrated *in vacuo.* The resulting residue was purified by a basic thin-layer chromatography (eluent: chloroform/methanol = 30/1) to give the title compound.

### (2) Synthesis of 6-((4-(2-fluoro-3-(trifluoromethyl)benzoyl)-6-hydroxy-1,4-diazepan-1-yl)methyl)-N-1 H-pyrazol-3-ylpyrazin-2-amine

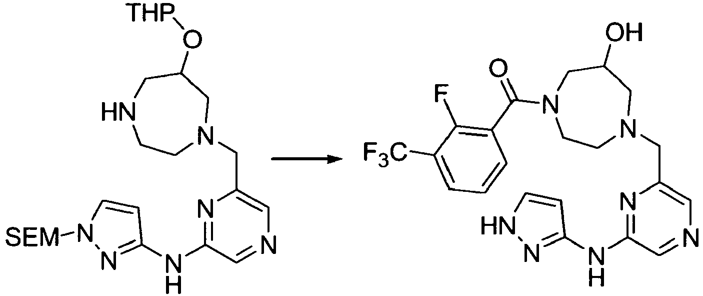

The amidation reaction was performed in the same manner as in Example 3-(2) using 6-((6-(tetrahydro-2H-pyran-2-yloxy)-1,4-diazepan-1-yl)methyl)-N-(1-((2-(trimethylsilyl)ethoxy)methyl)-1H-pyrazol-3-yl)pyrazin-2-amine and 2-fluoro-3-(trifluoromethyl) benzoic acid. Next, the deprotection reaction was performed in the same manner as in Example 16-(4) using trifluoroacetic acid to give the title compound.
Spectral data of the title compound are as follows.
¹H-NMR (CDCl₃) δ: 8.48-8.39 (m, 1H), 7.93 (s, 1H), 7.70-7.43 (m, 3H), 7.22-7.10 (m, 1H), 6.37-6.27 (m, 1H), 4.17-2.67 (m, 11H)
Mass: 480 (M + 1)⁺

### Example 56

### Synthesis of 6-((4-(3-chloro-2-fluorobenzoyl)piperazin-1-yl)methyl)-N-(5-methyl-1H-pyrazol-3-yl)pyrazin-2-amine

¹H-NMR (CDCl₃) δ: 8.48 (s, 1H), 8.06 (s, 1H), 7.48-7.33 (m, 2H), 7.33-7.25 (m, 1H), 7.15 (dd, J = 8.0,7.6 Hz, 1H), 6.02 (s, 1H), 3.87 (brs, 2H), 3.62 (s, 2H), 3.37 (brs, 2H), 2.64 (t, J = 5.1 Hz, 2H), 2.52 (brs, 2H), 2.31 (s, 3H)
Mass: 430 (M + 1)⁺

### Example 57

### Synthesis of 6-((4-(2-fluoro-(3-trifluoromethyl)benzoyl)piperazin-1-yl)methyl)-N-1,2,4-thiadiazol-5-ylpyrazin-2-amine

¹H-NMR (CDCl₃) δ: 12.16 (brs, 1H), 8.49 (s, 1H), 8.44-8.39 (m, 2H), 7.72-7.59 (m, 2H), 7.33 (dd, J = 8.0,7.6 Hz, 1H), 3.96-3.80 (m, 4H), 3.40 (brs, 2H), 2.78-2.51 (m, 4H)
Mass: 468 (M + 1)⁺

### Example 58

### Synthesis of 6-((4-(3-chloro-2-fluorobenzoyl)piperazin-1-yl)methyl)-N-thiazol-2-ylpyrazin-2-amine

¹H-NMR (CDCl₃) δ: 8.33 (s, 1H), 8.24 (s, 1H), 7.56 (d, J = 3.5 Hz, 1H), 7.49-7.42 (m, 1H), 7.30-7.23 (m, 2H), 7.14 (t, J = 8.1 Hz, 1H), 6.95 (d, J = 3.7 Hz, 1H), 3.87 (brs, 2H), 3.79 (s, 2H), 3.34 (brs, 2H), 2.72 (t, J = 5.2 Hz, 2H), 2.59 (brs, 2H)
Mass: 433 (M+ 1)⁺

### Example 59

### Synthesis of 2-((4-(3-chloro-2-fluorobenzoyl)piperazin-1-yl)methyl)-N-1H-pyrazo1-3-ylpyrimidin-4-amine

¹H-NMR (CDCl₃) δ: 8.35 (d, J = 5.9 Hz, 1H), 7.53 (d, J = 2.4 Hz, 2H), 7.46-7.42 (m, 1H), 7.28-7.26 (m, 1H), 7.15 (t, J = 7.8 Hz, 1H), 7.10-7.00 (m, 1H), 6.23 (s, 1H), 4.00-3.80 (m, 2H), 3.73 (s, 2H), 3.60-3.30 (m, 2H), 2.71 (t, J = 5.1 Hz, 2H), 2.71-2.51 (m, 2H)
Mass: 416 (M + 1)⁺

### Example 60

### Synthesis of 2-((4-(2-fluoro-3-(trifluoromethyl)benzoyl)piperazin-1-yl)methyl)-N-1H-pyrazol-3-ylpyrimidin-4-amine

¹H-NMR (CDCl₃) δ: 8.35 (d, J = 5.9 Hz, 1H), 7.66 (t, J = 7.1 Hz, 1H), 7.64-7.50 (m, 3H), 7.53 (d, J = 2.0 Hz, 1H), 7.32 (t, J = 7.8 Hz, 1H), 7.05 (s, 1H), 6.24 (s, 1H), 4.00-3.80 (m, 2H), 3.73 (s, 2H), 3.50-3.35 (m, 2H), 2.72 (t, J = 5.1 Hz, 2H), 2.70-2.55 (m, 2H)
Mass: 450 (M+ 1)⁺

### Example 61

### Synthesis of 6-((4-(3-furoyl)piperazin-1-yl)methyl)-N-(5-chlorothiazol-2-yl)pyridin-2-amine

### (1) Synthesis of 6-(((tert-butyl(dimethyl)silyl)oxy)methyl)-N-(5-chlorothiazol-2-yl)pyridin-2-amine

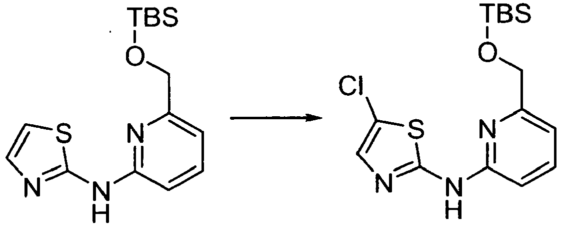

2 g of 6-(((tert-butyl(dimethyl)silyl)oxy)methyl)-N-thiazol-2-ylpyridin-2-amine obtained in Example 5-(2) was dissolved in 20 ml of 1,4-dioxane, and then 832 mg of N-chlorosuccinimide was added thereto at room temperature. The reaction mixture was heated under reflux for 2 hours, cooled to room temperature, diluted with ethyl acetate, and then washed with water and brine. The resulting organic layer was dried over anhydrous magnesium sulfate and filtered, and the filtrate was concentrated *in vacuo* to give the title compound.

### (2) Synthesis of 6-(chloromethyl)-N-(5-chlorothiazol-2-yl)pyridin-2-amine

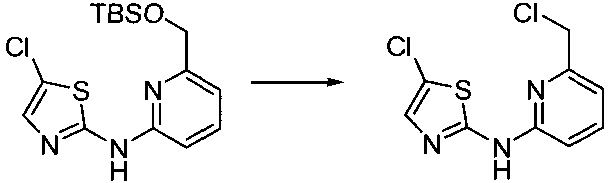

In the same manner as in Example 5-(3) and (4), the title compound was obtained using 6-(((tert-butyl(dimethyl)silyl)oxy)methyl)-N-(5-chlorothiazol-2-yl)pyridin-2-amine.

### (3) Synthesis of 6-((4-(3-furoyl)piperazin-1-yl)methyl)-N-(5-chlorothiazol-2-yl)pyridin-2-amine

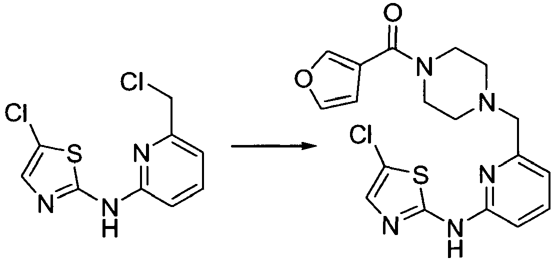

In the same manner as in Example 5-(5), the title compound was obtained using 6-(chloromethyl)-N-(5-chlorothiazol-2-yl)pyridin-2-amine and 1-(3-furoyl)piperazine synthesized in reference with the disclosed method in the process (7) to (8), similar to Reference Example 1.
Spectral data of the title compound are as follows.
¹H-NMR (DMSO-d₆) δ: 11.47 (s, 1H), 8.01 (s, 1H), 7.72-7.68 (m, 2H), 7.37 (s, 1H), 7.02 (d, J = 7.4 Hz, 1H), 6.89 (d, J = 8.2 Hz, 1H), 6.64-6.63 (m, 1H), 3.66 (s, 2H), 3.60-3.58 (m, 4H), 2.50-2.48 (m, 4H)
Mass: 404 (M + 1)⁺

Examples 62 and 64 to 77 were synthesized in the same manner as in Example 61 as follows.

### Example 62

### Synthesis of 6-((4-(2-furoyl)piperazin-1-yl)methyl-N-(5-chlorothiazol-2-yl)pyridin-2-amine

¹H-NMR (DMSO-d₆) δ: 11.48 (s, 1H), 7.82-7.80 (m, 1H), 7.71 (t, J = 7.8 Hz, 1H), 7.37 (s, 1H), 7.03 (d, J = 7.0 Hz, 1H), 6.97 (dd, J = 3.5, 0.8 Hz, 1H), 6.90 (d, J = 8.2 Hz, 1H), 6.61-6.59 (m, 1H), 3.73-3.64 (m, 6H), 2.53-2.47 (m, 4H)
Mass: 404 (M + 1)⁺

### Example 63

### Synthesis of 6-((4-(3-chloro-2-fluorobenzoyl)-1,4-diazepan-1-yl)methyl)-N-thiazol-2-ylpyridin-2-amine

¹H-NMR (CDCl₃) δ: 7.64-7.55 (m, 1H), 7.55-7.37 (m, 2H), 7.33-7.22 (m, 1H), 7.18-6.98 (m, 2H), 6.86-6.81 (m, 1H), 6.78 (dd, J = 9.2, 8.6 Hz, 1H), 3.92-3.78 (m, 4H), 3.49-3.36 (m, 2H), 2.96 (dd, J = 5.7, 4.1 Hz, 1H), 2.85 (t, J = 5.5 Hz, 1H), 2.81-2.70 (m, 2H), 2.07-1.78 (m, 2H)
Mass: 446 (M + 1)⁺

### Example 64

### Synthesis of 6-((4-(pyrazin-2-ylcarbonyl)piperazin-1-yl)methyl)-N-(5-chlorothiazol-2-yl)pyridin-2-amine

¹H-NMR (DMSO-d₆) δ: 11.48 (s, 1H), 8.83 (d, J = 1.2 Hz, 1H), 8.73 (d, J = 2.3 Hz, 1H), 8.65 (dd, J = 2.7, 1.6 Hz, 1H), 7.69 (t, J = 7.4 Hz, 1H), 7.38 (s, 1H), 7.02 (d, J = 7.4 Hz, 1H), 6.90 (d, J = 7.4 Hz, 1H), 3.74-3.64 (m, 4H), 3.48-3.40 (m, 2H), 2.62-2.53 (m, 2H), 2.51-2.44 (m, 2H)
Mass: 416 (M + 1)⁺

### Example 65

### Synthesis of 6-((4-(3-thienylcarbonyl)piperazin-1-yl)methyl)-N-(5-chlorothiazol-2-yl)pyridin-2-amine

¹H-NMR (DMSO-d₆) δ: 11.48 (s, 1H), 7.76 (d, J = 1.6 Hz, 1H), 7.70 (t, J = 7.6 Hz, 1H), 7.59 (dd, J = 4.7,2.7 Hz, 1H), 7.37 (s, 1H), 7.18 (dd, J = 5.1,1.2 Hz, 1H), 7.02 (d, J = 7.4 Hz, 1H), 6.90 (d, J = 8.2 Hz, 1H), 3.74 (s, 2H), 3.65-3.45 (m, 2H), 3.39-3.37 (m, 2H), 2.54-2.41 (m, 4H)
Mass: 420 (M + 1)⁺

### Example 66

### Synthesis of 6-((4-(2-thienylcarbonyl)piperazin-1-yl)methyl)-N-(5-chlorothiazol-2-yl)pyridin-2-amine

¹H-NMR (DMSO-d₆) δ: 11.48 (s, 1H), 7.76-7.67 (m, 2H), 7.15-7.05 (m, 2H), 7.10 (dd, J = 5.1, 3.9 Hz, 1H), 7.03 (d, J = 6.6 Hz, 1H), 6.90 (d, J = 8.6 Hz, 1H), 3.76-3.60 (m, 4H), 3.39-3.24 (m, 2H), 2.58-2.42 (m, 4H)
Mass: 420 (M + 1)⁺

### Example 67

### Synthesis of 6-((4-(thiazol-2-ylcarbonyl)piperazin-1-yl)methyl)-N-(5-chlorothiazol-2-yl)pyridin-2-amine

¹H-NMR (DMSO-d₆) δ: 11.48 (s, 1H), 8.03-7.97 (m, 2H), 7.71 (t, J = 7.8 Hz, 1H), 7.37 (s, 1H), 7.04 (d, J = 7.0 Hz, 1H), 6.90 (d, J = 8.2 Hz, 1H), 4.31 (brs, 2H), 3.68 (brs, 4H), 2.64-2.51 (m, 4H)
Mass: 421 (M + 1)⁺

### Example 68

### Synthesis of 6-((4-((2-methyl-thiazol-4-yl)carbonyl)piperazin-1-yl)methyl)-N-(5-chlorothiazol-2-yl)pyridin-2-amine

¹H-NMR (DMSO-d₆) δ: 11.48 (s, 1H), 7.90 (brs, 1H), 7.75-7.66 (m, 1H), 7.38 (s, 1H), 7.02 (d, J = 6.2 Hz, 1H), 6.89 (d, J = 8.6 Hz, 1H), 3.66 (brs, 4H), 3.36-3.25 (m, 2H), 2.66 (s, 3H), 2.51-2.45 (m, 4H)
Mass: 435 (M + 1)⁺

### Example 69

### Synthesis of 6-((4-((1-methyl-1H-pyrazol-3-yl)carbonyl)piperazin-1-yl)methyl)-N-(5-chlorothiazol-2-yl)pyridin-2-amine

¹H-NMR (DMSO-d₆) δ: 11.50 (brs, 1H), 7.75-7.69 (m, 2H), 7.38 (s, 1H), 7.04 (brs, 1H), 6.91 (brs, 1H), 6.52 (s, 1H), 4.07-3.80 (m, 2H), 3.86 (s, 3H), 3.79-3.56 (m, 2H), 3.38-3.21 (m, 2H), 2.51-2.38 (m, 4H)
Mass: 418 (M + 1)⁺

### Example 70

### Synthesis of 6-((4-(2-cyanobenzoyl)piperazin-1-yl)methyl)-N-(5-chlorothiazol-2-yl)pyridin-2-amine

¹H-NMR (DMSO-d₆) δ: 11.47 (s, 1H), 7.93 (dd, J = 7.8, 0.8 Hz, 1H), 7.77 (dt, J = 1.2, 7.6 Hz, 1H), 7.70 (dd, J = 8.2, 7.4 Hz, 1H), 7.62 (dt, J = 1.2, 7.6 Hz, 1H), 7.56 (dd, J = 7.8, 0.8 Hz, 1H), 7.37 (s, 1H), 7.02 (d, J = 7.0 Hz, 1H), 6.89 (d, J = 8.2 Hz, 1H), 3.74-3.64 (m, 4H), 3.26-3.20 (m, 2H), 2.60-2.53 (m, 2H), 2.51-2.43 (m, 2H)
Mass: 439 (M + 1)⁺

### Example 71

### Synthesis of 6-((4-(3-cyanobenzoyl)piperazin-1-yl)methyl)-N-(5-chlorothiazol-2-yl)pyridin-2-amine

¹H-NMR (DMSO-d₆) δ: 11.47 (s, 1H), 7.93-7.86 (m, 2H), 7.74-7.61 (m, 3H), 7.37 (s, 1H), 7.01 (d, J = 7.4 Hz, 1H), 6.89 (d, J = 8.2 Hz, 1H), 3.66 (s, 4H), 3.41-3.25 (m, 2H), 2.60-2.38 (m, 4H)
Mass: 439 (M + 1)⁺

### Example 72

### Synthesis of 6-((4-(3-(acetylamino)benzoyl)piperazin-1-yl)methyl)-N-(5-chlorothiazol-2-yl)pyridin-2-amine

¹H-NMR (DMSO-d₆) δ: 11.47 (s, 1H), 10.05 (s, 1H), 7.70 (dd, J = 8.2, 7.4 Hz, 1H), 7.65 (t, J = 1.6 Hz, 1H), 7.59-7.54 (m, 1H), 7.37 (s, 1H), 7.33 (t, J = 8.0 Hz, 1H), 7.01 (d, J = 7.4 Hz, 2H), 6.88 (d, J = 8.2 Hz, 1H), 3.77-3.56 (m, 4H), 3.48-3.22 (m, 2H), 2.59-2.37 (m, 4H), 2.03 (s, 3H)
Mass: 471 (M + 1)⁺

### Example 73

### Synthesis of 6-((4-(3-(dimethylamino)benzoyl)piperazin-1-yl)methyl)-N-(5-chlorothiazol-2-yl)pyridin-2-amine

¹H-NMR (DMSO-d₆) δ: 11.47 (s, 1H), 7.69 (t, J = 7.8 Hz, 1H), 7.37 (s, 1H), 7.20 (t, J = 8.0 Hz, 1H), 7.01 (d, J = 7.4 Hz, 1H), 6.88 (t, J = 8.4 Hz, 1H), 6.75 (dd, J = 8.6, 2.7 Hz, 1H), 6.63-6.56 (m, 2H), 3.73-3.53 (m, 4H), 3.48-3.20 (m, 2H), 2.87 (s, 6H), 2.53-2.46 (m, 4H)
Mass: 422 (M + 1)⁺

### Example 74

### Synthesis of 6-((4-(3-(methanesulfonyl)benzoyl)piperazin-1-yl)methyl)-N-(5-chlorothiazol-2-yl)pyridin-2-amine

¹H-NMR (DMSO-d₆) δ: 11.47 (s, 1H), 7.99 (dt, J = 7.4, 1.8 Hz, 1H), 7.91 (t, J = 1.4 Hz, 1H), 7.76-7.68 (m, 3H), 7.37 (s, 1H), 7.02 (d, J = 7.4 Hz, 1H), 6.89 (d, J = 8.2 Hz, 1H), 3.67 (s, 4H), 3.48-3.29 (m, 2H), 3.26 (s, 3H), 2.62-2.40 (m, 4H)
Mass: 492 (M + 1)⁺

### Example 75

### Synthesis of 6-((4-(3-chloro-2-fluorobenzoyl)piperazin-1-yl)methyl)-N-(5-chlorothiazol-2-yl)pyridin-2-amine

¹H-NMR (DMSO-d₆) δ: 11.46 (s, 1H), 7.73-7.62 (m, 2H), 7.41-7.33 (m, 2H), 7.29 (t, J = 8.0 Hz, 1H), 7.01 (d, J = 7.4 Hz, 1H), 6.89 (d, J = 8.2 Hz, 1H), 3.73-3.62 (m, 4H), 3.36-3.20 (m, 2H), 2.59-2.39 (m, 4H)
Mass: 466 (M + 1)⁺

### Example 76

### Synthesis of 6-((4-(2,3-dichlorobenzoyl)piperazin-1-yl)methyl)-N-(5-chlorothiazol-2-yl)pyridin-2-amine

¹H-NMR (DMSO-d₆) δ: 11.46 (s, 1H), 7.72-7.64 (m, 2H), 7.43 (t, J = 7.8 Hz, 1H), 7.37-7.34 (m, 2H), 7.01 (d, J = 7.4 Hz, 1H), 6.89 (d, J = 8.2 Hz, 1H), 3.76-3.59 (m, 4H), 3.21-3.11 (m, 2H), 2.61-2.37 (m, 4H)
Mass: 482 (M + 1)⁺

### Example 77

### Synthesis of 6-((4-((4-chlorophenyl)acetyl)plperazin-1-yl)methyl)-N-(5-chlorothiazol-2-yl)pyridin-2-amine trifluoroacetate

¹H-NMR (CD₃OD) δ: 7.81 (dd, J = 8.4, 7.6 Hz, 1H), 7.29 (d, J = 8.4 Hz, 2H), 7.28 (s, 1H), 7.21 (d, J = 8.4 Hz, 2H), 7.17 (d, J = 7.6 Hz, 1H), 7.10 (d, J = 8.4 Hz, 1H), 4.46 (s, 2H), 4.10-3.70 (m, 4H), 3.80 (s, 2H), 3.52-3.34 (m, 4H)
Mass: 462 (M + 1)⁺

### Example 78

### Synthesis of 6-((4-((2-fluoropyridin-3-yl)carbonyl)piperazin-1-yl)methyl)-N-(5-bromothiazol-2-yl)pyridin-2-amine

### (1) Synthesis of 6-(((tert-butyl(dimethyl)silyl)oxy)methyl)-N-(5-bromothiazol-2-yl)pyridin-2-amine

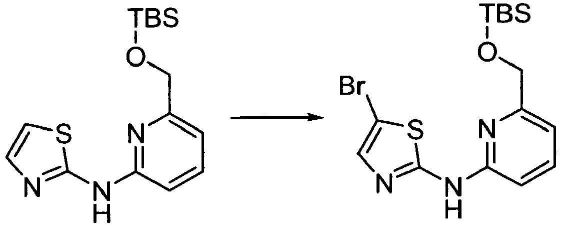

In the same manner as in Example 61-(1), the title compound was obtained using 6-(((tert-butyl(dimethyl)silyl)oxy)methyl)-N-thiazol-2-ylpyridin-2-amine obtained in Example 5-(2) and N-bromosuccinimide.

### (2) Synthesis of 6-(chloromethyl)-N-(5-bromothiazol-2-yl)pyridin-2-amine

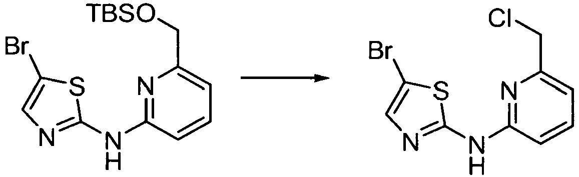

In the same manner as in Examples 5-(3) and (4), the title compound was synthesized using 6-(((tert-butyl(dimethyl)silyl)oxy)methyl)-N-(,5-bromothiazol-2-yl)pyridin-2-amine.

### (3) Synthesis of 6-((4-((2-fluoropyridin-3-yl)carbonyl)piperazin-1-yl)methyl)-N-(5-bromothiazol-2-yl)pyridin-2-amine

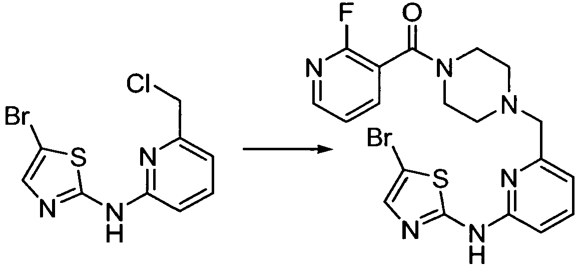

In the same manner as in Example 5-(5), the title compound was obtained using 6-(((tert-butyl(dimethyl)silyl)oxy)methyl)-N-(5-bromothiazol-2-yl)pyridin-2-amine and 1-((2-fluoropyridin-3-yl)carbonyl)piperazine synthesized with reference to the disclosed method in the processes (7) to (8), similar to Reference Example 1.
Spectral data of the title compound are as follows.
¹H-NMR (CDCl1₃) δ: 8.29 (d, J = 3.5 Hz, 1H), 7.89 (t, J = 7.6 Hz, 1H), 7.63 (t, J = 7.6 Hz, 1H), 7.35 (s, 1H), 7.33-7.20 (m, 1H), 7.05 (d, J = 7.4 Hz, 1H), 6.72 (d, J = 8.2 Hz, 1H), 3.87 (s, 2H), 3.77 (s, 2H), 3.39 (s, 2H), 2.69 (s, 2H), 2.58 (s, 2H)
Mass: 477, 479 (M + 1)⁺

Examples 79 and 80 were synthesized in the same manner as in Example 78 as follows.

### Example 79

### Synthesis of 6-((4-(2-fluoroisonicotinoyl)piperazin-1-yl)methyl)-N-(5-bromothiazol-2-yl)pyridin-2-amine

¹H-NMR (CDCl1₃) δ: 8.30 (d, J = 4.9 Hz, 1H), 7.64 (dd, J = 7.6, 7.2 Hz, 1H), 7.37 (s, 1H), 7.19 (d, J = 4.9 Hz, 1H), 7.03 (d, J = 7.2 Hz, 1H), 6.95 (s, 1H), 6.74 (d, J = 8.0 Hz, 1H), 3.85 (s, 2H), 3.77 (s, 2H), 3.41 (s, 2H), 2.70 (s, 2H), 2.55 (s, 2H)
Mass: 477, 479 (M + 1)⁺

### Example 80

### Synthesis of 6-((4-((6-fluoropyridin-2-yl)carbonyl)piperazin-1-yl)methyl)-N-(5-bromothiazol-2-yl)pyridin-2-amine

¹H-NMR (CDCl₃) δ: 7.95-7.88 (m, 1H), 7.63 (dd, J = 8.2, 7.6 Hz, 1H), 7.57 (d, J = 7.4 Hz, 1H), 7.35 (s, 1H), 7.06 (d, J = 8.0 Hz, 1H), 7.00 (d, J = 7.2 Hz, 1H), 6.72 (d, J = 7.6 Hz, 1H), 3.89-3.81 (m, 2H), 3.77 (s, 2H), 3.70-3.63 (m, 2H), 2.74-2.67 (m, 2H), 2.67-2.55 (m, 2H)
Mass: 477, 479 (M + 1)⁺

### Example 81

### Synthesis of 4-bromo-6-((4-(3-chloro-2-fluorobenzoyl)piperazin-1-yl)methyl)-N-thiazol-2-ylpyridin-2-amine

### (1) Synthesis of dimethyl 4-bromopyridine-2,6-dicarboxylate

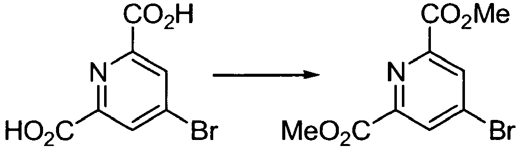

A mixture of 7.38 g of 4-bromopyridine-2,6-dicarboxylic acid synthesized in the method of Tetrahedron lett., 42 (29), 4849 (2001), 10 ml of a hydrochloric acid-methanol reagent and 100 ml of methanol was stirred at room temperature for 15 hours, and the reaction mixture was concentrated in *vacuo.* Ethyl acetate was added to the residue and the mixture was washed three times with a mixed solution of brine-saturated sodium bicarbonate (1 : 1). The organic layer was dried over anhydrous magnesium sulfate and filtered. The filtrate was then concentrated *in vacuo* to give the title compound.

### (2) Synthesis of methyl hydrogen 4-bromopyridine-2,6-dicarboxylate

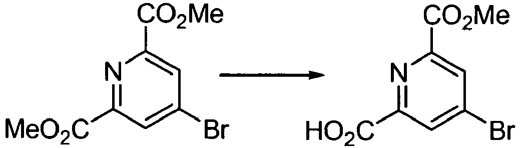

A mixture of 6.09 g of dimethyl 4-bromopyridine-2,6-dicarboxylate, 1.08 g of potassium hydroxide, 200 ml of methanol and 20 ml of dichloromethane was stirred at room temperature for 3 hours, and 200 ml of ether was added thereto. The resulting white solid was filtered, and then washed with ether. The obtained white solid was dissolved in water, and then 12 ml of hydrochloric acid (2 M) was added thereto. The resulting mixture was extracted with chloroform. The organic layer was dried over anhydrous magnesium sulfate and filtered, and the filtrate was concentrated *in vacuo* to give the title compound.

### (3) Synthesis of methyl 4-bromo-6-tert-butoxycarbonylaminopyridine-2-carboxylate

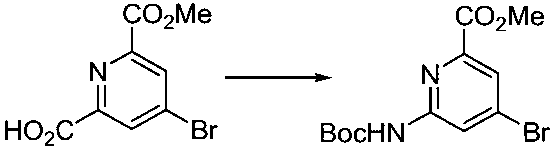

To a mixture of 4.62 g of methyl hydrogen 4-bromopyridine-2,6-dicarboxylate, 2.97 ml of triethylamine, 25 ml of t-butanol and 70 ml of 1,4-dioxane was added 4.59 ml of diphenylphosphoryl azide at room temperature. The reaction mixture was heated under reflux for 3 hours and cooled to room temperature. Water was added thereto and the resulting mixture was extracted with ethyl acetate. The obtained ethyl acetate solution was washed with water and brine, dried over anhydrous sodium sulfate and filtered. The filtrate was concentrated *in vacuo* to give the title compound.

### (4) Synthesis of methyl 6-amino-4-bromopyridine-2-carboxylate

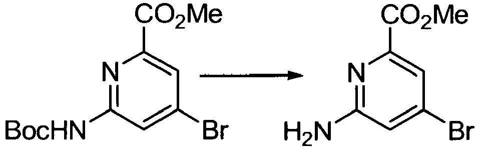

7.23 g of methyl 4-bromo-6-t-butoxycarbonylamino-pyridine-2-carboxylate was dissolved in 30 ml of chloroform, and then 15 ml of trifluoroacetic acid was added thereto, followed by stirring at room temperature for 1 hour. After concentrating the reaction mixture, the residue was dissolved in ethyl acetate and washed with saturated sodium bicarbonate and brine. The organic layer was dried over anhydrous magnesium sulfate, filtered, and then concentrated *in vacuo.* The residue was purified by a silica gel column chromatography (eluent: chloroform to chloroform/methanol = 20/1) to give the title compound.

### (5) Synthesis of methyl 6-(3-benzoylthioureido)-4-bromopyridine-2-carboxylate

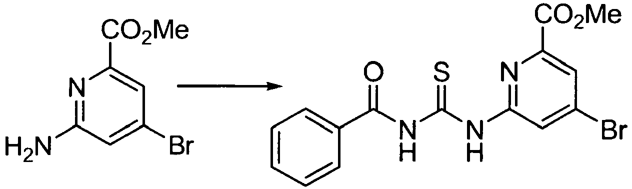

2.74 g of methyl 6-amino-4-bromopyridine-2-carboxylate was dissolved in 15 ml of tetrahydrofuran and 1.63 ml of benzoyl isothiocyanate was added thereto, followed by stirring at room temperature for 13 hours. To the reaction mixture was added 40 ml of hexane. The resulting solid was filtered and washed with hexane. The obtained solid was dried *in vacuo* to give the title compound.

### (6) Synthesis of methyl 4-bromo-6-(thiazol-2-ylamino)pyridine-2-carboxylate

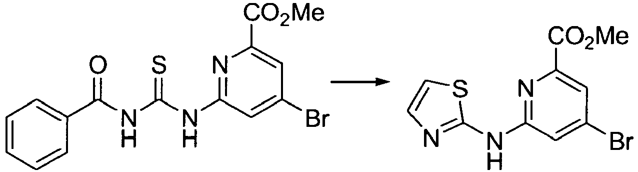

To a mixture of 2.37 g of methyl 6-(3-benzoylthioureido)-4-bromopyridine-2-carboxylate, 20 ml of tetrahydrofuran and 40 ml of methanol was added 673 mg of potassium hydroxide. The reaction mixture was stirred at room temperature for 1.5 hours, and acidified with a hydrochloric acid-methanol solution. The solvents were concentrated *in vacuo.* The resulting residue was dissolved in 60 ml of 1,4-dioxane and 3.53 ml of a 40% chloroacetaldehyde aqueous solution was added thereto. After the reaction mixture was heated under reflux for 1 hour, 40 ml of a hydrochloric acid-methanol solution and 60 ml of methanol were added thereto at room temperature, and the resulting mixture was stirred overnight. The reaction mixture was concentrated *in vacuo* and the residue was recrystallized from methanol-diethyl ether to give the title compound.

### (7) Synthesis of (4-bromo-6-((3-(methoxymethyl)-thiazol-2(3H)-ylidene)amino)pyridin-2-yl)methanol

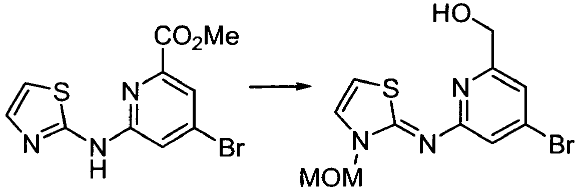

In the same manner as in Examples 1-(3) and (5), the title compound was obtained using methyl 4-bromo-6-(thiazol-2-ylamino)pyridine-2-carboxylate.

### (8) Synthesis of 4-bromo-6-(methanesulfonyloxymethyl)-N-(3-(methoxymethyl)-thiazol-2(3H)-ylidene)amino)pyridin-2-amine

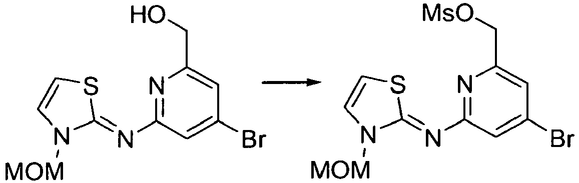

In the same manner as in Example 16-(2), the title compound was obtained using (4-bromo-6-((3-(methoxymethyl)-thiazol-2(3H)-ylidene)amino)pyridin-2-yl)methanol.

### (9) Synthesis of 4-bromo-6-((4-(3-chloro-2-fluorobenzoyl)piperazin-1-yl)methyl)-N-(3-(methoxymethyl)-thiazol-2(3H)-ylidene)amino)pyridin-2-amine

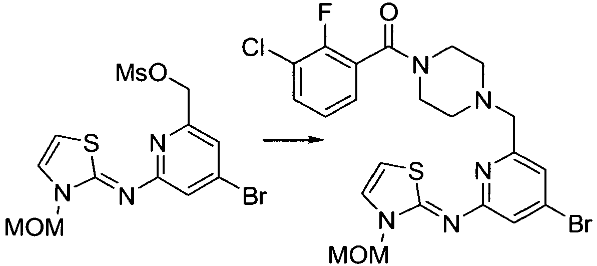

In the same manner as in example 16-(3), the title compound was obtained using 4-bromo-6-(methanesulfonyloxymethyl)-N-(3-(methoxymethyl)-thiazol-2(3H)-ylidene)amino)pyridin-2-amine.

### (10) Synthesis of 4-bromo-6-((4-(3-chloro-2-fluorobenzoyl)plperazin-1-yl)methyl)-N-thiazol-2-ylpyridin-2-amine

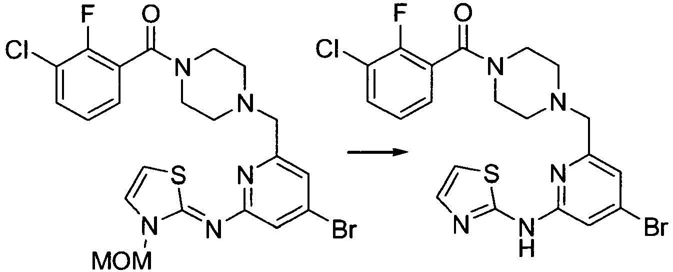

In the same manner as in Example 1-(8), the title compound was obtained using 4-bromo-6-((4-(3-chloro-2-fluorobenzoyl)piperazin-1-yl)methyl)-N-(3-(methoxymethyl)-thiazol-2(3H)-ylidene)amino)pyridin-2-amine.
Spectral data of the title compound are as follows.
Mass: 510, 512 (M + 1)⁺

### Example 82

### Synthesis of methyl 2-((4-(3-chloro-2-fluorobenzoyl)piperazin-1-yl)methyl)-6-(thiazol-2-ylamino)isonicotinate

### (1) Synthesis of methyl 2-((4-(3-chloro-2-fluorobenzoyl)piperazin-1-yl)methyl)-6-((3-(methoxymethyl)-thiazol-2(3H)-ylidene)amino)isonicotinate

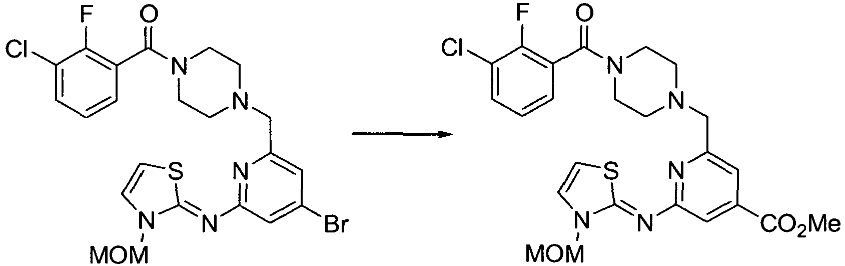

In the same manner as in Example 1-(2), the title compound was obtained using 4-bromo-6-((4-(3-chloro-2-fluorobenzoyl)piperazin-1-yl)methyl)-N-(3-(methoxymethyl)-thiazol-2(3H)-ylidene)amino)pyridin-2-amine obtained in Example 81-(9).

### (2) Synthesis of methyl 2-((4-(3-chloro-2-fluorobenzoyl)piperazin-1-yl)methyl)-6-(thiazol-2-ylamino)isonicotinate

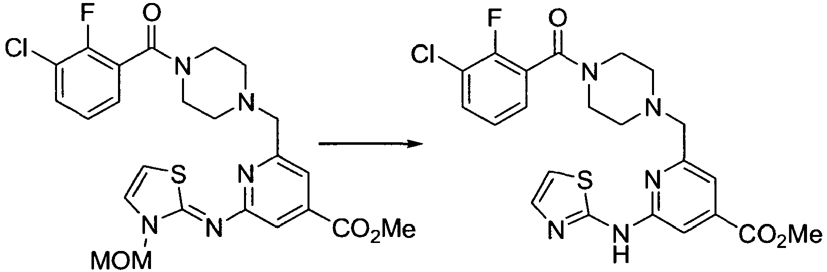

In the same manner as in Example 1-(8), the title compound was obtained using methyl 2-((4-(3-chloro-2-fluorobenzoyl)piperazin-1-yl)methyl)-6-((3-(methoxymethyl)-thiazol-2(3H)-ylidene)amino)isonicotinate.
Spectral data of the title compound are as follows.
¹H-NMR (CDCl₃) δ: 7.57-7.52 (m, 2H), 7.47-7.42 (m, 2H), 7.30-7.25 (m, 1H), 7.15 (t, J = 8.0 Hz, 1H), 6.91 (d, J = 3.6 Hz, 1H), 3.98 (s, 3H), 3.91-3.85 (m, 2H), 3.81 (s, 2H), 3.43-3.32 (m, 2H), 2.71 (brt, J = 4.8 Hz, 2H), 2.63-2.52 (m, 2H)
Mass: 490 (M + 1)⁺

### Example 83

### Synthesis of 2-((4-(3-chloro-2-fluorobenzoyl)piperazin-1-yl)methyl)-6-(thiazol-2-ylamino)isonicotinic acid

### (1) Synthesis of 2-((4-(3-chloro-2-fluorobenzoyl)piperazin-1-yl)methyl)-6-((3-(methoxymethyl)-thiazol-2(3H)-ylidene)amino)isonicotinic acid

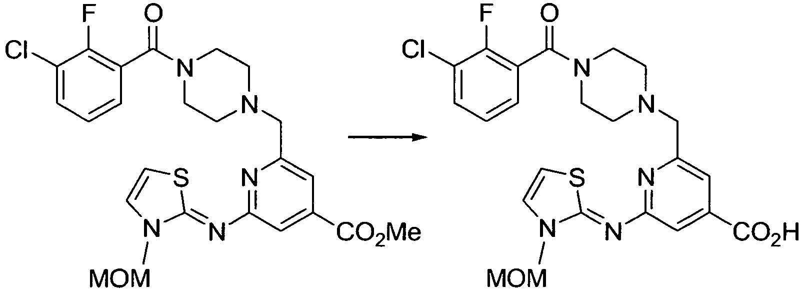

In the same manner as in Example 44-(3), the title compound was obtained using methyl 2-((4-(3-chloro-2-fluorobenzoyl)piperazin-1-yl)methyl)-6-((3-(methoxymethyl)-thiazol-2(3H)-ylidene)amino)isonicotinate obtained in Example 82-(1).

### (2) Synthesis of 2- ((4-(3-chloro-2-fluorobenzoyl)piperazin-1-yl)methyl)-6-(thiazol-2-ylamino)isonicotinic acid trifluoroacetate

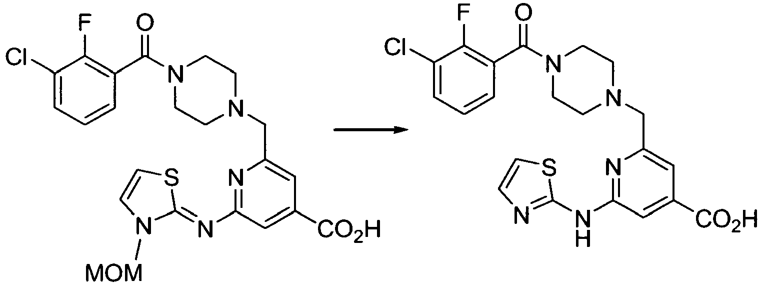

In the same manner as in Example 16-(4), the title compound was obtained using 2-((4-(3-chloro-2-fluorobenzoyl)piperazin-1-yl)methyl)-6-((3-(methoxymethyl)-thiazol-2(3H)-ylidene)amino)isonicotinic acid.
Spectral data of the title compound are as follows.
¹H-NMR (DMSO-d₆) δ: 7.74-7.68 (m, 1H), 7.63 (s, 1H), 7.61 (s, 1H), 7.47-7.41 (m, 2H), 7.33 (t, J = 8.0 Hz, 1H), 7.13 (d, J = 4.0 Hz, 1H), 4.49 (brs, 2H)
Mass: 476 (M + 1)⁺

### Example 84

### Synthesis of 6-((4-(3-ch)oro-2-fluorobenzoyl)piperazin-1-yl)methyl)-4-(pyrrolidin-1-ylcarbonyl)-N-thiazol-2-ylpyridin-2-amine trifluoroacetate

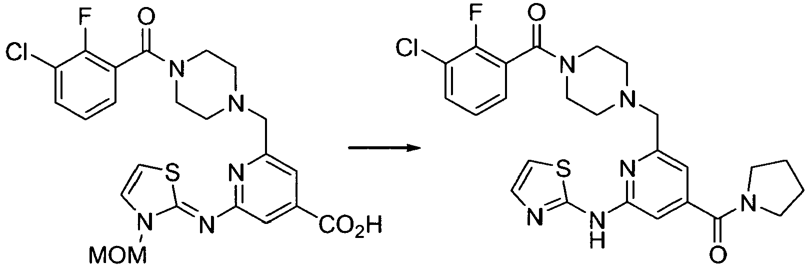

The amidation reaction was performed in the same manner as in Example 3-(2) using 2-((4-(3-chloro-2-fluorobenzoyl)piperazin-1-yl)methyl)-6-((3-(methoxymethyl)-thiazol-2(3H)-ylidene)amino)isonicotinic acid obtained in Example 83-(1) and pyrrolidine. The deprotection reaction was then performed to give the title compound.
Spectral data of the title compound are as follows.
¹H-NMR (CD₃OD) δ: 7.67-7.61 (m, 1H), 7.52-7.49 (m, 1H), 7.43-7.37 (m, 1H), 7.33-7.26 (m, 3H), 7.18-7.14 (m, 1H), 4.57 (s, 2H), 4.17-4.00 (m, 2H), 3.70 (brs, 2H), 3.64-3.57 (m, 4H), 3.52-3.43 (m, 4H), 2.06-1.90 (m, 4H)
Mass: 490 (M + 1)⁺

Examples 85 to 88 were synthesized in the same manner as in Example 84 as follows.

### Example 85

### Synthesis of 2-((4-(3-chloro-2-fluorobenzoyl)piperazin-1-yl)methyl)-N-methyl-6-(thiazol-2-ylamino)isonicotinamide trifluoroacetate

¹H-NMR (CD₃OD) δ: 7.67-7.60 (m, 1H), 7.54 (s, 1H), 7.52 (s, 1H), 7.51 (d, J = 4.0 Hz, 1H), 7.43-7.37 (m, 1H), 7.30 (t, J = 8.0 Hz, 1H), 7.16 (d, J = 4.0 Hz, 1H), 4.57 (s, 2H), 4.15-4.01 (m, 2H), 3.70 (brs, 2H), 3.60 (brs, 2H), 3.48 (brs, 2H), 2.95 (s, 3H)
Mass: 489 (M + 1)⁺

### Example 86

### Synthesis of 2-((4-(3-chloro-2-fluorobenzoyl)piperazin-1-yl)methyl)-N,N-dimethyl-6-(thiazol-2-ylamino)isonicotinamide trifluoroacetate

¹H-NMR (CD₃OD) δ: 7.66-7.60 (m, 1H), 7.54 (d, J = 4.0 Hz, 1H), 7.43-7.37 (m, 1H), 7.30 (t, J = 8.0 Hz, 1H), 7.29 (d, J = 1.2 Hz, 1H), 7.24 (d, J = 1.2 Hz, 1H), 7.20 (d, J = 4.0 Hz, 1H), 4.58 (s, 2H), 4.09 (brs, 2H), 3.70 (brs, 2H), 3.61 (brs, 2H), 3.49 (brs, 2H), 3.12 (s, 3H), 3.01 (s, 3H)
Mass: 503 (M + 1)⁺

### Example 87

### Synthesis of 2-((4-(3-chloro-2-fluorobenzoyl)piperazin-1-yl)methyl)-6-(thiazol-2-ylamino)isonicotinamide trifluoroacetate

¹H-NMR (CD₃OD) δ: 7.67-7.59 (m, 3H), 7.55 (d, J = 4.0 Hz, 1H), 7.43-7.37 (m, 1H), 7.30 (t, J = 8.0 Hz, 1H), 7.20 (d, J = 4.0 Hz, 1H), 4.60 (s, 2H), 4.09 (brs, 2H), 3.70 (brs, 2H), 3.60 (brs, 2H), 3.49 (brs, 2H)
Mass: 475 (M+1)⁺

### Example 88

### Synthesis of 2-((4-(3-chloro-2-fluorobenzoyl)piperazin-1-yl)methyl)-N-(2-hydroxyethyl)-6-(thiazol-2-ylamino)isonicotinamide trifluoroacetate

¹H-NMR (CD₃OD) δ: 7.66-7.60 (m, 1H), 7.49 (s, 2H), 7.45 (d, J = 3.6 Hz, 1H), 7.42-7.37 (m, 1H), 7.29 (t, J = 8.0 Hz, 1H), 7.09 (d, J = 3.6 Hz, 1H), 4.52 (s, 2H), 4.07 (brs, 2H), 3.72 (t, J = 5.6 Hz, 2H), 3.68 (brs, 2H), 3.56 (brs, 2H), 3.52 (t, J = 5.6 Hz, 2H), 3.44 (brs, 2H)
Mass: 519 (M + 1)⁺

### Example 89

### Synthesis of (2-((4-(3-chloro-2-fluorobenzoyl)piperazin-1-yl)methyl)-6-(thiazol-2-ylamino)pyridin-4-yl)methanol

### (1) Synthesis of (2-((4-(3-chloro-2-fluorobenzoyl)piperazin-1-yl)methyl)-6-((3-(methoxymethyl)-thiazol-2(3H)-ylidene)amino)pyridin-4-yl)methanol

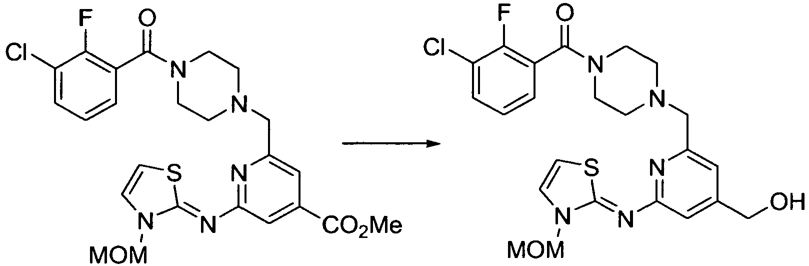

In the same manner as in Example 1-(5), the title compound was obtained using methyl 2-((4-(3-chloro-2-fluorobenzoyl)piperazin-1-yl)methyl)-6-((3-(methoxymethyl)-thiazol-2(3H)-ylidene)amino)isonicotinate obtained in Example 82-(1).

### (2) Synthesis of (2-((4-(3-chloro-2-fluorobenzoyl)piperazin-1-yl)methyl)-6-(thiazol-2-ylamino)pyridin-4-yl)methanol

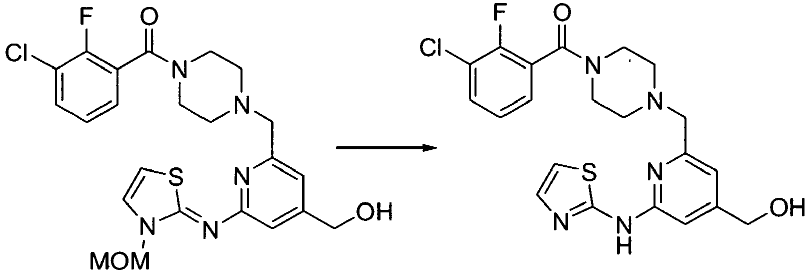

In the same manner as in Example 16-(4), the title compound was obtained using (2-((4-(3-chloro-2-fluorobenzoyl)piperazin-1-yl)methyl)-6-((3-(methoxymethyl)-thiazol-2(3H)-ylidene)amino)pyidin-4-yl)methanol.
Spectral data of the title compound are as follows.
¹H-NMR (CDCl₃) δ: 7.47-7.42 (m, 1H), 7.40 (d, J = 3.6 Hz, 1H), 7.30-7.23 (m, 1H), 7.15 (brt, J = 8.0 Hz, 1H), 6.97 (brs, 1H), 6.84-6.81 (m, 2H), 4.74 (s, 2H), 3.89-3,83 (m, 2H), 3.73 (s, 2H), 3.41-3.32 (m, 2H), 2.72-2.66 (m, 2H), 2.60-2.52 (m, 2H)
Mass: 462 (M + 1)⁺

### Example 90

### Synthesis of 6-((4-(3-chloro-2-fluorobenzoyl)piperazin-1-yl)methyl)-N-thiazol-2-yl-4-(1H-1,2,3-triazol-1-ylmethyl)pyridin-2-amine trifluoroacetate

### (1) Synthesis of 6-((4-(3-chloro-2-fluorobenzoyl)plperazin-1-yl)methyl)-N-(3-(Methoxymethyl)-thiazol-2(3H)-ylidene)-4-(methanesulfonyloxymethyl)pyridin-2-amine

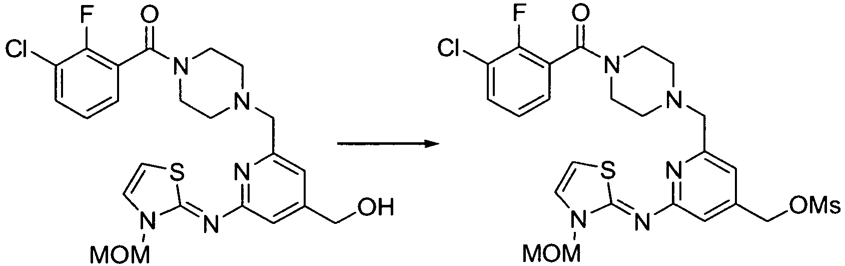

In the same manner as in Example 16-(2), the title compound was obtained using (2-((4-(3-chloro-2-fluorobenzoyl)piperazin-1-yl)methyl)-6-((3-(methoxymethyl)-thiazol-2(3H)-ylidene)amino)pyridin-4-yl)methanol obtained in Example 89-(1).

### (2) Synthesis of 6-((4-(3-chloro-2-fluorobenzoyl)piperazin-1-yl)methyl)-N-(3-(methoxymethyl)-thiazol-2(3H)-ylidene)-4-(1H-1,2,3-triazol-1-ylmethyl)pyridin-2-amine and 6-((4-(3-chloro-2-fluorobenzoyl)piperazin-1-yl)methyl)-N-(3-(methoxymethyl)-thiazol-2(3H)-ylidene)-4-(2H-1,2,3-triazol-2-ylmethyl)pyridin-2-amine

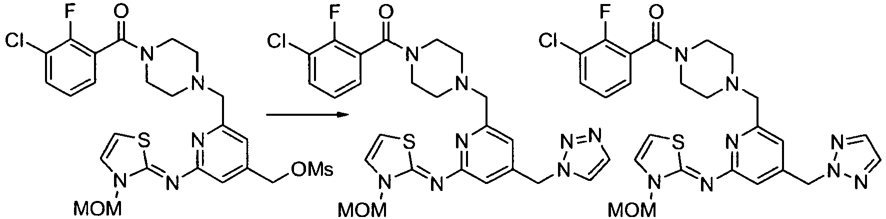

A mixture of 26 mg of 6-((4-(3-chloro-2-fluorobenzoyl)piperazin-1-yl)methyl)-N-(,3-(methoxymethyl)-thiazol-2(3H)-ylidene)-4-(methanesulfonyloxymethyl)pyridin-2-amine and 5.2 µl of 1H-1,2,3-triazole, 14 µl of 1,8-diazabicyclo[5.4.0]undeca-7-en and 0.47 ml of chloroform was stirred at room temperature for 19 hours. The reaction solution was purified by a silica gel column chromatography (eluent: chloroform to chloroform/methanol = 10/1) to give the title compound.

### (3) Synthesis of 6-((4-(3-chloro-2-fluorobenzoyl)piperazin-1-yl)methyl)-N-thiazol-2-yl-4-(1H-1,2,3-triazol- 1-ylmethyl)pyridin-2-amine trifluoroacetate

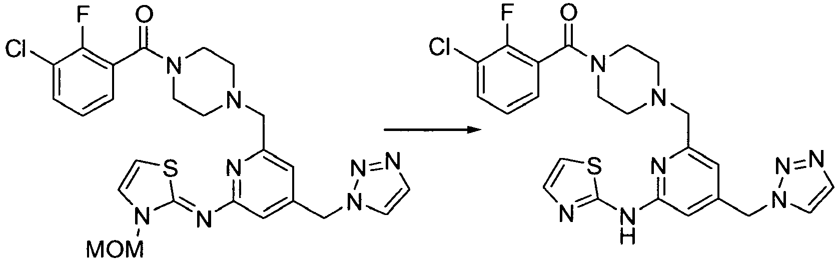

In the same manner as in Example 16-(4), the title compound was obtained using 6-((4-(3-chloro-2-fluorobenzoyl)piperazin-1-yl)methyl)-N-(3-(methoxymethyl)-thiazol-2(3H)-ylidene)-4-(1H-1,2,3-triazol-1 -ylmethyl)pyridin-2-amine.
Spectral data of the title compound are as follows.
¹H-NMR (CD₃OD) δ: 8.13 (s, 1H), 7.84 (d, J = 1.2 Hz, 1H), 7.66-7.60 (m, 1H), 7.55 (d, J = 3.6 Hz, 1H), 7.42-7.36 (m, 1H), 7.29 (brt, J = 7.6 Hz, 1H), 7.22 (d, J = 3.6 Hz, 1H), 7.21 (s, 1H), 7.04 (s, 1H), 5.79 (s, 2H), 4.54 (s, 2H), 4.07 (brs, 2H), 3.68 (brs, 2H), 3.57 (brs, 2H), 3.46 (brs, 2H)
Mass: 513 (M + 1)⁺

### Example 91

### Synthesis of 6-((4-(3-chloro-2-fluorobenzoyl)piperazin-1-yl)methyl)-N-thiazo1-2-yl-4-(2H-1,2,3-triazol-2-ylmethyl)pyridin-2-amine trifluoroacetate

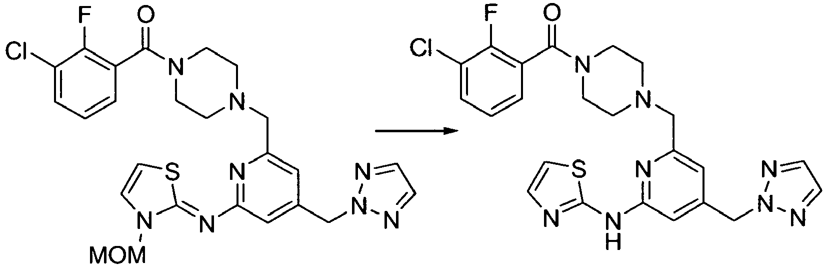

In the same manner as in Example 16-(4), the title compound was obtained using 6-((4-(3-chloro-2-fluorobenzoyl)piperazin-1-yl)methyl)-N-(3-(methoxymethyl)-thiazol-2(3H)-ylidene)-4-(2H-1,2,3-triazol-2-ylmethyl)pyridin-2-amine obtained in Example 90-(2).
Spectral data of the title compound are as follows.
¹H-NMR (CD₃OD) δ: 7.80 (s, 2H), 7.67-7.61 (m, 1H), 7.46 (d, J = 3.6 Hz, 1H), 7.43-7.37 (m, 1H), 7.30 (brt, J = 8.0 Hz, 1H), 7.11 (d, J = 3.6 Hz, 1H), 7.09 (s, 1H), 6.94 (s, 1H), 5.75 (s, 2H), 4.48 (s, 2H), 4.06 (brs, 2H), 3.67 (brs, 2H), 3.56 (brs, 2H), 3.44 (brs, 2H)
Mass: 513 (M + 1)⁺

Examples 92 to 95 were synthesized in the same manner as in Example 90 as follows.

### Example 92

Synthesis of 6-((4-(3-chloro-2-fluorobenzoyl)piperazin-1-yl)methyl)-N-thiazol-2-yl-4-(1H-1,2,4-triazol-1-ylmethyl)pyridin-2-amine trifluoroacetate
¹H-NMR (CD₃OD) δ: 8.71 (s, 1H), 8.12 (s, 1H), 7.66-7.60 (m, 1H), 7.56 (d, J = 3.6 Hz, 1H), 7.44-7.36 (m, 1H), 7.29 (t, J = 8.0 Hz, 1H), 7.23 (s, 1H), 7.22 (d, J = 3.6 Hz, 1H), 7.07 (s, 1H), 5.60 (s, 2H), 4.54 (s, 2H), 4.07 (brs, 2H), 3.69 (brs, 2H), 3.57 (brs, 2H), 3.46 (brs, 2H)
Mass: 513 (M + 1)⁺

### Example 93

Synthesis of 6-((4-(3-chloro-2-fluorobenzoyl)piperazin-1-yl)methyl)-4-((methanesulfonyl)methyl)-N-thiazol-2-ylpyridin-2-amine
¹H-NMR (CDCl₃) δ: 11.37 (brs, 1H), 7.69-7.63 (m, 1H), 7.40-7.34 (m, 2H), 7.30 (t, J = 8.0 Hz, 1H), 7.04 (s, 1H), 7.00 (d, J = 3.6 Hz, 1H), 6.96 (s, 1H), 4.53 (s, 2H), 3.72-3.64 (m, 4H), 2.97 (s, 3H)
Mass: 524 (M + 1)⁺

### Example 94

### Synthesis of 6-((4-(3-chloro-2-fluorobenzoyl)piperazin-1-yl)methyl)-4-((dimethylamino)methyl)-N-thiazol-2-ylpyridin-2-amine

¹H-NMR (CDCl₃) δ: 7.48 (d, J=4.0 Hz, 1H), 7.47-7.41 (m, 1H), 7.29-7.24 (m, 1H), 7.14 (brt, J = 8.0 Hz, 1H), 6.97 (s, 1H), 6.84 (d, J = 4.0 Hz, 1H), 6.81 (s, 1H), 3.86 (brt, J = 4.8 Hz, 2H), 3.74 (s, 2H), 3.42 (s, 2H), 3.37 (brs, 2H), 2.70 (brt, J = 4.8 Hz, 2H), 2.57 (brs, 2H), 2.27 (s, 6H)
Mass: 489 (M + 1)⁺

### Example 95

### Synthesis of 6-((4-(3-chloro-2-fluorobenzoyl)piperazin-1-yl)methyl)-4-(methoxymethyl)-N-thiazol-2-ylpyridin-2-amine

¹H-NMR (CDCl₃) δ: 7.49 (brd, J = 3.6 Hz, 1H), 7.47-7.41 (m, 1H), 7.30-7.24 (m, 1H), 7.14 (t, J = 8.0 Hz, 1H), 6.93 (s, 1H), 6.85 (d, J = 3.6 Hz, 1H), 6.83 (s, 1H), 4.47 (s, 2H), 3.87 (brt, J = 4.8 Hz, 2H), 3.74 (s, 2H), 3.46 (s, 3H), 3.37 (brs, 2H), 2.71 (brt, J = 4.8 Hz, 2H), 2.57 (brs, 2H)
Mass: 476 (M + 1)⁺

### Example 96

### Synthesis of 1-(2-((4-(3-chloro-2-fluorobenzoyl)piperazin-1-yl)methyl)-6-(thiazol-2-ylamino)pyridin-4-yl)pyrrolidin-2-one

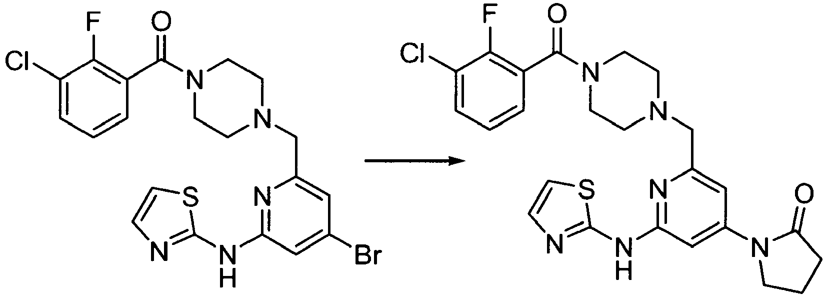

To a solution of 25.5 mg of 4-bromo-6-((4-(3-chloro-2-fluorobenzoyl)piperazin-1-yl)methyl)-N-thiazol-2-ylpyridin-2-amine obtained in Example 81 in 1 mL of 1,4-dioxane was added 0.011 mL of pyrrolidin-2-one, 32 mg of potassium phosphate, 8.7 mg of 9,9-dimethyl-4,5-bis(diphenylphosphino)xanthene and 7.8 mg of tris(dibenzylideneacetone)dipalladium(0)-chloroform complex, successively. The reaction solution was stirred at 100°C for 3 hours. The reaction solution was cooled, diluted with ethyl acetate, washed with water, and then dried over anhydrous magnesium sulfate. The solvent was concentrated *in vacuo* and the resulting residue was purified by a reversed phase medium pressure liquid chromatography [ODS-AS-360-CC (manufactured by YMC company), mobile phase: water-acetonitrile-0.1% trifluoroacetic acid]. The obtained fraction was diluted with ethyl acetate, washed with saturated sodium bicarbonate, and then dried over anhydrous magnesium sulfate. The solvent was concentrated *in vacuo* to give the title compound.
Spectral data of the title compound are as follows.
¹H-NMR (CDCl₃) δ: 7.62 (s, 1H), 7,50 (s, 1H), 7.44 (t, J = 6.8 Hz, 1H), 7.30-7.23 (m, 1H), 7.19-7.11 (m, 2H), 6.85 (s, 1H), 3.94-3.82 (m, 4H), 3.73 (s, 2H), 3.42-3.30 (m, 2H), 2.77-2.45 (m, 6H), 2.26-2.16 (m, 2H)
Mass: 515, 517 (M + 1)⁺

Examples 97 to 103 were synthesized in the same manner as in Example 96 as follows.

### Example 97

### Synthesis of 3-(2-((4-(3-chloro-2-fluorobenzoyl)piperazin-1-yl)methyl)-6-(thiazol-2-ylamino)pyridin-4-yl)- 1 ,3-oxazolidin-2-one

¹H-NMR (DMSO-d₆) δ: 7.67 (t, J = 8.0 Hz, 1H), 7.40-7.25 (m, 4H), 7.13 (s, 1H), 6.99 (d, J = 3.3 Hz, 1H), 4.46 (t, J = 7.8 Hz, 2H), 4.04 (t, J = 7.8 Hz, 2H), 3.72-3.65 (m, 2H), 3.64 (s, 2H), 3.30-3.20 (m, 2H), 2.60-2.41 (m, 4H)
Mass: 517, 519 (M+1)⁺

### Example 98

### Synthesis of 3-(2-((4-(3-chloro-2-fluorobenzoyl)piperazin-1-yl)methyl)-6-(thiazol-2-ylamino)pyridin-4-yl)-3-methylimidazolidin-2-one

¹H-NMR (DMSO-d₆) δ: 11.06 (s, 1H), 7.70-7.60 (m, 1H), 7.40-7.20 (m, 5H), 7.03 (s, 1H), 6.95-6.92 (m, 1H), 3.80-3.40 (m, 8H), 3.30-3.15 (m, 2H), 2.77 (s, 3H), 2.65-2.40 (m, 4H)
Mass: 530 (M+1)⁺

### Example 99

### Synthesis of 3-(2-((4-(3-chloro-2-fluorobenzoyl)piperazin-1-yl)methyl)-6-(thiazol-2-ylamino)pyridin-4-yl)piperidin-2-one

¹H-NMR (CDCl₃) δ: 7.47-7.40 (m, 2H), 7.29-7.22 (m, 1H), 7.14 (t, J = 7.8 Hz, 1H), 6.99 (s, 1H), 6.90 (s, 1H), 6.82 (s, 1H), 3.91-3.80 (m, 2H), 3.75-3.64 (m, 4H), 3.42-3.29 (m, 2H), 2.74-2.46 (m, 6H), 2.03-1.92 (m, 4H)
Mass: 529, 531 (M+1)⁺

### Example 100

### Synthesis of 6-((4-(2,3-difluorobenzoyl)piperazin-1-yl)methyl)-N⁴-(2,2-difluoroethyl)-N²-thiazol-2-ylpyridine-2,4-diamine

Mass: 495 (M+1)⁺

### Example 101

### Synthesis of 6-((4-(2,3-difluorobenzoyl)piperazin-1-yl)methyl)-4-piperazin-1-yl-N-thiazol-2-ylpyridin-2-amine

Mass: 500 (M+1)⁺

### Example 102

### Synthesis of 1-(2-((4-(2,3-difluorobenzoyl)piperazin-1-yl)methyl)-6-(thiazol-2-ylamino)pyridin-4-yl)-1,5-dihydro-2H-pyrrol-2-one

Mass: 497 (M+1)⁺

### Example 103

### Synthesis of 6-((4-(2,3-difluorobenzoyl)piperazin-1-yl)methyl)-4-morpholin-4-yl-N-thiazol-2-ylpyridin-2-amine

Mass: 501 (M+1)⁺

### Example 104

### Synthesis of 2-((4-(3-chloro-2-fluorobenzoyl)piperazin-1-yl)methyl)-6-(thiazol-2-ylamino)isonicotinonitrile

### (1) Synthesis of 4-bromo-6-(((tert-butyl(dimethyl)silyl)oxy)methyl)-N-(3-(methoxymethyl)-thiazol-2(3H)-ylidene)pyridin-2-amine

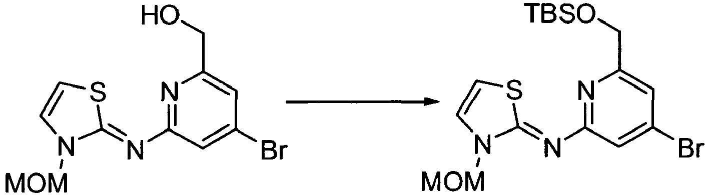

In the same manner as in Example 5-(1), the title compound was obtained using (4-bromo-6-((3-(methoxymethyl)-thiazol-2(3H)-ylidene)amino)pyridin-2-yl)methanol obtained in Example 81-(7).

### (2) Synthesis of 2-(((tert-butyl(dimethyl)silyl)oxy)methyl)-6-((3-(methoxymethyl)thiazol-2(3H)-ylidene)amino)isonicotinonitrile

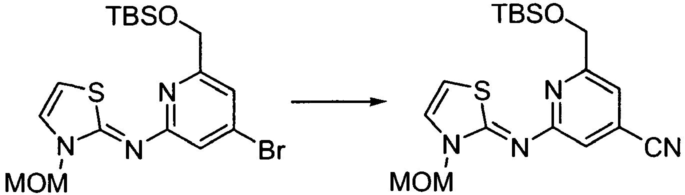

A mixture of 2.04 g of 4-bromo-6-(((tert-butyl(dimethyl)silyl)oxy)methyl)-N-(3-(methoxymethyl)-thiazol-2(3H)-ylidene)pyridin-2-amine, 379 mg of zinc cyanide, 266 mg of 4,5-bis(diphenylphosphino)-9,9-dimethylxanthene, 133 mg of bis(dibenzylideneacetone)palladium(0), 92 mg of zinc and 9.2 ml of N,N-dimethylacetamide was stirred at 140°C for 3 hours. The resulting reaction mixture was diluted with ethyl acetate, and then an insoluble matter was filtered off using Celite. The filtrate was washed with water and brine. The organic layer was dried over anhydrous magnesium sulfate and filtered, and the filtrate was concentrated. The residue was purified by a silica gel column chromatography (eluent: hexane to hexane/ethyl acetate = 3/1) to give the title compound.

### (3) Synthesis of 2-((4-(3-chloro-2-fluorobenzoyl)piperazin-1-yl)methyl)-6-(thiazol-2-ylamino)isonicotinonitrile

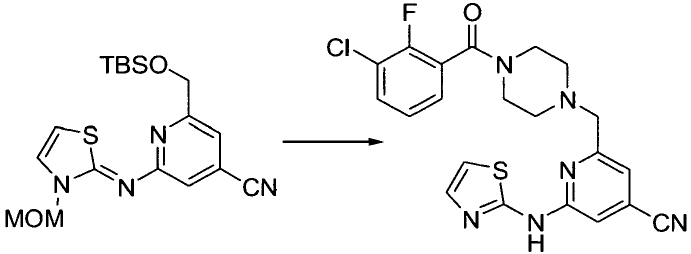

2-(((tert-butyl(dimethyl)silyl)oxy)methyl)-6-((3-(methoxymethyl)thiazol-2(3H)-ylidene)amino)isonicotinonitrile was subjected to the process similar to Example 5-(3) and subsequently the processes similar to Examples 16-(2) to (4) to give the title compound.
Spectral data of the title compound are as follows.
¹H-NMR (CDCl₃) δ: 7.53 (d, J = 3.7 Hz, 1H), 7.50-7.43 (m, 1H), 7.32-7.25 (m, 2H), 7.16 (t, J = 7.8 Hz, 1H), 7.05 (s, 1H), 6.97 (d, J = 3.5 Hz, 1H), 3.89 (brs, 2H), 3.79 (s, 2H), 3.39 (brs, 2H), 2.69 (t, J = 4.9 Hz, 2H), 2.57 (brs, 2H)
Mass: 457 (M+1)⁺

### Example 105

### Synthesis of 6-((4-(3-chloro-2-fluorobenzoyl)piperazin-1-yl)methyl)-4-(2H-tetrazol-5-yl)-N-thiazol-2-ylpyridin-2-amine

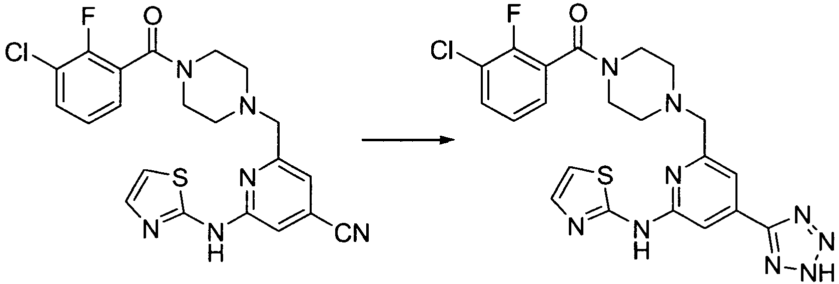

A mixture of 25.2 mg of 2-((4-(3-chloro-2-fluorobenzoyl)piperazin-1-yl)methyl)-6-(thiazol-2-ylamino)isonicotinonitrile obtained in Example 104, 17.9 mg of sodium azide, 38.0 mg of triethylamine hydrochloride and 2 ml of N,N-dimethylformamide was stirred at 110°C for 13 hours, and then the reaction solution was concentrated. The resulting residue was purified by a thin-layer chromatography for fractionation (eluent: chloroform/methanol = 5/1) to give the title compound.
Spectral data of the title compound are as follows.
¹H-NMR (CDCl₃) δ: 7.84 (s, 1H), 7.43 (dd, J = 7.0, 6.3 Hz, 1H), 7.38-7.23 (m, 3H), 7.13 (dd, J = 8.0, 7.6 Hz, 1H), 6.85-6.81 (m, 1H), 3.92-3.89 (m, 4H), 3.44-3.30 (m, 2H), 2.81-2.49 (m, 4H)
Mass: 500 (M+1)⁺

### Example 106

### Synthesis of 6-((4-(3-chloro-2-fluorobenzoyl)piperazin-1-yl)methyl)-4-(1-methyl-1H-tetrazol-5-yl)-N-thiazol-2-ylpyridin-2-amine

### (1) Synthesis of 6-(((tert-butyl(dimethyl)silyl)oxylmethyl)-N-(3-(methoxymethyl)thiazol-2(3H)-ylidene)-4-(2H-tetrazol-5-yl)pyridin-2-amine

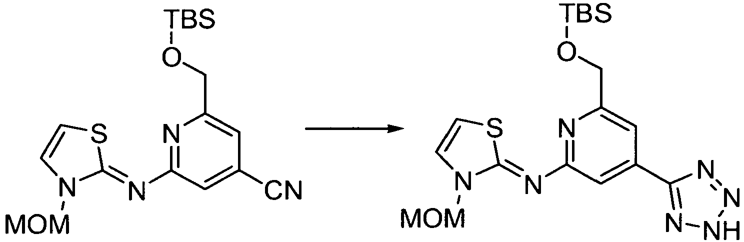

In the same manner as in Example 105, the title compound was obtained using 2-(((tert-butyl(dimethyl)silyl)oxy)methyl)-6-((3-(methoxymethyl)thiazol-2(3H)-ylidene)amino)isonicotinonitrile obtained in Example 104-(2).

### (2) Synthesis of 6-(((tert-butyl(dimethyl)silyl)oxy)methyl)-N-(3-(methoxymethyl)thiazol-2(3H)-ylidene)-4-(1-methyl-1H-tetrazol-5-yl)pyridin-2-amine and 6-(((tert-butyl(dimethyl)silyl)oxy)methyl)-N-(3-(methoxymethyl)thiazol-2(3H)-ylidene)-4-(2-methyl-2H-tetrazol-5-yl)pyridin-2-amine

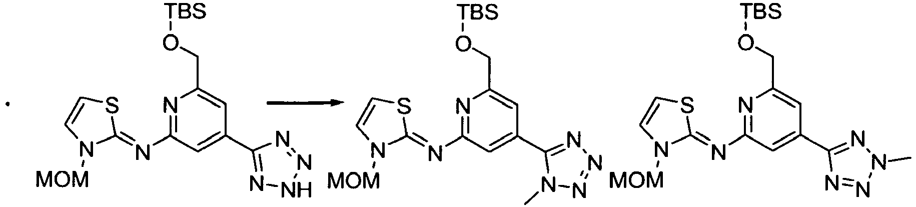

194 mg of 6-(((tert-butyl(dimethyl)silyl)oxy)methyl)-N-(3-(methoxymethyl)thiazol-2(3H)-ylidene)-4-(1H-tetrazol-5-yl)pyridin-2-amine was dissolved in 3 ml of N,N-dimethylformamide, and then 190 mg of cesium carbonate was added thereto. The reaction mixture was stirred at 60°C for 1.5 hours and cooled to room temperature. 29.3 µl of methyl iodide was added thereto, followed by stirring at room temperature for 1.5 hours. The reaction solution was diluted with ethyl acetate and then washed with water and brine. The resulting organic layer was dried over magnesium sulfate and filtered, and the filtrate was concentrated. The resulting residue was purified by a silica gel column chromatography (eluent: hexane to hexane/ethyl acetate = 2/1) to give the title compound, respectively.

### (3) Synthesis of 6-((4-(3-chloro-2-fluorobenzoyl)piperazin-1-yl)methyl)-4-(1-methyl-1H-tetrazol-5-yl)-N-thiazol-2-ylpyridin-2-amine

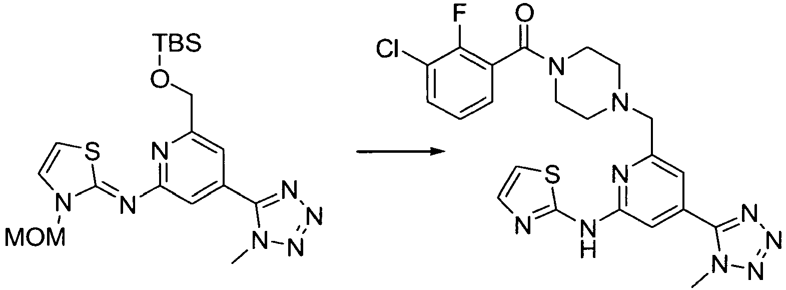

In the same manner as in Example 16, the title compound was obtained using 6-(((tert-butyl(dimethyl)silyl)oxy)methyl)-N-(3-(methoxymethyl)thiazol-2(3H)-ylidene)-4-(1-methyl- H-tetrazol-5-yl)pyridin-2-amine.
Spectral data of the title compound are as follows.
¹H-NMR (DMSO-d₆) δ: 11.56 (brs, 1H), 7.68-7.62 (m, 1H), 7.44-7.40 (m, 3H), 7.37 (dd, J = 8.0, 7.2 Hz, 1H), 7.30 (d, J = 7.8, 7.6 Hz, 1H), 7.07 (d, J = 3.3 Hz, 1H), 4.21 (s, 3H), 3.77 (s, 2H), 3.69 (brs, 2H), 3.30-3.23 (m, 2H), 2.64-2.45 (m, 4H)
Mass: 514 (M+1)⁺

### Example 107

### Synthesis of 6-((4-(3-chloro-2-fluorobenzoyl)piperazin-1-yl)methyl)-4-(2-methyl-2H-tetrazol-5-yl)-N-thiazol-2-ylpyridin-2-amine

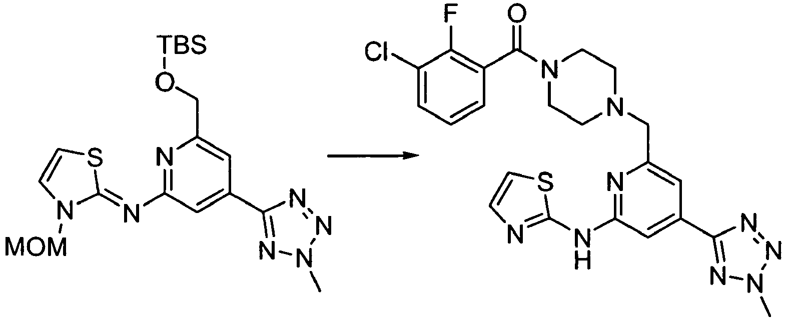

In the same manner as in Example 106, the title compound was obtained using 6-(((tert-butyl(dimethyl)silyl)oxy)methyl)-N-(3-(methoxymethyl)thiazol-2(3H)-ylidene)-4-(2-methyl-2H-tetrazol-5-yl)pyridin-2-amine obtained in Example 106-(2).
Spectral data of the title compound are as follows.
¹H-NMR (CDCl₃) δ: 7.73 (s, 1H), 7.59 (s, 1H), 7.57-7.41 (m, 2H), 7.30-7.10 (m, 2H), 6.89 (s, 1H), 4.46 (s, 3H), 3.89 (brs, 2H), 3.83 (s, 2H), 3.44-3.36 (m, 2H), 2.80-2.2.51 (m, 4H)
Mass: 514 (M+1)⁺

### Example 108

### Synthesis of 6-((4-(3-chloro-2-fluorobenzoyl)piperazin-1-yl)methyl)-N-thiazol-2-yl-4-(1H-1,2,4-triazol-5-yl)pyridin-2-amine

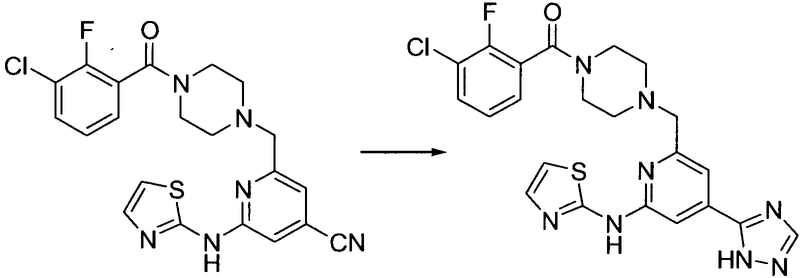

To a mixture of 25.2 mg of 2-((4-(3-chloro-2-fluorobenzoyl)piperazin-1-yl)methyl)-6-(thiazol-2-ylamino)isonicotinonitrile obtained in Example 104 and 1 ml of methanol was added 0.3 ml of a 28% sodium methoxide-methanol solution, followed by stirring at room temperature for 5 hours. To the reaction solution was added a solution of 3.3 mg of formohydrazide in 1 ml of methanol. The reaction mixture was stirred at room temperature for 17 hours, and then heated under reflux for 24 hours. To the reaction solution was added a solution of 16.6 mg of formohydrazide in 1.5 ml of methanol, and further heated under reflux for 22 hours. The reaction solution was cooled to room temperature, and then concentrated. The resulting residue was purified by a thin-layer chromatography for fractionation (eluent: chloroform/methanol = 7/1) to give the title compound.
Spectral data of the title compound are as follows.
¹H-NMR (DMSO-d₆) δ: 8.59 (s, 1H), 7.69-7.58 (m, 3H), 7.41-7.28 (m, 3H), 7.03-6.98 (m, 1H), 3.72 (s, 2H), 3.60-3.05 (m, 6H), 2.63-2.45 (m, 2H)
Mass: 499 (M+1)⁺

### Example 109

### Synthesis of 6-((4-(3-chloro-2-fluorobenzoyl)plperazin-1-yl)methyl)-4-(5-methyl-1,2,4-oxadiazol-3-yl)-N-thiazol-2-ylpyridin-2-amine

### (1) Synthesis of 6-((4-(3-chloro-2-fluorobenzoyl)piperazin-1-yl)methyl)-N-(3-(methoxymethyl)thiazol-2(3H)-ylidene)-4-(5-methyl-1,2,4-oxadiazol-3-yl)pyridin-2-amine

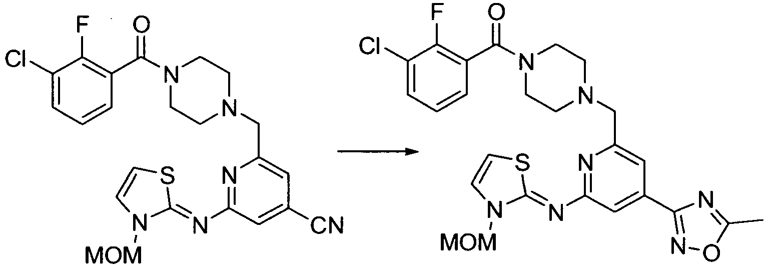

A mixture of 31.1 mg of 2-((4-(3-chloro-2-fluorobenzoyl)piperazin-1-yl)methyl)-6-((3-(methoxymethyl)-thiazol-2(3H)-ylidene)amino)isonicotinonitrile obtained in Example 104-(3), 13.0 mg of hydroxylammonium chloride, 17.2 mg of potassium carbonate and 3ml of ethanol was heated under reflux for 18 hours. The reaction solution was cooled to room temperature, and then concentrated. To the residue was added 3.0 ml of acetic anhydride followed by heating under reflux for 4 hours. The reaction solution was cooled to room temperature, and then concentrated. The reaction solution was diluted with chloroform and then washed with saturated potassium carbonate. The resulting organic layer was dried over magnesium sulfate and filtered, and the filtrate was concentrated. The resulting residue was purified by a thin-layer chromatography for fractionation (eluent: chloroform/methanol = 10/1) to give the title compound.

### (2) Synthesis of 6-((4-(3-chloro-2-fluorobenzoyl)piperazin-1-yl)methyl)-4-(5-methyl-1,2,4-oxadiazol-3-yl)-N-thiazol-2-ylpyridin-2-amine

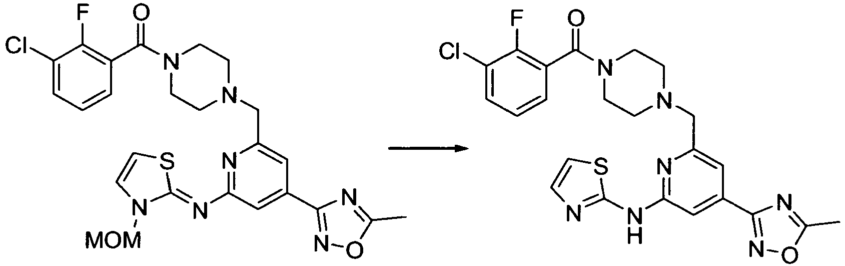

In the same manner as in Example 16-(4), the title compound was obtained using 6-((4-(3-chloro-2-fluorobenzoyl)piperazin-1-yl)methyl)-N-(3-(methoxymethyl)thiazol-2(3H)-ylidene)-4-(5-methyl-1,2,4-oxadiazol-3-yl)pyridin-2-amine.
Spectral data of the title compound are as follows.
¹H-NMR (CDCl₃) δ: 7.64 (s, 1H), 7.62-7.58 (m, 1H), 7.54 (s, 1H), 7.44 (dd, J = 8.0, 7.0 Hz, 1H), 7.28-7.23 (m, 1H), 7.14 (t, J = 7.8 Hz, 1H), 6.91 (d, J = 3.5 Hz, 1H), 3.91-3.84 (m, 2H), 3.82 (s, 2H), 3.38 (brs, 2H), 2.71 (s, 3H), 2.77-2.50 (m, 4H)
Mass: 514 (M + 1)⁺

### Example 110

### Synthesis of 6-((4-(3-chloro-2-fluorobenzoyl)piperazin-1-yl)methyl)-4-(3-methyl-1,2,4-oxadiazol-5-yl)-N-thiazol-2-ylpyridin-2-amine

### (1) Synthesis of 6-((4-(3-chloro-2-fluorobenzoyl)piperazin-1-yl)methyl)-N-(3-(methoxymethyl)thiazol-2(3H)-ylidene)-4-(3-methyl-1,2,4-oxadiazol-5-yl)pyridin-2-amine

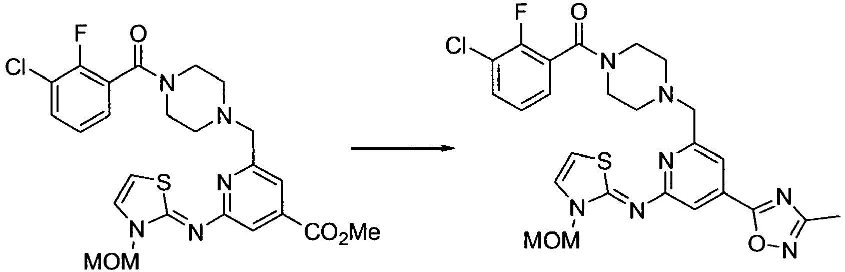

To a mixture of 9.4 mg ofN-hydroxyacetamidine, molecular sieves 4A and 1 ml of tetrahydrofuran was added sodium hydride followed by stirring at 65°C for 1 hour. To the reaction solution was added a solution of 22.6 mg of methyl 2-((4-(3-chloro-2-fluorobenzoyl)piperazin-1-yl)methyl)6-((3-(methoxymethyl)-thiazol-2(3H)-ylidene)amino)isonicotinate in 1 ml of tetrahydrofuran, followed by heating under reflux for 6 hours. The reaction solution was cooled to room temperature, diluted with ethyl acetate, and washed with saturated sodium bicarbonate and brine. The resulting organic layer was dried over magnesium sulfate and filtered, and the filtrate was concentrated. The resulting residue was purified by a thin-layer chromatography for fractionation (eluent: chloroform/methanol = 10/1) to give the title compound.

### (2) Synthesis of 6-((4-(3-chloro-2-fluorobenzoyl)piperazin-1-yl)methyl)-4-(3-methyl-1,2,4-oxadiazol-5-yl)-N-thiazol-2-ylpyridin-2-amine

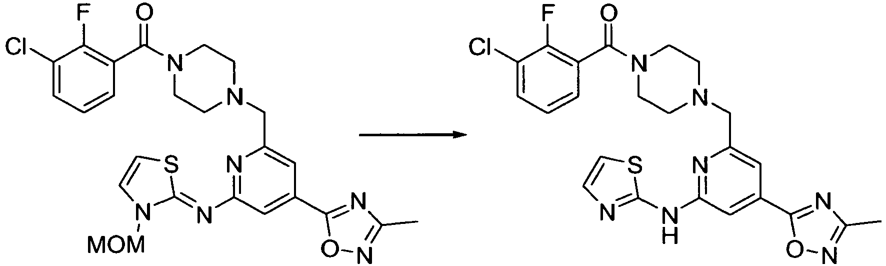

In the same manner as in Example 16-(4), the title compound was obtained using 6-((4-(3-chloro-2-fluorobenzoyl)piperazin-1-yl)methyl)-N-(3-(methoxymethyl)thiazol-2(3H)-ylidene)-4-(3-methyl-1,2,4-oxadiazol-5-yl)pyridin-2-amine.
Spectral data of the title compound are as follows.
¹H-NMR (CDCl₃) δ: 7.68 (s, 1H), 7.57-7.42 (m, 3H), 7.31-7.10 (m, 2H), 6.95-6.90 (m, 1H), 3.98-3.88 (m, 2H), 3.83 (s, 2H), 3.44-3.36 (m, 2H), 2.80-2.66 (m, 2H), 2.63-2.55 (m, 2H), 2.53 (s, 3H)
Mass: 514 (M + 1)⁺

### Example 111

### Synthesis of (2-((4-benzoylpiperazin-1-yl)methyl)-6-(1,2,4-thiadiazol-5-ylamino)pyridin-3-yl)methanol

### (1) Synthesis of (2,6-dibromopyridin-3-yl)methanol

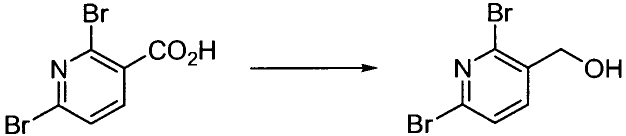

To a mixture of 1.51 g of 2,6-dibromonicotinic acid (Helvetica Chimica Acta, 1976, 59, 229), 0.72 ml of triethylamine and 30 ml of tetrahydrofuran was added 0.57 ml of ethyl chloroformate at 0°C. After stirring at room temperature for 30 minutes, an insoluble matter was filtered. To the filtrate was added 30 ml of tetrahydrofuran, and then 717 mg of sodium borohydride was added thereto. To this mixture was added 13 ml of methanol at 5°C followed by stirring for 15 minutes. The reaction mixture was diluted with ethyl acetate and then washed with an aqueous solution of saturated ammonium chloride and brine. The organic layer was dried over anhydrous magnesium sulfate and filtered, and the filtrate was concentrated to give the title compound.

### (2) Synthesis of 2,6-dibromo-3-(((tert-butyl(dimethyl)silyl)oxy)methyl)pyridine

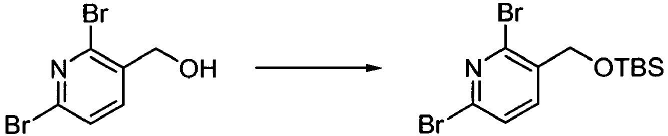

In the same manner as in Example 5-(1), the title compound was obtained using (2,6-dibromopyridin-3-yl)methanol.

### (3) Synthesis of 6-bromo-5-(((tert-butyl(dimethyl)silyl)oxy)methyl)-N-(1,2,4-thiadiazol-5-yl)pyridin-2-amine

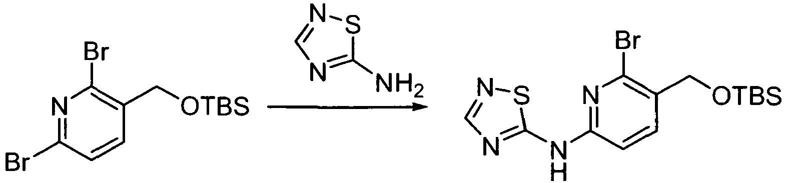

In the same manner as in Example 5-(2), the title compound was obtained using 2,6-dibromo-3-(((tert-butyl(dimethyl)silyl)oxy)methyl)pyridine and 1,2,4-thiadiazol-5-amine.

### (4) Synthesis of 6-(methanesulfonyloxymethyl)-5-(((tert-butyl(dimethyl)silyl)oxy)methyl)-N-(1,2,4-thiadiazol-5-yl)pyidin-2-amine

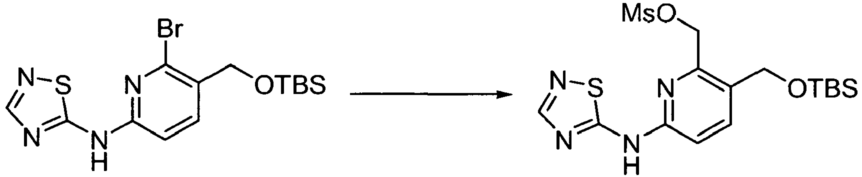

In the same manner as in Examples 1-(2), (5) and 16-(2), the title compound was obtained using 6-bromo-5-(((tert-butyl(dimethyl)silyl)oxy)methyl)-N-(1,2,4-thiadiazol-5-yl)pyridin-2-amine.

### (5) Synthesis of (2-((4-benzoytpiperazin-1-yl)methyl)-6-(1,2,4-thiadiazol-5-ylamino)pyridin-3-yl)methanol

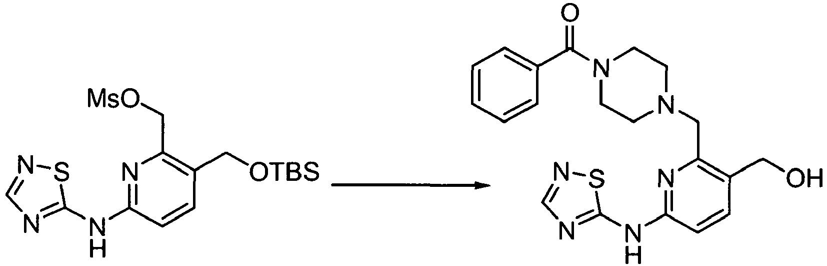

The amination reaction was performed in the same manner as in Example 16-(3) using 6-(methanesulfonyloxymethyl)-5-(((tert-butyl(dimethyl)silyl)oxy)methyl)-N-(1,2,4-thiadiazol-5-yl)pyridin-2-amine and 1-benzoylpiperazine hydrochloride obtained in the same manner as in Reference Example 1. Then, the deprotection reaction was performed in the same manner as in Example 5-(3) to give the title compound.
Spectral data of the title compound are as follows.
¹H-NMR (DMSO-d₆) δ: 12.11 (brs, 1H), 8.28 (s, 1H), 7.85 (d, J = 8.4 Hz, 1H), 7.45-7.35 (m, 5H), 7.07 (d, J = 8.4 Hz, 1H), 4.63 (s, 2H), 3.75 (s, 2H), 3.66-3.43 (m, 2H), 3.40-3.20 (m, 2H), 2.57-2.33 (m, 4H)
Mass: 411 (M+1)⁺

### Example 112

### Synthesis of 6-((4-(3-chloro-2-fluorobenzoyl)piperazin-1-yl)methyl)-N-(5-fluorothiazol-2-yl)pyridin-2-amine

### (1) Synthesis of 6-(((tert-butyl(dimethyl)silyl)oxy)methyl)-N-(3-(methoxymethyl)thiazol-2(3H)-ylidene)pyridin-2-amine

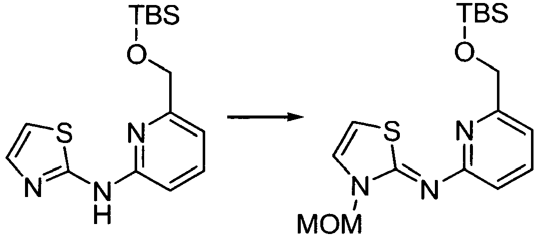

In the same manner as in Example 1-(3), the title compound was obtained using 6-(((tert-butyl(dimethyl)silyl)oxy)methyl)-N-thiazol-2-ylpyridin-2-amine obtained in Example 5-(2).

### (2) Synthesis of 6-(((tert-butyl(dimethyl)silyl)oxy)methyl)-N-(5-fluoro-3-(methoxymethyl)thiazol-2(3H)-ylidene)pyridin-2-amine

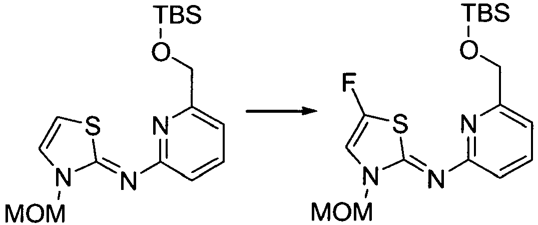

5 ml of tetrahydrofuran was cooled to -78°C, and then 1.05 ml of butyllithium (1.6 M, hexane solution) was dropped thereinto. Into the resulting reaction solution was dropped a solution of 280 mg of 6-(((tert-butyl(dimethyl)silyl)oxy)methyl)-N-(3-(methoxymethyl)thiazol-2(3H)-ylidene)pyridin-2-amine in 5 ml of tetrahydrofuran, followed by stirring at -78°C for 30 minutes. A solution of N-fluorobenzenesulfonimide in 5 ml of tetrahydrofuran was dropped thereinto. After having the temperature of the resulting reaction solution allowed to -30°C, 230 µl of acetic acid was dropped thereinto. The resulting reaction solution was diluted with ethyl acetate, and then washed with water and brine. The resulting organic layer was dried over anhydrous sodium sulfate and filtered, and the filtrate was concentrated *in vacuo.* The resulting residue was purified by a silica gel column chromatography (eluent: hexane to hexane/ethyl acetate = 3/1) to give the title compound.

### (3) Synthesis of 6-((4-(3-chloro-2-fluorobenzoyl)piperazin-1-yl)methyl)-N-(5-fluorothiazol-2-yl)pyidin-2-amine

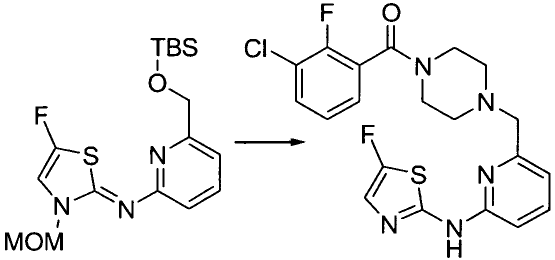

6-(((tert-Butyl(dimethyl)silyl)oxy)methyl)-N-(5-fluoro-3-(methoxymethyl)thiazol-2(3H)-ylidene)pyridin-2-amine was subjected to the process similar to Example 5-(3) and subsequently the processes similar to Examples 16-(2) to (4) to give the title compound.
Spectral data of the title compound are as follows.
Mass: 450 (M + 1)⁺

### Example 113

Synthesis of 4-((4-(3-chloro-2-fluorobenzoyl)piperazin-1-yl)methyl)-N-thiazol-2-ylpyrimidin-2-amine
¹H-NMR (CDCl₃) δ: 8.58 (d, J = 4.8 Hz, 1H), 7.55 (d, J = 3.2 Hz, 1H), 7.48-7.42 (m, 1H), 7.31-7.25 (m, 1H), 7.19-7.13 (m, 1H), 7.06 (d, J = 4.8 Hz, 1H), 6.90 (d, J = 3.2 Hz, 1H), 3.88 (brs, 2H), 3.70 (s, 2H), 3.39 (brs, 2H), 2.68 (t, J = 4.8 Hz, 2H), 2.55 (brs, 2H)
Mass: 433 (M + 1)⁺

### Example 114

### Synthesis of 6-((4-(2-fluoro-3-(trifluoromethyl)benzoyl)piperazin-1-yl)methyl)-N-(3 -methyl-1 H-pyrazol-5-yl)pyrazin-2-amine

¹H-NMR (CDCl₃) δ: 8.51 (s, 1H), 8.09 (s, 1H), 7.70-7.56 (m, 2H), 7.52 (s, 1H), 7.37-7.20 (m, 2H), 6.26 (s, 1H), 3.89 (brs, 2H), 3.64 (s, 2H), 3.37 (brs, 2H), 2.66 (dd, J = 5.1,4.9 Hz, 2H), 2.54 (brs, 2H)
Mass: 450 (M+1)⁺

### Example 115

### Synthesis of 6-(((1S,4S)-5-(3-chloro-2-fluorobenzoyl)-2,5-diazabicyclo[2.2.1]hept-2-yl)methyl)-N-(3-methyl-1H-pyrazol-5-yl)pyridin-2-amine

¹H-NMR (CDCl₃) δ: 8.45 (s, 1H), 8.08 (s, 1H), 7.47 (t, J = 6.8 Hz, 1H), 7.33 (t, J = 5.9 Hz, 1H), 7.19-7.14 (m, 2H), 6.07 (s, 1H), 4.94-2.71 (m, 8H), 2.32 (s, 3H), 2.07-1.99 (m, 1H), 1.88-1.79 (m, 1H)
Mass: 442 (M+1)⁺

### Example 116

### Synthesis of 2-{[4-(3-chloro-2-fluorobenzoyl)piperazin-1-yl]methyl}-N,N-dimethyl-6-(1H-pyrazol-3-ylamino)isonicotinamide trifluoroacetate

### (1) Synthesis of dimethyl 4-hydroxypyidine-2,6-dicarboxylate hydrochloride

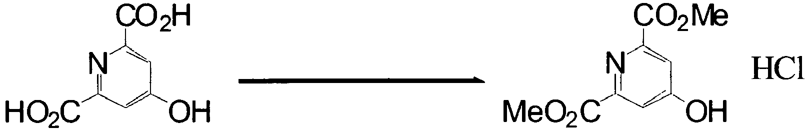

The tile compound was prepared from chelidamic acid in accordance with the method dislosed in J.Am.Chem. Soc.,70,3908(1948).

### (2) Synthesis of dimethyl 4-benzyloxypyridine-2,6-dicarboxylate

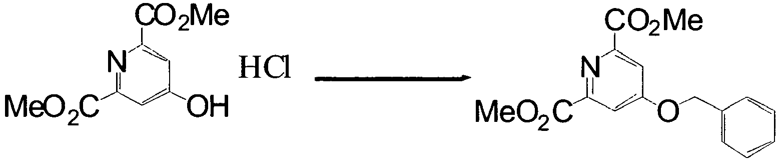

To a solution of 100 g of dimethyl 4-hydroxypyridine-2,6-dicarboxylate hydrochloride disloved in 500 ml of N,N-dimethylformamide, 122 g of potassium carbonate was added, and the mixture was stirred at room temperature for 1 hour. 52.5 ml of benzyl bromide was added to the resulting reaction mixture, and it was stirred at 50 °C overnight. The reaction mixture was poured into water; and then the resulting white solid was collected after filtration, washed with water, and dried in vacuo to afford the title compound.

### (3) Synthesis of monomethyl 4-benzyloxypyridine-2,6-dicarboxylate

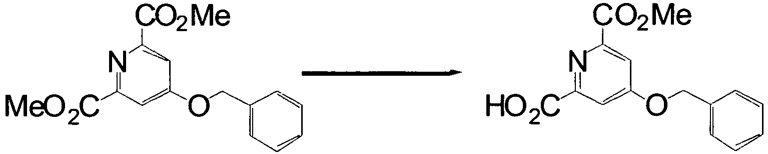

To a solution of 97 g of dimethyl 4-benzyloxypyridine-2,6-dicarboxylate dissolved in 1.51 of methanol, 18.0g of potassium hydroxide was added at 50 °C, and the mixture was stirred at 60 °C for 2 hours. The resulting reaction mixture was poured into a mixture of diethyl ether, hexane and water. The aqueous layer was separated, washed with diethyl ether., and neutralized with concentrated hydrochloric acid. The resulting white solid was collected after filtration, washed with water, and dried in vacuo to afford the title compound.

### (4) Synthesis of methyl 6-amino-4-(benzyloxy)pyridine-2-carboxylate

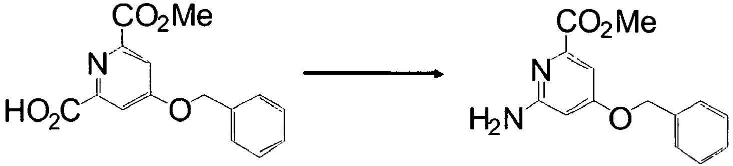

In accordance with the same manner as in Example 81-(3) and 81-(4), the title compound was obtained using monomethyl 4-benzyloxypyridine-2,6-dicarboxylate.

### (5) Synthesis of methyl 4-(benzyloxy)-6-chloropyridine-2-carboxylate

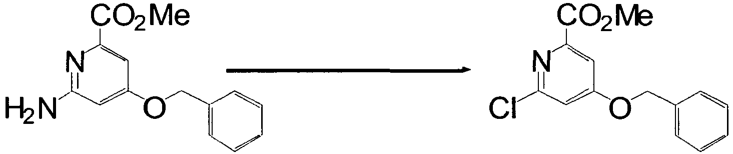

To a solution of 258 mg of methyl 6-amino-4-(benzyloxy)pyridine-2-carboxylate dissolved in 5 ml of chloroform, 170 mg of tert-butyl nitrate and 210 mg of copper(II) chloride were added, and the mixture was stirred for 6 hours with the light being shut out. Then saturated sodium bicarbonate was added to the resulting reaction mixture, and then the mixture was extracted with ethyl acetate. The organic layer was concentrated under a reduced pressure. The resulting residue was purified by flash chromatography (eluent: hexane/ethyl acetate) to yield the title compound.

### (6) Synthesis of methyl 4-(benzyloxy)-6-((1-((2-(trimethylsilyl)ethoxy)methyl)-1H-pyrazol-3-yl)amino)pyridine-2-carboxylate

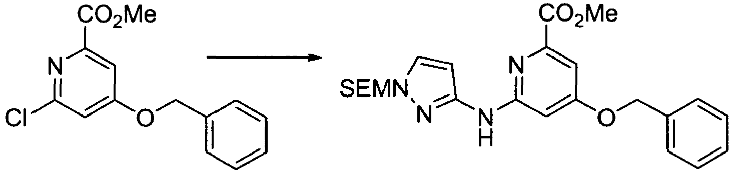

In accordance with the same manner as in Example 5-(2), the title compound was obtained using methyl 4-(benzyloxy)-6-chloropyridine-2-carboxylate and 1-((2-(trimethylsilyl)ethoxy)methyl)-1-H-pyrazol-3-amine obtained in Reference Example 2.

### (7) Synthesis of methyl 4-hydroxy-6-((1-((2-(trimethylsilyl)ethoxy)methyl)-1H-pyrazol-3-yl)amino)pyridine-2-carboxylate

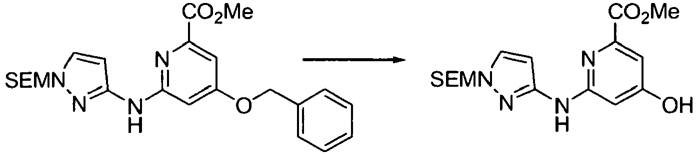

A mixture of 18 g of methyl 4-(benzyloxy)-6-((1-((2-(trimethylsilyl)ethoxy)methyl)-1H-pyrazol-3-yl)amino)pyridine-2-carboxylate dissolved in 200 ml of methanol with 200 ml of tetrahydrofuran was charged with 1.8 g of 20% palladium hydroxide on carbon catalyst, and the mixture was stirred at room temperature for 2 hours under hydrogen atmosphere at 1 atm. The reaction mixture was filtered and concentrated to give crude title compound.

### (8) Synthesis of 2-(hydroxymethyl)-6-((1-((2-(trimethylsilyl)ethoxy)methyl)-1H-pyrazol-3-yl)amino)pyridin-4-ol

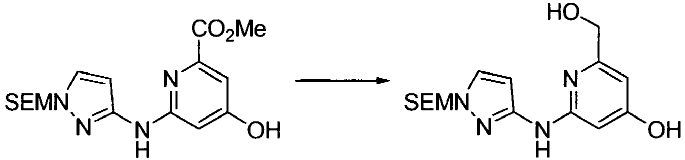

To 16 g of methyl 4-hydroxy-6-((1-((2-(trimethylsilyl)ethoxy)methyl)-1H-pyrazol-3-yl)amino)pyridine-2-carboxylate dissolved in 200 ml of tetrahydrofuran, 2.0 M of lithium borohydride dissolved in 30 ml of tetrahydrofuran solution was added, and the mixture was stirred at 60 °C for 1 hour. The resulting mixture was cooled to 0 °C, and then 10 % hydrochrolic adid in methanol was added thereto to adjust the pH of the solution to 4. After stirred 30 minutes at room temperature, the mixture was concentrated to give the title compound.

### (9) Synthesis of 2-(((tert-butyl(dimethyl)silyl)oxy)methyl)-6-((1-((2-(trimethylsilyl)ethoxy)methyl)-1H-pyrazol-3-yl)amino)pyridin-4-ol

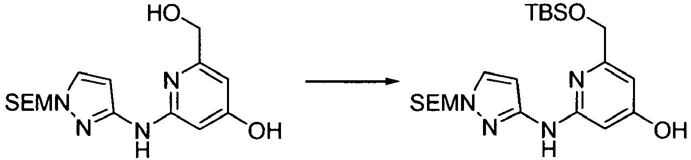

In the same manner as in Example 5-(1), the title compound was obtained using 2-(hydroxymethyl)-6-[(1-{[2-(trimethylsilyl)ethoxy]methyl}-1H-pyrazol-3-yl)amino]pyridin-4-ol.

### (10) Synthesis of 2-(((tert-butyl(dimethyl)silyl)oxy)methyl)-6-((1-((2-(trimethylsilyl)ethoxy]methyl)-1H-pyrazol-3-yl)amino)pyridin-4-yl trifluoromethanesulfonate

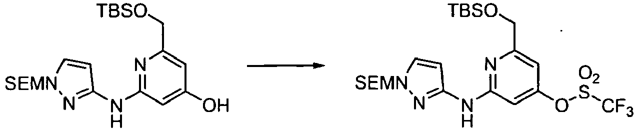

9.7 ml of trifluoromethanesulfonic anhydride was added to a mixture of 12.9 g of 2-(((tert-butyl(dimethyl)silyl)oxy)methyl)-6-((1-((2-(trimethylsilyl)ethoxy)methyl)-1H-pyrazol-3-yl)amino)pyridin-4-ol, 14.2 g of 4-(dimethylamino)pyridine and 290 ml of chloroform at 0 °C. The resulting mixture was stirred for 2 hours at room temperature and then diluted with ethyl acetate. The resulting solution was charged with 100 ml of 0.1 mol/l hydrochloric acid at 0 °C. The organic layer separated was washed with saturated sodium bicarbonate, water and brine. The resulting organic layer was dried over magnesium sulfate and filtered, and the filtrate was concentrated under a reduced pressure. The resulting residue was purified by a silica gel column chromatography (eluent: hexane/ethyl acetate = 10/1 to 1/1) to give the title compound.

### (11) Synthesis of methyl 2-(((tert-butyl(dimethyl)silyl)oxy)methyl)-6-((1-((2-(trimethylsilyl)ethoxy]methyl)-1H-pyrazol-3-yl)amino)isonicotinate

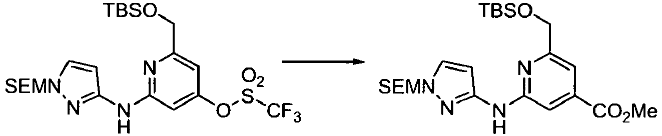

In the same manner as in Example 1-(2), the title compound was obtained using 2-(((tert-butyl(dimethyl)silyl)oxy)methyl)-6-((1-((2-(trimethylsilyl)ethoxy)methyl)-1H-pyrazol-3-yl)amino)pyridin-4-yl trifluoromethanesulfonate.

### (12) Synthesis of 2-(((tert-butyl(dimethyl)silyl)oxy)methyl)-N,N-dimethyl- 6-((1-((2-(trimethylsilyl)ethoxy)methyl)-1H-pyrazol-3-yl)amino)isonicotinamide

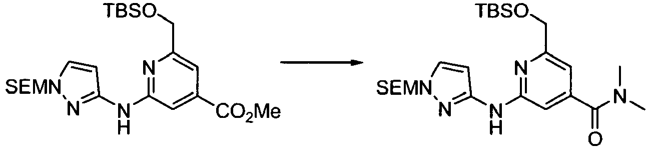

In the same manner as in Example 84, the title compound was obtained using methyl 2-(((tert-butyl(dimethyl)silyl)oxy)methyl)-6-((1-((2-(trimethylsilyl)ethoxy]methyl)-1H-pyrazol-3-yl)amino)isonicotinate.

### (13) Synthesis of 2-((4-(3-chloro-2-fluorobenzoyl)piperazin-1-yl)methyl)-N,N-dimethyl-6-(1H-pyrazol-3-ylamino)isonicotinamide

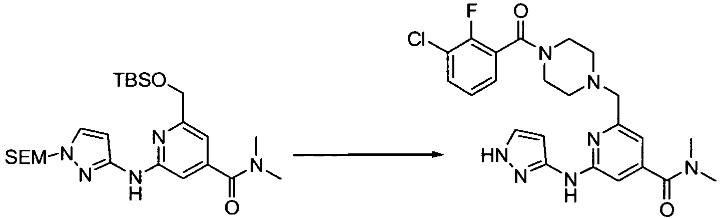

In the same manner as in Example 16, the title compound was obtained using 2-(((tert-butyl(dimethyl)silyl)oxy)methyl)-N,N-dimethyl-6-((1-((2-(trimethylsilyl)ethoxy)methyl)-1H-pyrazol-3-yl)amino)isonicotinamide.
Spectral data of the title compound are as follows.
¹H-NMR (CDCl₃) δ: 7.91 (brs, 1H), 7.47-7.41 (m, 2H), 7.29-7.24 (m, 1H), 7.15 (t, J=8.0Hz, 1H), 6.99 (brs, 1H), 6.78 (s, 1H),6.01 (brs, 1H), 3.85 (brs, 2H), 3.60 (s, 2H), 3.35 (brs, 2H), 3.10 (s, 3H), 2.95 (s, 3H), 2.61 (brt, J=5.2Hz, 2H), 2.48 (brs, 2H)
Mass: 486 (M + 1)⁺

Examples 117 to 121 were synthesized in the same manner as in Example 116 as follows.

### Example 117

Synthesis of 2-((4-(2-fluoro-3-(trifluoromethyl)benzoyl)piperazin-1-yl}methyl)-*N,N*-dimethyl-6-(1H-pyrazol-3-ylamino)isonicotinamide
¹H-NMR (CDCl₃) δ: 7.71-7.65 (m, 1H), 7.63-7.58 (m, 1H), 7.48 (brs, 1H), 7.33 (t, J=8.0Hz, 1H), 6.84 (s, 1H), 6.13 (brs, 1H), 3.90 (brs, 2H), 3.70 (s, 2H), 3.40 (brs, 2H), 3.11 (s, 3H), 2.98 (s, 3H), 2.74-2.67 (m, 2H), 2.59 (brs, 2H)
Mass: 520 (M + 1)⁺

### Example 118

Synthesis of 6-((4-(2-fluoro-3-(trifluoromethyl)benzoyl)piperazin-1-yl)methyl)-4-((4-methylpiperazin-1-yl)carbonyl)-*N*-1*H*-pyrazol-3-ylpyridin-2-amine trifluoroacetate
¹H-NMR (CD₃OD) δ: 7.85 (brt, J=6.8Hz, 1H), 7.77 (d, J=2.4Hz, 1H), 7.74 (brt, J=7.2Hz, 1H), 7.49 (t, J=8.0Hz, 1H), 7.19 (s, 1H), 7.01 (s, 1H), 6.21 (d, J=2.4Hz, 1H), 4.23 (s, 2H), 4.06 (brs, 2H), 3.63 (brs, 2H), 3.18 (brs, 2H), 3.06 (brs, 2H)2.95 (s, 3H)
Mass: 575 (M + 1)⁺

### Example 119

### Synthesis of 6-((4-(2-fluoro-3-(trifluoromethyl)benzoyl)piperazin-1-yl)methyl)-4-(piperazin-1-ylcarbonyl)-N-1H-pyrazol-3-ylpyridin-2-amine trifluoroacetate

¹H-NMR (CD₃OD) δ: 7.85 (brt, J=7.2Hz, 1H), 7.79 (d, J=2.4Hz, 1H), 7.74 (brt, J=6.4Hz, 1H), 7.49 (t, J=8.0Hz, 1H), 7.18 (s, 1H), 7.03 (s, 1H), 6.22 (d, J=2.4Hz, 1H), 4.24 (s, 2H), 4.06 (brs, 2H), 3.98 (brs, 2H), 3.71 (brs, 2H), 3.63 (brs, 2H), 3.19 (brs, 2H), 3.07 (brs, 2H)
Mass: 561 (M + 1)⁺

### Example 120

### Synthesis of 6-((4-(2-fluoro-3-(trifluoromethyl)benzoyl)piperazin-1-yl)methyl)-N-1H-pyrazol-3-yl-4-((4-pyridin-2-ylpiperazin-1-yl)carbonyl)pyridin-2-amine trifluoroacetate

¹H-NMR (CD₃OD) δ: 8.08 (ddd, J=9.2, 7.2, 1.6Hz, 1H), 8.01 (dd, J=6.4, 1.6Hz, 1H), 7.85 (brt, J=7.2Hz, 1H), 7.79 (d, J=2.4Hz, 1H), 7.74 (brt, J=6.8Hz, 1H), 7.49 (t, J=8.0Hz, 1H), 7.39 (d, J=9.2Hz, 1H), 7.19 (s, 1H), 7.08-7.02 (m, 2H), 6.22 (d, J=2.4Hz, 1H), 4.24 (s, 2H), 4.06 (s, 2H), 3.96 (s, 2H), 3.89 (s, 2H), 3.76 (brs, 2H), 3.73 (brs, 2H), 3.63 (brs, 2H), 3.18 (brs, 2H), 3.07 (brs, 2H)
Mass: 638 (M + 1)⁺

### Example 121

### Synthesis of 6-((4-(2-fluoro-3-(trifluoromethyl)benzoyl)piperazin-1-yl)methyl)-4-(morpholin-4-ylcarbonyl)-N-1H-pyrazol-3-ylpyridin-2-amine trifluoroacetate

¹H-NMR (CD₃OD) δ: 7.85 (brt, J=7.2Hz, 1H), 7.79 (d, J=2.4Hz, 1H), 7.73 (brt, J=6.8Hz, 1H), 7.49 (t, J=7.6Hz, 1H), 7.09 (s, 1H), 7.00 (s, 1H), 6.20 (d, J=2.4Hz, 1H), 4.19 (s, 2H), 4.05 (brs, 2H), 3.75 (brs, 4H), 3.66-3.58 (m, 4H), 3.47-3.41 (m, 2H), 3.16-3.10 (m, 2H), 3.03-2.97 (m, 2H)
Mass: 562 (M + 1)⁺

### Example 122

### Synthesis of 2-((4-(2-fluoro-3-(trifluoromethyl)benzoyl)piperazin-1-yl)methyl)-6-(1H-pyrazol-3-ylamino)isonicotinonitrile

In the same manner as in Example 104-(2) to (3), the title compound was obtained using 2-(((tert-butyl(dimethyl)silyl)oxy)methyl)-6-((1-((2-(trimethylsilyl)ethoxy]methyl)-1H-pyrazol-3-yl)amino)pyridin-4-yl trifluoromethanesulfonate obtained in Example 116-(10).
Spectral data of the title compound are as follows.
¹H-NMR (CDCl₃) δ: 7.71 (brs, 1H), 7.68 (brt, J=7.6Hz, 1H), 7.61 (brt, J=6.4Hz, 1H), 7.51 (brd, J=2.0Hz, 1H), 7.46 (brs, 1H), 7.33 (t, J=7.6Hz, 1H), 7.05 (brs, 1H), 6.15 (brs, 1H), 3.89 (brs, 2H), 3.62 (s, 2H), 3.38 (brs, 2H), 2.64 (brt, J=5.2Hz, 2H), 2.52 (brs, 2H)
Mass: 474 (M + 1)⁺

### Example 123

### Synthesis of 6-((4-(2-fluoro-3-(trifluoromethyl)benzoyl)piperazin-1-yl)methyl)-4-(2-methyl-2H-tetrazol-5-yl)-N-1H-pyrazol-3-ylpyridin-2-amine

In the same manner as in Example 106, the title compound was obtained using 2-(((tert-butyl(dimethyl)silyl)oxy)methyl)-6-((1-((2-(trimethylsilyl)ethoxy]methyl)-1H-pyrazol-3-yl)amino)isonicotinonitrile obtained in Example 122.
Spectral data of the title compound are as follows.
¹H-NMR (CDCl₃) δ: 7.71 (brs, 1H), 7.68 (brt, J=7.6Hz, 1H), 7.61 (brt, J=6.4Hz, 1H), 7.51 (brd, J=2.0Hz, 1H), 7.46 (brs, 1H), 7.33 (t, J=7.6Hz, 1H), 7.05 (brs, 1H), 6.15 (brs, 1H), 3.89 (brs, 2H), 3.62 (s, 2H), 3.38 (brs, 2H), 2.64 (brt, J=5.2Hz, 2H), 2.52 (brs, 2H)
Mass: 474 (M + 1)⁺

### Example 124

### Synthesis of (thiazol-2-yl)-(6-(4-(2,3-difluorobenzoyl)-piperazin-l-ylmethyl)-pyridin-2-yl)-amine hydrochloride

A mixture of 293 mg of (thiazol-2-yl)-(6-(4-(2,3-difluorobenzoyl)-piperazin-1-ylinethyl)-pyridin-2-yl)-amine (Example 4) with 1 ml of methanol was charged with a hydrochloric acid-1,4-dioxane solution (4 M, 3 ml), followed by stirring at room temperature for 1 hour. The reaction mixture was concentrated *in vacuo* and the residue was suspended in diethyl ether, filtered and collected to give the title compound.
Spectral data of the title compound are as follows.
¹H-NMR (DMSO-d₆) δ: 7.88-7.81 (m, 1H), 7.60-7.49 (m, 1H), 7.49-7.43 (m, 1H), 7.36-7.25 (m, 3H), 7.21-7.10 (m, 2H), 4.43 (s, 2H), 3.74-3.23 (m, 8H)
Mass: 416 (M + 1)⁺

### Example 125

### Synthesis of 6-((4-(3-chloro-2-fluorobenzoyl)piperazin-1-yl)methyl)-N-thiazol-2-ylpyridin-2-amine hydrochloride

5.09 g of 6-((4-(3-chloro-2-fluorobenzoyl)piperazin-1-yl)methyl)-N-thiazol-2-ylpyridin-2-amine (Example 5) was suspended in 100 ml of ethanol , and aqueous hydrogen chloride solution(1.0 M, 11.8 ml) was added thereto at room temperature. The reaction mixture was stirred at 80°C for 30 minutes and then cooled and evaporated. The resulting residue was dissolved in 250 ml of ethanol by heating under reflux. Then stirring and heating were stopped and the solution was cooled slowly to room temperature. The precipitate was filtered and dried to give 6-((4-(3-chloro-2-fluorobenzoyl)piperazin-1-yl)methyl)-N-thiazol-2-ylpyidin-2-amine hydrochloride as crystal.
Spectral data of the title compound are as follows.
¹H-NMR (DMSO-d₆) δ: 11.49 (brs, 1H), 7.79 (t, J = 7.8 Hz, 1H), 7.73-7.67 (m, 1H), 7.47-7.41 (m, 2H), 7.32 (t, J = 7.8 Hz, 1H), 7.27 (d, J = 7.0 Hz, 1H), 7.11 (d, J = 8.4 Hz, 1H), 7.07 (d, J = 3.5 Hz, 1H), 4.39 (s, 2H), 3.69-3.20 (m, 8H)
Mass: 432 (M + 1)⁺
m.p.: 141-167 °C(ethanol)

### Example 126

### Synthesis of 6-((4-(3-chloro-2-fluorobenzoyl)piperazin-1-yl)methyl)-N-1H-pyrazol-3-ylpyridin-2-amine hydrochloride

In the same manner as in Example 124, the title compound was obtained using the compound of Example 16.
Spectral data of the title compound are as follows.
¹H-NMR (DMSO-d₆) δ: 10.98 (brs, 1H), 7.91 (d, J = 2.3 Hz, 1H), 7.82 (dd, J = 8.5, 7.3 Hz, 1H), 7.77-7.65 (m, 1H), 7.47-7.41 (m, 1H), 7.39-7.30 (m, 1H), 7.15 (d, J = 8.6 Hz, 1H), 7.09 (d, J = 7.0 Hz, 1H), 6.27 (d, J = 2.5 Hz, 1H), 4.34 (s, 2H), 4.30-3.50 (m, 4H), 3.23 (brs, 2H), 3.11 (brs, 2H)
Mass: 415 (M + 1)⁺

### Example 127

### Synthesis of 6-((4-(3-chloro-2-fluorobenzoyl)piperazin-1-yl)methyl)-N-(5-methyl-1H-pyrazol-3-yl)pyridin-2-amine hydrochloride

In the same manner as in Example 124, the title compound was obtained using the compound of Example 25.
Spectral data of the title compound are as follows.
¹H-NMR (CD₃OD) δ: 7.88 (dd, J = 8.0, 7.8 Hz, 1H), 7.66-7.60 (m, 1H), 7.43-7.3 (m, 1H), 7.30 (bt, J = 8.8 Hz, 1H), 7.16 (d, J = 7.8 Hz, 1H), 7.05 (d, J = 8.0 Hz, 1H), 6.02 (s, 1H), 4.40 (bs, 2H), 3.73 (bs, 2H), 3.37-3.14 (m, 6H), 2.41 (s, 3H)
Mass: 429 (M + 1)⁺

### Example 128

### Synthesis of 6-((4-(2-fluoro-3 -(trifluoromethyl)benzoyl)piperazin-1-yl)methyl)-N-1H-pyrazol-3-ylpyrazin-2-amine hydrochloride

19.8 g of 6-((4-(2-fluoro-3-(trifluoromethyl)benzoyl)piperazin-1-yl)methyl)-N-1H-pyrazol-3-ylpyrazin-2-amine (Example 44) was suspended in 200 ml of ethanol, and aqueous hydrogen chloride solution(1.0 M, 44.1 ml) was added thereto. The reaction mixture was stirred at room temperature for 1 hour and then evaporated. The resulting residue was solidified with 200ml of heptane, evaporated and dried to give 6-((4-(4-fluoro-3-(trifluoromethyl)benzoyl)piperazin-1-yl)methyl)-N-1H-pyrazol-3-ylpyrazin-2-amine hydrochloride.
Spectral data of the title compound are as follows.
¹H-NMR (DMSO-d₆) δ: 10.10 (s, 1H), 8.45 (s, 1H), 8.12 (s, 1H), 7.91 (t, J = 7.3 Hz, 1H), 7.82 (t, J = 7.0 Hz, 1H), 7.64-7.61 (m, 1H), 7.52 (t, J = 7.7 Hz, 1H), 6.54 (s, 1H), 4.37 (s, 2H), 3.66-3.18 (m, 8H)
Mass: 450 (M + 1)⁺

### Example 129

### Synthesis of 6-((4-(3-chloro-2-fluorobenzoyl)piperazin-1-yl)methyl)-N-(5-methyl-1H-pyrazol-3-yl)pyrazin-2-amine hydrochloride

In the same manner as in Example 124, the title compound was obtained using the compound of Example 56.
Spectral data of the title compound are as follows.
¹H-NMR (CD₃OD) δ: 8.41 (s, 1H), 8.31 (s, 1H), 7.67-7.61 (m, 1H), 7.42 (t, J= 6.8 Hz, 1H), 7.31 (t, J = 7.8 Hz, 1H), 6.17 (s, 1H), 4.57 (s, 2H), 3.83-3.77 (m, 2H), 3.76-3.25 (m, 6H), 2.44 (s, 3H)
Mass: 430 (M + 1)⁺

### Example 130

Synthesis of 6-((4-(3-chloro-2-fluorobenzoyl)piperazin-1-yl)methyl)-N-(5-chlorothiazol-2-yl)pyridin-2-amine hydrochloride
In the same manner as in Example 124, the title compound was obtained using the compound of Example 75.
Spectral data of the title compound are as follows.
¹H-NMR (DMSO-d₆) δ: 11.68 (brs, 1H), 7.83 (dd, J = 8.0, 7.6 Hz, 1H), 7.70 (dd, J = 7.4, 7.0 Hz, 1H), 7.50-7.30 (m, 4H), 7.09 (d, J = 8.4 Hz, 1H), 4.44 (s, 2H), 3.90-3.18 (m, 8H)
Mass: 466 (M + 1)⁺

### Example 131

### Synthesis of 2-((4-(2-fluoro-3-(trifluoromethyl)benzoyl)piperazin-1-yl}methyl)-N,N-dimethyl-6-(1H-pyrazol-3-ylamino)isonicotinamide hydrochloride

In the same manner as in Example 128, the title compound was obtained using the compound of Example 117.
Spectral data of the title compound are as follows.
¹H-NMR (CD₃OD) δ: 7.85 (brt, J = 7.2 Hz, 1H), 7.82 (d, J = 2.4 Hz, 1H), 7.77-7.71 (m, 1H), 7.49 (brt, J = 8.0 Hz, 1H), 7.07 (s, 1H), 7.02 (s, 1H), 6.21 (d, J = 2.4 Hz, 1H), 4.21 (s, 2H), 4.07 (brs, 2H), 3.67-3.60 (m, 2H), 3.17-3.08 (m, 5H), 3.04-2.97 (m, 5H)
Mass: 520 (M + 1)⁺

### [Reference Example]

### Reference Example 1

### Synthesis of 1-(3-chloro-2-fluorobenzoyl)piperazine hydrochloride

### (1) Synthesis oftert-butyl 4-(3-chloro-2-fluorobenzoyl)piperazine-1-carboxylate

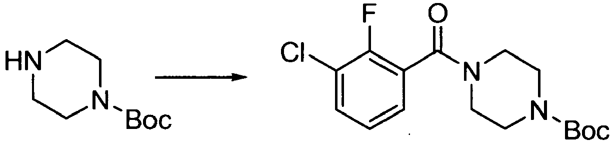

A mixture of 19.4 g of 1-Boc-piperazine, 24.0 g of 1-(3-dimethylaminopropyl)-3-ethylcarbodiimide hydrochloride, 19.1 g of 1-hydroxybenzotriazole, 20.0 g of 3-chloro-2-fluorobenzoic acid and 200 ml of chloroform was stirred at room temperature for 4 hours. The resulting mixture was diluted with chloroform, and an insoluble matter was filtered off using Celite. Then, the filtrate was washed with water and brine. The resulting organic layer was dried over magnesium sulfate and filtered, and the filtrate was concentrated *in vacuo.* The resulting residue was purified by a silica gel column chromatography (eluent: hexane/ethyl acetate = 9/1 to 1/1) to give title compound.

### (2) Synthesis of 1-(3-chloro-2-fluorobenzoyl)piperazine hydrochloride

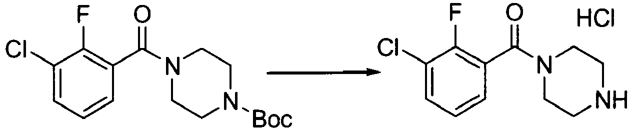

To a mixture of 35.1 g of tert-butyl 4-(3-chloro-2-fluorobenzoyl)piperazine-1-carboxylate and 50 ml of methanol was added a hydrochloric acid-1,4-dioxane solution (4 M, 100 ml) followed by stirring at room temperature for 2.5 hours. The reaction mixture was concentrated *in vacuo* and the residue was suspended in diethyl ether, filtered and collected to give the title compound.
Spectral data of the title compound are as follows.
¹H-NMR (DMSO-d₆) δ: 9.68 (br ,1 H) ,7.74-7.65s (m, 1H), 7.50-7.41 (m, 1H), 7.37-7.28 (m, 1H), 3.88 (br, 2H), 3.57-3.30 (m, 2H), 3.16 (br, 2H), 3.03 (br, 2H)
Mass: 243 (M + 1)⁺

### Reference Example 2

### Synthesis of 1-((2-(trimethylsilyl)ethoxy)methyl)-1H-pyrazol-3-amine

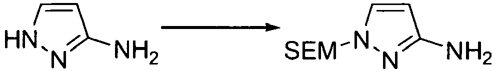

To a solution of 10 g of 1 H-pyrazol-3-amine in 100 ml of N,N-dimethylformamide was added 9.6 g of sodium hydride (60%, in oil) under cooling with ice. The reaction mixture was stirred for 30 minutes, and then 21.3 ml of 2-(trimethylsilyl)ethoxymethyl chloride was added thereto. After stirring the resulting mixture at room temperature for 1 hour, aqueous ammonium chloride was added thereto, and the mixture was extracted with chloroform. The resulting organic layer was washed with water and brine, and then dried over magnesium sulfate. The organic layer was filtered and concentrated *in vacuo.* The resulting residue was purified by a silica gel column chromatography (eluent: hexane/ethyl acetate = 4/1 to 1/2) to give the title compound.

### Reference Example 3

### Synthesis of 5-methyl-1-((2-(trimethylsilyl)ethoxy)methyl)-1H-pyrazol-3-amine

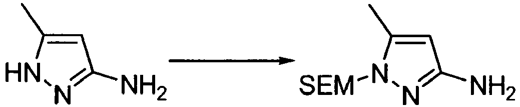

In accordance with the manner of Reference Example 2, the title compound was obtained from 5-methyl-1H-pyrazol-3-amine.

### Reference Example 4

### Synthesis of 5-cyclopropyl-1-((2-(trimethylsilyl)ethoxy)methyl)-1-H-pyrazol-3-amine or 3-cyclopropyl-1-((2-(trimethylsilyl)ethoxy)methyl)-1H-pyrazol-5-amine

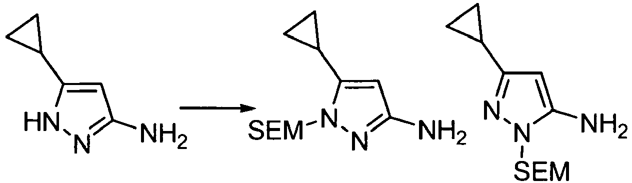

In accordance with the manner of Reference Example 2, the title compound was obtained from 5-cyclopropyl-1H-pyrazol-3-amine.
Spectral data of the title compound are as follows.
¹H-NMR (CDCl₃) δ: 5.31 (s, 2H), 5.26 (s, 1H), 3.62-3.56 (m, 2H), 1.84-1.76 (m, 1H), 0.97-0.88 (m, 4H), 0.67-0.62 (m, 2H), -0.02s (s, 9H)
Mass: 254 (M + 1)⁺

### Reference Example 5

### Synthesis of 6-(tetrahydro-2H-pyran-2-yloxy)-1,4-diazepane

### (1) Synthesis of 2-(2-bromo-1-(bromomethyl)ethoxy)tetrahydro-2H-pyran

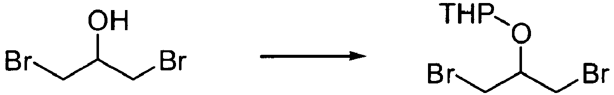

To a mixture of 10 g of 1,3-dibromopropan-2-ol, 5.0 ml of 3,4-dihydro-2H-pyran and 50 ml of chloroform was added a catalytic amount of p-toluenesulfonic acid monohydrate followed by stirring at room temperature for 3 hours. The reaction solution was concentrated, diluted with diethyl ether and washed with saturated sodium bicarbonate and brine. The resulting organic layer was dried over magnesium sulfate and filtered, and the filtrate was concentrated to give the title compound.

### (2) Synthesis of 1,4-dibenzyl-6-(tetrahydro-2H-pyran-2-yloxy)-1,4-diazepane

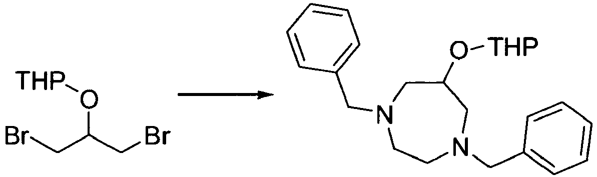

A mixture of 2-(2-bromo-1-(bromomethyl)ethoxy)tetrahydro-2H-pyran obtained above, 10.8 ml of N,N'-dibenzylethane-1,2-diamine, 19.0 g of potassium carbonate and 50 ml ofN,N'-dimethylformamide was stirred at 120°C for 3 hours. The reaction solution was diluted with diethyl ether and washed with water and brine. The resulting organic layer was dried over magnesium sulfate and filtered, and the filtrate was concentrated. The resulting residue was purified by a silica gel column chromatography (eluent: hexane to hexane/ethyl acetate = 4/1) to give the title compound.

### (3) Synthesis of 6-(tetrahydro-2H-pyran-2-yloxy)-1,4-diazepane

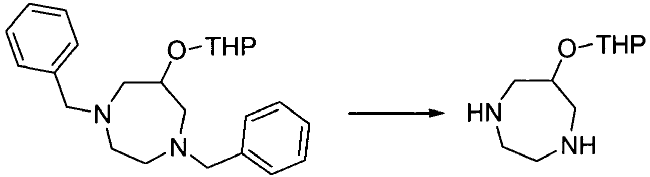

3.06 g of 1,4-dibenzyl-6-(tetrahydro-2H-pyran-2-yloxy)-1,4-diazepane was dissolved in 50 ml of methanol and 1 g of a 20% palladium hydroxide on carbon catalyst was added thereto. The resulting mixture was stirred under a hydrogen atmosphere at ordinary pressure and room temperature for 5 hours. The reaction solution was filtered and the solvent was concentrated to give the title compound.

### Reference Example 6

### Synthesis of 1-(2-fluoro-3-(difluoromethyl)benzoyl)piperazine hydrochloride

### (1) Synthesis of tert-butyl 4-(3-bromo-2-fluorobenzoyl)piperazine-1-carboxylate

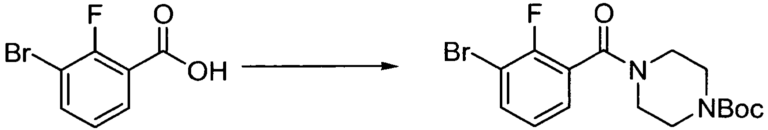

To a mixture of 220 mg of 3-bromo-2-fluorobenzoic acid, 199 mg of tert-butyl piperazine-1-carboxylate and 5.0 ml of chloroform was added 171 mg of hydroxybenzotriazole monohydrate and 215 mg of 1-(3-dimethylaminopropyl)-3-ethylcarbodiimide hydrochloride under cooling with ice, successively. After stirring at room temperature for 4 hours, saturated sodium bicarbonate was added to the reaction mixture, and the resulting mixture was extracted with chloroform. The organic layer was dried over anhydrous magnesium sulfate and filtered, and the filtrate was concentrated. The residue was purified by a silica gel column chromatography (eluent: hexane/ethyl acetate = 9/1 to 1/1) to give the title compound.

### (2) Synthesis of tert-butyl 4-(2-fluoro-3-formylbenzoyl)piperazine-1-carboxylate

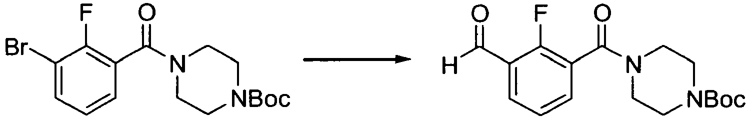

194 mg of tert-butyl 4-(3-bromo-2-fluorobenzoyl)piperazine-1-carboxylate was dissolved in 5.0 ml of tetrahydrofuran, and 1.25 ml of a solution of isobutylmagnesium chloride in tetrahydrofuran (2.0 M) was added thereto at -78°C. After stirring at the same temperature for 2 hours, 0.39 ml (5.0 mmol) of N,N-dimethylformamide was added thereto. The resulting mixture was stirred at the same temperature for 30 minutes and further stirred at 0°C for 30 minutes. To the reaction mixture was added saturated ammonium chloride, and the mixture was extracted with chloroform. The organic layer was washed with brine and then concentrated. The residue was purified by a silica gel column chromatography (eluent: hexane/ethyl acetate = 9/1 to 1/1) to give the title compound.

### (3) Synthesis oftert-butyl 4-(3-difluoromethyl-2-fluorobenzoyl)piperazine-1-carboxylate

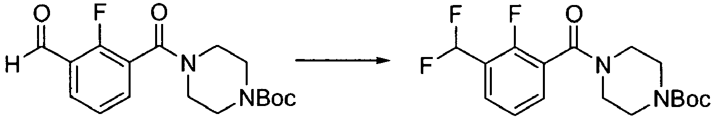

130 mg of tert-butyl 4-(2-fluoro-3-formylbenzoyl)piperazine-1-carboxylate was dissolved in 4.8 ml of dichloromethane, and 0.13 ml of diethylaminosulfur trifluoride was added thereto at room temperature. After stirring at the same temperature for 2 hours, 0.13 ml of diethylaminosulfur trifluoride was added thereto and the resulting mixture was further stirred at the same temperature for 2 hours. The reaction mixture was purified by a silica gel column chromatography (eluent: hexane/ethyl acetate = 9/1 to 1/1) to give the title compound.

### (4) Synthesis of 1-(3-(difluoromethyl)-2-fluorobenzoyl)piperazine hydrochloride

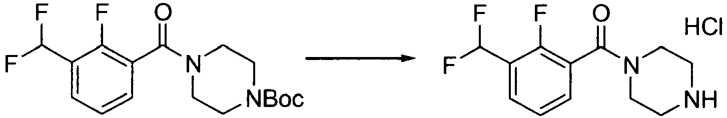

In accordance with the manner of Reference Example 1-(2), the title compound was obtained from tert-butyl 4-(3-(difluoromethyl)-2-fluorobenzoyl)piperazine-1-carboxylate.

### Reference Example 7

### Synthesis of 1-(2-fluoro-3-(trifluoromethyl)benzoyl)piperazine hydrochloride

### (1) Synthesis of tert-butyl 4-(2-fluoro-3-(trifluoromethyl)benzoyl)piperazine-1-carboxylate

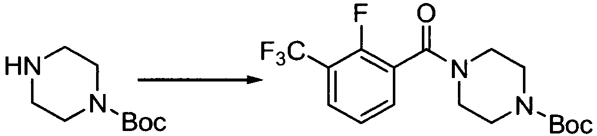

A mixture of 3.66 g of 1-Boc-piperazine, 4.53 g of 1-(3-dimethylaminopropyl)-3-ethylcarbodiimide hydrochloride, 3.61 g of 1-hydroxybenzotriazole, 4.50 g of 2-fluoro-3-(trifluoromethyl)benzoic acid and 40 ml of chloroform was stirred at room temperature for 4 hours. The reaction mixture was diluted with chloroform and then was washed with water and brine. The resulting organic layer was dried over magnesium sulfate and filtered, and the filtrate was concentrated *in vacuo.* The resulting residue was purified by a silica gel column chromatography (eluent: hexane/ethyl acetate = 4/1 to 2/1) to give the title compound.

### (2) Synthesis of 1-(2-fluoro-3-(trifluoromethyl)benzoyl)piperazine hydrochloride

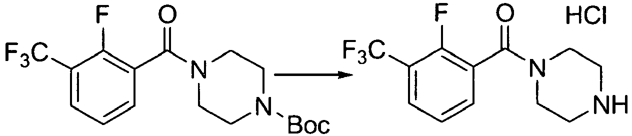

To a mixture of 7.74 g of tert-butyl 4-(2-fluoro-3-(trifluoromethyl)benzoyl)piperazine-1-carboxylate and 10 ml of methanol was added a hydrochloric acid-1,4-dioxane solution (4 M, 20 ml) followed by stirring at room temperature for 4 hours. The reaction mixture was concentrated *in vacuo* to give the title compound.
Spectral data of the title compound are as follows.
¹H-NMR (DMSO-d₆) δ: 7.95-7.81 (m, 2H), 7.56-7.49 (m, 1H), 3.92-3.79 (m, 2H), 3.45 (br, 2H), 3.19 (br, 2H), 3.04 (br, 2H)
Mass: 277 (M + 1)⁺

### Reference Example 8

### Synthesis of tert-butyl 4-(3-cyclopropyl-2-fluorobenzoyl)piperazine-1-carboxylate

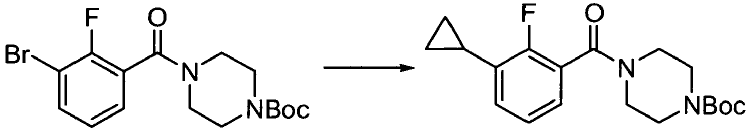

To a solution of 107 mg of tert-butyl 4-(3-bromo-2-fluorobenzoyl)piperazine-1-carboxylate in 2.0 ml of toluene and 0.1 ml of water was added 72 mg of cyclopropylboronic acid, 3.2 mg of palladium acetate, 0.052 ml of tricyclohexylphosphine (15% toluene solution) and 207 mg of potassium phosphate, successively. The reaction solution was stirred at 150°C for 10 minutes using a microwave reaction apparatus. Water was added to the reaction mixture, and the mixture was extracted with ethyl acetate. The organic layer was washed with brine and then concentrated. The residue was purified by a silica gel column chromatography (eluent: hexane to hexane/ethyl acetate = 1/1) to give the title compound.

### Industrial Applicability

The compound of the invention is characterized in that it has cell growth inhibitory action as well as synergistic action with other antitumor agents, based on excellent Aurora A selective inhibitory action, and thus it is expected as a useful antitumor agent in the field of pharmaceuticals.

## Claims

1. A compound of general formula I: 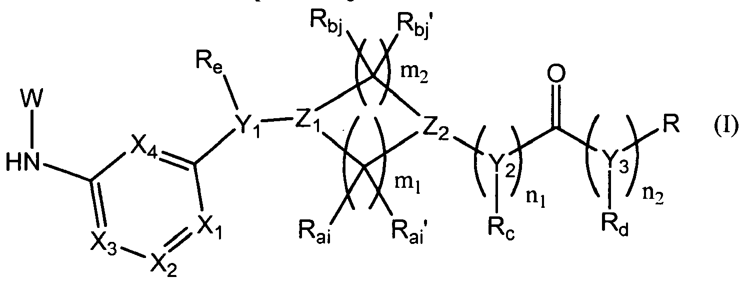 wherein:
m₁ is 2 or 3;
m₂ is 2;
n₁ is 0;
n₂ is 0;
i is an integer of any of 1 to m₁;
j is an integer of any of 1 to m₂;
R is phenyl of which 2^{nd} and 3^{rd} positions are substituted with the same or different halogen atoms or alternatively substituted with halogen atom and methyl substituted with one to three of the same or different halogen atoms, respectively;
Rₐᵢ and Rₐᵢ' are each independently hydrogen atom and lower alkyl;
R_{bj} and R_{bj}' are each independently hydrogen atom and lower alkyl;
wherein:
i is i₀ wherein io is an integer of any 1 to m₁, and j is j₀ wherein jo is an integer of any 1 to m₂, so that one of Rₐᵢ₀ and Rₐᵢ₀' and one of R_{bj0} and R_{bj0}' may be combined to form -(CH₂)ₙ- wherein n is 1 or 2; and
Rₑ is a hydrogen atom or lower alkyl;
X₁ is CH, CX₁ₐ, or N wherein X₁ₐ is lower alkyl;
X₂ is CH or N;
X₃ is CH;
X₄ is N;
the number of nitrogen atoms among X₁, X₂ and X₄ is one or two;
Y₁, is CH or N; however, if Y₁ is CH and Rₑ is hydrogen atom, then the two hydrogen atoms may be substituted with oxo;
Z₁ is N and Z₂ is CH or N;
W is selected from: 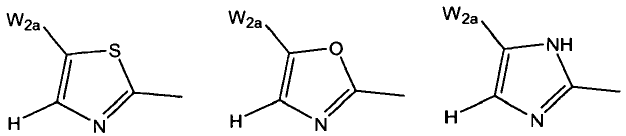 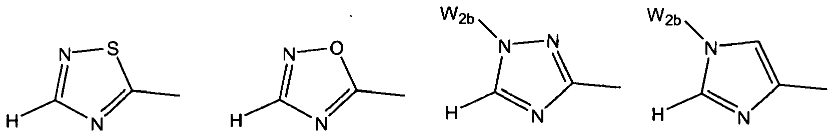 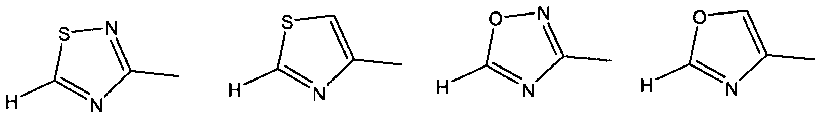 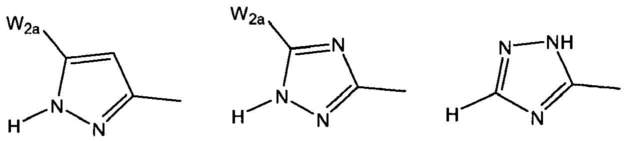 wherein W₂ₐ and W_{2b} are each independently hydrogen atom, halogen atom, cyano, C₁₋₂ lower alkyl, C₃₋₅ cycloalkyl, or C₁₋₂ lower alkyl which may be substituted with one or more halogen atoms; and wherein 'lower alkyl' means a linear or branched alkyl group having 1 to 6 carbon atoms;
with the proviso that when W is: 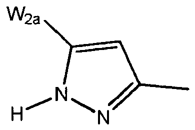 then X, is CH or CX₁ₐ and X₂ is N;
or a pharmaceutically acceptable salt thereof;

2. The compound according to Claim 1 or a pharmaceutically acceptable salt thereof, wherein:
Y₁ is CH; and
Rₑ is hydrogen atom.

3. The compound according to Claim 1 or 2 or a pharmaceutically acceptable salt thereof, wherein:
m₁ is 2; and
Rₐᵢ, Rₐᵢ', Rₐ₂, Rₐ₂', R_{b1}, R_{b1}', R_{b2}, and R_{b2}' are hydrogen atom.

4. The compound according to Claim 1, 2 or 3 or a pharmaceutically acceptable salt thereof, wherein:
W is selected from: 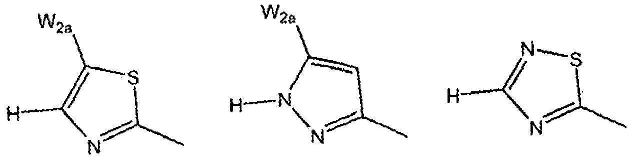
wherein W₂ₐ is hydrogen atom, halogen atom, cyano, methyl which may be substituted with one to three fluorine atoms.

5. The compound according to any preceding Claim or a pharmaceutically acceptable salt thereof, wherein both of Z₁ and Z₂ are N.

6. A compound according to claim 1 which is:
(a) 6-((4-(3-chloro-2-fluorobenzoyl)piperazin-1-yl)methyl)-N-thiazol-2-ylpyridin-2-amine,
(b) 6-((4-(2,3-dichlorobenxoyl)piperazin-1-yl)methyl)-N-thiazol-2-ylpyridin-2-amine,
(c) 6-(((1S,4S)-5-(3-chloro-2-fluorobenzoyl)-2,5-diazabicyclo[2.2.1]hept-2-yl)methyl)-N-thiazol-2-ylpyridin-2-amine.
(d) 6-((4-(3-chloro-2-fluorobenzoyl)piperazin-1-yl)methyl)-N-(1H-pyrazol-3-yl)pyridin-2-amine,
(e) 6-((4-(2-fluoro-3-(trifluoromethyl)benzoyl)piperazin-1-yl)methyl)-N-1H-pyrazol-3-ylpyridin-2-amine,
(f) 6-((4-(3-(difluoromethyl)-2-fluorobenzoyl)piperazin-1-yl)methyl)-N-1H-pyrazol-3-ylpyridin-2-amine,
(g) 6-((4-(3-chloro-2-fluorobenzoyl)piperazin-1-yl)methyl)-N-(5-methyl-1H-pyrazol-3-yl) pyridin-2-amine,
(h) 6-((4-(2-fluoro-3-(trifluoromethyl)benzoyl)piperazin-1-yl)methyl)-N-(5-methyl-1H-pyrazol-3-yl)pyridin-2-amine,
(i) 6-((4-(2-chloro-3-(trifluoromethyl)benzoyl)piperazin-1-yl)methyl)-N-(5-methyl-1H-pyrazol-3-yl)pyridin-2-amine.
(j) 6-((4-(2,3-dichlorobenzoyl)piperazin-1-yl)methyl)-N-1,2,4-thiadiazol-5-ylpyridin-2-amine,
(k) 6-((4-(2-fluoro-3-(trifluuromethyl)benzoyl)piperazin-1-yl)methyl)-N-1,2,4-thiadiazol-5-ylpyridin-2-amine,
(l) 6-((4-(3-chloro-2-fluorobenzoyl)piperazin-1-yl)methyl)-N-1H-pyrazol-3-ylpyrazin-2-amine,
(m) 6-(((1S,4S)-5-(3-chloro-2-fluorobenzoyl)-2,5-diazabicyclo[2.2.1]hept-2-yl)methyl)-N-1H-pyrazol-3-ylpyrazin-2-amine,
(n) 6-((4-(2-fluoro-3-(trifluoromethyl)benzoyl)piperazin-1-yl)methyl)-N-1H-pyrazol-3-ylpyrazin-2-amine,
(o) 6-((4-(3-chloro-2-fluorobenzoyl)piperazin-1-yl)methyl)-N-(5-methyl-1H-pyrazol-3-yl)pyrazin-2-amine,
(p) 6-((4-(2-fluoro-3-(trifluoromethyl)benzoyl)piperazin-1-yl)methyl)-N-1,2,4-thiadiazol-5-ylpyrazin-2-amine,
(q) 6-((4-(3-chloro-2-fluorobenzoyl)piperazin-1-yl)methyl)-N-thiazol-2-ylpyrazin-2-amine,
(r) 6-((4-(3-chloro-2-fluorobenzoyl)piperazin-1-yl)methyl)-N-(5-chlorothiazol-2-yl)pyridin-2-amine,
(s) 6-((4-(3-chloro-2-fluorobenzoyl)piperazin-1-yl)methyl)-N-(5-fluorothiazol-2-yl) pyridin-2-amine; or
(t) 6-((4-(2-imono-3-trifluoromethylbenzoyl)piperazin-1-yl)methyl)-N-(5-methyl-1H-pyrazol-3-yl)pyrazin-2-amine;
or a pharmaceutically acceptable salt thereof.

7. The compound according to Claim 6 which is:
6-((4-(3-chloro-2-fluorobenzoyl)piperazin-1-yl)methyl)-N-thiazol-2-ylpyridin-2-amine,
or a pharmaceutically acceptable salt thereof.

8. The compound according to Claim 6 which is:
6-((4-(2-fluoro-3-(trifluoromethyl)benzoyl)piperazin-1-yl)methyl)-N-1H-pyrazol-3-ylpyrazin-2-amine,
or a pharmaceutically acceptable salt thereof.

9. The compound according to Claim 6 which is:
6-((4-(2-fluoro-3-(trifloromethyl)benzoyl)piperazin-1-yl)methyl)-N-1H-pyrazol-3-ylpyridin-2-amine,
or a pharmaceutically acceptable salt thereof.

10. The compound according to Claim 6 which is:
6-((4-(3-chloro-2-fluorobenzoyl)piperazin-1-yl)methyl)-N-(5-fluorothiazol-2-yl)pyridin-2-amine, or a pharmaceutically acceptable salt thereof.

11. The compound according to Claim 6 which is:
6-((4-(2,3-dichlorobenzoyl)piperazin-1-yl)methyl)-N-1,2,4-thiadiazol-5-ylpyridin-2-amine, or a pharmaceutically acceptable salt thereof.

12. The compound according to Claim 6 which is:
6-((1S,4S)-5 (3-chloro-2-fluorobenzoyl)-2,5-diazabicyclo[2.2.1]hept-2-yl)methyl)-N-1H-pyrazol-3-ylpyrazin-2-amine, or a pharmaceutically acceptable salt thereof.

13. A pharmaceutical composition comprising a compound of any previous claim, or a pharmaceutically acceptable salt thereof, together with a pharmaceutically acceptable carrier or diluent.

14. A compound of any one of claims 1 to 12, or a pharmaceutically acceptable salt thereof for use in therapy.

15. A combination of a compound of any one of claims 1 to 12, or a pharmaceutically acceptable salt thereof and at least one other antitumor agent for simultaneous, separate or sequential administration.

16. A combination of claim 15 wherein the other antitumor agent is selected from the group consisting of antitumor alkylating agents, antitumor antimetabolites, antitumor antibiotics, plant-derived antitumor agents, antitumor platinum-complex compounds, antitumor campthotecin derivatives, antitumor tyrosine kinase inhibitors, monoclonal antibodies, interferons, biological response modifiers, and other antitumor agents or a pharmaceutically acceptable salt thereof, wherein:
the antitumor alkylating agents are nitrogen mustard N-oxide, cyclophosphamide, ifosfamide, melphalan, busulfan, mitobronitol, carboquone, thiotepa, ranimustine, nimustine, temozolomide, and carmustine;
the antitumor antimetabolites are methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, and pemetrexed disodium;
the antitumor antibiotics are actinomycin D, doxorubicin, daunorubicin, neocarzinostatin, bleomycin, peplomycin, mitomycin C, aclarubicin, pirarubicin, epirubicin, zinostatin stimalamer, idarubicin, sirolimus, and valrubicin;
the plant-derived antitumor agents arc vincristine, vinblastine, vindeshine, etoposide, sobuzoxane, docetaxel, paclitaxel, and vinorelbine;
the antitumor platinum-complex compounds are cisplatin, carboplatin, nedaplatin, and oxaliplatin;
the antitumor campthotecin derivatives are irinotecan, topotecan, and campthotecin;
the antitumor tyrosine kinase inhibitors are gefitinib, imatinib, and erloinib;
the monoclonal antibodies are cetuximab, bevacizumab, rituximab, bevacizumab, alemtuzumab, and trastuzumab;
the interferons are interferon α, interferon α-2a, interferon α-2b, interferon β, interferon γ-1a, and mterfefon γ-n1,
the biological response modifiers are krestin, lentinan, sixofiran, picibanil, or ubenimex, and
the other antitumor agents are mitoxantrone, L-asparaginase, procarbazine, dacarbazine, hydroxycarbamide, pentostatin, tretinoin, alefacept, darbepoetin alfa, anastrozole, exemestane, bicalutamide, leuprorelin, flutamide, fulvestrant, pegaptanib octasodium, denileukin diftitox, aldesleukin, thyrotropin alfa, arsenic trioxide, bortezomib, capecitabine, and goserelin.

17. The combination according to Claim 15 or 16 wherein one of or both of the two separate compounds is/are in (a) parenteral preparation(s).

18. The combination according to Claim 17 wherein one of or both of the two separate preparations is/are an injection or an infusion.

19. A combination of a compound of any one of claims 1 to 12, or a pharmaceutically acceptable salt thereof and paclitaxel or docetaxel, for simultaneous, separate or sequential administration.

20. The use of a compound of any one of the claims 1 to 12, or a pharmaceutically acceptable salt thereof for the manufacture of a medicament for treating or preventing cancer.

21. A compound of any one of Claim 1 to 12 or a pharmaceutically acceptable salt thereof for use in a method of treating or preventing cancer.

## Patentansprüche

1. Eine Verbindung der allgemeinen Formel I: 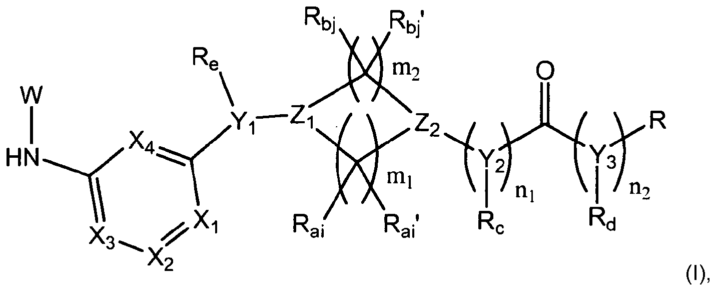 wobei:
m₁ 2 oder 3 ist,
m₂ 2 ist,
n₁ 0 ist,
n₂ 0 ist,
i eine beliebige ganze Zahl von 1 bis m₁ ist,
j eine beliebige ganze Zahl von 1 bis m₂ ist,
R Phenyl ist, worin die 2. und die 3. Position mit den gleichen oder mit unterschiedlichen Halogenatomen substituiert sind oder alternativ substituiert sind mit Halogenatom und Methyl, das mit einem bis drei gleichen oder unterschiedlichen Halogenatomen substituiert ist,
Rₐᵢ und Rₐᵢ' jeweils unabhängig ein Wasserstoffatom und Niederalkyl sind,
R_{bj} und R_{bj}' jeweils unabhängig ein Wasserstoffatom und Niederalkyl sind,
wobei:
iiₒ ist, wobei iₒ eine beliebige ganze Zahl von 1 bis m₁ ist, und j j₀ ist, wobei j₀ eine beliebige ganze Zahl von 1 bis m₂ ist, so dass eines von Rₐᵢ₀ und Rₐᵢ₀' und eines von R_{bj0} und R_{bj0}' vereint sein können unter Bildung von -(CH₂)ₙ-, wobei n 1 oder 2 ist, und
Rₑ ein Wasserstoffatom oder Niederalkyl ist,
X₁ CH, CX₁ₐ oder N ist, wobei X₁ₐ Niederalkyl ist,
X₂ CH oder N ist,
X₃ CH ist,
X₄ N ist,
die Anzahl an Stickstoffatomen bei X₁, X₂ und X₄ eins oder zwei beträgt,
Y₁ CH oder N ist, wobei jedoch, wenn Y₁ CH ist und Rₑ ein Wasserstoffatom ist, die zwei Wasserstoffatome dann mit Oxo substituiert sein können,
Z₁ N ist und Z₂ CH oder N ist,
W ausgewählt ist aus: 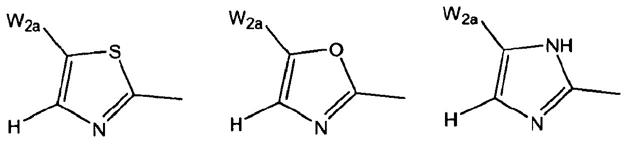 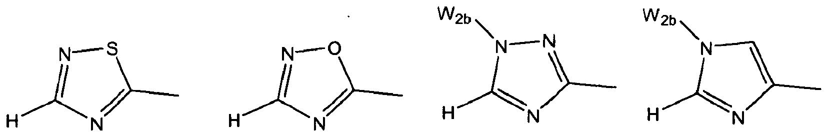 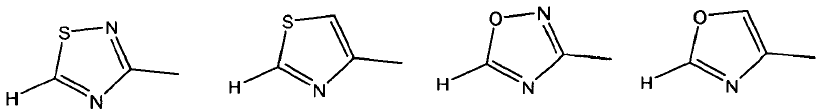 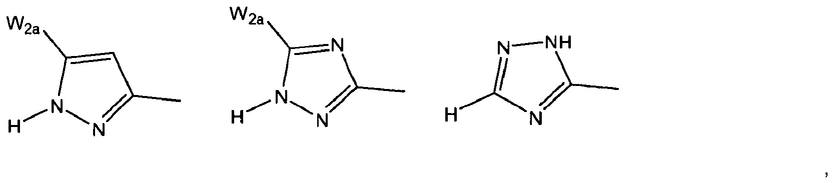 wobei W₂ₐ und W_{2b} jeweils unabhängig ein Wasserstoffatom, Halogenatom, Cyano, C₁₋₂-Niederalkyl, C₃₋₅-Cycloalkyl oder C₁₋₂-Niederalkyl, das substituiert sein kann mit einem oder mehreren Halogenatomen, sind und wobei "Niederalkyl" eine lineare oder verzweigte Alkylgruppe mit 1 bis 6 Kohlenstoffatomen bedeutet,
mit der Maßgabe, dass, wenn W 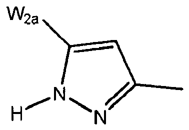 ist, X dann CH oder CX₁ₐ ist und X₂ N ist,
oder ein pharmazeutisch annehmbares Salz davon.

2. Die Verbindung gemäß Anspruch 1 oder ein pharmazeutisch annehmbares Salz davon, wobei:
Y₁ CH ist und
Rₑ ein Wasserstoffatom ist.

3. Die Verbindung gemäß Anspruch 1 oder 2 oder ein pharmazeutisch annehmbares Salz davon, wobei:
m₁ 2 ist und
Rₐ₁, Rₐ₁', Rₐ₂, Rₐ₂', R_{b1}, R_{b1}', R_{b2} und R_{b2}' Wasserstoffatome sind.

4. Die Verbindung gemäß Anspruch 1, 2 oder 3 oder ein pharmazeutisch annehmbares Salz davon, wobei:
W ausgewählt ist aus: 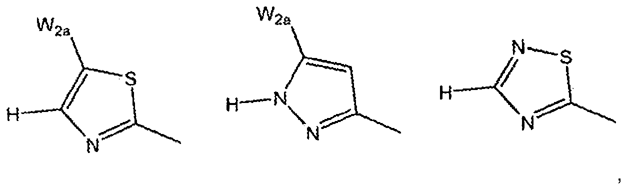
wobei W₂ₐ ein Wasserstoffatom, Halogenatom, Cyano, Methyl, das mit einem bis drei Fluoratomen substituiert sein kann, ist.

5. Die Verbindung gemäß einem vorhergehenden Anspruch oder ein pharmazeutisch annehmbares Salz davon, wobei sowohl Z₁ als auch Z₂ N sind.

6. Eine Verbindung gemäß Anspruch 1, die ist:
(a) 6-((4-(3-Chlor-2-fluorbenzoyl)piperazin-1-yl)methyl)-N-thiazol-2-ylpyridin-2-amin,
(b) 6-((4-(2,3-Dichlorbenzoyl)piperazin-1-yl)methyl)-N-thiazol-2-ylpyridin-2-amin,
(c) 6-(((1S,4S)-5-(3-Chlor-2-fluorbenzoyl)-2,5-diazabicyclo[2.2.1]hept-2-yl)methyl)-N-thiazol-2-ylpyridin-2-amin,
(d) 6-((4-(3-Chlor-2-fluorbenzoyl)piperazin-1-yl)methyl)-N-(1H-pyrazol-3-yl)pyridin-2-amin,
(e) 6-((4-(2-Fluor-3-(trifluormethyl)benzoyl)piperazin-1-yl)methyl)-N-1H-pyrazol-3-ylpyridin-2-amin,
(f) 6-((4-(3-(Difluormethyl)-2-fluorbenzoyl)piperazin-1-yl)methyl)-N-1H-pyrazol-3-ylpyridin-2-amin,
(g) 6-((4-(3-Chlor-2-fluorbenzoyl)piperazin-1-yl)methyl)-N-(5-methyl-1 H-pyrazol-3-yl)pyridin-2-amin,
(h) 6-((4-(2-Fluor-3-(trifluormethyl)benzoyl)piperazin-1-yl)methyl)-N-(5-methyl-1H-pyrazol-3-yl)pyridin-2-amin,
(i) 6-((4-(2-Chlor-3-(trifluormethyl)benzoyl)piperazin-1-yl)methyl)-N-(5-methyl-1 H-pyrazol-3-yl)pyridin-2-amin,
(j) 6-((4-(2,3-Dichlorbenzoyl)piperazin-1-yl)methyl)-N-1,2,4-thiadiazol-5-ylpyridin-2-amin,
(k) 6-((4-(2-Fluor-3-(trifluormethyl)benzoyl)piperazin-1-yl)methyl)-N-1,2,4-thiadiazol-5-ylpyridin-2-amin,
(l) 6-((4-(3-Chlor-2-fluorbenzoyl)piperazin-1-yl)methyl)-N-1H-pyrazol-3-ylpyrazin-2-amin,
(m) 6-(((1S,4S)-5-(3-Chlor-2-fluorbenzoyl)-2,5-diazabicyclo[2.2.1]hept-2-yl)methyl)-N-1H-pyrazol-3-ylpyrazin-2-amin,
(n) 6-((4-(2-Fluor-3-(trifluormethyl)benzoyl)piperazin-1-yl)methyl)-N-1H-pyrazol-3-ylpyrazin-2-amin,
(o) 6-((4-(3-Chlor-2-fluorbenzoyl)piperazin-1-yl)methyl)-N-(5-methyl-1H-pyrazol-3-yl)pyrazin-2-amin,
(p) 6-((4-(2-Fluor-3-(trifluormethyl)benzoyl)piperazin-1-yl)methyl)-N-1,2,4-thiadiazol-5-ylpyrazin-2-amin,
(q) 6-((4-(3-Chlor-2-fluorbenzoyl)piperazin-1-yl)methyl)-N-thiazol-2-ylpyrazin-2-amin,
(r) 6-((4-(3-Chlor-2-fluorbenzoyl)piperazin-1-yl)methyl)-N-(5-chlorthiazol-2-yl)pyridin-2-amin,
(s) 6-((4-(3-Chlor-2-fluorbenzoyl)piperazin-1-yl)methyl)-N-(5-fluorthiazol-2-yl)pyridin-2-amin oder
(l) 6-((4-(2-Fluor-3-trifluormethylbenzoyl)piperazin-1-yl)methyl)-N-(5-methyl-1H-pyrazol-3-yl)pyrazin-2-amin,
oder ein pharmazeutisch annehmbares Salz davon.

7. Die Verbindung gemäß Anspruch 6, die ist:
6-((4-(3-Chlor-2-fluorbenzoyl)piperazin-1-yl)methyl)-N-thiazol-2-ylpyridin-2-amin,
oder ein pharmazeutisch annehmbares Salz davon.

8. Die Verbindung gemäß Anspruch 6, die ist:
6-((4-(2-Fluor-3-(trifluormethyl)benzoyl)piperazin-1-yl)methyl)-N-1H-pyrazol-3-ylpyrazin-2-amin,
oder ein pharmazeutisch annehmbares Salz davon.

9. Die Verbindung gemäß Anspruch 6, die ist:
6-((4-(2-Fluor-3-(trifluormethyl)benzoyl)piperazin-1-yl)methyl)-N-1H-pyrazol-3-ylpyridin-2-amin,
oder ein pharmazeutisch annehmbares Salz davon.

10. Die Verbindung gemäß Anspruch 6, die ist:
6-((4-(3-Chlor-2-fluorbenzoyl)piperazin-1-yl)methyl)-N-(5-fluorthiazol-2-yl)pyridin-2-amin, oder ein pharmazeutisch annehmbares Salz davon.

11. Die Verbindung gemäß Anspruch 6, die ist:
6-((4-(2,3-Dichlorbenzoyl)piperazin-1-yl)methyl)-N-1,2,4-thiadiazol-5-ylpyridin-2-amin,
oder ein pharmazeutisch annehmbares Salz davon.

12. Die Verbindung gemäß Anspruch 6, die ist:
6-(((1S,4S)-5-(3-Chlor-2-fluorbenzoyl)-2,5-diazabicyclo[2.2.1]hept-2-yl)methyl)-N-1H-pyrazol-3-ylpyrazin-2-amin,
oder ein pharmazeutisch annehmbares Salz davon.

13. Eine pharmazeutische Zusammensetzung, die eine Verbindung nach einem vorhergehenden Anspruch oder ein pharmazeutisch annehmbares Salz davon zusammen mit einem pharmazeutisch annehmbaren Träger oder Verdünnungsmittel umfasst.

14. Eine Verbindung nach einem der Ansprüche 1 bis 12 oder ein pharmazeutisch annehmbares Salz davon zur Verwendung in der Therapie.

15. Eine Kombination aus einer Verbindung nach einem der Ansprüche 1 bis 12 oder einem pharmazeutisch annehmbaren Salz davon und wenigstens einem weiteren Antitumormittel zur gleichzeitigen, separaten oder sequentiellen Verabreichung.

16. Eine Kombination nach Anspruch 15, wobei das weitere Antitumormittel ausgewählt ist aus der Gruppe, bestehend aus antitumoralen Alkylierungsmitteln, antitumoralen Antimetaboliten, antitumoralen Antibiotika, Antitumormitteln pflanzlichen Ursprungs, antitumoralen Platinkomplexverbindungen, antitumoralen Campthotecin-Derivaten, antitumoralen Tyrosinkinaseinhibitoren, monoklonalen Antikörpern, Interferonen, Biomodulatoren und anderen Antitumormitteln oder einem pharmazeutisch annehmbares Salz davon, wobei:
die antitumoralen Alkylierungsmittel Stickstofflost-N-oxid, Cyclophosphamid, Ifosfamid, Melphalan, Busulfan, Mitobronitol, Carboquon, Thiotepa, Ranimustin, Nimustin, Temozolomid und Carmustin sind,
die antitumoralen Antimetabolite Methotrexat, 6-Mercaptopurinribosid, Mercaptopurin, 5-Fluorouracil, Tegafur, Doxifluridin, Carmofur, Cytarabin, Cytarabinocfosfat, Enocitabin, S-1, Gemcitabin, Fludarabin und Pemetrexed-Dinatrium sind,
die antitumoralen Antibiotika Actinomycin D, Doxorubicin, Daunorubicin, Neocarzinostatin, Bleomycin, Peplomycin, Mitomycin C, Aclarubicin, Pirarubicin, Epirubicin, Zinostatin-Stimalamer, Idarubicin, Sirolimus und Valrubicin sind,
die Antitumormittel pflanzlichen Ursprungs Vincristin, Vinblastin, Vindesin, Etoposid, Sobuzoxan, Docetaxel, Paclitaxel und Vinorelbin sind,
die antitumoralen Platinkomplexverbindungen Cisplatin, Carboplatin, Nedaplatin und Oxaliplatin sind,
die antitumoralen Campthotecin-Derivate Irinotecan, Topotecan und Campthotecin sind,
die antitumoralen Tyrosinkinaseinhibitoren Gefitinib, Imatinib und Erlotinib sind,
die monoklonalen Antikörper Cetuximab, Bevacizumab, Rituximab, Bevacizumab, Alemtuzumab und Trastuzumab sind,
die Interferone Interferon α, Interferon α-2a, Interferon α-2b, Interferon β, Interferon-γ-1a und Interferon γ-n1 sind,
die Biomodulatoren Krestin, Lentinan, Sizofiran, Picibanil oder Ubenimex sind und
die anderen Antitumormittel Mitoxantron, L-Asparaginase, Procarbazin, Dacarbazin, Hydroxycarbamid, Pentostatin, Tretinoin, Alefacept, Darbepoetin-alfa, Anastrozol, Exemestan, Bicalutamid, Leuprorelin, Flutamid, Fulvestrant, Pegaptanib-Octanatrium, Denileukin-Diftitox, Aldesleukin, Thyrotropin-alfa, Arsentrioxid, Bortezomib, Capecitabin und Goserelin sind.

17. Die Kombination gemäß Anspruch 15 oder 16, wobei eine oder beide der zwei separaten Verbindungen in (einem) parenteralen Präparat(en) vorliegt/vorliegen.

18. Die Kombination gemäß Anspruch 17, wobei eine oder beide der zwei separaten Präparate eine Injektion oder eine Infusion ist/sind.

19. Eine Kombination aus einer Verbindung nach einem der Ansprüche 1 bis 12 oder ein pharmazeutisch annehmbares Salz davon und Paclitaxel oder Docetaxel zur gleichzeitigen, separaten oder sequentiellen Verabreichung.

20. Die Verwendung einer Verbindung nach einem der Ansprüche 1 bis 12 oder eines pharmazeutisch annehmbaren Salzes davon zur Herstellung eines Medikaments zur Behandlung oder Prävention von Krebs.

21. Eine Verbindung nach einem der Ansprüche 1 bis 12 oder ein pharmazeutisch annehmbares Salz davon zur Verwendung bei einem Verfahren zur Behandlung oder Prävention von Krebs.

## Revendications

1. Composé de la formule générale I: 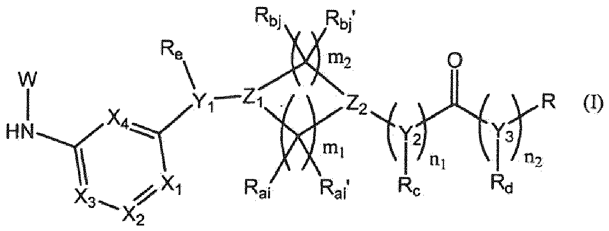 où:
m₁ est 2 ou 3;
m₂ est 2;
n₁ est 0;
n₂ est 0;
i est un nombre entier de l'un quelconque de 1 à m₁;
j est un nombre entier de l'un quelconque de 1 à m₂;
R est phényle dont les 2^{e} et 3^{e} positions sont substituées par les mêmes ou de différents atomes d'halogène ou bien alternativement substituées par un atome d'halogène et méthyle substitué par un à trois des mêmes ou de différents atomes d'halogène, respectivement;
Rₐᵢ et Rₐᵢ' sont chacun indépendamment un atome d'hydrogène et un alkyle inférieur;
R_{bj} et R_{bj}' sont chacun indépendamment un atome d'hydrogène et un alkyle inférieur;
où:
i est i₀ où i₀ est un nombre entier de l'un quelconque de 1 à m₁ et j est j₀ où j₀ est un nombre entier de l'un quelconque de 1 à m₂, de sorte que l'un de Rₐᵢ₀ et Rₐᵢ₀' et l'un de R_{bj0} et R_{bj0}' peuvent être combinés pour former -(CH₂)ₐ-, où n est 1 ou 2; et
Rₑ est un atome d'hydrogène ou un alkyle inférieur;
X1 est CH, CX₁ₐ ou bien N dans lequel X₁ₐ est un alkyle inférieur;
X₂ est CH ou N;
X₃ est CH;
X₄ est N;
le nombre d'atomes d'azote parmi X₁, X₂ et X₄ est de un ou deux;
Y₁ est CH ou N; toutefois, si Y₁ est CH et Rₑ est un atome d'hydrogène, alors les deux atomes d'hydrogène peuvent être substitués par oxo;
Z₁ est N et Z₂ est CH ou N;
W est sélectionné parmi: 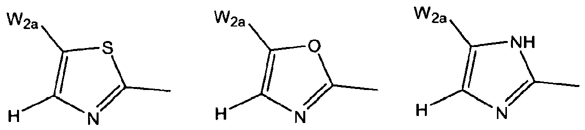 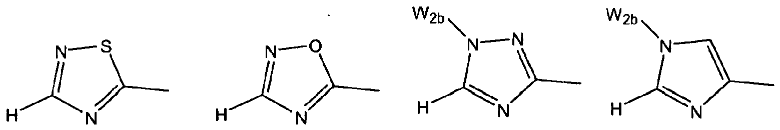 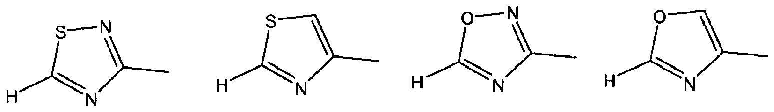 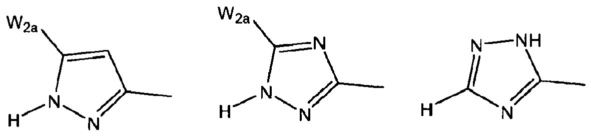 où W₂ₐ et W_{2b} sont chacun indépendamment un atome d'hydrogène, un atome d'halogène, cyano, alkyle inférieur C₁₋₂, cycloalkyle C₃₋₅ ou alkyle inférieur C₁₋₂ qui peut être substitué par un ou plusieurs atomes d'halogène; et où 'alkyle inférieur' signifie un groupe alkyle linéaire ou ramifié ayant 1 à 6 atomes de carbone;
à condition que lorsque W est: 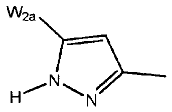 alors X₁ est CH ou CX₁ₐ et X₂ est N;
ou un sel pharmaceutiquement acceptable de celui-ci.

2. Composé selon la revendication 1 ou un sel pharmaceutiquement acceptable de celui-ci, dans lequel:
Y₁ est CH; et
Rₑ est un atome d'hydrogène.

3. Composé selon la revendication 1 ou 2 ou un sel pharmaceutiquement acceptable de celui-ci, dans lequel:
m₁ est 2; et
Rₐᵢ, Rₐᵢ', Rₐ₂, Rₐ₂', R_{b1}, R_{b1}', R_{b2} et R_{b2}' sont un atome d'hydrogène.

4. Composé selon la revendication 1, 2 ou 3 ou un sel pharmaceutiquement acceptable de celui-ci, dans lequel:
W est sélectionné parmi: 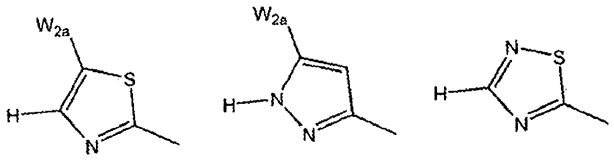
où W₂ₐ est un atome d'hydrogène, un atome d'halogène, cyano, méthyle qui peut être substitué par un à trois atomes de fluor.

5. Composé selon l'une quelconque des revendications précédentes ou un sel pharmaceutiquement acceptable de celui-ci, dans lequel Z₁ et Z₂ sont tous les deux N.

6. Composé selon la revendication 1, qui est de la:
(a) 6-((4-(3-chloro-2-fluorobenzoyl)pipérazin-1-yl)méthyl)-N-thiazol-2-ylpyridin-2-amine,
(b) 6-((4-(2,3-dichlorobenzoyl)pipérazin-1-yl)méthyl)-N-thiazol-2-ylpyridin-2-amine,
(c) 6-(((1S,4S)-5-(3-chloro-2-fluorobenzoyl)-2,5-diazabicyclo[2.2.1]hept-2-yl)méthyl)-N-thiazol-2-ylpyridin-2-amine,
(d) 6-((4-(3-chloro-2-fluorobenzoyl)pipérazin-1-yl)méthyl)-N-(1H-pyrazol-3-yl)pyridin-2-amine,
(e) 6-((4-(2-fluoro-3-(trifluorométhyl)benzoyl)pipérazin-1-yl)méthyl)-N-1H-pyrazol-3-ylpyridin-2-amine,
(f) 6-((4-(3-(difluorométhyl)-2-fluorobenzoyl)pipérazin-1-yl)méthyl)-N-1H-pyrazol-3-ylpyridin-2-amine,
(g) 6-((4-(3-chloro-2-fluorobenzoyl)pipérazin-1-yl)méthyl)-N-(5-méthyl-1H-pyrazol-3-yl)pyridin-2-amine,
(h) 6-((4-(2-fluoro-3-(trifluorométhyl)benzoyl)pipérazin-1-yl)méthyl)-N-(5-méthyl-1H-pyrazol-3-yl)pyridin-2-amine,
(i) 6-((4-(2-chloro-3-(trifluorométhyl)benzoyl)pipérazin-1-yl)méthyl)-N-(5-méthyl-1H-pyrazol-3-yl)pyridin-2-amine,
(j) 6-((4-(2,3-dichlorobenzoyl)pipérazin-1-yl)méthyl)-N-1,2,4-thiadiazol-5-ylpyridin-2-amine,
(k) 6-((4-(2-fluoro-3-(trifluorométhyl)benzoyl)pipérazin-1-yl)méthyl)-N-1,2,4-thiadiazol-5-ylpyridin-2-amine,
(l) 6-((4-(3-chloro-2-fluorobenzoyl)pipérazin-1-yl)méthyl)- N-1H-pyrazol-3-ylpyrazin-2-amine,
(m) 6-(((1S,45)-5-(3-chloro-2-fluorobenzoyl)-2,5-diazabicyclo[2.2.1]hept-2-yl)méthyl)-N-1H-pyrazol-3-ylpyrazin-2-amine,
(n) 6-((4-(2-fluoro-3-(trifluorométhyl)benzoyl)pipérazin-1-yl)méthyl)-N-1H-pyrazol-3-ylpyrazin-2-amine,
(o) 6-((4-(3-chloro-2-fluorobenzoyl)pipérazin-1-yl)méthyl)-N-(5-méthyl-1H-pyrazol-3-ylpyrazin-2-amine,
(p) 6-((4-(2-fluoro-3-(trifluorométhyl)benzoyl)pipérazin-1-yl)méthyl)-N-1,2,4-thiadiazol-5-ylpyrazin-2-amine,
(q) 6-((4-(2-chloro-2-fluorobenzoyl)pipérazin-1-yl)méthyl)-N-thiazol-2-ylpyrazin-2-amine,
(r) 6-((4-(3-chloro-2-fluorobenzoyl)pipérazin-1-yl)méthyl)-N-(5-chlorothiazol-2-yl)pyridin-2-amine,
(s) 6-((4-(3-chloro-2-fluorobenzoyl)pipérazin-1-yl)méthyl)-N-(5-fluororothiazol-2-yl)pyridin-2-amine, ou
(t) 6-((4-(2-fluoro-3-trifluorométhylbenzoyl)pipérazin-1-yl)méthyl)-N-(5-méthyl-1H-pyrazol-3-yl)pyrazin-2-amine;
ou un sel pharmaceutiquement acceptable de celles-ci.

7. Composé selon la revendication 6, qui est de la:
6-((4-(3-chloro-2-fluorobenzoyl)pipérazin-1-yl)méthyl)-N-thiazol-2-ylpyridin-2-amine ou un sel pharmaceutiquement acceptable de celle-ci.

8. Composé selon la revendication 6, qui est de la:
6-((4-(2-fluoro-3-(trifluorométhyl)benzoyl)pipérazin-1-yl)méthyl)-N-1H-pyrazol-3-ylpyrazin-2-amine ou un sel pharmaceutiquement acceptable de celle-ci.

9. Composé selon la revendication 6, qui est de la:
6-((4-(2-fluoro-3-(trifluorométhyl)benzoyl)pipérazin-1-yl)méthyl)-N-1H-pyrazol-3-ylpyridin-2-amine ou un sel pharmaceutiquement acceptable de celle-ci.

10. Composé selon la revendication 6, qui est de la:
6-((4-(3-chloro-2-fluorobenzoyl)pipérazin-1-yl)méthyl)-N-(5-fluorothiazol-2-yl)pyridin-2-amine ou un sel pharmaceutiquement acceptable de celle-ci.

11. Composé selon la revendication 6, qui est de la:
6-((4-(2,3-dichlorobenzoyl)pipérazin-1-yl)méthyl)-N-1,2,4-thiadiazol-5-ylpyridin-2-amine ou un sel pharmaceutiquement acceptable de celle-ci.

12. Composé selon la revendication 6, qui est de la:
6-(((1S,4S)-5-(3-chloro-2-fluorobenzoyl)-2,5-diazabicyclo[2.2.1]hept-2-yl)méthyl)-N-1H-pyrazol-3-ylpyrazin-2-amine ou un sel pharmaceutiquement acceptable de celle-ci.

13. Composition pharmaceutique comprenant un composé selon l'une quelconque des revendications précédentes ou un sel pharmaceutiquement acceptable de celui-ci, ensemble avec un véhicule ou un diluant pharmaceutiquement acceptable.

14. Composé selon l'une quelconque des revendications 1 à 12 ou un sel pharmaceutiquement acceptable de celui-ci, à utiliser en thérapie.

15. Combinaison d'un composé selon l'une quelconque des revendications 1 à 12 ou d'un sel pharmaceutiquement acceptable de celui-ci et d'au moins un autre agent antitumoral pour administration simultanée, séparée ou séquentielle.

16. Combinaison selon la revendication 15, dans laquelle l'autre agent antitumoral est sélectionné parmi le groupe consistant en agents alkylants antitumoraux, antimétabolites antitumoraux, antibiotiques antitumoraux, agents antitumoraux dérivés de plantes, composés antitumoraux de complexes de platine, dérivés antitumoraux de camphotécine, inhibiteurs antitumoraux de tyrosine kinase, anticorps monoclonaux, interférons, modificateurs de réponse biologique et autres agents antitumoraux ou un sel pharmaceutiquement acceptable de ceux-ci, dans laquelle:
les agents alkylants antitumoraux sont le N-oxyde de moutarde azotée, le cyclophosphamide, l'ifosfamid, le melphalan, le busulfan, le mitobronitol, le carboquone, le thiotépa, la ranimustine, la nimustine, le témozolomide et la carmustine;
les antimétabolites antitumoraux sont le méthotrexate, le 6-mercaptopurine riboside, la mercaptopurine, le 5-fluorouracile, le tégafur, la doxifluridine, le carmofur, la cytarabine, l'ocfosfate de cytarabine, l'énocitabine, S-1, la gemcitabine, la fludarabine et le permetrexed disodium;
les antibiotiques antitumoraux sont l'actinomycine D, la doxorubicine, la daunorubicine, la néocarzinostatine, la bléomycine, la péplomycine, la mitomycine C, l'aclarubicine, la pirarubicine, l'épirubicine, le stimalamère de zinostatine, l'idarubicine, le sirolimus et la valrubicine;
les agents antitumoraux dérivés de plantes sont la vincristine, la vinblastine, la vindeshine, l'étoposide, le sobuzoxane, le docétaxel, le paclitaxel et la vinorelbine;
les composés antitumoraux de complexes de platine sont le cisplatine, le carboplatine, le nédaplatine et foxaliplatine;
les dérivés antitumoraux de camptothécine sont l'irinotécan, le topotécan et la camptothécine;
les inhibiteurs antitumoraux de tyrosine kinase sont le gefitinib, l'imatinib et ferlotinib;
les anticorps monoclonaux sont le cétuximab, le bévacizumab, le rituximab, le bévacizumab, l'alemtuzumab et le trastuzumab;
les interférons sont l'interféron α, l'interféron α-2a, l'interféron α-2b, l'interféron β, l'interféron γ-1a et l'interféron γ-n1;
les modificateurs de réponse biologique sont la krestine, le lentinane, le sizofiran, le picibanil ou l'ubénimex, et
les autres agents antitumoraux sont le mitoxantrone, la L-asparaginase, la procarbazine, la dacarbazine, l'hydroxycarbamide, la pentostatine, la trétinoïne, l'aléfacept, la darbépoétine alfa, l'anastrozole, l'exémestane, le bicalutamide, la leuproréline, le flutamide, le fulvestrant, le pégaptanib octasodium, le dénileukine diftitox, l'aldesleukine, la thyrotropine alfa, le trioxyde d'arsenic, le bortézomib, la capécitabine et la goséréline.

17. Combinaison selon la revendication 15 ou 16, dans laquelle un ou deux des deux composés séparés est/sont dans une/des préparation(s) parentérale(s).

18. Combinaison selon la revendication 17, dans laquelle une ou deux des deux préparations séparées est/sont une injection ou une perfusion.

19. Combinaison d'un composé selon l'une quelconque des revendications 1 à 12 ou d'un sel pharmaceutiquement acceptable de celui-ci et de paclitaxel ou de docétaxel pour administration simultanée, séparée ou séquentielle.

20. Utilisation d'un composé selon l'une quelconque des revendications 1 à 12 ou d'un sel pharmaceutiquement acceptable de celui-ci, dans la fabrication d'un médicament pour le traitement ou la prévention du cancer.

21. Composé selon l'une quelconque des revendications 1 à 12 ou un sel pharmaceutiquement acceptable de celui-ci, à utiliser dans une méthode de traitement ou de prévention du cancer.
